# EUROPEAN PATENT SPECIFICATION

(11) **EP 4 358 995 B1**
(45) Date of publication and mention of the grant of the patent: **03.12.2025**
(21) Application number: 22744054.2
(22) Date of filing: 22.06.2022
(51) Int. Cl.: A61K 38/26, C07K 16/18, C07K 16/24, A61P 3/04, A61P 3/06, A61P 3/10, A61P 21/06

(54) **A MYOSTATIN PATHWAY INHIBITOR IN COMBINATION WITH A GLP-1 PATHWAY ACTIVATOR FOR USE IN TREATING METABOLIC DISORDERS**
MYOSTATIN-PFADHEMMER IN KOMBINATION MIT EINEM GLP-1-PFADAKTIVATOR ZUR VERWENDUNG BEI DER BEHANDLUNG VON STOFFWECHSELERKRANKUNGEN
INHIBITEUR DE LA VOIE DE PASSAGE DE LA MYOSTATINE EN COMBINAISON AVEC UN ACTIVATEUR DE LA VOIE DE PASSAGE GLP-1 POUR L'UTILISATION DANS LE TRAITEMENT DE TROUBLES MÉTABOLIQUES

(30) Priority: 23.06.2021 US 202163214234 P; 10.08.2021 US 202163260136 P; 13.08.2021 US 202163260254 P; 03.01.2022 US 202263266348 P; 21.03.2022 US 202263269702 P
(43) Date of publication of application: 01.05.2024
(73) Proprietor: Scholar Rock, Inc., Cambridge, MA 02142 (US)
(72) Inventor: LONG, Kimberly, Cambridge, Massachusetts 02142 (US); CHYUNG, Yung, Cambridge, Massachusetts 02142 (US); NOMIKOS, George, Cambridge, Massachusetts 02142 (US); FLIER, Jeffrey S., Cambridge, Massachusetts 02142 (US); WEBSTER, Micah T., Cambridge, Massachusetts 02142 (US); CHAPRON, Christopher, Cambridge, Massachusetts 02142 (US)
(74) Representative: Carpmaels & Ransford LLP
(86) International application number: PCT/US2022/034588
(87) International publication number: WO 2022/271867

(56) References cited:
- WO-A1-2018/116201
- WO-A1-2018/129395
- WO-A1-2019/193204
- WO-A2-2020/160291

## Description

### FIELD

The invention relates to use of myostatin pathway inhibitors and GLP-1 pathway activators for treating metabolic disorders, including obesity, metabolic syndrome, and type 2 diabetes mellitus.

### BACKGROUND

Among metabolic diseases, obesity is a chronic condition that affects more than one in three adults and about 17 percent of children and adolescents in the United States alone. More than one in three adults is overweight. Being overweight or obese increases the risk of type 2 diabetes, heart disease, stroke, fatty liver disease, kidney disease, and other health issues.

Weight loss efforts often face challenges. For example, extreme dieting such as ultra-low caloric restriction, is impractical, if not unfeasible, for most patients. For example, when a person with obesity syndrome goes on a diet, 10% weight loss can result in about 20-30% decrease in overall energy expenditure, accompanied by reduced basal metabolic rate, reduced leptin concentrations, and reduced lean mass. It is well known that weight loss from dietary/caloric restriction causes not only adipose (fat) tissue mass reduction, but also results in loss of skeletal muscle mass. Coupled with the body's natural propensity towards maintaining homeostasis by adapting to lower energy supply, reduced skeletal muscle causes a decrease in metabolic rate, lowering energy expenditure. In fact, a 10% loss in overall weight results in a 30-40% reduction in energy expanded during physical activity above resting energy expenditure.

In addition to more invasive procedures such as gastric bypass surgery, several weight loss medications have been approved by regulatory authorities (e.g., FDA) to treat overweight and obesity (see, e.g., Williams. Diabetes Ther (2020) 11:1199-1216). These include orlistat (XENICAL^{®}, ALLI^{®}), phentermine and topiramate (QSYMIA^{®}), naltrexone, HCl/bupropion, HCl (CONTRAVE^{®}), liraglutide (SAXENDA), semaglutide (WEGOVY^{®}, OZEMPIC^{®}, RYBELSUS^{®}), dulaglutide (TRULICITY^{®}), and setmelanotide (IMCIVREE^{®}). Other medications that are aimed to curb appetite include phentermine, benzphetamine, diethylpropion and phendimetrazine. Many side effects have been reported with the use of the above-listed medications, and some of them come with warnings such as severe liver injury, birth defects, suicidal thoughts, pancreatitis, and thyroid tumor.

Recently, semaglutide (available as WEGOVY^{®}, 2.4 mg once-weekly subcutaneous injection) was approved by the U.S. Food and Drug Administration (FDA) for chronic weight management in adults with obesity or who are overweight with at least one weight-related condition (such as high blood pressure, type 2 diabetes, and high cholesterol), to be used in addition to a reduced calorie diet and increased physical activity. The prescribing information for WEGOVY contains a boxed warning to inform healthcare professionals and patients about the potential risk of thyroid C-cell tumors, pancreatitis, among other risks.

Myostatin, also known as growth differentiation factor 8 or GDF-8, is a member of the transforming growth factor-β (TGF-β) superfamily. Myostatin is a negative regulator of skeletal muscle growth, although there have been suggestions that Activin A may play a more prominent role in regulating muscle mass than myostatin in primates (Latres et al., 2017, Nature Communications, 8:15153). Garito et al. (Diabetes Obes Metab. 2018 Jan; 20(1):94-102. Epub 2017) and Heymsfield et al. (JAMA Network Open. 2021; 4(1):e2033457) reported that bimagrumab, an antibody that binds Activin Receptor type IIB, thereby interfering with ligand binding, showed clinically beneficial effects in improving weight, reducing adiposity and preserving lean mass. Because multiple ligands (including Activins, myostatin, GDF11 and others) signal through this receptor, bimagrumab presumably inhibits activities of all of these growth factors, raising safety concerns. Indeed, clinical studies revealed increased serious adverse events in bimagrumab-treated patients over placebo. WO 2018/116201 A1 describes myostatin, activin or activin receptor antagonists for use in treating obesity and related conditions. WO 2018/129395 A1 describes methods for treating metabolic diseases by inhibiting myostatin activation.

Accordingly, there exists an unmet need for therapies for subjects suffering from metabolic diseases such as obesity, type 2 diabetes or obesity associated with type 2 diabetes.

### SUMMARY

The invention provides a myostatin-selective inhibitor for use in the treatment of a metabolic disorder in a subject, wherein the treatment comprises administration of the myostatin-selective inhibitor to the subject in conjunction with a GLP-1 analog, wherein the myostatin-selective inhibitor and the GLP-1 analog are administered in amounts sufficient to treat the metabolic disorder; wherein, optionally, the metabolic disorder is type 2 diabetes mellitus (T2D), obesity, obesity associated with T2D, or metabolic syndrome,
wherein the myostatin-selective inhibitor is a myostatin-selective antibody or antigen-binding fragment thereof that binds to myostatin and exhibits no detectable binding or potency towards GDF11 or Activin A; and
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117).

The invention also provides a GLP-1 analog for use in the treatment of a metabolic disorder in a subject, wherein the treatment comprises administration of the GLP-1 analog to the subject in conjunction with a myostatin-selective inhibitor, wherein the myostatin-selective inhibitor and the GLP-1 analog are administered in amounts sufficient to treat the metabolic disorder; wherein, optionally, the metabolic disorder is type 2 diabetes mellitus (T2D), obesity, obesity associated with T2D, or metabolic syndrome,
wherein the myostatin-selective inhibitor is a myostatin-selective antibody or antigen-binding fragment thereof that binds to myostatin and exhibits no detectable binding or potency towards GDF11 or Activin A; and
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117).

The invention also provides a myostatin-selective inhibitor and a GLP-1 analog for use in the treatment of a metabolic disorder in a subject, wherein the treatment comprises administration of the myostatin-selective inhibitor to the subject in conjunction with the GLP-1 analog, wherein the myostatin-selective inhibitor and the GLP-1 analog are administered in amounts sufficient to treat the metabolic disorder; wherein, optionally, the metabolic disorder is type 2 diabetes mellitus (T2D), obesity, obesity associated with T2D, or metabolic syndrome,
wherein the myostatin-selective inhibitor is a myostatin-selective antibody or antigen-binding fragment thereof that binds to myostatin and exhibits no detectable binding or potency towards GDF11 or Activin A; and
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117).

The invention also provides a composition comprising a myostatin-selective inhibitor and a GLP-1 analog;
wherein the myostatin-selective inhibitor is a myostatin-selective antibody or antigen-binding fragment thereof that binds to myostatin and exhibits no detectable binding or potency towards GDF11 or Activin A; and
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117), optionally wherein the GLP-1 analog is semaglutide, exenatide ER, liraglutide, lixisenatide, tirzepatide, XW003, Noiiglutide, MEDI0382, dulaglutide, or albiglutide.

Herein, the terminologies "embodiment" and "embodiments" are to be construed as embodiment(s) of the invention only in as far as they fall within the scope of the appended claims. In particular, references to a "myostatin pathway inhibitor" or "an inhibitor of the myostatin signaling pathway" in embodiments of the compositions and compositions for use as described herein are to be interpreted as references to a myostatin-selective inhibitor as defined in the claims. Likewise, references to a "GLP-1 pathway activator" or "an activator of the GLP-1 signaling pathway" in embodiments of the compositions and compositions for use as described herein are to be interpreted as references to a GLP-1 analog as defined in the claims. The references to methods of treatment of the human or animal body by surgery or therapy herein are to be interpreted as references to a myostatin-selective inhibitor and/or a GLP-1 analog for use in those methods.

The present disclosure provides novel therapy to improve metabolic conditions, in which an inhibitor of the myostatin signaling pathway and an insulin secretion-promoting agent (e.g., an activator of the glucagon-like peptide-1 (GLP-1) signaling pathway in particular) are used in conjunction for the treatment of obesity/overweight or weight management in a subject. In the context of the present disclosure, weight management encompasses not only overall weight loss, but improvements to body composition, e.g., enhanced lean mass (muscle) relative to fat mass (adipose). Combination therapies, as well as add-on/adjunct therapies are contemplated herein.

The data disclosed herein show that when used in conjunction with a GLP-1 receptor agonist (e.g., GLP-1 analogs), a myostatin inhibitor can enhance overall weight loss. Advantageously, the weight loss is achieved while substantially maintaining or enhancing lean mass (e.g., muscle) in a diet-induced obese rodent model, demonstrating preferential metabolism of adipose tissue over muscle tissue, leading to improved body composition (e.g., increased lean mass-to-fat mass ratios). Surprisingly, these synergistic effects are attributable to myostatin inhibition alone, due to the selectivity of the myostatin inhibitor used, without contribution from other structurally related growth factors such as GDF11 and Activins, which signal through the same receptors. This suggests that GLP-1 pathway activators, when used in conjunction with myostatin pathway inhibitors, can enhance overall weight loss, alter body composition by increasing the muscle to fat ratio, and/or otherwise improve treatments for the obese or overweight patient. Additionally, the current disclosure examines the effect of treatment with a myostatin inhibitor on the metabolic changes associated with weight loss in obese or overweight subjects. It is contemplated that treatment with a myostatin pathway inhibitor (e.g., a myostatin inhibitor, e.g., a myostatin-selective inhibitor) may reverse metabolic changes associated with weight loss and help maintain weight loss in obese or overweight subjects, e.g., after changes to diet and/or exercise regimen alone or in combination with other therapeutic interventions.

The present disclosure provides an inhibitor of the myostatin signaling pathway (e.g., a myostatin inhibitor, e.g., a myostatin-selective inhibitor) for use in the treatment of obesity or overweight in a subject as a monotherapy. According to some embodiments of the invention, the myostatin signaling pathway inhibitor (i.e., a myostatin-selective inhibitor) is used as an adjunct therapy, wherein the subject has received or is treated with an activator of the GLP-1 signaling pathway. According to some embodiments of the invention, the myostatin signaling pathway inhibitor (i.e., a myostatin-selective inhibitor) is used in conjunction with an activator of the GLP-1 signaling pathway. In some embodiments, the present disclosure encompasses use of a myostatin signaling pathway inhibitor (i.e., a myostatin-selective inhibitor) for treating a subject with obesity. According to the invention, the myostatin signaling pathway inhibitor is a myostatin-selective inhibitor, such as apitegromab, GYM-329, trevogrumab, or an MST1032 variant as described in PCT/JP2015/006323. In some embodiments, use of a myostatin-selective inhibitor may provide a safer therapeutic option (e.g., reduced toxicity, an improved safety profile, reduced side effects, and/or improved patient tolerance) as compared to non-selective myostatin inhibitors (e.g., myostatin inhibitors that also inhibit GDF11 and/or Activin A). In some embodiments, use of a myostatin-selective inhibitor may provide superior therapeutic efficacy as compared to non-selective myostatin inhibitors (e.g., myostatin inhibitors that also inhibit GDF11 and/or Activin A) (Muramatsu et al. Sci Rep. 2021 Jan 25;11(1):2160).

In some embodiments, the myostatin signaling pathway inhibitor is administered intravenously or subcutaneously. In some embodiments, the subject has obesity with type 2 diabetes. In some embodiments, the present disclosure encompasses use of apitegromab for treating a subject with obesity, wherein the subject optionally has obesity with type 2 diabetes. In some embodiments, the present disclosure encompasses the use of apitegromab in conjunction with a GLP-1 pathway activator for treating a subject with obesity, wherein the subject optionally has obesity with type 2 diabetes. In some embodiments, the present disclosure encompasses the use of GYM-329 for treating a subject with obesity, wherein the subject optionally has obesity with type 2 diabetes. In some embodiments, the present disclosure encompasses the use of GYM-329 in conjunction with a GLP-1 pathway activator for treating a subject with obesity, wherein the subject optionally has obesity with type 2 diabetes. In some embodiments, the present disclosure encompasses use of a MST1032 variant for treating a subject with obesity, wherein the subject optionally has obesity with type 2 diabetes. In some embodiments, the present disclosure encompasses use of a MST1032 variant in conjunction with a GLP-1 pathway activator for treating a subject with obesity, wherein the subject optionally has obesity with type 2 diabetes.

In some embodiments, the subject being given the inhibitor of the myostatin signaling pathway previously received a GLP-1 pathway activator (such as a GLP-1 analog) but failed to achieve weight loss of at least 5% of baseline body weight after 24 weeks of treatment comprising the GLP-1 pathway activator and/or was unable to tolerate a therapeutic dose of the GLP-1 pathway activator. In some embodiments, the subject received a GLP-1 pathway activator (such as a GLP-1 analog) but failed to achieve weight loss of at least 10% of baseline body weight after 24 weeks of treatment comprising the GLP-1 pathway activator. The "baseline body weight" refers to the subject's body weight measured just prior to or at the time of the initiation of the treatment (e.g., before a 24-week treatment), against which changes in body weight in response to the treatment are assessed.

The data provided herein raises the possibility that patients who do not respond adequately to a GLP-1 pathway activator, such as those who do not meet 5-10% weight loss goals with a treatment comprising a GLP-1 pathway activator, or those who cannot tolerate a required dose of the GLP-1 pathway activator, may benefit from a myostatin pathway inhibitor (e.g., a myostatin inhibitor, e.g., a myostatin-selective inhibitor) used in conjunction with a GLP-1 pathway activator. Without being bound by a particular theory, it is contemplated herein that concurrent inhibition of the myostatin pathway may enhance the effect of GLP-1 pathway activation and improve a therapeutic outcome or allow for a therapeutic outcome at a lower dose or less frequent administration of the GLP-1 pathway activator. Therefore, patients who respond poorly to the GLP-1 activation therapy may benefit from a myostatin pathway inhibitor in conjunction, either as a combination therapy or an adjunct therapy. In the context of the present disclosure, the expression "patients who respond poorly to a GLP-1 activation therapy" refers to those who fail to meet an intended weight management goal following the treatment that includes a GLP-1 pathway activator (such as 3 months, 6 months, 9 months and 12 months treatment), such as at least 5% or at least 10% body weight loss or who are unable to tolerate the treatment regimen required to attain an intended weight management goal. According to the invention, the myostatin pathway inhibitor is an antibody or antigen-binding fragment that selectively binds to myostatin, e.g., apitegromab, GYM329 (also known as RO7204239), trevogrumab, or any variants thereof. In another preferred embodiment, the myostatin pathway inhibitor is a MST1032 variant as disclosed in PCT/JP2015/006323. According to the invention, the GLP-1 pathway activator is a GLP-1 analog such as semaglutide, exenatide ER, liraglutide, lixisenatide, tirzepatide, dulaglutide, XW003, Noiiglutide, MEDI0382, and albiglutide. The GLP-1 pathway activator may be an agent that affects the GLP-1 pathway among other activities.

In some embodiments, the treatment comprising the GLP-1 pathway activator further includes a caloric restriction regimen (e.g., reduced calorie diet) and/or an exercise regimen (e.g., increased physical activity) as part of the treatment. In some embodiments, the subject who has received or is on the GLP-1 pathway activator treatment is unable to perform or adhere to the caloric restriction regimen and/or the exercise regimen as part of the GLP-1 pathway activator treatment. The myostatin pathway inhibitor, used in conjunction, may enhance the effect of the GLP-1 pathway activator even in the absence of or incomplete adherence to the caloric restriction regimen and/or the exercise regimen. According to the invention, the myostatin pathway inhibitor is a myostatin-selective antibody or antigen-binding fragment that selectively binds to myostatin, e.g., apitegromab, GYM329, trevogrumab, MST1032, or any variants thereof. According to the invention, the GLP-1 pathway activator is a GLP-1 analog such as semaglutide, exenatide ER, liraglutide, tirzepatide, lixisenatide, dulaglutide, XW003, Noiiglutide, MEDI0382, and albiglutide.

The present disclosure also provides an activator of the GLP-1 signaling pathway for use in the treatment of obesity or overweight in a subject as an adjunct therapy, wherein the subject is treated with an inhibitor of the myostatin signaling pathway.

The present disclosure further provides an inhibitor of the myostatin signaling pathway and an activator of the GLP-1 signaling pathway as a combination therapy for use in the treatment of obesity or overweight in the subject. The present disclosure also provides an inhibitor of the myostatin signaling pathway in conjunction with an activator of the GLP-1 signaling pathway for use in the treatment of obesity or overweight in the subject. The myostatin signaling inhibitor and the GLP-1 signaling activator can be formulated in a single pharmaceutical composition or can be formulated in separate pharmaceutical compositions and are used in amounts sufficient to treat obesity or overweight as weight management.

In various embodiments, use of inhibitors of the myostatin signaling pathway and activators of the GLP-1 signaling pathway may be optionally coupled with calorie restriction (e.g., diet) and/or moderate exercise to further enhance the effects. In some embodiments, the calorie restriction and/or the physical activity requirements for the approved GLP-1 analogs (such as semaglutide) for the treatment of obesity or overweight may be replaced with a myostatin pathway inhibitor (e.g., a myostatin inhibitor), used in conjunction with the approved GLP-1 analogs. In some embodiments, the degree (amount) of the calorie restriction and physical activity requirements for the approved GLP-1 analogs may be reduced with the use of a myostatin pathway inhibitor, such that less stringent caloric restriction and/or less stringent physical activity may be required to achieve equivalent weight loss benefits.

In some embodiments, overall weight loss is achieved with the use of an inhibitor of the myostatin signaling pathway (i.e., a myostatin-selective inhibitor) and an activator of the GLP-1 signaling pathway, while lean mass is increased during the same duration of use. In some embodiments, lean mass (e.g., % change in lean mass) is measured with the use of suitable techniques, such as ultrasound, quantitative nuclear magnetic resonance (qNMR), dual-energy X-ray absorptiometry (DXA), magnetic resonance imaging (MRI)-derived hepatic fat fraction, hydrodensitometry, air displacement plethysmography (ADP), bioelectrical impedance analysis (BIA), bioimpedance spectroscopy (BIS), electrical impedance myography (EIM), 3D body scanners, and multi-compartment models (e.g., 3-compartment and 4-compartment models). Thus, the myostatin pathway inhibitor may be used in conjunction with the GLP-1 pathway activators to prevent muscle loss while achieving overall weight loss.

In some embodiments, the use of an inhibitor of the myostatin signaling pathway (i.e., a myostatin-selective inhibitor) and an activator of the GLP-1 signaling pathway can reduce or slow fat mass gain, as compared to the latter alone. In some embodiments, the use of an inhibitor of the myostatin signaling pathway (i.e., a myostatin-selective inhibitor) can help maintain weight loss in a subject that has previously achieved weight loss. In some embodiments, fat mass (e.g., % change in fat mass) is measured with the use of suitable techniques, such as ultrasound, quantitative nuclear magnetic resonance (qNMR), dual-energy X-ray absorptiometry (DXA), magnetic resonance imaging (MRI)-derived hepatic fat fraction, hydrodensitometry, air displacement plethysmography (ADP), bioelectrical impedance analysis (BIA), bioimpedance spectroscopy (BIS), electrical impedance myography (EIM), 3D body scanners, and multi-compartment models (e.g., 3-compartment and 4-compartment models).

In some embodiments, the current disclosure encompasses the use of a myostatin signaling pathway inhibitor (i.e., a myostatin-selective inhibitor) for maintaining weight loss in an obese or overweight subject. Without wishing to be bound by theory, it is contemplated that the myostatin inhibition can lead to a reversal of certain metabolic changes associated with weight loss in a subject that otherwise favor weight regain. Such metabolic changes include but are not limited to decreased energy expenditure, decreased lean muscle mass, decreased mechanical efficiency of skeletal muscles, decreased insulin sensitivity, decreased circulating leptin levels, and/or decreased circulating adiponectin levels relative to the level(s) seen in the subject prior to treatment, i.e., in the absence of a myostatin inhibitor. Thus, in certain embodiments, the current disclosure encompasses the use of a myostatin signaling pathway inhibitor (i.e., a myostatin-selective inhibitor) for reversing metabolic changes associated with weight loss in an obese or overweight subject. The effects of such a use may include increasing energy expenditure, increasing lean muscle mass, increasing overall skeletal muscle function, increasing insulin sensitivity, increasing circulating leptin levels, and/or increasing circulating adiponectin levels as compared to baseline, i.e., in the absence of a myostatin inhibitor. In certain embodiments, the current disclosure encompasses use of a myostatin signaling pathway inhibitor (e.g., a myostatin inhibitor, e.g., a myostatin-selective inhibitor) for maintaining weight loss in an obese or overweight subject. According to the invention, the myostatin signaling pathway inhibitor is an antibody or antigen-binding fragment that selectively binds to myostatin, e.g., apitegromab, GYM-329, travogrumab, or a MST1032 variant. In certain embodiments, the myostatin signaling pathway inhibitor is administered intravenously or subcutaneously. In certain embodiments, the subject has previously received at least one dose of a GLP-1 pathway activator. In certain embodiments, the subject is receiving a GLP-1 pathway activator. In certain embodiments, the myostatin signaling pathway inhibitor (i.e., a myostatin-selective inhibitor) is used in conjunction with a GLP-1 pathway activator.

In some embodiments, the current disclosure encompasses the use of apitegromab for maintaining weight loss in an obese or overweight subject, wherein the obese or overweight subject optionally has type 2 diabetes. In some embodiments, the apitegromab is administered in conjunction with or complementary to a GLP-1 pathway activator. In some embodiments, the current disclosure encompasses the use of GYM-329 for maintaining weight loss in an obese or overweight subject, wherein the obese or overweight subject optionally has type 2 diabetes. In some embodiments, the GYM-329 is administered in conjunction with or complementary to a GLP-1 pathway activator. In some embodiments, the current disclosure encompasses the use of trevogrumab for maintaining weight loss in an obese or overweight subject, wherein the obese or overweight subject optionally has type 2 diabetes. In some embodiments, the trevogrumab is administered in conjunction with a GLP-1 pathway activator. In some embodiments, the current disclosure encompasses the use of a MST1032 variant for maintaining weight loss in an obese or overweight subject, wherein the obese or overweight subject optionally has type 2 diabetes. In some embodiments, the MST1032 variant is administered in conjunction with a GLP-1 pathway activator.

In some embodiments, the current disclosure encompasses the use of a myostatin signaling pathway inhibitor (i.e., a myostatin-selective inhibitor) for treating certain metabolic phenotypes in a leptin deficient subject. Such a subject may exhibit a metabolic phenotype that includes but is not limited to, low energy expenditure, low lean muscle mass, low mechanical efficiency of skeletal muscles, low insulin sensitivity, low circulating leptin levels, and/or low circulating adiponectin levels as compared to an individual with normal leptin levels (Rosenbaum et al. J Clin Invest. 2005 Dec; 115(12):3579-86). Thus, in certain embodiments, the current disclosure encompasses the use of a myostatin signaling pathway inhibitor (i.e., a myostatin-selective inhibitor) for reversing metabolic changes associated with leptin deficiency in a subject. The effects of such a use may include increasing energy expenditure, increasing lean muscle mass, increasing overall skeletal muscle function, increasing insulin sensitivity, and/or increasing circulating adiponectin levels as compared to baseline. In certain embodiments, the leptin deficient subject is obese or overweight. In certain embodiments, the current disclosure encompasses use of a myostatin signaling pathway inhibitor (i.e., a myostatin-selective inhibitor) for weight loss in the obese or overweight subject who is leptin deficient. In certain embodiments, the use of the myostatin signaling pathway inhibitor maintains weight loss in the leptin deficient subject. In certain embodiments, the subject has previously received at least one dose of a GLP-1 pathway activator. In certain embodiments, the subject is receiving a GLP-1 pathway activator. In certain embodiments, the myostatin signaling pathway inhibitor (i.e., a myostatin-selective inhibitor) is used in conjunction with a GLP-1 pathway activator.

According to the present disclosure, an inhibitor of the myostatin signaling pathway is used in conjunction with an activator of the GLP-1 signaling pathway to treat obesity (e.g., excess adiposity, overweight) in a subject. The inhibitor of the myostatin signaling pathway may be any agent capable of suppressing myostatin signaling. The inhibitors of the myostatin signaling pathway useful for carrying out the present invention are myostatin-selective inhibitors. Other inhibitors of the myostatin signaling pathway may be myostatin non-selective inhibitors. Generally, inhibitors of the myostatin signaling pathway may include low molecular weight compounds (i.e., small molecules) that inhibit the pathway, as well as biologics, such as antibodies or antigen-binding fragments thereof, and engineered protein constructs that comprise ligand-binding domains (such as soluble receptor ligand traps, follistatin-based engineered constructs, and adnectins). In some embodiments, the myostatin pathway inhibitors are antibodies (e.g., antigen-binding fragments thereof or engineered constructs that comprise such fragments) that bind mature myostatin (also known as GDF-8 or GDF8). Such antibodies are typically referred to as neutralizing antibodies because they bind the growth factor thereby blocking its ability to bind endogenous receptors for activating the signaling pathway. A myostatin pathway inhibitor may bind a myostatin receptor thereby interfering with ligand binding. These include antibodies that bind the extracellular portion(s) of the receptor. Non-limiting examples of such antibodies include bimagrumab (BYM338) which binds ActRIIB. The inhibitors of the myostatin signaling pathway useful for carrying out the invention are myostatin-selective inhibitors. In some embodiments, the myostatin-selective inhibitors are neutralizing antibodies that selectively bind mature myostatin (but not other growth factors such as GDF11 or Activin A). One example of such myostatin-selective antibody is trevogrumab, also known as REGN1033. In some embodiments, the myostatin-selective inhibitors are antibodies or antigen-binding fragments thereof that bind the pro/latent myostatin complex, thereby inhibiting activation (e.g., release) of mature myostatin. Such antibodies or antigen-binding fragments are referred to as "activation inhibitors" of myostatin. Non-limiting examples of myostatin-selective activation inhibitors include apitegromab (also known as SRK-015) and variants thereof, GYM329 and variants thereof, or a MST1032 variant as described in PCT/JP2015/006323.

According to the present disclosure, an activator of the GLP-1 signaling pathway is used in conjunction with an inhibitor of the myostatin signaling pathway to treat obesity (e.g., excess adiposity, overweight) in a subject. The activator of the GLP-1 signaling pathway may include any agent capable of enhancing the GLP-1 signaling. Activators of the GLP-1 signaling pathway useful for carrying out the invention are GLP-1 analogs. Endogenous GLP-1 is susceptible to degradation by peptidases and has a short half-life. Therefore, certain modifications can be introduced to the peptide hormone to generate GLP-1 analogs, aimed to prolong or stabilize its activity. In some embodiments, the GLP-1 analog comprises the amino acid sequence EGTFTSD (SEQ ID NO: 116). In some embodiments, GLP-1 analogs comprise the amino acid sequence HXXGXFTXD (SEQ ID NO: 117), wherein X is any amino acid residue. Non-limiting examples of GLP-1 analogs include semaglutide, exenatide ER, liraglutide, lixisenatide, tirzepatide, dulaglutide, XW003, Noiiglutide, MEDI0382, and albiglutide. Activators of the GLP-1 signaling pathway may be inhibitors of peptidases responsible for the degradation of GLP-1. The peptidase inhibitor may inhibit dipeptidyl peptidase 4 (DPP-4) and/or neutral endopeptidase (NEP). Activators of the GLP-1 signaling pathway may be agents capable of modulating GLP-1 receptor trafficking (e.g., agents that cause slower internalization of the receptor); agents that increase expression of cell-surface GLP-1 receptors; agents that enhance ligand-induced activation of the GLP-1 receptor; agents that enhance pathways such as cAMP signaling that are downstream of the GLP-1 receptor, and/or agents that antagonize the phosphatase responsible for de-phosphorylating the GLP-1 receptor.

Therapeutic methods disclosed herein are aimed to provide improved weight management regimen. Thus, inhibitors of the myostatin signaling pathway are used in conjunction with activators of the GLP-1 signaling pathway to treat obesity or overweight for weight management, e.g., chronic weight management in subject in need thereof.

In some embodiments, assessment of obesity and overweight is based on body mass index (BMI). BMI is the tool most commonly used to estimate and screen for overweight and obesity in adults and children. In some embodiments, BMI of the adult subject to be treated with the combination or adjunct therapy according to the present disclosure is 25 kg/m² or above (overweight). In some embodiments, the adult subject has a BMI between 25-29.9 kg/m² (overweight). In some embodiments, BMI of the adult subject to be treated with the combination or adjunct therapy of the present disclosure is 30 kg/m² or above (obesity). In some embodiments, BMI of the adult subject to be treated with the combination or adjunct therapy of the present disclosure is between 30 and 40. In some embodiments, BMI of the adult subject to be treated with the combination or adjunct therapy of the present disclosure is 40 or above (extreme obesity). In some embodiments, BMI of a child or adolescent subject aged 2-19 years to be treated with the combination or adjunct therapy according to the present disclosure is at or above the 85th percentile of children or young adults of the same sex and age (e.g., BMI at or above 85th percentile on the CDC growth charts). In some embodiments, BMI of a subject aged 2-19 years to be treated with the combination or adjunct therapy according to the present disclosure is at or above the 95th percentile of children or young adults of the same sex and age (e.g., BMI at or above 95th percentile on the CDC growth charts). In some embodiments, BMI of a subject aged 2-19 years to be treated with the combination or adjunct therapy according to the present disclosure is at or above 120 percent of the 95th percentile of children or young adults of the same sex and age (e.g., BMI at or above 120 percent of the 95th percentile on the CDC growth charts).

In some embodiments, the subject also suffers from or is at risk of developing one or more weight-related condition(s) (such as high blood pressure, type 2 diabetes, heart disease, stroke, fatty liver disease, kidney disease or high cholesterol). In some embodiments, the subject is an adult subject. In some embodiments, the subject is a child or adolescent subject aged 2-19 years. In some embodiments, the adult subject has a body mass index (BMI) of 27 kg/m² or greater with at least one weight-related ailment, or, the adult subject has a BMI of 30 kg/m² or greater. In some embodiments, the adult subject has BMI ranging from 28-40 kg/m², and optionally the subject has HbA1c of 6.5-10%. In some embodiments, the subject aged 2-19 years has a BMI that is at or above the 85th percentile of children or young adults of the same sex and age (e.g., BMI at or above 85th percentile on the CDC growth charts). In some embodiments, the subject aged 2-19 years has a BMI that is at or above the 95th percentile of children or young adults of the same sex and age (e.g., BMI at or above 95th percentile on the CDC growth charts). In some embodiments, the subject aged 2-19 years has a BMI that is at or above 120 percent of the 95th percentile of children or young adults of the same sex and age (e.g., BMI at or above 120 percent of the 95th percentile on the CDC growth charts). In some embodiments, the subject is on a diet (e.g., calorie restriction). In some embodiments, the subject is on an exercise regimen.

Thus, the therapeutic methods disclosed herein may be useful for chronic weight management aimed to treat excess adiposity and metabolic disturbances of subjects with obesity, wherein optionally the subject has or is at risk of developing type 2 diabetes associated with obesity, while maintaining or improving the body composition (e.g., lean-to-fat mass ratios) of the subject.

The present disclosure also encompasses various embodiments of therapeutic use in which an inhibitor of the myostatin signaling pathway is administered in conjunction with an activator of the GLP-1 signaling pathway for improving body composition in a subject. In some embodiments, the subject may be obese or overweight. In some embodiments, the subject may have or is at risk of developing metabolic condition(s) such as type 2 diabetes or metabolic syndrome. In some embodiments, the therapeutic use disclosed herein can improve body composition even if there is no overall weight loss (e.g., neutral effects on body weight) or even if there is overall weight gain in the subject. In some embodiments, the therapeutic use increases lean mass. In some embodiments, the therapeutic use decreases fat mass. In some embodiments, the therapeutic use increases lean-to-fat mass ratios. In some embodiments, the therapeutic use has a neutral effect on body weight. In some embodiments, the therapeutic use achieves overall weight loss in a subject. In some embodiments, therapeutic use maintains weight loss in a subject that has previously achieved weight loss. Body composition may be assessed or measured using suitable techniques, such as qNMR, dual energy x-ray absorptiometry (DXA), magnetic resonance imaging (MRI)-derived hepatic fat fraction, hydrodensitometry, air displacement plethysmography (ADP), bioelectrical impedance analysis (BIA), bioimpedance spectroscopy (BIS), electrical impedance myography (EIM), 3D body scanners, and multi-compartment models (e.g., 3-compartment and 4-compartment models).

Advantageously, a myostatin-selective inhibitor is used, which provides improved safety profiles, as compared to a non-selective inhibitor of myostatin.

### BRIEF DESCRIPTION OF THE FIGURES

FIG. 1A shows overall (whole body) weight loss in groups on a 20% or 30% calorie restriction (CR) treated with either the myostatin-selective inhibitor Ab2 or a control antibody. The left panel shows the body weight of the animals over the course of the study and the right panel shows the percent change in body weight.
FIG. 1B shows the percent change in lean muscle weight in groups on a 20% or 30% calorie restriction (CR) treated with either the myostatin-selective inhibitor Ab2 or a control antibody as measured by qNMR.
FIG. 1C shows a comparison of the percent change of lean mass weight during the study, measured on day 44, compared to baseline.
FIG. 1D shows the weight in milligrams of the average of left and right gastrocnemius muscle samples collected at the end of the study.
FIG. 1E shows the percentage difference in weight from the gastrocnemius muscles shown in FIG. 1D compared to the gastrocnemius muscle weight of the control mice receiving the high fat diet ad lib.
FIG. 1F shows the percentage of fat mass lost compared to the percentage of fat mass lost by the control mice receiving the high fat diet ad lib.
FIG. 1G shows that Ab2 significantly increased the percent loss of fat mass in the animals that were calorie restricted by 20%.
FIG. 1H shows the weight of the inguinal fat pads (average of both left & right fat pads) gathered at the end of the study. Ab2 significantly decreased the inguinal fat pad weight in animals that were calorie restricted by 20%.
FIG. 1I (left panel) shows serum leptin levels in mice that were calorie restricted by 20% compared to mice receiving the high fat diet ad lib. FIG. 1I (right panel) shows the leptin level normalized to the percentage of fat.
FIG. 2A shows the results of an initial dose-determining study for liraglutide in the C57BL/6NTac DIO mouse model.
FIG. 2B shows the % body weight change from baseline at the end of a 30-day study in mice receiving the control IgG1 alone, Ab2 alone, or IgG1 or Ab2 in conjunction with liraglutide at 0.03, 0.06, or 0.10 mg/kg. Liraglutide induced a dose proportional decrease in body weight and Ab2 enhanced body weight loss in the 0.1 mg/kg liraglutide group relative to the IgG control.
FIG. 2C shows the percentage of change in lean mass compared to baseline as measured by qNMR.
FIG. 2D shows the percent change in mass of the gastrocnemius muscle.
FIG. 2E shows the full body percentage change in fat mass compared to baseline, as measured by qNMR.
FIG. 2F shows the percent change in weight of the inguinal fat pad.
FIG. 2G shows the overall lipid count in livers from the mice at the end of the study, as measured by histology.
FIG. 3A shows that the myostatin inhibiting antibody Ab2 increased VO₂ in animals that were calorie restricted by 30%.
FIG. 3B shows that the myostatin inhibiting antibody Ab2 increased VO₂ in animals that were calorie restricted by 30%, when normalized to weight loss.
FIG. 3C shows that the myostatin inhibiting antibody Ab2 had no effect on VO₂ in animals that were calorie restricted by 30%, when normalized to lean mass.
FIG. 3D shows that the myostatin inhibiting antibody Ab2 increased energy expenditure in animals that were calorie restricted by 30%.
FIG. 3E shows that the myostatin inhibiting antibody Ab2 did not affect the respiratory exchange ratio (RER) in animals that were calorie restricted by 30%.
FIG. 4 shows body weight of mice undergoing 20% calorie restriction over time and treated with antibody Ab2. The left panel shows the whole body weight over the 44 days of the study and the right panel shows the percent change.
FIG. 5A shows that the myostatin inhibiting antibody Ab2 preserved lean body mass in animals that were calorie restricted by 20%.
FIG. 5B shows that animals treated with the anti-myostatin antibody Ab2 lost significantly less lean body mass than animals treated with a control antibody.
FIG. 5C shows that the myostatin inhibitor Ab2 increased gastrocnemius muscle weight in animals that were calorie restricted by 20%.
FIG. 5D shows that calorie restricted animals treated with a control antibody lost gastrocnemius muscle weight while the calorie restricted animals treated with Ab2 gained a significant amount of gastrocnemius muscle weight.
FIG. 5E shows that the myostatin inhibitor Ab2 decreased both O₂ consumption and CO₂ output compared to mice treated with control antibody in the animals that were calorie restricted by 20%.
FIG. 5F shows the average respiratory exchange ratio as a function of time over the course of the 72 hours that the mice spent in the metabolic cage
FIG. 5G shows the physical activity levels of mice that were calorie restricted by 20% and treated with antibody Ab2
FIG. 6 shows the reduction in fat mass in mice treated with Ab2 and/or 20% calorie restriction.
FIG. 7 shows oxygen and energy consumption in mice treated with Ab2 and/or 20% calorie restriction.

### DETAILED DESCRIPTION OF CERTAIN EMBODIMENTS

The present invention is based, at least in part, on the discovery that administration of a myostatin pathway inhibitor, e.g., a myostatin inhibitor such as a myostatin-selective inhibitor, in subjects having a metabolic disease, e.g., obesity and/or type 2 diabetes mellitus (T2DM) significantly improves both the physiological and the functional characteristics (e.g., improvements in body composition, glucose tolerance, etc.) of the affected subjects (e.g., a mammalian subject). In particular, the present inventors have surprisingly discovered that administration of a combination of a myostatin pathway inhibitor and a GLP-1 pathway activator can help preserve lean mass in such subjects, can prevent or reduce gain of fat mass, improve body composition by increasing the muscle to fat ratio, and/or obviate or reduce the need, or provide additional benefit in conjunction with calorie restriction and/or increased exercise.

### Definitions

The articles "a" and "an" are used herein to refer to one or to more than one (i.e., to at least one) of the grammatical object of the article. By way of example, "an element" means one element or more than one element.

Other than in the operating examples, or where otherwise indicated, all numbers expressing quantities of ingredients or reaction conditions used herein should be understood as modified in all instances by the term "about." The term "about" when used in connection with percentages may mean ±1%. Furthermore, the term "about" can mean within ±1% of a value.

*Activator* of *the GLP-1 signaling pathway*: The terms "activator of the GLP-1 signaling pathway" and "GLP-1 pathway activator" are used interchangeably herein and encompass any agent that increases or enhances the activity of the GLP-1 signaling pathway, irrespective of the mechanism of action. Increased or enhanced activity of the GLP-1 signaling pathway may be a result of, for example, a greater degree of activity, longer duration of activity, increased availability of one or more components of the signaling pathway, etc.

*Adjunct therapy*: The terms "adjunct therapy" and "add-on" therapy are used interchangeably herein and are intended to refer to a therapeutic regimen in which a second agent (used as an adjunct therapy) is administered to a subject who is on, has received, or to be treated with, a first agent (e.g., background therapy). The terms "in conjunction with" and "complementary to" are used interchangeably herein and intended to refer to therapies used together, whether concurrent or partially overlapping in time.

*Administer*/*administration:* The terms "administer", "administering" or "administration" include any method or act of delivery of a pharmacological agent (e.g., medicament) to an intended subject, e.g., patient. The pharmacological agent can be biologic, such as an antibody or an antigen-binding fragment thereof (e.g., a pharmaceutical composition comprising such an antibody or antigen-binding fragment, peptide agents, such as hormones and modified or synthetic analogs thereof, or low molecular weight agents (i.e., structurally-defined small molecules or chemical entities), into a subject's system or to a particular region in or on a subject (systemic and local administration, respectively).

*Adult:* As used herein, in the context of weight management, an "adult" patient refers to an individual who is older than 19 years of age (i.e., 20 or older) unless otherwise specified.

*Antibody*: As used herein the term "antibody" encompasses full-length immunoglobulins, antigen-binding portion(s)/fragment(s) thereof, and any variants thereof that retain the ability to bind a target antigen. Antibodies include human antibodies and humanized antibodies.

*Background therapy:* In the context of add-on or adjunct therapy, the term "background therapy" refers to approved treatment available to a patient or patient population, for a particular indication, or a condition associated with the indication, which the patient has received or is receiving prior to a second therapy to be added on as adjunct therapy for the same indication (e.g., prior to adjunct administration of a myostatin pathway inhibitor). Typically, background therapies are standard-on-care for the condition. For example, patients with type 2 diabetes may be on a biguanide, such as metformin (e.g., Fortamet, Glucophage, Glucophage XR, Glumetza, Riomet, Obimet, Gluformin, Dianben, Diabex, Diaformin, Metsol, Siofor, Metforgamma and Glifor) or metformin-containing medications that include one or more additional active agents (e.g., thiazolidinediones (glitazones) and rosiglitazone), as a background therapy. For example, patients with type 2 diabetes may be on a sulfonylurea (e.g., glimepiride, glipizide, glyburide, glipalamide, or chlorpropamide. Also by way of example, patients with type 2 diabetes may be on a thiazolidinedione, e.g., pioglitazone.

*Baseline:* The terms "baseline" and "baseline measurement" refer to measurement of a particular metric in a subject or a population of subjects before an action occurs to modify that metric, e.g., at the beginning of a study (prior to dosing). For example, in studying a treatment for a metabolic disease or disorder, a baseline or baseline measurement may refer to measurement of leptin levels, ghrelin levels, adiponectin levels, body weight, fat mass (e.g., total fat mass or visceral fat mass), lean mass (e.g., muscle mass), insulin levels (fasted or non-fasted), insulin sensitivity, glucose levels (fasted or non-fasted), rate of gastric emptying, metabolic rate (e.g., as measured by oxygen consumed and carbon dioxide expelled during a defined period of time), HbA1c levels, blood pressure and pulse, lipoprotein lipids, and/or measurement of waist circumference, among other measurements.

*Body composition:* The term "body composition" refers to relative components that make up a body, including fat mass, muscle (lean) mass, bone and water etc. In particular, in the context of weight management, body composition refers to ratios of lean vs fat mass in the body. Unless explicitly stated, as used herein the term refers to the body composition of the whole body (total body). Body composition can be measured by various suitable methods and techniques, including, body density, dual energy X-ray absorptiometry (DEXA), air displacement plethysmography (ADP), bioelectrical impedance analysis (BIA), body volume indicator (BVI), skin folds (with measuring caliper), Ultrasound, quantitative magnetic resonance (QMR), circumferences (e.g., as measured at waistline), and other measurements.

*Body* mass *index (BMI):* The term "body mass index" or "BMI" is a numerical value derived from the mass and height of a person and is defined as weight in kilograms divided by height in meters squared (expressed in units of kg/m²). BMI provides general body weight-height relationships which can be used to categorize a person as underweight, normal weight, overweight, obese or extreme obese, based on tissue mass (muscle, fat and bone) relative to height.

*Calorie restriction*/*caloric restriction:* The term refers to a form of dieting that requires a reduced overall calorie intake.

*Combination therapy*: As used herein, "combination therapy" refers to a therapeutic regimen involving administration of two or more active agents (e.g., two or more pharmacological agents) intended to treat a predetermined indication and/or conditions associated therewith. The two or more agents may be formulated as separate compositions (e.g., formulations) or may be formulated as a single composition (formulation). "Combination therapy" encompasses therapies used in conjunction with each other and complementary to each other.

The term "decrease" or"reduce", as used herein, in the context of a disease symptom refers to a statistically significant decrease in such level. The decrease can be, for example, at least 5%, 10%, 15%, 20%, 25%, 30%, 35%, 40%, 45%, 50%, 55%, 60%, 65%, 70%, 75%, 80%, 85%, 90%, or 95%, or below the level of detection for the detection method. The decrease can also be, for example, about 1-10%, 10-20%, 1-30%, 20-50%, 30-60%, 40-70%, 50-80%, or 60-90%. In certain embodiments, the reduction in an individual with a disorder may achieve a level accepted as within the normal range for an individual without such disorder.

*Dieting*/*diet regimen:* In the context of the present disclosure, certain dieting may be incorporated as part of weight management, e.g., obesity treatment which includes a pharmacological intervention. Dieting may include caloric/calorie restriction (i.e., reduced calorie intake or reduced absorption of calories) as well as alterations in choices about the type of food consumed (e.g., high protein, lower fat or lower carbohydrate regimens). Thus, a patient is on a "diet or reduced calorie regimen" when the patient incorporates or is instructed by a physician or equivalent to incorporate dieting into overall therapeutic regimen, e.g., as part of weight management,

*Effective amount:* As used herein, the terms "effective amount" and "effective dose" refer to any amount or dose of a compound or composition that is sufficient to fulfill its intended purpose(s), i.e., a desired biological or medicinal response in a tissue or subject at an acceptable benefit/risk ratio. For example, in certain embodiments of the present invention, the intended purpose may be to inhibit activation of myostatin in vivo, to achieve a clinically meaningful outcome associated with the myostatin inhibition.

In some embodiments, an effective amount is an amount that, when administered according to a particular regimen, produces a positive clinical outcome with a reasonably acceptable level of adverse effects (e.g., toxicity), such that the adverse effects, if present, are tolerable enough for a patient to continue with the therapeutic regimen, and the benefit of the therapy overweighs risk of toxicity. Those of ordinary skill in the art will appreciate that in some embodiments of the invention, a unit dosage may be considered to contain an effective amount if it contains an amount appropriate for administration in the context of a dosage regimen correlated with a positive outcome.

A therapeutically effective amount is commonly administered in a dosing regimen that may comprise multiple unit doses. For any particular pharmaceutical agent, a therapeutically effective amount (and/or an appropriate unit dose within an effective dosing regimen) may vary, for example, depending on route of administration, on combination with other pharmaceutical agents. In some embodiments, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the patient; the time of administration, route of administration, and/or rate of excretion or metabolism of the specific pharmaceutical agent employed; the duration of the treatment; and like factors as is well known in the medical arts.

An "effective amount" as used herein may also refer to the amount of each active agent required to confer a therapeutic effect on the subject, either alone or in combination with one or more other active agents. For example, an effective amount refers to the amount of a myostatin inhibitor, e.g., an antibody, or antigen-binding fragment thereof, of the present disclosure which is sufficient to achieve a biological effect, e.g., an increase in muscle mass or muscle fiber diameter, a switch in muscle fiber type, an increase in the amount of force generated by the muscle, an increase in mass and/or function of a muscle tissue in the subject; an increase in the metabolic rate of the subject; an increase in insulin sensitivity of the subject; an increase in a level of brown adipose tissue in the subject; an increase in a level of beige adipose tissue in the subject; a decrease in a level of white adipose tissue in the subject; a decrease in a level of visceral adipose tissue in the subject; a decrease in ratio of adipose-to-muscle tissue in the subject; an increase in glucose uptake by a brown adipose tissue, a beige adipose tissue, or a muscle tissue in the subject; a decrease in glucose uptake by a white adipose tissue or a liver tissue; a decrease in muscle catabolism of protein and/or muscle release of amino acids in the subject; an increase in insulin dependent glycemic control in the subject; a decrease in intramuscular fat infiltration in the subject; or a clinically significant outcome, e.g., partial and complete reversal of insulin resistance, overweight, or glycemic control, or the prevention of muscle loss or atrophy in the subject; and/or prevention of developing a metabolic disease in the subject.

Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation.

*Efficacy:* The term "efficacy" refers to a measurable biological or medicinal response in a subject as the result of administering a therapy in an amount that is sufficient to fulfill its intended purpose(s). For example, in certain embodiments of the present invention, the efficacy of a myostatin pathway inhibitor may refer to any desired clinically meaningful outcome that is associated with use of the inhibitor. In some embodiments, the efficacy of a GLP-1 pathway activator may refer to any desired clinically meaningful outcome that is associated with use of the GLP-1 pathway activator. In some embodiments, efficacy of a myostatin pathway inhibitor used in conjunction with a GLP-1 pathway activator is measured in a patient or patient population. In some embodiments, efficacy of a combination therapy comprising a myostatin pathway inhibitor and a GLP-1 pathway activator is measured in a patient or patient population who is treated with the combination. In some embodiments, efficacy of an adjunct therapy comprising a myostatin pathway inhibitor is measured in a patient or patient population who is treated with a GLP-1 pathway activator. In some embodiments, efficacy of an adjunct therapy comprising a GLP-1 pathway activator is measured in a patient or patient population who is treated with a myostatin pathway inhibitor. In a combination or adjunct therapy, the measured efficacy is preferably greater (more clinically meaningful) than monotherapy of either. In some embodiments, therapeutic efficacy refers to a clinically meaningful outcome for a subject (e.g., a mammalian subject) having a metabolic disorder, such as alleviation, reduction, delay, or prevention of one or more symptoms of a metabolic disorder in a subject receiving a myostatin pathway inhibitor in conjunction with a GLP-1 pathway activator. In some embodiments, such efficacy is evaluated by a measuring a change in body composition (e.g., a change in the muscle to fat ratio) at the end of a study or a treatment regimen, e.g., by DXA, qNMR, etc. In some embodiments, therapeutic efficacy is assessed by measuring glycemic control/insulin sensitivity (e.g., HbA1c measurements, fasting glucose and insulin, etc.), anthropometric measurements (body weight, BMI, waist circumference, weight to hip ratio, loss, stabilization, or gain of fat or muscle), each as compared to baseline. In some embodiments, PK/PD markers can be used as a measurement of efficacy, e.g., measurements of serum latent myostatin. In some embodiments, a therapeutically effective outcome of treatment with a myostatin pathway inhibitor and a GLP-1 pathway activator is an increase in the muscle to fat ratio, regardless of change in other properties such as body weight relative to baseline. In some embodiments, a therapeutically effective outcome comprises a decrease in body weight, a reduction or stabilization in fat mass, and/or an increase in muscle mass, relative to baseline.

*Exercise*/*exercise regimen:* As used herein, the term "exercise" includes any physical activities. A patient is on an "exercise regimen" when the patient incorporates or is instructed by a physician or equivalent to incorporate increased physical activity into overall therapeutic regimen, e.g., as part of weight management.

*Fc variant:* The term "Fc variant" refers to an antibody (immunoglobulins) comprising one or more mutations within the Fc region thereof, typically having the same CDR sequences as the parental (reference) antibody from which it is derived. Fc variants can be generated to have altered (e.g., increased) affinities to the FcR, such as FcRn. In some embodiments, altered affinities to FcRn result in altered biological activities in vivo, such as a longer serum half-life of the Fc variant, as compared to its parental antibody without the Fc mutation(s).

The term "gastric emptying" refers to the process by which the contents of the stomach are moved into the small intestine (i.e., the duodenum). The term "postprandial gastric emptying" refers to the rate at which gastric emptying takes place after a meal. Gastric emptying may be measured by any known method in the art, such as scintigraphy, magnetic resonance imaging (MRI), and real-time ultrasonography.

*GLP-1 analog:* As used herein, the term "GLP-1 analog" refers to a peptide or modified peptide bearing structural and functional similarities to the naturally-occurring GLP-1 and is capable of binding to and activating the GLP-1 receptor. Non-limiting examples of GLP-1 analogs include, semaglutide, exenatide ER, liraglutide, lixisenatide, tirzepatide, dulaglutide, XW003, Noiiglutide, MEDI0382, and albiglutide. GLP-1 analogs shall include peptides or modified peptides comprising an amino acid sequence EGTFTSD (SEQ ID NO: 116). GLP-1 analogs shall include peptides or modified peptides comprising an amino acid sequence HXXGXFTXD (SEQ ID NO: 117), wherein X is any amino acid residue.

*GLP-1 receptor agonist:* The term "GLP-1 receptor agonist" means an agent capable of binding to and activating the GLP-1 receptor. GLP-1 is a naturally-occurring agonist of the GLP-1 receptor. GLP-1 receptor agonists encompass GLP-1 analogs. A GLP-1 receptor agonist may be a small molecule GLP-1 receptor agonist. A GLP-1 receptor agonist may be a long-acting small molecule GLP-1 receptor agonist.

*GLP-1 pathway activator.* The terms "GLP-1 pathway activator" and "activator of the GLP-1 signaling pathway" are used interchangeably herein and encompass any agent that increases or enhances the activity of the GLP-1 signaling pathway, irrespective of the mechanism of action. Increased or enhanced activity of the GLP-1 signaling pathway may be a result of, for example, a greater degree of activity, longer duration of activity, increased availability of one or more components of the signaling pathway, etc. GLP-1 pathway activators may include agents that modulate upstream regulators of GLP-1 (e.g., dipeptididyl peptidase (DPP-IV) inhibitors). GLP-1 pathway activators may include agents that regulate the amount or activity of GLP-1 (e.g., agents that increase production of or secretion of GLP-1; GLP-1 stabilizers). GLP-1 pathway activators may include agents that increase activation of the GLP-1 receptor (GLP-1R). Agents that increase activation of the GLP-1R include GLP-1 agonists, which include GLP-1 analogs. GLP-1 pathway activators may include agents that activate signaling downstream of the GLP-1R (e.g., activators of PI3K, PKC, cAMP, etc.). GLP-1 pathway activators may include agents that modulate receptor GLP-1R expression and/or trafficking (e.g., inhibitors of GLP-1R internalization; see, e.g., Jones et al., Nat. Comm. (2018)9:1602). GLP-1 pathway activators encompass activators of the GLP-1R. A GLP-1 pathway activator may be a GLP-1 receptor agonist. GLP-1 pathway activators include, but are not limited to, antibodies and antigen binding fragments thereof, engineered protein constructs (such as Fc conjugates and multi-functional molecules comprising a GLP-1 analog), peptides, and small molecules. Therapeutic regimen that comprises a GLP-1 pathway activator (such as GLP-1 analogs) may further include dieting (e.g., reduced calorie diet or calorie restriction) and/or exercising (e.g., increased physical activity) as part of the treatment. In some embodiments, patients are provided with dieting and/or exercising counseling.

*Human antibody*/*humanized antibody*: The term "human antibody", as used herein, is intended to include antibodies having variable and constant regions derived from human germline immunoglobulin sequences and fragments thereof. The human antibodies of the disclosure may include amino acid residues not encoded by human germline immunoglobulin sequences (e.g., mutations introduced by random or site-specific mutagenesis in vitro or by somatic mutation in vivo), for example in the CDRs and in particular CDR3. The term "humanized antibody", as used herein, refers to antibodies from non-human species whose protein sequences have been modified to increase their similarity to antibodies produced in humans. "Humanized antibodies" may refer to antibodies in which CDR sequences derived from the germline of another mammalian species, such as a mouse, have been grafted onto human framework sequences.

The term "increase" in the context, e.g., of a disease in which a symptom can be a loss of function or loss of mass, e.g., muscle mass associated with a disease, refers to a statistically significant increase in such level. The increase can be, for example, at least 5%, 10%, 15%, 20%, 25%, 30%, 35%, 40%, 45%, 50%, 55%, 60%, 65%, 70%, 75%, 80%, 85%, 90%, or 95%, or above the level of detection for the detection method. The increase can also be, for example, about 1-10%, 10-20%, 1-30%, 20-50%, 30-60%, 40-70%, 50-80%, or 60-90%. In certain embodiments, the increase is up to a level accepted as within the range of normal for an individual without such disorder which can also be referred to as a normalization of a level. In certain embodiments, the increase is the normalization of the level of a sign or symptom of a disease, an increase in the difference between the subject level of a sign of the disease and the normal level of the sign for the disease. In certain embodiments, the methods include an increase in the mass and/or function of the muscle tissue after treatment of a subject with an antibody that specifically binds pro/latent myostatin. In certain embodiments, the methods include an increase in a level of pro-myostatin in a target muscle, as compared to a control level of pro-myostatin.

*Inhibitor* of *the myostatin signaling pathway*: The terms "inhibitor of the myostatin signaling pathway" and "myostatin pathway inhibitor" are used interchangeably herein and encompass any agent that decreases (reduces) or suppresses the activity of the myostatin signaling pathway, irrespective of the mechanism of action. Decreased or suppressed activity of the myostatin signaling pathway may be a result of, for example, a reduced degree of activity, shorter duration of activity, reduced availability of one or more components of the signaling pathway, etc.

*Insulin sensitivity; insulin resistance:* The term "insulin sensitivity" refers to the metabolic actions of insulin to promote glucose disposal in a subject's body. A subject is said to have increased insulin sensitivity if the subject requires smaller amounts of insulin to lower blood glucose levels as compared to the average in a human population. In contrast, a subject is said to have decreased insulin sensitivity if the subject requires higher amounts of insulin to lower blood glucose levels. A subject is said to have "insulin resistance" if the quantity of exogenous or endogenous insulin required to increase glucose uptake and utilization in a subject is significantly higher than that in a healthy subject. For instance, a subject is said to have "insulin resistance" if the quantity of exogenous or endogenous insulin required to increase glucose uptake and utilization in a subject is 10%, 20%, 30%, 40% 50%, 60%, 70%, 80%, 90%, 100%, or higher as compared to that in a healthy subject.

*Leanllean mass:* As used herein, "lean" mass or tissue generally corresponds to muscle mass or muscle tissue, as opposed to fat mass or fat tissue (e.g., adipose). Typically, greater ratios of lean mass relative to fat mass in the body indicate more desirable body composition.

*Ligand trap:* As used herein, the term "ligand trap" refers to a molecule or class of molecules comprising a ligand-binding fragment/moiety that serves as a "trap" to sequester ligands (such as growth factors), so as to prevent the ligand from binding to and activating endogenous receptors. Ligand traps may be derived from the naturally-occurring receptors of the ligand by incorporating the ligand-binding portion thereof. Examples of myostatin ligand traps include, without limitation, soluble receptor-based ligand traps, follistatin-based ligand traps, etc. Myostatin ligand traps may not be selective for myostatin but may also bind and inhibit additional ligands such as GDF11 and Activin A.

*Mature myostatin:* The term "mature myostatin" refers to the dimeric growth factor, which is also known as GDF8, and is released from the latent myostatin complex. Mature myostatin is the soluble and biologically active ligand capable of binding to and activating its receptors. Unless explicitly stated otherwise, the term "mature myostatin" refers to a fully processed, biologically active form of myostatin. A wildtype sequence of mature myostatin is provided as SEQ ID NO: 56. In some cases, mature myostatin may contain one or more mutations, which may exhibit altered structure/function or stability.

SEQ ID NO: 56
DFGLDCDEHSTESRCCRYPLTVDFEAFGWDWIIAPKRYKANYCSGECEFVFLQKYPHTHLVHQANPRGSAGPC
CTPTKMSPINMLYFNGKEQIIYGKIPAMVVDRCGCS

*Metabolic disorder:* The term "metabolic disorder" may also be referred to as metabolic disease or metabolic condition and encompasses any conditions involving dysregulation of the body's metabolic function, resulting in perturbation of the normal physiological state of homeostasis due to an alteration in metabolism (anabolism and/or catabolism). Metabolic disorders may be inherited or acquired. Non-limiting examples of metabolic disorders include obesity/overweight, type 2 diabetes mellitus, type 2 diabetes mellitus associated with obesity, and metabolic syndrome.

*Metabolic rate:* The term "metabolic rate" refers to the amount of energy expended over a specific period of time. It is typically measured in calories, kilocalories, or joules. Metabolic rate may be expressed as oxygen consumed or carbon dioxide produced per unit time.

*Metabolism:* The term "metabolism" refers to the processes involved in the biosynthesis and breakdown of components that make up a body, such as fats (e.g., adipose tissue), muscle and bones. "Fat metabolism" therefore means the process of biosynthesis and breakdown of fats.

*Myostatin:* In the context of the present disclosure, unless explicitly defined otherwise, the term "myostatin" can refer to any forms of the myostatin protein, such as pro-myostatin, latent myostatin and mature myostatin, each of which exists in dimers in vivo.

*Myostatin inhibitor:* As used herein, the term "myostatin inhibitor" refers to any agent that inhibits one or more forms of myostatin (e.g., pro-myostatin, latent myostatin and/or mature myostatin) and encompasses both selective inhibitors of myostatin and non-selective inhibitors of myostatin. Selective inhibitors of myostatin (i.e., myostatin-selective inhibitors) are substantially specific to and potent towards myostatin over other structurally related members of the TGFβ superfamily at given concentrations. In some embodiments, a myostatin inhibitor is selective if it binds to myostatin with 2-fold, 5-fold, 10-fold, 50-fold, 100-fold, 200-fold, 500-fold, 1,000-fold, or greater affinity as compared to another member of the TGFβ superfamily (e.g., GDF11 or Activin A). Examples of myostatin-selective inhibitors known in the art include apitegromab, trevogrumab, and GYM329. By contrast, non-selective inhibitors of myostatin inhibit myostatin, as well as one or more additional members of the TGFβ superfamily, such as GDF11, Activin A, Activin B, BMPs, etc. at given concentrations. Most of the myostatin inhibitors in the literature fall within the non-selective category. The term myostatin inhibitor encompasses any molecular modalities such as large molecules (biologics, such as antibodies and engineered protein constructs) and small molecules (such as structurally-defined low molecular weight chemical entities). A myostatin inhibitor may be an anti-myostatin antibody, or antigen binding fragment thereof, that binds pro- and/or latent myostatin and/or mature myostatin. In some embodiments, the myostatin inhibitor may be an anti-pro/latent myostatin antibody, or antigen binding fragment thereof, that preferentially (e.g., selectively) binds pro- and/or latent myostatin over mature myostatin. A myostatin inhibitor may be an antibody (such as a neutralizing antibody), an activation inhibitor (e.g., an antibody that inhibits activation of pro- and/or latent-myostatin), an adnectin, a peptibody, a receptor trap, or a ligand trap. A myostatin inhibitor may be a small molecule inhibitor. A myostatin inhibitor may refer to a gene therapy.

*Myostatin pathway inhibitor:* The terms "myostatin pathway inhibitor" and "inhibitor of the myostatin signaling pathway" are used interchangeably herein and encompass any agent that decreases (reduces) or suppresses the activity of the myostatin signaling pathway, irrespective of the mechanism of action. Decreased or suppressed activity of the myostatin signaling pathway may be a result of, for example, a reduced degree of activity, shorter duration of activity, reduced availability of one or more components of the signaling pathway, etc. Such inhibitors may be selective to myostatin per se, or may be non-selective such that they also inhibit at least one additional growth factor, such as GDF11 and Activin A. Such agents may reduce myostatin-related signaling by acting upstream of myostatin, on myostatin, or downstream of myostatin via a member of the myostatin-induced signaling cascade to alter a signaling function of the myostatin pathway. Myostatin pathway inhibitors may encompass inhibitors that act on molecules upstream of myostatin, e.g., to prevent activation of proteases that cleave pro- and/or latent-myostatin to its active form. In some embodiments, myostatin pathway inhibitors encompass myostatin inhibitors that act on myostatin to prevent its activation or to prevent its interaction with receptors. Myostatin pathway inhibitors may encompass antibodies and other inhibitors that act on myostatin receptors or downstream to prevent one or more effects of the myostatin signaling cascade. Myostatin pathway inhibitors include, but are not limited, antibodies or antigen-binding fragments thereof, small molecules, receptor traps, adnectins, affibodies, DARPins, Anticalins, Avimers, Versabodies, receptor inhibitors (such as a receptor antibody or a receptor kinase inhibitor), or gene therapies.

*Myostatin-selective inhibitor.* The term "myostatin-selective inhibitor" refers to an inhibitor of the myostatin signaling pathway (i.e., myostatin pathway inhibitor) which is selective to myostatin, but not GDF11, Activin A, or other TGFβ family members. In some embodiments, a myostatin inhibitor is selective if it binds to myostatin with a 2-fold, 5-fold, 10-fold, 50-fold, 100-fold, 200-fold, 500-fold, 1,000-fold, or greater affinity toward as compared to another member of the TGFβ superfamily (e.g., GDF11 or Activin A). In preferred embodiments, a selective myostatin inhibitor exhibits no detectable binding or potency towards other TGFβ family members. In some embodiments, myostatin-selective inhibitors are neutralizing antibodies that bind mature myostatin thereby inhibiting its activity. In some embodiments, myostatin-selective inhibitors are antibodies that bind pro- and/or latent myostatin, thereby inhibiting the activation step of myostatin. In some embodiments, the myostatin-selective inhibitor is Ab2, apitegromab, trevogrumab, GYM329, or a variant of the any one of the foregoing.

*Overweight*/*obesity*: A person whose weight is higher than what is considered as a normal weight adjusted for height is described as being overweight or having obesity. Using the BMI-based classification, for human adults (ages 20 and older), BMI of 18.5 to 24.9 is considered *normal weight;* BMI of 25 to 29.9 is considered *overweight;* BMI of 30+ is considered obese (including extreme obesity); and BMI of 40+ is considered *extremely obese.* For children and adolescents (ages 2-19), BMI at or above the 85^{th} percentile on the CDC growth chart is considered overweight or obese; BMI at or above the 95^{th} percentile on the CDC growth charts is considered obese (including extreme obesity); and, BMI at or above 120 percent of the 95^{th} percentile on the CDC growth chart is considered extremely obese.

*Prollatent myostatin:* As used herein, the term "pro/latent myostatin" refers to pro-myostatin, latent myostatin, or both (i.e., pro-forms or precursors of myostatin), but excludes mature myostatin. The pro- and latent forms of myostatin remain associated with a prodomain (e.g., LAP) and are *inactive* in that they are incapable of binding to the cellular myostatin receptors.

"Specific" and "specificity" in the context of an interaction between members of a specific binding pair (e.g., a ligand and a binding site, an antibody and an antigen, biotin and avidin) refer to the selective reactivity of the interaction. The phrase "specifically binds to" and analogous phrases, in the context of antibodies, refer to the ability of antibodies (or antigenically reactive fragments thereof) to bind specifically to an antigen (or a fragment thereof) and not bind specifically to other entities. Specific binding is understood as a preference for binding a certain antigen, epitope, receptor ligand, or binding partner with, for example, at least 2-fold, 5-fold, 10-fold, 50-fold, 100-fold, 200-fold, 500-fold, or 1,000-fold preference over a control non-specific antigen, epitope, receptor ligand, or binding partner. "Specific binding" as used herein can also refer to binding pairs based on binding kinetics such as Kₒₙ, K_{off}, and K_{D}. For example, a ligand can be understood to bind specifically to its target site if it has a K_{off} of 10⁻²sec⁻¹ or less, 10⁻³sec⁻¹ or less, 10⁻⁴sec⁻¹ or less, 10⁻⁵sec⁻¹ or less, or 10⁻⁶ sec⁻¹ or less; and/ or a K_{D} of 10⁻⁸ M or less, 10⁻⁹ M or less, 10⁻¹⁰ M or less, or 10⁻¹¹ M or less, or 10⁻¹¹ M or less, e.g., as measured by ELISA and Octet. It is understood that various proteins can share common epitopes or other binding sites (e.g., kinase reactive sites). In certain embodiments, binding sites may bind more than one ligand, but still can be considered to have specificity based on binding preference as compared to a non-specific antigen and/ or by having certain binding kinetic parameters. Methods of selecting appropriate non-specific controls are within the ability of those of skill in the art. Binding assays are typically performed under physiological conditions. In some embodiments, an antibody may also "selectively" (i.e., "preferentially") bind a target antigen if it binds that target with a comparatively greater strength than the strength of binding shown to other antigens, e.g., a 2-fold, 5-fold, 10-fold, 50-fold, 100-fold, 200-fold, 500-fold, 1000-fold, or greater comparative affinity for a target antigen (e.g., pro/latent myostatin) than for a non-target antigen (e.g., GDF11, Activin A, or other TGFβ superfamily members). In preferred embodiments, a selective myostatin inhibitor exhibits no detectable binding or potency towards other TGFβ family members.

*Subject:* As used herein, the term "subject" is a target to whom the therapy or therapies described herein may be administered. In a clinical context, the terms "subject" (e.g., human subjects) and "patient" may be used interchangeably. In some embodiments, a subject is a vertebrate, in particular a mammal, in need of treatment, e.g., companion animals (e.g., dogs, cats and the like), farm animals (e.g., cows, pigs, horses, sheep, goats, poultry and the like) and laboratory animals (e.g., rats, mice, guinea pigs and the like). In some embodiments, the subject is a human who will benefit from or in need of treatment. In some embodiments, a subject is a human subject. In some embodiments, the subject is a child or adolescent subject aged 2-19 years. In some embodiments, the subject, is a subject aged 12 years or older. In some embodiments, the subject is a subject aged 12-17. In some embodiments, the subject is an adult subject aged 20 years or older. In some embodiments, the subject is an overweight, obese, or extremely obese human. In some embodiments, the subject has metabolic syndrome, type 2 diabetes, or type 2 diabetes associated with obesity.

The term "total fat mass" refers to the cumulative fat content in a subject's body. Total fat mass includes fat made up of various types of fat cells, such as white fat, brown fat, and beige fat, and includes fat storage in different body compartments, such as essential fat, subcutaneous fat, and visceral fat. Total fat mass may be measured or estimated by any method known in the art, including by skinfold measurements using calipers, measuring the circumference of certain body parts, dual-energy X-ray absorptiometry (DXA), hydrostatic weighing, air displacement plethysmography, bioelectric impedance analysis, bioimpedance spectroscopy, electrical impedance myography, 3-dimensional body scanners, multi-compartment models, or magnetic resonance imaging. The term "fat mass gain" or "fat mass loss" refers to a change in the amount of fat mass measured as compared to a baseline measurement. For example, a subject having a metabolic disorder may exhibit fat mass loss or visceral fat mass loss after a treatment for the metabolic disorder (e.g., treatment with a myostatin pathway inhibitor, e.g., in conjunction with a GLP-1 pathway activator). The term "visceral fat" refers to fat content that is predominantly made up of white fat cells and is mainly found in the abdominal area of a subject's body and around the subject's major organs, such as the liver, kidneys, pancreas, intestines, and heart.

*Treat*/*treating*/*treatment:* Treatment of a condition or disease in a subject refers to the act of providing a therapeutic regimen aimed to alleviate, prevent, or improve a medical condition. Thus, the terms treating or treatment do not necessarily require a complete treatment or prevention of the disease or disorder. In one embodiment, the symptoms of a disease or disorder are alleviated by at least 5%, at least 10%, at least 20%, at least 30%, at least 40%, or at least 50%.

*Weight loss:* Weight loss refers to a reduction of body weight, irrespective of particular tissue(s) being lost. For example, weight loss per se does not distinguish between loss in fat mass vs muscle mass. Overall loss of total body weight does not necessarily reflect improved body composition,

*Weight management:* As used herein, the term "weight management" encompasses measures taken to lose weight, sustain weight, as well as to reduce adipose tissue, increase lean mass, or otherwise to improve or sustain body composition, Successful weight management that is clinically meaningful may or may not accompany overall weight loss. Thus, weight management can include diet (e.g., a calorie restriction diet, e.g., reduced calorie intake or reduced absorption of calories), an exercise regimen, and/or medication (e.g., a myostatin pathway inhibitor and/or a GLP-1 pathway activator) in order to reduce the amount of total body weight, reduce the amount of total fat mass, reduce the amount of visceral fat mass, increase the metabolic rate, increase the amount of lean mass, and/or increase the ratio of muscle to fat, or otherwise to improve body composition, in a subject.

*Weight-related condition:* The term "weight-related condition" or "weight-related problem" as used herein, refers to one or more medical condition(s) associated with excess fat mass (i.e., in addition to being overweight or obese), where the excess fat mass of the subject is a contributing factor. Non-limiting examples of weight-related conditions include type 2 diabetes mellitus, high blood pressure, high triglyceride or cholesterol level, heart disease, stroke, kidney disease, fatty liver (e.g., NAFLD and NASH) and sleep apnea.

### Glucagon Like Peptide 1 Receptor (GLP-1R)

In various embodiments agents useful for carrying out the present invention include agents that enhance the GLP-1 signaling pathway. GLP-1 signals through its endogenous receptor, GLP-1 receptor (GLP-1R). Human GLP-1R (UniProt Accession No. P43220) is a 463-amino acid, G protein-coupled receptor formed by eight hydrophobic domains, mainly expressed in pancreatic islets. This receptor, which functions at the cell surface, becomes internalized in response to binding by peptide hormone glucagon-like peptide 1 (GLP-1) and GLP-1 analogs, and it plays an important role in the signaling cascades leading to insulin secretion.

GLP-1Rs are particularly abundant in pancreatic β-cells, where they drive glucose-dependent insulin secretion, but are also present in pancreatic α-cells, where they mediate the inhibition of glucagon secretion, and in the intestine, lung, kidney, heart, blood vessels, lymphocytes, and peripheral and central nervous systems. In the cardiovascular system, GLP-1R is abundant in the vasculature, mainly in arteries and arterioles where GLP-1 exerts vasodilatory effects, whereas, in the heart, GLP-1R expression is about sixfold greater in atrial than ventricular myocytes.

Wild-type glucagon-like peptide-1(7-36)amide (GLP-1) is a secreted peptide that acts as a key determinant of blood glucose homeostasis by virtue of its abilities to slow gastric emptying, to enhance pancreatic insulin secretion, and to suppress pancreatic glucagon secretion (see, e.g., Prog Mol Biol Transl Sci. 2014; 121: 23-65.). GLP-1 is secreted from L cells of the gastrointestinal mucosa in response to food consumption and acts to lower blood glucose. Wild-type GLP-1 is quickly inactivated due to its enzymatic degradation by dipeptidyl-peptidase-IV (DPP-IV). Released GLP-1 activates enteric and autonomic reflexes while also circulating as an incretin hormone to control endocrine pancreas function. Upon ligand (GLP-1) binding, GLP-1R initiates a cascade that involves activation of membrane bound adenyl cyclase and consequent production of cyclic adenosine monophosphate (cAMP). Downstream of cAMP formation, several signal transduction pathways can be initiated, which generally require activation of either one or both of the cellular cAMP effectors, protein kinase A (PKA) and exchange protein directly activated by cAMP (EPAC). The downstream signaling cascade, can induce glucose stimulated insulin secretion (GSIS) in β-cells, as well as inhibition of α-cell glucagon release. In addition, binding of GLP-1R by GLP-1 and its analogs also initiates a variety of anti-diabetic effects, including, but not limited to, reduction in gastric emptying, increase in satiety and inhibition of food motivated behavior, replenishment of insulin stores, as well as cytoprotective and anti-inflammatory actions on β-cells.

### GLP-1pathway activators

The present disclosure provides use of agents that activate or enhance the GLP-1 signaling pathway, in conjunction with myostatin pathway inhibitors, for weight management and improved body composition. Use in conjunction may involve combination therapy or adjunct therapy. Because of the potential beneficial effects on glucose homeostasis, primarily through its regulation of beta cell mass, function, and viability, the GLP-1 signaling pathway is an attractive therapeutic target for development of treatments of metabolic disorders, such as treatments including (but not limited to) activators of GLP-1/GLP-1R and the associated signaling pathway. For example, use of GLP-1 pathway activators may provide a number of benefits for a subject (e.g., a mammal) having type 2 diabetes mellitus (T2DM), such as enhanced appropriate pancreatic beta cell (insulin and amylin) secretion, pancreatic alpha cell (glucagon) suppression, decreased liver glucose production, increased satiety through the central nervous system, slowed gastric emptying time, and increased insulin uptake in peripheral tissue via weight loss, limitations include requirements for concurrent dieting and increased physical activity, as well as safety concerns. Novel weight management approach disclosed herein combines the benefit of GLP-1 pathway activators with myostatin pathway inhibitors in order to enhance clinical benefit. Without wishing to be limited by theory, it is contemplated that at least some of the weight loss effects of GLP1R agonists are mediated via direct action on CNS GLP1R or the result of downstream activation of afferent neuronal GLP1R.

According to the present disclosure, "activators of the GLP-1 pathway" or "GLP-1 pathway activators" include (but are not limited to) insulin production pathway activators (e.g., activators of cAMP, EPAC, CREB, or EGFR signaling pathways) and agents that act on multiple pathways that include an effect on insulin production, such as metformin. Also included are GLP-1 enhancers (e.g., agents that increase production of or secretion of GLP-1, GLP-1 stabilizers); modulators of GLP-1R trafficking (e.g., enhancers of receptor membrane localization, inhibitors of receptor internalization), GLP-1 degradation inhibitors (e.g., DPP-IV inhibitors); GLP-1 secretagogues, and GLP-1 receptor agonists.

"Insulin secretion" refers to the level of insulin released by the pancreas. Levels of insulin secretion in a subject may be estimated by proxy using measurements of fasting and/or non-fasting blood glucose concentrations. For example, the normal fasting blood glucose concentration in humans is between 80 mg/100 mL and 90 mg/100 mL, which is linked to very low levels of insulin secretion. Serum insulin levels may also be measured using any known method in the art, such as by enzyme-linked immunoassay (ELISA). Agents that enhance the GLP-1 signaling pathway, including GLP-1 (and GLP-1 analogs) and gastroinhibitory intestinal polypeptide (GIP), may promote/stimulate insulin secretion.

Useful activators of the GLP-1 signaling pathway include GLP-1 receptor agonists, such as GLP-1 analogs. GLP-1 analogs share certain structural similarities with the GLP-1 peptide and include modified peptides that retain the ability to bind to and activate the GLP-1 receptors. In some embodiments, GLP-1 analogs comprise the amino acid sequence EGTFTSD (SEQ ID NO: 116). In some embodiments, GLP-1 analogs comprise the amino acid sequence HXXGXFTXD, wherein X is any amino acid residue (SEQ ID NO: 117). According to the present disclosure, GLP-1 signaling pathway activators may be used in conjunction with a myostatin pathway inhibitor. In some embodiments, the GLP-1 pathway activator and the myostatin pathway inhibitor may be used as a combination therapy or adjunct (or add-on) therapy in the treatment of a metabolic disorder such as obesity in a subject. In some embodiments, the GLP-1 pathway activator and the myostatin pathway inhibitor may be used as a combination therapy or adjunct (or add-on) therapy or in conjunction with or complementary to in a weight management regimen in a subject. In some embodiments, the GLP-1 pathway activator and the myostatin pathway inhibitor may be used as a combination therapy or adjunct (or add-on) therapy or in conjunction with or complementary to, in the treatment of a metabolic disorder to improve body composition in a subject.

Non-limiting examples of GLP-1 analogs include albiglutide, taspoglutide, semaglutide, exenatide, BPI-3016, GW002, glutazumab, exendin-4, exenatide, GLP-1 (7-36)NH2, everestmab, liraglutide, lixisenatide, tirzepatide, XW003, Noiiglutide, MEDI0382, and dulaglutide. In one embodiment, the GLP-1 analog is liraglutide, which is currently approved for type 2 diabetes and obesity.

Liraglutide (Victoza^{®}, Saxenda^{®}, CAS Number 204656-20-2) is an acylated GLP-1 agonist derived from GLP-1(7-37). While wild-type GLP-1 has a plasma half-life of 1.5-2 minutes due to degradation by the enzyme dipeptidyl peptidase-4 (DPP-IV) and/or neutral endopeptidases, liraglutide is an acylated form of GLP-1 engineered to bind albumin when administered to a subject, thus greatly extending its half-life compared to GLP-1(7-37), with a plasma half-life of 13 hours. Liraglutide is also available in combination with insulin degludec under the name Xultophy^{®}, Semaglutide (Ozempic^{®}, Rybelsus^{®}, Wegovy^{®}, CAS Number 910463-68-2) is a modified GLP-1 peptide comprising two amino acid replacements: 2-aminoisobutyric acid replaces the alanine moiety at position 8 and arginine replaces the lysine moiety at position 34. The substitution at position 8 prevents breakdown by the enzyme DPP-IV. Semaglutide was approved by the USFDA in 2017. See, e.g., Novo Nordisk A/S. Ozempic (semaglutide) injection, for subcutaneous use; prescribing information; 2020.

Dulaglutide (Trulicity^{®}, CAS Number 923950-08-7) comprises GLP-1(7-37) covalently linked to an Fc fragment of human IgG4. Dulaglutide is approved for adults with type 2 diabetes mellitus (combined with diet and exercise). See, e.g., Meece J., Adv Ther. 2017;34:638-657.

Exenatide (Byetta^{®}, Bydureon^{®}, CAS Number 141758-74-9) is a synthetic form of the GLP-1 mimetic exendin-4 (a Gila monster saliva hormone) approved by the USFDA in 2005 for treatment of diabetes mellitus type 2 combined with diet and exercise.

Glutazumab is an antibody fusion protein engineered by linking the human GLP-1 derivative to a humanized GLP-1R antibody via a peptide linker. Li et al., Biochem Pharmacol. 2018 Apr;150:46-53.

Lixisenatide (Lyxumia^{®}, Adlyxin^{®}, CAS Number 320367-13-3) is a GLP-1 mimetic approved for treatment of diabetes mellitus type 2 in conjunction with diet and exercise. See, e.g., Sanofi-Aventis US. Adlyxin (lixisenatide), prescribing information, 2016. Lixisenatide is also available in combination with insulin glargine under the name Soliqua^{®}/Suliqua^{®}.

Everestmab is a long-acting GLP-1/anti-GLP1R fusion protein comprising a mutated GLP-1(A8G) fused to tandem bispecific humanized GLP-1R targeting and albumin-binding nanobodies designed for treatment of diabetes mellitus type 2. Pan et al., ARTIFICIAL CELLS, NANOMEDICINE, AND BIOTECHNOLOGY 2020, VOL. 48, NO. 1, 854-866.

Albiglutide (Eperzan^{®}, Tanzeum^{®}, CAS Number 782500-75-8) is a GLP-1R agonist that was approved for type 2 diabetes mellitus. Its manufacture was discontinued in 2018 due to lack of sales. See, e.g., GlaxoSmithKline LLC. Tanzeum^{®} (albiglutide) for injection, for subcutaneous use prescribing information; 2017.

Taspoglutide is a GLP-1R agonist that was previously under clinical investigation for treatment of diabetes mellitus type 2.

Tirzepatide (LY3298176) is a dual glucose-dependent insulinotropic polypeptide, GIP and GLP-1 receptor agonist that is under clinical investigation for the treatment of type 2 diabetes. It was made by engineering GLP-1 activity into the GIP sequence and has been shown to improve glycemic control and to reduce body weight (Rosenstock et al., Lancet (2020) 398:143-155).

BP3016 is a long-acting hGLP-1 analog comprising human GLP-1(7-37) with engineered resistance to DPP-IV cleavage. See, e.g., Pharmacol Res. 2017 Aug;122:130-139.

XW003 is an acylated human glucagon-like peptide-1 (GLP-1) analog that has been shown to induce dose-dependent weight reductions in early clinical trials. The drug is currently in phase 2 stage of development for the treatment of obesity. See, e.g., ClinicalTrials.gov identifier NCT05111912.

Noiiglutide is a 40-mer exenatide analog, which acts as a glucagon-like peptide-1 (GLP-1) agonist. The drug is being developed by Jiangsu Hansoh Pharmaceutical for the treatment of obesity. It is currently in phase 2 clinical development. See, e.g., Cabri et al. Front Mol Biosci. 2021; 8: 697586.

MEDI0382 is an oxyntomodulin-like peptide with targeted GLP-1 and glucagon receptor activity. It is currently in phase 2 stage of clinical development for the treatment of obesity. See, e.g., Ambery et al. Lancet. 2018 Jun 30;391(10140):2607-2618.

According to the present disclosure, GLP-1 pathway activators include danuglipron (Pfizer), GLP-1 receptor agonist/GIP receptor antagonist combination such as AMG 133 (Amgen), mas receptor activators, e.g., Sarconeos, GLP-1/GIP combination such as tirzepatide (Eli Lilly), amylin/GLP-1 combination such as cagrilintide/semaglutide combination (Novo Nordisk), GLP-1/glucagon combination such as DD01 (Neuraly), GLP-1/glucagon combination such as ALT-801 (Altimmune), GLP-1/GIP such as CT-388 (Carmot), GLP-1/glucagon dual agonist such as IBI362 (mazdutide) (LY-330567 (Innovent/Eli Lilly), danuglipron (PF-06882961) (Pfizer), setmelanotide (Rhythm), GLP-1/GIP/Glucagon triple receptor agonist such as LY3437943 (Eli Lilly) and a dual GIP/GLP-1 receptor agonist such as LY3298176 (Eli Lilly).

GLP-1 pathway activators further encompass agents that act on multiple pathways, including the GLP-1 pathway, such as metformin and derivatives thereof. Rena et al., Diabetologia 60:1577-85 (2017). Non-limiting examples of metaformin include Fortamet, Glucophage, Glucophage XR, Glumetza, Riomet, Obimet, Gluformin, Dianben, Diabex, Diaformin, Metsol, Siofor, Metforgamma and Glifor. GLP-1 pathway activators also include metformin-containing medications that include additional active agents. Examples include, but are not limited to, thiazolidinediones (glitazones) and rosiglitazone. A GLP-1 pathway activator may be FGF-2 or a peptide derived therefrom

### Myostatin

Myostatin, also known as GDF8 (or GDF-8), is a member of the TGFβ superfamily and belongs to a subfamily including two members: myostatin and GDF11. Like other members of the TGFβ superfamily, myostatin and GDF11 are both initially expressed as inactive precursor polypeptides (termed pro-myostatin and proGDF11, respectively). In the overall structure of pro-myostatin, the mature growth factor is held locked in a cage comprised **of two alpha** helices connected by a loop termed the "latency lasso". Human myostatin corresponds to UniProt Accession No. 014793; murine myostatin corresponds to UniProt Accession No. O08689.

Myostatin is a well-characterized negative regulator of skeletal muscle mass that is released from an autoinhibitory N-terminal prodomain by two separate protease cleavage steps. These cleavage events, within the muscle fiber microenvironment, for example, may be referred to as supracellular activation. Following activation, mature myostatin signals by binding to a complex of Type I and II cell surface receptors (Alk4/5 and ActRIIB) whose downstream signaling induces muscle breakdown and atrophy. There has long been interest in myostatin as a potential target for the treatment of muscle wasting; however, almost all clinical programs have failed and discontinued to date, casting doubt as to its potential. Indeed, some reports have suggested that myostatin expression levels appear to be reduced in a number of muscle disorders, leading to increased skepticism of myostatin as a therapeutic target (see, for example, Mariot et al. (2017) Nat. Com. 8:1859; Burch et al. (2017) J. Neurol). Moreover, in a 2017 paper, Latres et al. suggested that Activin A, not myostatin, appears to be the prominent regulator of skeletal muscle growth. Notwithstanding the foregoing, data presented herein support the notion that myostatin inhibition may be an effective approach to weight management and its effects may be enhanced when the GLP-1 signaling pathway is concurrently activated. Accordingly, the present disclosure includes the use of any agents that inhibit or suppress the myostatin signaling pathway, used in conjunction with activators of the GLP-1 signaling pathway as described herein.

Activation and release of mature myostatin is accomplished by several discrete protease cleavage events. The first cleavage step of pro-myostatin and proGDF11 is carried out by a proprotein convertase, which cuts at a conserved RXXR site between the prodomain and mature growth factor. This cleavage produces a "latent-myostatin," in which the mature myostatin is shielded from binding to its receptors by the prodomain. Activation and release of the mature, active myostatin growth factor is accomplished after cleavage of latent-myostatin by an additional protease from the BMP/tolloid family, such as mTLL-2. As used herein, the term "mature myostatin" can refer to both full-length mature myostatin, as well as fragments of the full-length mature myostatin which retain biological activity.

The term "pro-myostatin," also known as "proGDF8," refers to an inactive precursor of mature myostatin which comprises a disulfide-linked homodimer, each molecule of the homodimer comprising the amino terminal prodomain covalently bound to the carboxyl terminal mature myostatin domain. In one embodiment, "pro-myostatin" has not been cleaved by either a proprotein convertase, or a protease from the BMP/tolloid family. Exemplary pro-myostatin sequences, variants thereof, and methods of generating pro-myostatin are well known in the art and described in more detail herein.

As used herein the term "latent-myostatin" refers to an inactive precursor of mature myostatin which comprises a disulfide-linked homodimer, each molecule of the homodimer comprising the amino terminal prodomain non-covalently bound to the carboxyl terminal mature myostatin domain. In one embodiment, "latent-myostatin" is generated from a pro-myostatin that has been cleaved by a proprotein convertase, but which has not been cleaved by a protease from the BMP/tolloid family. In another embodiment, "latent-myostatin" can be generated by combining the prodomain and the carboxy terminal mature myostatin domain in vitro and allowing them to fold properly. See, for example, Sengle et al., J. Biol. Chem., 286(7):5087-5099, 2011. Exemplary latent-myostatin sequences, variants thereof, and methods of generating latent-myostatin are well known in the art and described in more detail herein.
proGDF8 (human): 




 (SEQ ID NO: 52).
proGDF8 (rat): 
NEDSEREANVEKEGLCNACAWRQNTRYSRIEAIKIQILSKLRLETAPNISKDAIRQLLPRAPPLRELIDQYDV
QRDDSSDGSLEDDDYHATTETIITMPTESDFLMQADGKPKCCFFKFSSKIQYNKVVKAQLWIYLRAVKTPTTVFVQ
ILRLIKPMKDGTRYTGIRSLKLDMSPGTGIWQSIDVKTVLQNWLKQPESNLGIEIKALDENGHDLAVTFPGPGEDGL
NPFLEVKVTDTPKRSRRDFGLDCDEHSTESRCCRYPLTVDFEAFGWDWIIAPKRYKANYCSGECEFVFLQKYPH
THLVHQANPRGSAGPCCTPTKMSPINMLYFNGKEQIIYGKIPAMVVDRCGCS (SEQ ID NO: 53).
proGDF8 (mouse): 
NEGSEREENVEKEGLCNACAWRQNTRYSRIEAIKIQILSKLRLETAPNISKDAIRQLLPRAPPLRELIDQYDV
QRDDSSDGSLEDDDYHATTETIITMPTESDFLMQADGKPKCCFFKFSSKIQYNKVVKAQLWIYLRPVKTPTTVFVQ
ILRLIKPMKDGTRYTGIRSLKLDMSPGTGIWQSIDVKTVLQNWLKQPESNLGIEIKALDENGHDLAVTFPGPGEDGL
NPFLEVKVTDTPKRSRRDFGLDCDEHSTESRCCRYPLTVDFEAFGWDWIIAPKRYKANYCSGECEFVFLQKYPH
THLVHQANPRGSAGPCCTPTKMSPINMLYFNGKEQIIYGKIPAMVVDRCGCS (SEQ ID NO: 54).
proGDF8 (cynomolgus):

NENSEQKENVEKEGLCNACTWRQNTKSSRIEAIKIQILSKLRLETAPNISKDAIRQLLPKAPPLRELIDQYDV
QRDDSSDGSLEDDDYHATTETIITMPTESDFLMQVDGKPKCCFFKFSSKIQYNKVVKAQLWIYLRPVETPTTVFVQ
ILRLIKPMKDGTRYTGIRSLKLDMNPGTGIWQSIDVKTVLQNWLKQPESNLGIEIKALDENGHDLAVTFPGPGEDG
LNPFLEVKVTDTPKRSRRDFGLDCDEHSTESRCCRYPLTVDFEAFGWDWIIA (SEQ ID NO: 55).

Exemplary proGDF8 sequences in the human, rat, mouse and cynomolgus are provided above. In these proGDF8 sequences, a proprotein convertase cleavage site is indicated in bold and a tolloid protease site is indicated by underlining. In some embodiments, the proprotein convertase cleavage site comprises amino acid residues 240 to 243 of SEQ ID NOs: 52-55. In some embodiments, the tolloid protease site comprises amino acid residues 74-75 of SEQ ID NOs: 52-55. It should be appreciated that the exemplary proGDF8 sequences provided herein are not intended to be limiting and additional proGDF8 sequences from other species, including any isoforms thereof, are within the scope of this disclosure.

The prodomain of the myostatin polypeptide is comprised of several structural domains as described previously (WO 2014/182676). These include, for example, Straight Jacket region, Fastner region, Arm region, Fingers region 1, Fingers region 2, Latency Loop, Alpha-1 Helical region, and Bowtie region. In some embodiments, preferred antibodies or fragments thereof bind an epitope within the Arm region of the myostatin prodomain. In some embodiments, the epitope includes at least one amino acid residue from the "KALDEN" (SEQ ID NO: 118) polypeptide stretch within the Arm region of the prodomain. In some embodiments, the amino acid residue within the Arm region of the prodomain making contact with the antibody when bound to the antigen is a residue that is not conserved between myostatin and GDF11. In some embodiments, such residue(s) is/are K, E, and/or N of the polypeptide stretch (shown in bold type above). In some embodiments, the epitope includes at least one amino acid residue from the "**F**V**Q**I**L**RLIKPMKDGTR**Y**TGI**RS**L**K**" (SEQ ID NO: 57) polypeptide stretch within the Arm region of the prodomain. In some embodiments, such residue(s) is/are F, Q, L, Y, R, S and/or K of the polypeptide stretch (shown in bold type above). See Dagbay et al. (J. Biol. Chem. (2020) 295(16): 5404-5418). In some embodiments, an antibody or antigen-binding fragment thereof that binds such epitope is apitegromab or a variant thereof, wherein optionally the variant is an Fc variant. In other embodiments, an antibody or antigen-binding fragment thereof that binds such epitope is not apitegromab or its variant.

### Myostatin pathway inhibitors

There are several myostatin pathway inhibitors, such as small molecules, antibodies, or antigen-binding portions thereof, and gene therapies, in various stages of clinical development towards the treatment of muscle-related conditions. Such inhibitors of the signaling pathway target either the mature growth factor or its type II receptor. Notably, most of these antagonists are not myostatin-specific, such that they antagonize the signaling of multiple TGFβ family members. For example, a number of current clinical candidates block additional growth factors such as Activin A, GDF11, and BMPs 9 and 10, which are regulators of reproductive biology, wound healing, erythropoiesis and blood vessel formation, respectively.

In some embodiments, as disclosed herein, myostatin pathway inhibitors, such as anti-myostatin antibodies, or antigen binding fragments thereof, bind, e.g., selectively bind, to pro-myostatin and/or latent myostatin, thereby inhibiting myostatin activation In some embodiments, given the prevalence of the latent complex in circulation, treatments are provided herein that specifically target more abundant and longer-lived myostatin precursors e.g., pro-myostatin and latent myostatin, rather than the mature growth factor. In some embodiments, myostatin inhibitors, such as antibodies, or antigen binding fragments thereof, provided herein may prevent the proteolytic activation of pro-myostatin and/or latent myostatin into mature myostatin which is considered the "active" form of myostatin, capable of activating the myostatin pathway, e.g., by binding Type I (ALK4/5) and Type II (ACTRIIA/B) receptors.

Myostatin inhibitors may be an antibody (including fragments thereof, such as Domain Antibodies (dAbs) as described in, for example, U.S. Patent 6,291,158; 6,582,915; 6,593,081; 6,172,197; and 6,696,245), a small molecule inhibitor, an Adnectin, an Affibody, a DARPin, an Anticalin, an Avimer, a Versabody or a gene therapy.

A myostatin pathway inhibitor may be a non-selective myostatin inhibitor, e.g., an inhibitor that also inhibits Activin A and/or GDF11. A non-selective myostatin inhibitor may be a ligand trap (e.g., ACE-031, ACE-083, and BIIB-110/ALG-801); an anti-ActRllb antibody (e.g., bimagrumab); a neutralizing antibody that binds mature myostatin (e.g., stamulumab (MYO-029), domagrozumab (PF-06252616), landogrozumab (LY2495655), AMG-745/PINTA-745 (Myostatin peptibody), RG6206 (an antimyostatin adnectin, which is a single-strand fusion protein containing domains of fibronectin), BMS-986089 (an anti-myostatin adnectin also known as taldefgrobep alfa). A myostatin pathway inhibitor may be a receptor antagonist (e.g., Alk4/5 inhibitors).

A myostatin pathway inhibitor may comprise stamulumab, trevogrumab, LY2495655, AMG 745, bimagrumab, BIIB-110, domagrozumab (PF-06252616), apitegromab, GYM329, taldefgrobep alfa (also known as BMS-986089), or efmitermant alfa. In some embodiments, the myostatin pathway inhibitor comprises a pH-dependent anti-latent myostatin antibody, such as GYM329 or another MST1032 variant as disclosed in International Publication No. WO2016/098357. In some embodiments, the myostatin pathway inhibitor comprises a MST1032 variant as disclosed in International Patent Application No. PCT/JP2015/006323. In some embodiments, the myostatin pathway inhibitor comprises an antibody or antigen-binding fragment comprising additional sequences found in, e.g., in International Patent Publication Nos. WO 2018/129395; WO 2017/218592; WO 2017/120523; WO 2017/049011; WO 2016073853; or WO 2014182676.

A "MST1032 variant", as used herein, refers to an antibody or antigen-binding fragment comprising any of the sequences disclosed in PCT/JP2015/006323, including but not limited to sequences of MS1032LO01-SG1, MS1032LO06-SG1, MS1032LO11-SG1, MS1032LO18-SG1, MS1032LO19-SG1, MS1032LO21-SG1, MS1032LO25-SG1 , and sequences provided in Table 2a, Table 11a, Table 11b, or Table 13 of PCT/JP2015/006323. In certain embodiments, a MST1032 variant comprises an antibody or antigen-binding fragment comprising a heavy chain variable domain comprising three CDR sequences of HCDR1, HCDR2, and HCDR3, and a light chain variable domain comprising three CDR sequences of LCDR1, LCDR2, and LCDR3, wherein the heavy chain CDRs comprise the amino acid sequences of: X1X2DIS (HCDR1; SEQ ID NO: 17); IISYAGSTYYASWAKG (HCDR2; SEQ ID NO: 18); GVPAYSX3GGDL (HCDR3; SEQ ID NO: 19), respectively; and the light chain CDRs comprise amino acid sequences of: X4X5SQSVYX6X7NWLS (LCDR1; SEQ ID NO: 20); WASTLAX8 (LCDR2; SEQ ID NO: 21); and AGGYGGGX9YA (LCDR3; SEQ ID NO: 22), respectively, wherein each of X1-X9 is any amino acid residue. In certain embodiments, X1 is S or H; X2 is Y, T, or D; X3 is T or H; X4 is Q or T; X5 is S or T; X6 is D or H; X7 is N or E; X8 is S or Y; X9 is L or R. In certain embodiments, a MST1032 variant comprises the six CDR sequences of SEQ ID NOs: 18, 66, 67, 68-70; SEQ ID Nos: 119, 18, 120, 68, 121, 122; or SEQ ID Nos: 123, 18, 120, 124, 121, 122. In certain embodiments, a MST1032 variant comprises a heavy chain variable domain comprising the amino acid sequence of any one of SEQ ID NOs: 125-128. In certain embodiments, a MST1032 variant comprises a light chain variable domain comprising the amino acid sequence of any one of SEQ ID NOs: 129, 126, 127 and 130. In certain embodiments, a MST1032 variant comprises a set of six CDRs (e.g., from the same MST1032 variant antibody) or a paired VH/VL (e.g., from the same MST1032 variant antibody) from those listed in the tables below.

**Table 1A.**

| | **VHCDR1** | | **VHCDR2** | | **VHCDR3** | |
|---|---|---|---|---|---|---|
| | SEQ ID NO: | Amino Acid Sequence | SEQ ID NO: | Amino Acid Sequence | SEQ ID NO: | Amino Acid Sequence |
| **Consensus Sequences** | 17 | X₁X₂DIS | 18 | IISYAGSTYY ASWAKG | 19 | GVPAYSX₃GGDL |

**Table 1B.**

| | **VLCDR1** | | **VLCDR2** | | **VLCDR3** | |
|---|---|---|---|---|---|---|
| | SEQ ID NO: | Amino Acid Sequence | SEQ ID NO: | Amino Acid Sequence | SEQ ID NO: | Amino Acid Sequence |
| **Consensus Sequences** | 20 | X4X5SQSVYX6X7NWL S | 21 | WASTLAX₈ | 22 | AGGYGGGX₉Y A |

**Table 1C.**

| | VHCDR1 | | VHCDR2 | | VHCDR3 | |
|---|---|---|---|---|---|---|
| | SEQ ID NO: | Amino Acid Sequence | SEQ ID NO: | Amino Acid Sequence | SEQ ID NO: | Amino Acid Sequence |
| **MST1032-G1m** | 66 | SYDIS | 18 | IISYAGSTYY ASWAKG | 67 | GVPAYSTGGD L |
| **MS1032LO00-SG1** | 66 | SYDIS | 18 | IISYAGSTYY ASWAKG | 67 | GVPAYSTGGD L |
| **MS1032LO01-SG1** | 119 | HTDIS | 18 | IISYAGSTYY ASWAKG | 120 | GVPAYSHGGD L |
| **MS1032LO19-SG1** | 123 | HDDIS | 18 | IISYAGSTYY ASWAKG | 120 | GVPAYSHGGD L |

**Table 1D.**

| | VLCDR1 | | VLCDR2 | | VLCDR3 | |
|---|---|---|---|---|---|---|
| | SEQ ID NO: | Amino Acid Sequence | SEQ ID NO: | Amino Acid Sequence | SEQ ID NO: | Amino Acid Sequence |
| **MST1032-G1m** | 68 | QSSQSVYDNN WLS | 69 | WASTLAS | 70 | AGGYGGGLYA |
| **MS1032LO00-SG1** | 68 | QSSQSVYDNN WLS | 69 | WASTLAS | 70 | AGGYGGGLYA |
| **MS1032LO01-SG1** | 68 | QSSQSVYDNN WLS | 121 | WASTLAY | 122 | AGGYGGGRYA |
| **MS1032LO19-SG1** | 124 | TTSQSVYHEN WLS | 121 | WASTLAY | 122 | AGGYGGGRYA |

**Table 1E.**

| | Heavy Chain Variable Domain (VH) | |
|---|---|---|
| | SEQ ID NO: | Amino Acid Sequence |
| **MST1032-G1m** | 125 | QSLEESGGRLVTPGGSLTVTCRVSGIDLSSYDISWVRQAPGKGLEWIGIISYAGS TYYAS WAKGRFTISKTSTTVDLKMTSLTTEDTATYFCARGVPAYSTGGDLWGPGTLVTVS S |
| **MS1032LO00-SG1** | 126 | QVTLKESGPVLVKPTETLTLTCTVSGIDLS SYDISWVRQAPGKGLEWVGIISYA GSTYYASWAKGRLTISKDTSKNQVVLTMTNMDPVDTATYYCARGVPAYSTGGDLW GQGTLVTVSS |
| **MS1032LO01-SG1** | 127 | QVTLKESGPVLVKPTETLTLTCTVSGIDLSHTDISWVRQAPGKGLEWVGIISYAG STYYASWAKGRLTISKDTSKNQVVLTMTNMDPVDTATYYCARGVPAYSHGGDLWG QGTLVTVSS |
| **MS1032LO19-SG1** | 128 | QVQLVESGGGLVQPGGSLRLSCAVSGIDLSHDDISWVRQAPGKGLEWVSIISYAG STYYASWAKGRLTISKDTSKNQVVLTMTNMDPVDTATYYCARGVPAYSHGGDLWG QGTLVTVSS |

**Table 1F**

| | **Light Chain Variable Domain (VL)** | |
|---|---|---|
| | SEQ ID NO: | Amino Acid Sequence |
| **MST1032-G1m** | 129 | AQVLTQTASSVSAAVGGTVTINCQSSQSVYDNNWLSWFQQKPGQPPKLLIYWASTLA SGVPSRFKGSGSGTQFTLTISDLECDDAATYYCAGGYGGGLYAFGGGTKVEIK |
| **MS1032LO00-SG1N** | 132 | DIVMTQSPATLSLSPGERATLSCQSSQSVYDNNWLSWFQQKPGQPPKLLI YWASTLASGVPSRFSGSGSGTDFTLTISSLQPEDAATYYCAGGYGGGLYA FGQGTKVEIK |
| **MS1032LO01-SG1** | 133 | DIVMTQSPATLSLSPGERATLSCTSSQSVYHNNWLSWFQQKPGQPPKLLI YWASTLAYGVPSRFSGSGSGTDFTLTISSLQPEDAATYYCAGGYGGGRYA FGQGTKVEIK |
| **MS1032LO19-SG1** | 130 | DIVMTQSPATLSLSPGERATLSCTTSQSVYHENWLSWFQQKPGQPPKLLIYWASTLA YGVPSRFSGSGSGTDFTLTISSLQPEDAATYYCAGGYGGGRYAFGQGTKVEIK |

In some embodiments, a myostatin antibody or antigen binding fragment thereof may include or may be engineered to include a mutation or modification that causes an extended half-life of the antibody. In some embodiments, such mutations or modifications may be within the Fc domain of the antibodies (e.g., Fc-modified antibodies), e.g., to promote circulating half life or other PK properties. In some embodiments, the mutation is the YTE mutation (see, e.g., Saunders, KO (2019) "Conceptual Approaches to Modulating Antibody Effector Functions and Circulation Half-Life," Frontiers in Immunology 10:1296 and U.S. Patent No. 7,083,784.

In some embodiments, an anti-pro/latent myostatin antibody, or antigen binding fragment thereof, binds specifically to latent myostatin. In some embodiments, an anti-pro/latent myostatin antibody, or antigen binding fragment thereof, binds specifically to both latent myostatin and pro-myostatin. In preferred embodiments, the anti-pro/latent myostatin antibody, or antigen binding fragment thereof, that binds specifically to pro-myostatin and/or latent myostatin does not bind mature myostatin. In preferred embodiments, the anti-pro/latent myostatin antibody, or antigen binding fragment thereof, that binds specifically to pro-myostatin and/or latent myostatin does not bind pro/latent GDF11 or mature GDF11.

### Anti-Pro/Latent Myostatin Antibodies and Antigen-Binding Fragments Thereof

In some embodiments, antibodies, or antigen binding fragments thereof, described herein are capable of binding to a pro/latent-myostatin, thereby inhibiting the proteolytic activation of pro/latent-myostatin into mature myostatin. In some instances, antibodies, or antigen binding fragments thereof, described herein can inhibit the proteolytic activation of pro/latent-myostatin by at least 20%, e.g., 30%, 40%, 50%, 60%, 70%, 80%, 90%, 95%, or higher. In some instances, antibodies described herein can inhibit the proteolytic cleavage of pro-myostatin by a proprotein convertase (e.g., furin) by at least 20%, e.g., 30%, 40%, 50%, 60%, 70%, 80%, 90%, 95%, or higher. In some instances, antibodies, or antigen binding fragments thereof, described herein can inhibit the proteolytic cleavage of pro-myostatin or latent myostatin by a tolloid protease (e.g., mTLL2) by at least 20%, e.g., 30%, 40%, 50%, 60%, 70%, 80%, 90%, 95%, or higher.

In some embodiments, antibodies, or antigen binding fragments thereof, described herein are capable of binding to a pro/latent-myostatin, thereby inhibiting myostatin activity. In some instances, the antibodies, or antigen binding fragments thereof, described herein can inhibit myostatin signaling by at least 20%, e.g., 30%, 40%, 50%, 60%, 70%, 80%, 90%, 95%, or higher. In some embodiments, inhibition of myostatin signaling can be measured by routine methods, for example, using a myostatin activation assay as described in Example 1 disclosed in WO 2016/073853. However, it should be appreciated that additional methods may be used for measuring myostatin signaling activity.

It should be appreciated that the extent of proteolytic cleavage of myostatin, e.g., by a proprotein convertase and/or a tolloid protease, can be measured and/or quantified using any suitable method. In some embodiments, the extent of proteolytic cleavage of myostatin is measured and/or quantified using an enzyme-linked immunosorbent assay (ELISA). For example, an ELISA may be used to measure the level of released growth factor (e.g., mature myostatin). As another example, an antibody, or antigen binding fragment thereof, that specifically binds to pro-myostatin, latent myostatin and/or mature myostatin can be used in an ELISA to measure the level of a specific form of myostatin (e.g., pro/latent/mature-myostatin), to quantify the extent of proteolytic cleavage of myostatin. In some embodiments, the extent of proteolytic cleavage of myostatin is measured and/or quantified using immunoprecipitation followed by SDS-PAGE or mass spectrometry of tryptic peptides, fluorescence anisotropy-based techniques, FRET assays, hydrogen-deuterium-exchange mass spectrometry, and/or NMR spectroscopy.

Anti-pro/latent-myostatin antibodies, or antigen binding fragments thereof, suitable for use in the methods of the present invention include those described in International Patent Application Nos. PCT/US15/59468 and PCT/US16/52014.

According to the present disclosure, an antibody or antigen-binding fragment that binds human pro/latent myostatin may be used to carry out various embodiments disclosed herein. In some embodiments, such antibody and antigen-binding fragment binds an epitope within the prodomain, wherein the epitope comprises one or more amino acid residues F147, Q149, L151, Y186, S168, K170, K205, and/or L207, as numbered according to SEQ ID NO: 52 (Dagbay et al. J Biol Chem. 2020 Apr 17;295(16):5404-5418).

In some embodiments, anti-pro/latent-myostatin antibodies, or antigen binding fragments thereof, of the present disclosure and the nucleic acid molecules of the present disclosure that encode the antibodies, or antigen binding fragments thereof, include the CDR amino acid sequences shown in Tables 1G-1 to 1G-3.

**Table 1G-1.**

| Antibody | CDRH1 | CDRH2 | CDRH3 | CDRL1 | CDRL2 | CDRL3 |
|---|---|---|---|---|---|---|
| Ab1 Kabat: IMGT: | SSYGMH (SEQ ID NO: 1) | VISYDGSNKY YADSVKG (SEQ ID NO: 4) | DLLVRFLEWS HYYGMDV (SEQ ID NO: 10) | SGSSSNIGSN TVH (SEQ ID NO: 12) | SDNQRPS (SEQ ID NO: 60) | AAWDDSLNG V (SEQ ID NO: 65) |
| | GFTFSSYGM H (SEQ ID NO: 2) | ISYDGSN (SEQ ID NO: 5) | | SSNIGSNT (SEQ ID NO: 13) | SDN (SEQ ID NO: 61) | |
| Ab2 Kabat: IMGT: | SSYGMH (SEQ ID NO: 1) | VISYDGSNKY YADSVKG (SEQ ID NO: 4) | DLLVRFLEWS HYYGMDV (SEQ ID NO: 10) | SGSSSNIGSN TVH (SEQ ID NO: 12) | SDNQRPS (SEQ ID NO: 60) | AAWDDSLNG V (SEQ ID NO: 65) |
| | GFTFSSYGM H (SEQ ID NO: 2) | ISYDGSN (SEQ ID NO: 5) | | SSNIGSNT (SEQ ID NO: 13) | SDN (SEQ ID NO: 61) | |
| Ab3 Kabat: IMGT: | SSYGMH (SEQ ID NO: 1) | VISYDGSIKYY ADSVKG (SEQ ID NO: 6) | DLLVRFLEWS HKYGMDV (SEQ ID NO: 11) | SGSTSNIGSN TVH (SEQ ID NO: 14) | SDDQRPS (SEQ ID NO: 131) | AAWDESLNG V (SEQ ID NO: 23) |
| | GFAFSSYGM H (SEQ ID NO: 3) | ISYDGSI (SEQ ID NO: 7) | | TSNIGSNT (SEQ ID NO: 15) | SDD (SEQ ID NO: 64) | |
| Ab4 Kabat: IMGT: | SSYGMH (SEQ ID NO: 1) | VISYDGSIKYY ADSVKG (SEQ ID NO: 6) | DLLVRFLEWS HKYGMDV (SEQ ID NO: 11) | SGSTSNIGSN TVH (SEQ ID NO: 14) | SDDQRPS (SEQ ID NO: 131) | AAWDESLNG V (SEQ ID NO: 23) |
| | GFAFSSYGM H (SEQ ID NO: 3) | ISYDGSI (SEQ ID NO: 7) | | TSNIGSNT (SEQ ID NO: 15) | SDD (SEQ ID NO: 64) | |
| Ab5 Kabat: IMGT: | SSYGMH (SEQ ID NO: 1) | VISYDGNNKY YADSVKG (SEQ ID NO: 8) | DLLVRFLEWS HKYGMDV (SEQ ID NO: 11) | SGSSSNIGGN TVH (SEQ ID NO: 16) | SDDQRPS (SEQ ID NO: 131) | |
| | GFAFSSYGM H (SEQ ID NO: 3) | ISYDGNN (SEQ ID NO: 9) | | SSNIGGNT (SEQ ID NO: 59) | SDD (SEQ ID NO: 64) | |

In Table 1, the single sequences of CDRH3 and CDRL3 reflect Kabat and IMGT.

**Table 1G-2.**

| Description | Amino Acid Sequence (SEQ ID NO) | Nucleic Acid Sequence (SEQ ID NO) |
|---|---|---|
| Heavy chain variable region - Ab1 parental | QIQLVQSGGGVVQPGRSLRLSCA ASGFTFSSYGMHWVRQAPGKGLE WVAVISYDGSNKYYADSVKGRFTI SRDNSKNTLYLQMNSLRAEDTAVY YCARDLLVRFLEWSHYYGMDVWG QGTTVTVSS (SEQ ID NO: 24) | CAGATCCAGCTGGTGCAGTCTGGGGGAGGCGTGGTC CAGCCTGGGAGGTCCCTGAGACTCTCCTGTGCAGCG TCTGGATTCACCTTCAGTAGCTATGGCATGCACTGGG TCCGCCAGGCTCCAGGCAAGGGGCTGGAGTGGGTG GCAGTTATATCATATGATGGAAGTAATAAATACTATGC AGACTCCGTGAAGGGCCGATTCACCATCTCCAGAGA CAATTCCAAGAACACGCTGTATCTGCAAATGAACAGC CTGAGAGCCGAGGACACGGCTGTGTATTACTGTGCG AGAGATCTCCTGGTGCGATTTTTGGAGTGGTCGCACT ACTACGGTATGGACGTCTGGGGCCAAGGGACCACGG TCACCGTCTCCTCA (SEQ ID NO: 38) |
| Heavy chain variable region - Ab2 germline | QVQLVESGGGVVQPGRSLRLSCA ASGFTFSSYGMHWVRQAPGKGLE WVAVISYDGSNKYYADSVKGRFTI SRDNSKNTLYLQMNSLRAEDTAVY YCARDLLVRFLEWSHYYGMDVWG QGTTVTVSS (SEQ ID NO: 25) | CAGGTGCAGCTGGTGGAGTCTGGGGGAGGCGTGGT CCAGCCTGGGAGGTCCCTGAGACTCTCCTGTGCAGC GTCTGGATTCACCTTCAGTAGCTATGGCATGCACTGG GTCCGCCAGGCTCCAGGCAAGGGGCTGGAGTGGGT GGCAGTTATATCATATGATGGAAGTAATAAATACTATG CAGACTCCGTGAAGGGCCGATTCACCATCTCCAGAG ACAATTCCAAGAACACGCTGTATCTGCAAATGAACAG CCTGAGAGCCGAGGACACGGCTGTGTATTACTGTGC GAGAGATCTCCTGGTGCGATTTTTGGAGTGGTCGCAC TACTACGGTATGGACGTCTGGGGCCAAGGGACCACG GTCACCGTCTCCTCA (SEQ ID NO: 39) |
| Heavy chain variable region - Ab3 parental | QIQLVQSGGGVVQPGRSLRLSCA ASGFAFSSYGMHWVRQAPGKGLE WVAVISYDGSIKYYADSVKGRFTIS RDNSKNTLYLQMNSLRAEDTAVYY CARDLLVRFLEWSHKYGMDVWG QGTTVTVSS (SEQ ID NO: 26) | CAGATCCAGCTGGTGCAGTCTGGGGGAGGCGTGGTC CAGCCTGGGAGGTCCCTGAGACTCTCCTGTGCAGCG TCTGGATTCGCCTTCAGTAGCTATGGCATGCACTGGG TCCGCCAGGCTCCAGGCAAGGGGCTGGAGTGGGTG GCAGTTATATCATATGATGGAAGTATCAAATACTATGC AGACTCCGTGAAGGGCCGATTCACCATCTCCAGAGA CAATTCCAAGAACACGCTGTATCTGCAAATGAACAGC CTGAGAGCCGAGGACACGGCTGTGTATTACTGTGCG AGAGATCTCCTGGTGCGATTTTTGGAGTGGTCGCACA AGTACGGTATGGACGTCTGGGGCCAAGGGACCACGG TCACCGTCTCCTCA (SEQ ID NO: 40) |
| Heavy chain variable region - Ab4 germline | QVQLVESGGGVVQPGRSLRLSCA ASGFAFSSYGMHWVRQAPGKGLE WVAVISYDGSIKYYADSVKGRFTIS RDNSKNTLYLQMNSLRAEDTAVYY CARDLLVRFLEWSHKYGMDVWG QGTTVTVSS (SEQ ID NO: 27) | CAGGTGCAGCTGGTGGAGTCTGGGGGAGGCGTGGT CCAGCCTGGGAGGTCCCTGAGACTCTCCTGTGCAGC GTCTGGATTCGCCTTCAGTAGCTATGGCATGCACTGG GTCCGCCAGGCTCCAGGCAAGGGGCTGGAGTGGGT GGCAGTTATATCATATGATGGAAGTATCAAATACTATG CAGACTCCGTGAAGGGCCGATTCACCATCTCCAGAG ACAATTCCAAGAACACGCTGTATCTGCAAATGAACAG CCTGAGAGCCGAGGACACGGCTGTGTATTACTGTGC GAGAGATCTCCTGGTGCGATTTTTGGAGTGGTCGCAC AAGTACGGTATGGACGTCTGGGGCCAAGGGACCACG GTCACCGTCTCCTCA (SEQ ID NO: 41) |
| Heavy chain variable region - Ab5 parental | QIQLVQSGGGVVQPGRSLRLSCA ASGFAFSSYGMHWVRQAPGKGLE WVAVISYDGNNKYYADSVKGRFTI SRDNSKNTLYLQMNSLRAEDTAVY YCARDLLVRFLEWSHKYGMDVWG QGTTVTVSS (SEQ ID NO: 28) | CAGATCCAGCTGGTGCAGTCTGGGGGAGGCGTGGTC CAGCCTGGGAGGTCCCTGAGACTCTCCTGTGCAGCG TCTGGATTCGCCTTCAGTAGCTATGGCATGCACTGGG TCCGCCAGGCTCCAGGCAAGGGGCTGGAGTGGGTG GCAGTTATATCATATGATGGAAATAATAAATACTATGC AGACTCCGTGAAGGGCCGATTCACCATCTCCAGAGA CAATTCCAAGAACACGCTGTATCTGCAAATGAACAGC CTGAGAGCCGAGGACACGGCTGTGTATTACTGTGCG AGAGATCTCCTGGTGCGATTTTTGGAGTGGTCGCACA AGTACGGTATGGACGTCTGGGGCCAAGGGACCACGG TCACCGTCTCCTCA (SEQ ID NO: 42) |
| Heavy chain variable region - Ab6 germline | QVQLVESGGGVVQPGRSLRLSCA ASGFAFSSYGMHWVRQAPGKGLE WVAVISYDGNNKYYADSVKGRFTI SRDNSKNTLYLQMNSLRAEDTAVY YCARDLLVRFLEWSHKYGMDVWG QGTTVTVSS (SEQ ID NO: 29) | CAGGTGCAGCTGGTGGAGTCTGGGGGAGGCGTGGT CCAGCCTGGGAGGTCCCTGAGACTCTCCTGTGCAGC GTCTGGATTCGCCTTCAGTAGCTATGGCATGCACTGG GTCCGCCAGGCTCCAGGCAAGGGGCTGGAGTGGGT GGCAGTTATATCATATGATGGAAATAATAAATACTATG CAGACTCCGTGAAGGGCCGATTCACCATCTCCAGAG ACAATTCCAAGAACACGCTGTATCTGCAAATGAACAG CCTGAGAGCCGAGGACACGGCTGTGTATTACTGTGC GAGAGATCTCCTGGTGCGATTTTTGGAGTGGTCGCAC AAGTACGGTATGGACGTCTGGGGCCAAGGGACCACG GTCACCGTCTCCTCA (SEQ ID NO: 43) |
| Light chain variable region - Ab1 parental | QPVLTQPPSASGTPGQRVTISCSG SSSNIGSNTVHWYQQLPGTAPKLL IYSDNQRPSGVPDRFSGSKSGTSA SLVISGLQSDDEADYYCAAWDDSL NGVFGGGTKLTVL (SEQ ID NO: 30) | CAGCCTGTGCTGACTCAGCCACCCTCAGCGTCTGGG ACCCCCGGGCAGAGGGTCACCATCTCTTGTTCTGGA AGCAGCTCCAACATCGGAAGTAATACTGTCCACTGGT ACCAGCAACTCCCAGGAACGGCCCCCAAACTCCTCAT CTATAGTGATAATCAGCGCCCCTCAGGGGTCCCTGAC CGATTCTCTGGCTCCAAGTCTGGCACCTCAGCCTCCC TGGTCATCAGTGGGCTCCAGTCTGACGATGAGGCTG ATTATTACTGTGCAGCATGGGATGACAGCCTGAATGG GGTGTTCGGCGGAGGGACCAAGCTGACCGTCCTA (SEQ ID NO: 44) |
| Light chain variable region - Ab2 germline | QSVLTQPPSASGTPGQRVTISCSG SSSNIGSNTVHWYQQLPGTAPKLL IYSDNQRPSGVPDRFSGSKSGTSA SLAISGLQSEDEADYYCAAWDDSL NGVFGGGTKLTVL (SEQ ID NO: 31) | CAGTCTGTGCTGACTCAGCCACCCTCAGCGTCTGGG ACCCCCGGGCAGAGGGTCACCATCTCTTGTTCTGGA AGCAGCTCCAACATCGGAAGTAATACTGTCCACTGGT ACCAGCAACTCCCAGGAACGGCCCCCAAACTCCTCAT CTATAGTGATAATCAGCGCCCCTCAGGGGTCCCTGAC CGATTCTCTGGCTCCAAGTCTGGCACCTCAGCCTCCC TGGCCATCAGTGGGCTCCAGTCTGAGGATGAGGCTG ATTATTACTGTGCAGCATGGGATGACAGCCTGAATGG GGTGTTCGGCGGAGGGACCAAGCTGACCGTCCTA (SEQ ID NO: 45) |
| Light chain variable region - Ab3 parental | QPVLTQPPSASGTPGQRVTISCSG STSNIGSNTVHWYQQLPGTAPKLLI YSDDQRPSGVPDRFSGSKSGTSA SLVISGLQSDDEADYYCAAWDESL NGVFGGGTKLTVL (SEQ ID NO: 32) | CAGCCTGTGCTGACTCAGCCACCCTCAGCGTCTGGG ACCCCCGGGCAGAGGGTCACCATCTCTTGTTCTGGA AGCACCTCCAACATCGGAAGTAATACTGTCCACTGGT ACCAGCAACTCCCAGGAACGGCCCCCAAACTCCTCAT CTATAGTGATGATCAGCGCCCCTCAGGGGTCCCTGA CCGATTCTCTGGCTCCAAGTCTGGCACCTCAGCCTCC CTGGTCATCAGTGGGCTCCAGTCTGACGATGAGGCT GATTATTACTGTGCAGCATGGGATGAGAGCCTGAATG GGGTGTTCGGCGGAGGGACCAAGCTGACCGTCCTA (SEQ ID NO: 46) |
| Light chain variable region - Ab4 germline | QSVLTQPPSASGTPGQRVTISCSG STSNIGSNTVHWYQQLPGTAPKLLI YSDDQRPSGVPDRFSGSKSGTSA SLAISGLQSEDEADYYCAAWDESL NGVFGGGTKLTVL (SEQ ID NO: 33) | CAGTCTGTGCTGACTCAGCCACCCTCAGCGTCTGGG ACCCCCGGGCAGAGGGTCACCATCTCTTGTTCTGGA AGCACCTCCAACATCGGAAGTAATACTGTCCACTGGT ACCAGCAACTCCCAGGAACGGCCCCCAAACTCCTCAT CTATAGTGATGATCAGCGCCCCTCAGGGGTCCCTGA CCGATTCTCTGGCTCCAAGTCTGGCACCTCAGCCTCC CTGGCCATCAGTGGGCTCCAGTCTGAGGATGAGGCT GATTATTACTGTGCAGCATGGGATGAGAGCCTGAATG GGGTGTTCGGCGGAGGGACCAAGCTGACCGTCCTA (SEQ ID NO: 47) |
| Light chain variable region - Ab5 parental | QPVLTQPPSASGTPGQRVTISCSG SSSNIGGNTVHWYQQLPGTAPKLL IYSDDQRPSGVPDRFSGSKSGTSA SLVISGLQSDDEADYYCAAWDESL NGVFGGGTKLTVL (SEQ ID NO: 34) | CAGCCTGTGCTGACTCAGCCACCCTCAGCGTCTGGG ACCCCCGGGCAGAGGGTCACCATCTCTTGTTCTGGA AGCAGCTCCAACATCGGAGGAAATACTGTCCACTGGT ACCAGCAACTCCCAGGAACGGCCCCCAAACTCCTCAT CTATAGTGATGATCAGCGCCCCTCAGGGGTCCCTGA CCGATTCTCTGGCTCCAAGTCTGGCACCTCAGCCTCC CTGGTCATCAGTGGGCTCCAGTCTGACGATGAGGCT GATTATTACTGTGCAGCATGGGATGAGAGCCTGAATG GGGTGTTCGGCGGAGGGACCAAGCTGACCGTCCTA (SEQ ID NO: 48) |
| Light chain variable region - Ab6 germline | QSVLTQPPSASGTPGQRVTISCSG SSSNIGGNTVHWYQQLPGTAPKLL IYSDDQRPSGVPDRFSGSKSGTSA SLAISGLQSEDEADYYCAAWDESL NGVFGGGTKLTVL (SEQ ID NO: 35) | CAGTCTGTGCTGACTCAGCCACCCTCAGCGTCTGGG ACCCCCGGGCAGAGGGTCACCATCTCTTGTTCTGGA AGCAGCTCCAACATCGGAGGAAATACTGTCCACTGGT ACCAGCAACTCCCAGGAACGGCCCCCAAACTCCTCAT CTATAGTGATGATCAGCGCCCCTCAGGGGTCCCTGA CCGATTCTCTGGCTCCAAGTCTGGCACCTCAGCCTCC CTGGCCATCAGTGGGCTCCAGTCTGAGGATGAGGCT GATTATTACTGTGCAGCATGGGATGAGAGCCTGAATG GGGTGTTCGGCGGAGGGACCAAGCTGACCGTCCTA (SEQ ID NO: 49) |
| Ab2-Heavy Chain | QVQLVESGGGVVQPGRSLRLSCA ASGFTFSSYGMHWVRQAPGKGLE WVAVISYDGSNKYYADSVKGRFTI SRDNSKNTLYLQMNSLRAEDTAVY YCARDLLVRFLEWSHYYGMDVWG QGTTVTVSSASTKGPSVFPLAPCS RSTSESTAALGCLVKDYFPEPVTV SWNSGALTSGVHTFPAVLQSSGL YSLSSVVTVPSSSLGTKTYTCNVD HKPSNTKVDKRVESKYGPPCPPC PAPEFLGGPSVFLFPPKPKDTLMIS RTPEVTCVVVDVSQEDPEVQFNW YVDGVEVHNAKTKPREEQFNSTY RVVSVLTVLHQDWLNGKEYKCKV SNKGLPSSIEKTISKAKGQPREPQV YTLPPSQEEMTKNQVSLTCLVKGF YPSDIAVEWESNGQPENNYKTTPP VLDSDGSFFLYSRLTVDKSRWQE GNVFSCSVMHEALHNHYTQKSLSL SLG (SEQ ID NO: 50) | |
| Ab2-Light Chain | QSVLTQPPSASGTPGQRVTISCSG SSSNIGSNTVHWYQQLPGTAPKLL IYSDNQRPSGVPDRFSGSKSGTSA SLAISGLQSEDEADYYCAAWDDSL NGVFGGGTKLTVLGQPKAAPSVTL FPPSSEELQANKATLVCLISDFYPG AVTVAWKADSSPVKAGVETTTPSK QSNNKYAASSYLSLTPEQWKSHR SYSCQVTHEGSTVEKTVAPTECS (SEQ ID NO: 51) | |

**Table 1G-3.**

| | CDR-H3 | CDR-L3 | VH | VL | scFV | SEQ ID NOs: from left to right |
|---|---|---|---|---|---|---|
| Ab7 | ESLIRFLE DPQQG GMDV | NSWTRS NNYI | QVQLQQSGAEV KKPGASVKVSC KASGYTFTSYY MHWVRQAPGQ GLEWMGIINPS GGSTSYAQKFQ GRVTMTRDTST STVYMELSSLR SEDTAVYYCAR ESLIRFLEDPQQ GGMDVWGQGT TVTVSS | QSALTQPASVS GSPGQSLTISC TGTSSDIGGY NYVSWYQQH PGKAPKLIIYD VTDRPSGVSG RFSGSKSGNT ASLTISGLQTE DEAEYFCNSW TRSNNYIFGG GTKLTVLGQP KAAPSVTL | QVQLQQSGAEVKKPGASV KVSCKASGYTFTSYYMH WVRQAPGQGLEWMGIIN PSGGSTSYAQKFQGRVT MTRDTSTSTVYMELSSLR SEDTAVYYCARESLIRFLE DPQQGGMDVWGQGTTV TVSSGSASAPTLGGGGS GGGGSAAAQSALTQPAS VSGSPGQSLTISCTGTSS DIGGYNYVSWYQQHPGK APKLIIYDVTDRPSGVSGR FSGSKSGNTASLTISGLQ TEDEAEYFCNSWTRSNN YIFGGGTKLTVLGQPKAA PSVTLFPPSS | 71-75 |
| Ab10 | DRYSSS WGGGF DY | QSYDAS SLWV | EVQLVQSGGGV VQSGRSLRLSC VASGFSFSNYG MHWVRQAPGK GLEWLAFIWYD GSNKWYADSV KGRFTISRDNS KNALYLQMNSL RAEDTAVYYCA RDRYSSSWGG GFDYWGQGTV LTVSS | NFMLTQPHSVS ESPGRTVTIPC SGRGGSIASD SVQWYQQRP GSAPTTIIYED NQRPSGVPDR FSGSVDSSSN SASLTISGLRT EDEADYYCQS YDASSLWVFG GKTKLTVLGQ PKAAPSVTL | EVQLVQSGGGVVQSGRSL RLSCVASGFSFSNYGMH WVRQAPGKGLEWLAFIW YDGSNKWYADSVKGRFTI SRDNSKNALYLQMNSLR AEDTAVYYCARDRYSSS WGGGFDYWGQGTVLTV SSGSASAPTLGGGGSGG GGSAAANFMLTQPHSVS ESPGRTVTIPCSGRGGSI ASDSVQWYQQRPGSAPT TIIYEDNQRPSGVPDRFS GSVDSSSNSASLTISGLR TEDEADYYCQSYDASSL WVFGGKTKLTVLGQPKA APSVTLFPPSSKASGA | 76-80 |
| Ab11 | DRHSLG DFDY | QAWDST TVV | QLQLQQSGGGL VKPGGSLRLSC AASGFTFSSYS MNWVRQAPGK GLEWVSSISSS SSYIYYADSVKG RFTISRDNAKNS LYLQMNSLRAE DTAVYYCVRDR HSLGDFDYWG QGTLVTVSSGS | SSELTQPSVSV SPGQTATITCS GDKLGDKYAS WYQQKPGQS PVLVIY QDTKRPSGIP ARFSGSNSGN TATLTISGTQA MDEAAYYCQA WDSTTVVF GGGTKLTVLG QPKAAPSVTL FPPSS | QLQLQQSGGGLVKPGGSL RLSCAASGFTFSSYSMN WVRQAPGKGLEWVSSIS SSSSYIYYADSVKGRFTIS RDNAKNSLYLQMNSLRA EDTAVYYCVRDRHSLGD FDYWGQGTLVTVSSGSA SAPTLGGGGSGGGGSAA ASSELTQPPSVSVSPGQT ATITCSGDKLGDKYASWY QQKPGQSPVLVIYQDTKR PSGIPARF | 81-85 |
| Ab9 | HGLMDD SSGYYL SNAFDI | ATWDDS LTGVV | QVQLVQSGAEV KKPGSSVKVSC KASGGTFSSYAI SWVRQAPGQG LEWMGGIIPIFG TANYAQKFQGR VTITADESTSTA YMELSSLRSED TAVYYCANHGL MDDSSGYYLSN AFDIWGQGTMV TVSSGS | QPVLTQPPSAS GTPGQRVTIS CSGSSSNIGS NTVEWYQQLP GTAPKLLIHSN NQRPSGVPDR FSGSRSGTSA SLAISGLQSED EADYFCATWD DSLTGVVFGG GTTLTVLGQP KAAPSVTLFPP SS | QVQLVQSGAEVKKPGSSV KVSCKASGGTFSSYAISW VRQAPGQGLEWMGGIIPI FGTANYAQKFQGRVTITA DESTSTAYMELSSLRSED TAVYYCANHGLMDDSSG YYLSNAFDIWGQGTMVTV SSGSASAPTLGGGGSGG GGSAAAQPVLTQPPSAS GTPGQRVTISCSGSSSNI GSNTVEWYQQLPGTAPK LLIHSNNQRPSGVPDRFS GSRSGTSASLAISGLQSE DEADYFCATWDDSLTGV VFGGGTTLTVLGQPKAAP SVTLFPPSS | 86-90 |
| Ab12 | VGTAAA GDAFDI | AAWDDS LSGWV | QVQLVQSGGGLI QPGGSLRLSCA ASGFTVSSYSM NWVRQAPGKG LEWVSYISSSG STIYYADSVKGR FTISRDNAKNSL YLQMNSLRAED TALYYCAKVGT AAAGDAFDIWG QGTMVTVSSGS | QPVLTQPPSAS GTPGQRVTIS CFGSSSNIGS NYVYWYQQLP GTAPKLLIYRN NQRPSGVPDR FSGSKSGTSA SLAISGLRSED EADYYCAAWD DSLSGWVFGG GTKLTVLGQP KAAPSVTLFPP SS | QVQLVQSGGGLIQPGGSL RLSCAASGFTVSSYSMN WVRQAPGKGLEWVSYIS SSGSTIYYADSVKGRFTIS RDNAKNSLYLQMNSLRA EDTALYYCAKVGTAAAGD AFDIWGQGTMVTVSSGS ASAPTLGGGGSGGGGSA AAQPVLTQPPSASGTPG QRVTISCFGSSSNIGSNY VYWYQQLPGTAPKLLIYR NNQRPSGVPDRFSGSKS GTSASLAISGLRSEDEAD YYCAAWDDSLSGWVFGG GTKLTVLGQPKAAPSVTL FPPSS | 91-95 |
| Ab8 | VGFYDYV WGSYP YDAFDI | QQYGTS PLT | QIQLVQSGAEVK KPGASVKVSCK ASGYTFTSYGIS WVRQAPGQGL EWMGWISAYN GNTNYAQKLQG RVTMTTDTSTS TAYMELSSLRS EDTAVYYCARV GFYDYVWGSY PYDAFDIWGQG TMVTVSS | EIVMTQSPGTL SLSPGERATL SCRASQSVSS NYLAWYQQKP GQAPRLLIYDA SNRATGIPARF SGSGSGTDFT LTISSLEPEDF ALYYCQQYGT SPLTFGGGTK LEIK | QIQLVQSGAEVKKPGASVK VSCKASGYTFTSYGISWV RQAPGQGLEWMGWISAY NGNTNYAQKLQGRVTMT TDTSTSTAYMELSSLRSE DTAVYYCARVGFYDYVW GSYPYDAFDIWGQGTMV TVSSGSASAPTLGGGGS GGGGSAAAEIVMTQSPG TLSLSPGERATLSCRASQ SVSSNYLAWYQQKPGQA PRLLIYDASNRATGIPARF SGSGSGTDFTLTISSLEPE DFALYYCQQYGTSPLTFG GGTKLEIKRTVAAPSVF | 96-100 |
| Ab13 | DTSNGG YSSSSF DY | SSYTSSS TLV | EVQLVQSGGGL VQPGRSLRLSC AASGFTFDDYA MHWVRQAPGK GLEWVSGISWN SGSIGYADSVK GRFTISRDNAK NSLYLQMNSLR AEDTALYYCAK DTSNGGYSSSS FDYWGQGTLVT VSS | QSALTQPASVS GSPGQSITISC TGTSSDVGGY NYVSWYQQH PGTAPKLMIYD VSYRPSGVSN RFSGSKSGNT ASLTISGLQAE DEADYYCSSY TSSSTLVFGT GTKVTVL | EVQLVQSGGGLVQPGRSL RLSCAASGFTFDDYAMH WVRQAPGKGLEWVSGIS WNSGSIGYADSVKGRFTI SRDNAKNSLYLQMNSLR AEDTALYYCAKDTSNGGY SSSSFDYWGQGTLVTVS SGSASAPTLGGGGSGGG GSAAAQSALTQPASVSG SPGQSITISCTGTSSDVG GYNYVSWYQQHPGTAPK LMIYDVSYRPSGVSNRFS GSKSGNTASLTISGLQAE DEADYYCSSYTSSSTLVF GTGTKVTVLGQPKANPTV TLFPPSS | 101-105 |
| Ab14 | LVYGGY DEPGYY FDY | AAWDDS LNGWV | EVQLLESRAEVK KPGESLKISCKG SGYSFTSYWIG WVRQMPGKGP EWMGIIYPGDS DTRYSPSFQGQ VTISADKSISTA YLQWSSLKASD TAMYYCARLVY GGYDEPGYYFD YWGQGTLVTVS S | QSVLTQPPSAS GTPGQRVTIS CSGSSSNIRS NTVNWYQQLP GTAPKLLIYSN NQRPSGVPDR FSGSKSGTSA SLAISGLQSED EADYYCAAWD DSLNGWVFG GGTKLTVL | EVQLLESRAEVKKPGESLK ISCKGSGYSFTSYWIGWV RQMPGKGPEWMGIIYPG DSDTRYSPSFQGQVTISA DKSISTAYLQWSSLKASD TAMYYCARLVYGGYDEP GYYFDYWGQGTLVTVSS GSASAPTLGGGGSGGGG SAAAQSVLTQPPSASGTP GQRVTISCSGSSSNIRSN TVNWYQQLPGTAPKLLIY SNNQRPSGVPDRFSGSK SGTSASLAISGLQSEDEA DYYCAAWDDSLNGWVFG GGTKLTVLGQPKAAPSVT LFPPSSKASGA | 106-110 |
| Ab15 | VDGLEYS SGHNFD Y | SSYAGSY TWV | EVQLVQSGGGL VQPGRSLRLSC AASGFTFDDYA MHWVRQAPGK GLEWVSGISWN SGSIGYADSVK GRFTISRDNSK NTLYLQMNSLR AEDTAVYYCAK VDGLEYSSGHN FDYWGQGTLVT VSS | QSALTQPPSVS GSPGQSVTIS CTGSSSDVGY YDHVSWYQH HPGRAPKVIIY DVTKRPSGVP DRFSGSKSGN TASLTISGLQA EDEADYYCSS YAGSYTWVFG GGTELTVL | EVQLVQSGGGLVQPGRSL RLSCAASGFTFDDYAMH WVRQAPGKGLEWVSGIS WNSGSIGYADSVKGRFTI SRDNSKNTLYLQMNSLRA EDTAVYYCAKVDGLEYSS GHNFDYWGQGTLVTVSS GSASAPTLGGGGSGGGG SAAAQSALTQPPSVSGSP GQSVTISCTGSSSDVGYY DHVSWYQHHPGRAPKVII YDVTKRPSGVPDRFSGS KSGNTASLTISGLQAEDE ADYYCSSYAGSYTWVFG GGTELTVLGQPKAAPSVT LFPPSS | 111-115 |

In some embodiments, anti-pro/latent-myostatin antibody, or antigen-binding portion thereof, of the disclosure include any antibody, or antigen binding fragment thereof, that includes a CDRH1, CDRH2, CDRH3, CDRL1, CDRL2, or CDRL3, or combinations thereof, as provided for any one of the antibodies shown in Tables 1G-1 to 1G-3. In some embodiments, anti-pro/latent-myostatin antibodies, or antigen-binding portions thereof, comprise the CDRH1, CDRH2, CDRH3, CDRL1, CDRL2, and CDRL3 of any one of the antibodies shown in Tables 1G-1 to 1G-3. The disclosure also includes any nucleic acid sequence that encodes a molecule comprising a CDRH1, CDRH2, CDRH3, CDRL1, CDRL2, or CDRL3 as provided for any one of the antibodies shown in Tables 1G-1 to 1G-3. Antibody heavy and light chain CDR3 domains may play a particularly important role in the binding specificity/affinity of an antibody for an antigen. Accordingly, the anti-pro/latent myostatin antibodies, or antigen-binding portions thereof, of the disclosure, or the nucleic acid molecules thereof, may include at least the heavy and/or light chain CDR3s of antibodies as shown in Tables 1G-1 to 1G-3.

Aspects of the disclosure relate to a monoclonal antibody, or antigen binding fragment, that binds to pro/latent-myostatin protein and that comprises six complementarity determining regions (CDRs): CDRH1, CDRH2, CDRH3, CDRL1, CDRL2, and CDRL3.

In some embodiments, CDRH1 comprises a sequence as set forth in any one of SEQ ID NOs: 1-3. In some embodiments, CDRH2 comprises a sequence as set forth in any one of SEQ ID NOs: 4-9. In some embodiments, CDRH3 comprises a sequence as set forth in any one of SEQ ID NOs: 10-11, 66, 71, 76, 81, 86, 91, 96, 101, 106 and 111. CDRL1 comprises a sequence as set forth in any one of SEQ ID NOs: 12-16 and 59. In some embodiments, CDRL2 comprises a sequence as set forth in any one of SEQ ID NOs: 60, 61, 131 and 64. In some embodiments, CDRL3 comprises a sequence as set forth in any one of SEQ ID NOs: 65, 23, 67, 72, 77, 82, 87, 92, 97, 102, 107 and 112.

In some embodiments (e.g., as for anti-pro/latent-myostatin antibody Ab1, shown in Table 1G, or an antigen-binding portion thereof), CDRH1 comprises a sequence as set forth in SEQ ID NO: 1 or 2, CDRH2 comprises a sequence as set forth in SEQ ID NO: 4 or 5, CDRH3 comprises a sequence as set forth in SEQ ID NO: 10, CDRL1 comprises a sequence as set forth in SEQ ID NO: 12, or 13, CDRL2 comprises a sequence as set forth in SEQ ID NO: 60 or 61, and CDRL3 comprises a sequence as set forth in SEQ ID NO: 65, and the antibody, or an antigen-binding portion thereof, binds to pro/latent-myostatin.

In some embodiments (e.g., as for anti-pro/latent-myostatin antibody Ab2, shown in Table 1G, or an antigen-binding portion thereof), CDRH1 comprises a sequence as set forth in SEQ ID NO: 1 or 2, CDRH2 comprises a sequence as set forth in SEQ ID NO: 4 or 5, CDRH3 comprises a sequence as set forth in SEQ ID NO: 66, CDRL1 comprises a sequence as set forth in SEQ ID NO: 12, or 13, CDRL2 comprises a sequence as set forth in SEQ ID NO: 60 or 61, and CDRL3 comprises a sequence as set forth in SEQ ID NO: 67, and the antibody, or an antigen-binding portion thereof, binds to pro/latent-myostatin.

In some embodiments (e.g., as for anti-pro/latent-myostatin antibody Ab3, shown in Table 1G, or an antigen-binding portion thereof), CDRH1 comprises a sequence as set forth in SEQ ID NO: 1 or 3, CDRH2 comprises a sequence as set forth in SEQ ID NO: 6 or 7, CDRH3 comprises a sequence as set forth in SEQ ID NO: 11, CDRL1 comprises a sequence as set forth in SEQ ID NO: 14, or 15, CDRL2 comprises a sequence as set forth in SEQ ID NO: 131 or 64, and CDRL3 comprises a sequence as set forth in SEQ ID NO: 23, and the antibody, or an antigen-binding portion thereof, binds to pro/latent-myostatin.

In some embodiments (e.g., as for anti-pro/latent-myostatin antibody Ab5, shown in Table 1G, or an antigen-binding portion thereof), CDRH1 comprises a sequence as set forth in SEQ ID NO: 1 or 3, CDRH2 comprises a sequence as set forth in SEQ ID NO: 8 or 9, CDRH3 comprises a sequence as set forth in SEQ ID NO: 11, CDRL1 comprises a sequence as set forth in SEQ ID NO: 16, or 59, CDRL2 comprises a sequence as set forth in SEQ ID NO: 131 or 64, and CDRL3 comprises a sequence as set forth in SEQ ID NO: 23, and the antibody, or an antigen-binding portion thereof, binds to pro/latent-myostatin.

In some examples, any of the anti-pro/latent-myostatin antibodies, or antigen-binding portions thereof, of the disclosure include any antibody or antigen binding fragment having one or more CDR (e.g., CDRH or CDRL) sequences substantially similar to CDRH1, CDRH2, CDRH3, CDRL1, CDRL2, and/or CDRL3. For example, the antibodies may include one or more CDR sequences as shown in Tables 1A to 1G-3 (SEQ ID NOs: 1-16, 18, 23, 59-61, 64-72, 76, 77, 81, 82, 86, 87, 91, 92, 96, 97, 101, 102, 106, 107, 111, 112, and 119-124) containing up to 5, 4, 3, 2, or 1 amino acid residue variations as compared to the corresponding CDR region in any one of the SEQ ID NOs: shown in Tables 1A to 1G3 (1-16, 18, 23, 59-61, 64-72, 76, 77, 81, 82, 86, 87, 91, 92, 96, 97, 101, 102, 106, 107, 111, 112, and 119-124).

In some embodiments, an anti-pro/latent myostatin antibody or antigen-binding portion thereof described herein comprises three heavy chain complementarity determining regions (HCDRs) comprising amino acid sequences of SEQ ID NO: 1 (HCDR1), SEQ ID NO: 4 (HCDR2), and SEQ ID NO: 10 (HCDR3); and three light chain complementarity determining regions (LCDRs) comprising amino acid sequences of SEQ ID NO: 12 (LCDR1), SEQ ID NO: 60 (LCDR2), and SEQ ID NO: 65 (LCDR3), as defined by the Kabat numbering system.

In some embodiments, an anti-pro/latent myostatin antibody or antigen-binding portion thereof described herein comprises three heavy chain complementarity determining regions (HCDRs) comprising amino acid sequences of SEQ ID NO: 2 (HCDR1), SEQ ID NO: 5 (HCDR2), and SEQ ID NO: 10 (HCDR3); and three light chain complementarity determining regions (LCDRs) comprising amino acid sequences of SEQ ID NO: 13 (LCDR1), SEQ ID NO: 61 (LCDR2), and SEQ ID NO: 65 (LCDR3), as defined by the IMGT numbering system.

In various embodiments, the anti-pro/latent myostatin antibody or antigen-binding portion thereof comprises a heavy chain variable region comprising an amino acid sequence of SEQ ID NO: 25, and/or a light chain variable region comprising an amino acid sequence of SEQ ID NO: 31.

In various embodiments, the anti-pro/latent myostatin antibody or antigen-binding portion thereof comprises a heavy chain region comprising an amino acid sequence of SEQ ID NO: 50, and/or a light chain region comprising an amino acid sequence of SEQ ID NO: 51.

In some embodiments, anti-pro/latent-myostatin antibodies, or antigen-binding portions thereof, of the disclosure include any antibody that includes a heavy chain variable domain of any one of SEQ ID NOs: 24-29, 73, 78, 83, 88, 93, 98, 103, 108, 113, 125-128 or a light chain variable domain of any one of SEQ ID NOs: 30-35, 74, 79, 84, 89, 94, 99, 104, 109, 114, 129-130, 132, 133. In some embodiments, anti-pro/latent-myostatin antibodies, or antigen-binding portions thereof, of the disclosure include any antibody that includes the heavy chain variable and light chain variable pairs of SEQ ID NOs: 24 and 30; 25 and 31; 26 and 32; 27 and 33; 28 and 34; 29 and 35; 125 and 129; 126 and 132; 127 and 133; 128 and 130).

Aspects of the disclosure provide anti-pro/latent-myostatin antibodies, or antigen-binding portions thereof, having a heavy chain variable and/or a light chain variable amino acid sequence homologous to any of those described herein. In some embodiments, the anti-pro/latent-myostatin antibody, or antigen-binding portions thereof, comprises a heavy chain variable sequence or a light chain variable sequence that is at least 75% (e.g., 80%, 85%, 90%, 95%, 98%, or 99%) identical to the heavy chain variable sequence of any of SEQ ID NOs: 24-29, 73, 78, 83, 88, 93, 98, 103, 108, 113, 125-128 or a light chain variable sequence of any one of SEQ ID NOs: 30-35, 74, 79, 84, 89, 94, 99, 104, 109, 114, 129-130, 132, 133. In some embodiments, the homologous heavy chain variable and/or a light chain variable amino acid sequences do not vary within any of the CDR sequences provided herein. For example, in some embodiments, the degree of sequence variation (e.g., 75%, 80%, 85%, 90%, 95%, 98%, or 99%) may occur within a heavy chain variable and/or a light chain variable sequence excluding any of the CDR sequences provided herein.

The "percent identity" of two amino acid sequences is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, word length=3 to obtain amino acid sequences homologous to the protein molecules of interest. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (e.g., XBLAST and NBLAST) can be used.

In some embodiments, conservative mutations can be introduced into the CDRs or framework sequences at positions where the residues are not likely to be involved in interacting with pro/latent-myostatin as determined based on the crystal structure. As used herein, a "conservative amino acid substitution" refers to an amino acid substitution that does not alter the relative charge or size characteristics of the protein in which the amino acid substitution is made. Variants can be prepared according to methods for altering polypeptide sequence known to one of ordinary skill in the art such as are found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

In some embodiments, the antibodies, or antigen binding fragments thereof, provided herein comprise mutations that confer desirable properties to the antibodies, or antigen binding fragments thereof. For example, to avoid potential complications due to Fab-arm exchange, which is known to occur with native IgG4 mAbs, the antibodies, or antigen binding fragments thereof, provided herein may comprise a stabilizing 'Adair' mutation (Angal S., et al., "A single amino acid substitution abolishes the heterogeneity of chimeric mouse/human (IgG4) antibody," Mol Immunol 30, 105-108; 1993), where serine 228 (EU numbering; residue 241 Kabat numbering) is converted to proline resulting in an IgG1-like (CPPCP (SEQ ID NO: 58)) hinge sequence. Accordingly, any of the antibodies may include a stabilizing 'Adair' mutation or the amino acid sequence CPPCP (SEQ ID NO: 58).

Anti-pro/latent-myostatin antibodies, or antigen-binding portions thereof, of this disclosure may optionally comprise antibody constant regions or parts thereof. For example, a VL domain may be attached at its C-terminal end to a light chain constant domain like C_{κ} or C_{λ}. Similarly, a VH domain or portion thereof may be attached to all or part of a heavy chain like IgA, IgD, IgE, IgG, and IgM, and any isotype subclass. Antibodies may include suitable constant regions (see, for example, Kabat et al., Sequences of Proteins of Immunological Interest, No. 91-3242, National Institutes of Health Publications, Bethesda, Md. (1991)). Therefore, antibodies within the scope of this may disclosure include VH and VL domains, or an antigen binding portion thereof, combined with any suitable constant regions.

In certain embodiments, the VH and/or VL domains may be reverted to germline sequence, e.g., the FR of these domains are mutated using conventional molecular biology techniques to match those produced by the germline cells. For example, the VH and/or VL domains may be reverted to germline sequence of IgHV3-30 (SEQ ID NO: 36) and/or IgLV1-44 (SEQ ID NO: 37), respectively. It should be appreciated that any of the VH and/or VL domains may be reverted to any suitable germline sequence. In other embodiments, the FR sequences remain diverged from the consensus germline sequences.
IgHV3-30 
QVQLVESGGGVVQPGRSLRLSCAASGFTFSSYGMHWVRQAPGKGLEWVAVISYDGSNKYYADSVKGR
FTISRDNSKNTLYLQMNSLRAEDTAVYYCAR (SEQ ID NO: 36)
IgLV1-44

QSVLTQPPSASGTPGQRVTISCSGSSSNIGSNTVNWYQQLPGTAPKLLIYSNNQRPSGVPDRFSGSKSG
TSASLAISGLQSEDEADYYCAAWDDSLNG (SEQ ID NO: 37)

In some embodiments, anti-pro/latent-myostatin antibodies or antigen binding fragments may or may not include the framework region of the antibodies shown in SEQ ID NOs: 24-35. In some embodiments, anti-pro-latent-myostatin antibodies are murine antibodies and include murine framework region sequences.

In some embodiments, an anti-pro/latent-myostatin antibodies, or antigen binding fragments thereof, can bind to pro/latent-myostatin with relatively high affinity, e.g., with a K_{D} less than 10⁻⁸ M, 10⁻⁹ M, 10⁻¹⁰ M, 10⁻¹¹ M or lower. For example, anti-pro/latent-myostatin antibodies, or antigen binding fragments thereof, can bind to pro/latent-myostatin with an affinity between 5 pM and 500 nM, e.g., between 50 pM and 100 nM, e.g., between 500 pM and 50 nM. The invention also includes antibodies or antigen binding fragments that compete with any of the antibodies described herein for binding to pro/latent-myostatin and that have an affinity of 50 nM or lower (e.g., 20 nM or lower, 10 nM or lower, 500 pM or lower, 50 pM or lower, or 5 pM or lower). The affinity and binding kinetics of the anti-pro/latent-myostatin antibody can be tested using any suitable method including but not limited to biosensor technology (e.g., OCTET or BIACORE). When such binding profiles are measured with the use of OCTET or BIACORE, the assay is performed typically in accordance with the manufacturer's instructions, unless otherwise specified.

In some embodiments, antibodies, or antigen binding fragments thereof, are disclosed herein that specifically bind pro/latent-myostatin. In some embodiments, any of the antibodies, or antigen binding fragments thereof, provided herein bind at or near a tolloid cleavage site or at or near a tolloid docking site of pro/latent-myostatin. In some embodiments, an antibody binds near a tolloid cleavage site or near a tolloid docking site if it binds within 15 or fewer amino acid residues of the tolloid cleavage site or tolloid docking site. In some embodiments, any of the antibodies, or antigen binding fragments thereof, provided herein bind within 1, 2, 3, 4, 5, 6, 7, 8, 9, 10, 11, 12, 13, 14 or 15 amino acid residues of a tolloid cleavage site or tolloid docking site. In some embodiments, an antibody binds at or near a tolloid cleavage site of GDF8. For example, an antibody may bind an amino acid sequence as set forth in SEQ ID NO: 62 PKAPPLRELIDQYDVQRDDSSDGSLEDDDYHAT (SEQ ID NO: 62). In other embodiments, any of the antibodies, or antigen binding fragments thereof, provided herein bind at or near a proprotein convertase cleavage site or at or near a proprotein convertase docking site of pro/latent-myostatin. In some embodiments, an antibody binds near a proprotein convertase cleavage site or near a proprotein convertase docking site if it binds within 15 or fewer amino acid residues of the proprotein convertase cleavage site or proprotein convertase docking site. In some embodiments, any of the antibodies, or antigen binding fragments thereof, provided herein bind within 1, 2, 3, 4, 5, 6, 7, 8, 9, 10, 11, 12, 13, 14 or 15 amino acid residues of a proprotein convertase cleavage site or proprotein convertase docking site. In some embodiments, an antibody binds at or near a proprotein convertase cleavage site of GDF8. For example, an antibody may bind an amino acid sequence as set forth in SEQ ID NO: 63 (GLNPFLEVKVTDTPKRSRRDFGLDCDEHSTESRC).

In some embodiments, the myostatin-selective inhibitor according to the present disclosure is an antibody or antigen-binding fragment thereof, which is optionally a myostatin-selective inhibitor, that binds an epitope that includes at least one amino acid residue of "KALDEN" (SEQ ID NO: 118) and/or "FVQILRLIKPMKDGTRYTGIRSLK" (SEQ ID NO: 57). In some embodiments, the antibody is apitegromab or a variant thereof, wherein optionally the variant is an Fc variant. In some embodiments, the antibody is not apitegromab or its variant.

In some embodiments, the myostatin-selective inhibitor according to the present disclosure is an antibody or antigen-binding fragment thereof, which is optionally a myostatin-selective inhibitor, that binds an epitope that includes one or more amino acid residues of F147, Q149, L151, Y163, R167, S168, K170, K205, L207, E209 and N210, based on the numbering of the human proGDF8 sequence as set forth in SEQ ID NO; 52, which correspond to: F170, Q172, L174, Y186, R190, S191, K193, K228, L230, E232, and N233, respectively, based on the numbering of Dagbay et al. J. Biol. Chem. (2020), 295(16): 5404-5418. In preferred embodiments, such antibody binds an epitope that includes 10 or more, 9 or more, 8 or more, 7 or more, 6 or more, 5 or more, 4 or more, or 3 or more of the amino acid residues shown above. In some embodiments, the antibody is apitegromab or a variant thereof, wherein optionally the variant is an Fc variant. In some embodiments, the antibody is not apitegromab or its variant.

In one example, the anti-pro/latent-myostatin antibodies, or antigen binding fragments thereof, described herein specifically bind pro/latent-myostatin as compared to other forms of Myostatin and/or other members of the TGFβ family of growth factors. Members of the TGFβ family of growth factors include, without limitation AMH, ARTN, BMP10, BMP15, BMP2, BMP3, BMP4, BMP5, BMP6, BMP7, BMP8A, BMP8B, GDF1, GDF10, GDF11, GDF15, GDF2, GDF3, GDF3A, GDF5, GDF6, GDF7, GDF8, GDF9, GDNF, INHA, INHBA, INHBB, INHBC, INHBE, LEFTY1, LEFTY2, NODAL, NRTN, PSPN, TGFβ1, TGFβ2, and TGFβ3 protein. Such antibodies, or antigen binding fragments thereof, may bind pro/latent-myostatin at a much higher affinity as compared to other members of the TGFβ family of growth factors (e.g., at least 2-fold, 5-fold, 10-fold, 50-fold, 100-fold, 200-fold, 500-fold, or 1,000-fold higher). Such antibodies, or antigen binding fragments thereof, may bind pro/latent-myostatin with an affinity of at least 1000-fold higher as compared to other members of the TGFβ family of growth factors. In some embodiments, antibodies, or antigen binding fragments thereof, provided herein may bind to pro/latent-myostatin at a much higher affinity as compared to one or more forms of mature myostatin (e.g., at least 2-fold, 5-fold, 10-fold, 50-fold, 100-fold, 200-fold, 500-fold, or 1,000-fold higher). In some embodiments, antibodies, or antigen binding fragments thereof, provided herein may bind to pro/latent-myostatin with an affinity of at least 1,000-fold higher as compared to mature myostatin. Antibodies, or antigen binding fragments thereof, may exhibit a much higher inhibitory activity against proteolytic cleavage of pro/latent-myostatin (e.g., by a proprotein convertase or tolloid protease) as compared with other members of the TGFβ family, such as pro/latent GDF11 (e.g., at least 2-fold, 5-fold, 10-fold, 50-fold, 100-fold, 200-fold, 500-fold, 1,000-fold higher). In another embodiment, the antibodies, or antigen binding fragments thereof, disclosed herein do not bind to GDF11. This avoids potential toxicity issues associated with antibodies that cross-react with both myostatin and GDF11. An example of one such potential toxicity relates to impaired bone strength associated with GDF11 inhibition as recently reported in Suh et al. Proceedings of the National Academy of Sciences Mar 2020, 117 (9) 4910-4920.

In some embodiments, antibodies bind an antigen but cannot effectively eliminate the antigen from the plasma. Thus, in some embodiments, the concentration of the antigen in the plasma may be increased by reducing the clearance of the antigen. However, in some embodiments, antibodies (e.g., sweeping antibodies) provided herein have an affinity to an antigen that is sensitive to pH. Such pH sensitive antibodies may bind to the antigen in plasma at neutral pH and dissociate from the antigen in an acidic endosome, thus reducing antibody-mediated antigen accumulation and/or promoting antigen clearance from the plasma.

Aspects of the disclosure relate to sweeping antibodies. As used herein "sweeping antibodies" or antigen-binding fragments thereof refer to antibodies, or antigen-binding fragments thereof, having both pH-sensitive antigen binding and at least a threshold level of binding to cell surface neonatal Fc receptor (FcRn) at neutral or physiological pH. In some embodiments, sweeping antibodies, or an antigen-binding portion thereof, bind to the neonatal Fc receptor FcRn at neutral pH. For example, sweeping antibodies may bind to the FcRn at a pH ranging from 7.0 to 7.6. In some embodiments, sweeping antibodies, or an antigen-binding portion thereof, can bind to an antigen at an antigen binding site and bind to a cellular FcRn via an Fc portion of the antibody. In some embodiments, sweeping antibodies, or an antigen-binding portion thereof, may then be internalized, releasing antigen in an acidic endosome, which may be degraded. In some embodiments, a sweeping antibody, or an antigen-binding portion thereof, no longer bound to the antigen, may then be released (e.g., by exocytosis) by the cell back into the serum.

In some embodiments, FcRn in the vascular endothelia (e.g., of a subject) extends the half-life of a sweeping antibody, or an antigen-binding portion thereof. In some embodiments, vascular endothelial cells internalize sweeping antibodies, or antigen-binding portions thereof, which in some embodiments are bound to an antigen such as myostatin (e.g., pro-myostatin, latent myostatin or primed myostatin). In some embodiments, a sweeping antibody, or an antigen-binding portion thereof, is recycled back into the bloodstream. In some embodiments, a sweeping antibody, or an antigen-binding portion thereof, has an increased half-life (e.g., in the serum of a subject) as compared to its conventional counterpart. In some embodiments, a conventional counterpart of a sweeping antibody refers the antibody, or an antigen-binding portion thereof, from which the sweeping antibody, or an antigen-binding portion thereof, was derived (e.g., prior to engineering the Fc portion of the conventional antibody to bind FcRn with greater affinity at pH 7). In some embodiments, a sweeping antibody, or an antigen-binding portion thereof, has a half-life in the serum of a subject that is at least 1%, 5%, 10%, 15%, 20%, 25%, 35%, 50%, 75%, 100%, 150%, 200% or 250% longer as compared to its conventional counterpart.

In some embodiments, an Fc portion of a sweeping antibody binds FcRn. In some embodiments, the Fc portion of a sweeping antibody binds to FcRn at a pH of 7.4 with a K_{D} ranging from 10⁻³ M to 10⁻⁸ M. In some embodiments, a sweeping antibody binds to FcRn at a pH of 7.4 with a K_{D} ranging from 10⁻³ M to 10⁻⁷ M, from 10⁻³ M to 10⁻⁶ M, from 10⁻³ M to 10⁻⁵ M, from 10⁻³ M to 10⁻⁴ M, from 10⁻⁴ M to 10⁻⁸ M, from 10⁻⁴ M to 10⁻⁷ M, from 10⁻⁴ M to 10⁻⁶ M, from 10⁻⁴ M to 10⁻⁵ M, from 10⁻⁵ M to 10⁻⁸ M, from 10⁻⁵ M to 10⁻⁷ M, from 10⁻⁵ M to 10⁻⁶ M, from 10⁻⁶ M to 10⁻⁸ M, from 10⁻⁶ M to 10⁻⁷ M, or from 10⁻⁷ M to 10⁻⁸ M. In some embodiments, FcRn binds to the CH2-CH3 hinge region of a sweeping antibody. In some embodiments, FcRn binds to the same region as protein A or protein G. In some embodiments, FcRn binds to a different binding site from FcyRs. In some embodiments, the amino acid residues AA of a sweeping antibody Fc region are required for binding to FcRn. In some embodiments, the amino acid residues AA of a sweeping antibody Fc region affect binding to FcRn.

In some embodiments, any of the antibodies, or antigen binding fragments thereof, provided herein are engineered to bind FcRn with greater affinity. In some embodiments, any of the antibodies, or antigen binding fragments thereof, provided herein are engineered to bind FcRn with greater affinity at pH 7.4. In some embodiments, the affinity of antibodies, or antigen binding fragments thereof, to FcRn is increased to extend their pharmacokinetic (PK) properties as compared to their conventional counterparts. For example, in some embodiments, sweeping antibodies elicit less adverse reactions due to their efficacy at lower doses. In some embodiments, sweeping antibodies, or an antigen-binding portion thereof, are administered less frequently. In some embodiments, transcytosis of sweeping antibodies, or an antigen-binding portion thereof, to certain tissue types are increased. In some embodiments, sweeping antibodies, or antigen-binding portions thereof, enhance efficiency of trans-placental delivery. In some embodiments, sweeping antibodies, or antigen-binding portions thereof, are less costly to produce.

In some embodiments, any of the antibodies, or antigen binding fragments thereof, provided herein are engineered to bind FcRn with lower affinity. In some embodiments, any of the antibodies, or antigen binding fragments thereof, provided herein are engineered to bind FcRn with lower affinity at pH 7.4. In some embodiments, the affinity of sweeping antibodies, or an antigen-binding portion thereof, to FcRn is decreased to shorten their pharmacokinetic (PK) properties as compared to their conventional counterparts. For example, in some embodiments, sweeping antibodies, or an antigen-binding portion thereof, are more rapidly cleared for imaging and/or radioimmunotherapy. In some embodiments, sweeping antibodies, or an antigen-binding portion thereof, promote clearance of endogenous pathogenic antibodies as a treatment for autoimmune diseases. In some embodiments, sweeping antibodies, or antigen-binding portions thereof, reduce the risk of adverse pregnancy outcome, which may be caused by trans-placental transport of material fetus-specific antibodies.

In some embodiments, sweeping antibodies, or an antigen-binding portion thereof, have decreased affinity to an antigen at low pH as compared to a neutral or physiological pH (e.g., pH 7.4). In some embodiments, sweeping antibodies, or an antigen-binding portion thereof, have a decreased affinity to an antigen at an acidic pH (e.g., a pH ranging from 5.5 to 6.5) as compared to a physiological pH (e.g., pH 7.4).

It should be appreciated that any of the antibodies, or antigen binding fragments thereof, provided herein can be engineered to dissociate from the antigen depending on changes in pH (e.g., pH-sensitive antibodies). In some embodiments, sweeping antibodies, or an antigen-binding portion thereof, provided herein are engineered to bind antigen in a pH-dependent manner. In some embodiments, sweeping antibodies, or an antigen-binding portion thereof, provided herein are engineered to bind FcRn in a pH-dependent manner. In some embodiments, sweeping antibodies, or an antigen-binding portion thereof, provided herein are internalized by endocytosis. In some embodiments, sweeping antibodies, or an antigen-binding portion thereof, provided here are internalized by FcRn binding. In some embodiments, endocytosed sweeping antibodies, or antigen-binding portion thereof, release antigen in an endosome. In some embodiments, sweeping antibodies, or antigen-binding portions thereof, are recycled back to the cell surface. In some embodiments, sweeping antibodies remain attached to cells. In some embodiments, endocytosed sweeping antibodies, or an antigen-binding portion thereof, are recycled back to the plasma. It should be appreciated that the Fc portion of any of the antibodies, or antigen binding fragments thereof, provided herein may be engineered to have different FcRn binding activity. In some embodiments, FcRn binding activity affects the clearance time of an antigen by a sweeping antibody. In some embodiments, sweeping antibodies may be long-acting or rapid-acting sweeping antibodies.

In some embodiments, converting a conventional therapeutic antibody, or an antigen-binding portion thereof, into a sweeping antibody, or an antigen-binding portion thereof, reduces the efficacious dose. In some embodiments, converting a conventional therapeutic antibody, or an antigen-binding portion thereof, into a sweeping antibody, or an antigen-binding portion thereof, reduces the efficacious dose by at least 1%, 2%, 5%, 10%, 20%, 30%, 40%, 50%, 60%, 70%, 80%, 90%, 95%, or 99%. In some embodiments, converting a conventional therapeutic antibody, or an antigen-binding portion thereof, into a sweeping antibody, or an antigen-binding portion thereof, reduces the efficacious dose by at least 1.5-fold, 2-fold, 3-fold, 4-fold, 5-fold, 6-fold, 8-fold, 10-fold, 15-fold, 20-fold, 50-fold or 100-fold.

In some embodiments, selecting an appropriate dose of a sweeping antibody, or an antigen-binding portion thereof, for therapy may be performed empirically. In some embodiments, a high dose of a sweeping antibody, or an antigen-binding portion thereof, may saturate FcRn, resulting in antibodies which stabilize antigen in serum without being internalized. In some embodiments, a low dose of a sweeping antibody, or an antigen-binding portion thereof, may not be therapeutically effective. In some embodiments, sweeping antibodies, or antigen-binding portions thereof, are administered once a day, once a week, once every two weeks, once every three weeks, once every four weeks, once every 6 weeks, once every 8 weeks, once every 10 weeks, once every 12 weeks, once every 16 weeks, once every 20 weeks, or once every 24 weeks.

In some embodiments, any of the antibodies, or antigen binding fragments thereof, provided herein may be modified or engineered to be sweeping antibodies. In some embodiments, any of the antibodies, or antigen binding fragments thereof, provided herein may be converted into a sweeping antibody using any suitable method. For example, suitable methods for making sweeping antibodies, or antigen-binding portions thereof, have been previously described in Igawa et al., (2013) "Engineered Monoclonal Antibody with Novel Antigen-Sweeping Activity In vivo," PLoS ONE 8(5): e63236; and Igawa et al., "pH-dependent antigen-binding antibodies as a novel therapeutic modality," Biochimica et Biophysica Acta 1844 (2014) 1943-1950. It should be appreciated, however, that the methods for making sweeping antibodies, or an antigen-binding portion thereof, as provided herein are not meant to be limiting. Thus, additional methods for making sweeping antibodies, or an antigen-binding portion thereof, are within the scope of this disclosure.

Some aspects of the disclosure are based on the recognition that the affinity (e.g., as expressed as K_{D}) of any of the anti-pro/latent-myostatin antibodies, or antigen binding fragments thereof, provided herein are sensitive to changes in pH. In some embodiments, the antibodies, or antigen binding fragments thereof, provided herein have an increased K_{D} of binding to pro/latent-myostatin at a relatively low pH (e.g., a pH ranging from 4.0-6.5) as compared to a relatively high pH (e.g., a pH ranging from 7.0-7.4). In some embodiments, the antibodies, or antigen binding fragments thereof, provided herein have a K_{D} of binding to pro/latent-myostatin ranging from 10⁻³ M, 10⁻⁴ M, 10⁻⁵ M, 10⁻⁶ M, 10⁻⁷ M, 10⁻⁸ M when the pH is between 4.0 and 6.5. In some embodiments, the antibodies, or antigen binding fragments thereof, provided herein have a K_{D} of binding to pro/latent-myostatin ranging from 10⁻⁶ M, 10⁻⁷ M, 10⁻⁸ M, 10⁻⁹ M, 10⁻¹⁰ M, 10⁻¹¹ M when the pH is between 7.0 and 7.4. In some embodiments, the antibodies, or antigen binding fragments thereof, provided herein have a K_{D} of binding to pro/latent-Myostatin that is at least 2-fold, at least 10-fold, at least 50-fold, at least 100-fold, at least 500-fold, at least 1000-fold, at least 5000-fold, or at least 10000-fold greater at a pH between 4.0 and 6.5 as compared to a pH between 7.0 and 7.4.

In some embodiments, pro/latent-myostatin antibodies, or antigen binding fragments thereof, are provided herein that specifically bind to the same epitope as an antibody described in Table 2a, 11a, 11b, or 13 of International Patent Application Publication No. WO 2016/098357, which was published on June 23, 2016, and which is based on International Patent Application No. PCT/JP2015/006323, which was filed on December 18, 2015. In some embodiments, pro/latent-myostatin antibodies, or antigen binding fragments thereof, are provided herein that compete for binding with such an antibody.

In some embodiments, pro/latent-myostatin antibodies, or antigen binding fragments thereof, provided herein do not specifically bind to the same epitope as an antibody described in Table 2a, 11a, 11b, or 13 of International Patent Application Publication No. WO 2016/098357, which was published on June 23, 2016, and which is based on International Patent Application No. PCT/JP2015/006323, which was filed on December 18, 2015. In some embodiments, pro/latent-myostatin antibodies, or antigen binding fragments thereof, provided herein do not compete or do not cross-compete for binding to the same epitope as an antibody described in Table 2a, 11a, 11b, or 13 of International Patent Application Publication No. WO 2016/098357, which was published on June 23, 2016, and which is based on International Patent Application No. PCT/JP2015/006323, which was filed on December 18, 2015. In some embodiments, pro/latent-myostatin antibodies, or antigen binding fragments thereof, provided herein do not specifically bind to the same epitope as an antibody comprising a VH and a VL pair described in Table 2a, 11a, 11b, or 13 of International Patent Application Publication No. WO 2016/098357, which was published on June 23, 2016, and which is based on International Patent Application No. PCT/JP2015/006323, which was filed on December 18, 2015. In some embodiments, pro/latent-myostatin antibodies, or antigen binding fragments thereof, provided herein do not compete or do not cross-compete for binding to the same epitope as an antibody comprising a VH and a VL pair described in Table 2a, 11a, 11b, or 13 of International Patent Application Publication No. WO 2016/098357, which was published on June 23, 2016, and which is based on International Patent Application No. PCT/JP2015/006323, which was filed on December 18, 2015.

### Antibodies, and Antigen-Binding Fragments, that Compete with Anti-pro/latent-Myostatin Antibodies, or Antigen Binding Fragments Thereof

Aspects of the disclosure relate to antibodies, and antigen-binding fragments thereof, that compete or cross-compete with any of the antibodies, or antigen binding fragments thereof, provided herein. The term "compete", as used herein with regard to an antibody, means that a first antibody binds to an epitope of a protein (e.g., latent **myostatin)** in a manner sufficiently similar to the binding of a second antibody, such that the result of binding of the first antibody with its epitope is detectably decreased in the presence of the second antibody compared to the binding of the first antibody in the absence of the second antibody. The alternative, where the binding of the second antibody to its epitope is also detectably decreased in the presence of the first antibody, can, but need not be the case. That is, a first antibody can inhibit the binding of a second antibody to its epitope without that second antibody inhibiting the binding of the first antibody to its respective epitope. However, where each antibody detectably inhibits the binding of the other antibody with its epitope or ligand, whether to the same, greater, or lesser extent, the antibodies are said to "cross-compete" with each other for binding of their respective epitope(s). Both competing and cross-competing antibodies are within the scope of this disclosure. Regardless of the mechanism by which such competition or cross-competition occurs (e.g., steric hindrance, conformational change, or binding to a common epitope, or portion thereof), the skilled artisan would appreciate that such competing and/or cross-competing antibodies are encompassed and can be useful for the methods and/or compositions provided herein.

Aspects of the disclosure relate to antibodies, or antigen-binding portions thereof, that compete or cross-compete with any of the antibodies, or antigen binding fragments thereof, provided herein. In some embodiments, an antibody, or an antigen-binding portion thereof, binds at or near the same epitope as any of the antibodies provided herein. In some embodiments, an antibody, or an antigen-binding portion thereof, binds near an epitope if it binds within 15 or fewer amino acid residues of the epitope. In some embodiments, any of the antibodies, or antigen binding fragments thereof, provided herein bind within 1, 2, 3, 4, 5, 6, 7, 8, 9, 10, 11, 12, 13, 14 or 15 amino acid residues of an epitope that is bound by any of the antibodies, or antigen binding fragments thereof, provided herein. In preferred embodiments, such antibody or antigen-binding fragment cross-competes with apitegromab for binding to human pro/latent myostatin. In some embodiments, such antibody or antigen-binding fragment binds an epitope within the prodomain of human myostatin, wherein the epitope comprises one or more amino acid residues F147, Q149, L151, Y186, S168, K170, K205, and/or L207, as numbered according to SEQ ID NO: 52 disclosed herein. In some embodiments, such antibody or antigen-binding fragment binds an epitope within the prodomain of human myostatin, wherein the epitope comprises one or more amino acid residues F147, Q149, L151, Y186, R167, S168, K170, K205, L207, E209, and/or N210, as numbered according to SEQ ID NO: 52. In some embodiments, such antibody or antigen-binding fragment comprises an HCDR3 paratope that is a variant of SEQ ID NO: 10, wherein the variant contains of up to two amino acid substitutions as compared to SEQ ID NO: 10. In some embodiments, such antibody or antigen-binding fragment comprises an HCDR3 sequence comprising a leucine at amino acid position 3 and a tryptophan at amino acid position 9, as numbered according to SEQ ID NO: 10. In some embodiments, such antibody or antigen-binding fragment comprises an HCDR3 sequence comprising a leucine at amino acid position 3, a valine at amino acid position 4, a leucine at amino acid position 7, a tyrosine at amino acid position 8, and/or a tryptophan at amino acid position 9, as numbered according to SEQ ID NO: 10.

In another embodiment, an antibody, or an antigen-binding portion thereof, competes or cross-competes for binding to any of the antigens provided herein (e.g., pro/latent-myostatin) with an equilibrium dissociation constant, K_{D}, between the antibody and the protein of less than 10⁻⁸ M. In other embodiments, an antibody, or an antigen-binding portion thereof, competes or cross-competes for binding to any of the antigens provided herein with a K_{D} in a range from 10⁻¹¹ M to 10⁻⁸ M.

Aspects of the disclosure relate to antibodies, or antigen-binding portions thereof, that compete for binding to pro/latent-myostatin with any of the antibodies, or antigen binding fragments thereof, provided herein. In some embodiments, the antibody, or an antigen-binding portion thereof, binds to pro/latent-myostatin at the same epitope as any of the antibodies, or antigen-binding portions thereof, provided herein. For example, in some embodiments any of the antibodies provided herein bind at or near a tolloid cleavage site or at or near a tolloid docking site of pro/latent-myostatin. In other embodiments, any of the antibodies, or antigen binding fragments thereof, provided herein bind at or near a proprotein convertase cleavage site or at or near a proprotein convertase docking site of pro/latent-myostatin. In another embodiment, an antibody, or an antigen-binding portion thereof, competes for binding to pro/latent-myostatin with an equilibrium dissociation constant, K_{D}, between the antibody, or antigen-binding portion thereof, and pro/latent-myostatin of less than 10⁻⁶ M. In other embodiments, the antibody, or antigen-binding portion thereof, that competes with any of the antibodies, or antigen-binding portions thereof, provided herein binds to pro/latent-myostatin with a K_{D} in ranging from 10⁻¹¹ M to 10⁻⁸ M.

Any of the antibodies, or antigen binding fragments thereof, provided herein can be characterized using any suitable methods. For example, one method is to identify the epitope to which the antigen binds, or "epitope mapping." There are many suitable methods for mapping and characterizing the location of epitopes on proteins, including solving the crystal structure of an antibody-antigen complex, competition assays, gene fragment expression assays, and synthetic peptide-based assays, as described, for example, in Chapter 11 of Harlow and Lane, Using Antibodies, a Laboratory Manual, Cold Spring Harbor Laboratory Press, Cold Spring Harbor, N.Y., 1999. In an additional example, epitope mapping can be used to determine the sequence to which an antibody, or an antigen-binding portion thereof, binds. The epitope can be a linear epitope, i.e., contained in a single stretch of amino acids, or a conformational epitope formed by a three-dimensional interaction of amino acids that may not necessarily be contained in a single stretch (primary structure linear sequence). Peptides of varying lengths (e.g., at least 4-6 amino acids long) can be isolated or synthesized (e.g., recombinantly) and used for binding assays with an antibody. In another example, the epitope to which the antibody, or an antigen-binding portion thereof, binds can be determined in a systematic screen by using overlapping peptides derived from the target antigen sequence and determining binding by the antibody, or an antigen-binding portion thereof. According to the gene fragment expression assays, the open reading frame encoding the target antigen is fragmented either randomly or by specific genetic constructions and the reactivity of the expressed fragments of the antigen with the antibody to be tested is determined. The gene fragments may, for example, be produced by PCR and then transcribed and translated into protein in vitro, in the presence of radioactive amino acids. The binding of the antibody, or an antigen-binding portion thereof, to the radioactively labeled antigen fragments is then determined by immunoprecipitation and gel electrophoresis. Certain epitopes can also be identified by using large libraries of random peptide sequences displayed on the surface of phage particles (phage libraries). Alternatively, a defined library of overlapping peptide fragments can be tested for binding to the test antibody, or an antigen-binding portion thereof, in simple binding assays. In an additional example, mutagenesis of an antigen binding domain, domain swapping experiments and alanine scanning mutagenesis can be performed to identify residues required, sufficient, and/or necessary for epitope binding. For example, domain swapping experiments can be performed using a mutant of a target antigen in which various fragments of the pro/latent-myostatin polypeptide have been replaced (swapped) with sequences from a closely related, but antigenically distinct protein, such as another member of the TGFβ protein family (e.g., GDF11). By assessing binding of the antibody, or antigen-binding portion thereof, to the mutant pro/latent-myostatin, the importance of the particular antigen fragment to antibody, or antigen-binding portion thereof, binding can be assessed.

Alternatively, competition assays can be performed using other antibodies known to bind to the same antigen to determine whether an antibody, or an antigen-binding portion thereof, binds to the same epitope as the other antibodies, or antigen-binding portions thereof. Competition assays are well known to those of skill in the art.

Any of the suitable methods, e.g., the epitope mapping methods as described herein, can be applied to determine whether an anti-pro/latent-myostatin antibody, or an antigen-binding portion thereof, binds one or more of the specific residues/segments in pro/latent-myostatin as described herein. Further, the interaction of the antibody, or an antigen-binding portion thereof, with one or more of those defined residues in pro/latent-myostatin can be determined by routine technology. For example, a crystal structure can be determined, and the distances between the residues in pro/latent-myostatin and one or more residues in the antibody, or antigen-binding portion thereof, can be determined accordingly. Based on such distance, whether a specific residue in pro/latent-myostatin interacts with one or more residues in the antibody, or antigen-binding portion thereof, can be determined. Further, suitable methods, such as competition assays and target mutagenesis assays can be applied to determine the preferential binding of a candidate anti-pro/latent-myostatin antibody, or an antigen-binding portion thereof, to pro/latent-myostatin as compared to another target such as a mutant pro/latent-myostatin.

### Production of Anti-pro/latent-Myostatin Antibodies or Antigen Binding Fragments Thereof

Numerous methods may be used for obtaining antibodies, or antigen binding fragments thereof, of the disclosure. For example, antibodies, and antigen-binding fragments thereof, can be produced using recombinant DNA methods. Monoclonal antibodies, and antigen-binding fragments thereof, may also be produced by generation of hybridomas (see e.g., Kohler and Milstein (1975) Nature, 256: 495-499) in accordance with known methods. Hybridomas formed in this manner are then screened using standard methods, such as enzyme-linked immunosorbent assay (ELISA) and surface plasmon resonance (e.g., OCTET or BIACORE) analysis, to identify one or more hybridomas that produce an antibody, or an antigen-binding portion thereof, that specifically binds to a specified antigen. Any form of the specified antigen may be used as the immunogen, e.g., recombinant antigen, naturally occurring forms, any variants or fragments thereof, as well as antigenic peptide thereof (e.g., any of the epitopes described herein as a linear epitope or within a scaffold as a conformational epitope). One exemplary method of making antibodies, and antigen-binding portions thereof, includes screening protein expression libraries that express antibodies or fragments thereof (e.g., scFv), e.g., phage or ribosome display libraries. Phage display is described, for example, in Ladner et al., U.S. Pat. No. 5,223,409; Smith (1985) Science 228:1315-1317; Clackson et al. (1991) Nature, 352: 624-628; Marks et al. (1991) J. Mol. Biol., 222: 581-597; WO92/18619; WO 91/17271; WO 92/20791; WO 92/15679; WO 93/01288; WO 92/01047; WO 92/09690; and WO 90/02809.

In addition to the use of display libraries, the specified antigen (e.g., pro-myostatin) can be used to immunize a non-human animal, e.g., a rodent, e.g., a mouse, hamster, or rat. In one embodiment, the non-human animal is a mouse.

In another embodiment, a monoclonal antibody is obtained from the non-human animal, and then modified, e.g., chimeric, using suitable recombinant DNA techniques. A variety of approaches for making chimeric antibodies have been described. See e.g., Morrison et al., Proc. Natl. Acad. Sci. U.S.A. 81:6851, 1985; Takeda et al., Nature 314:452, 1985, Cabilly et al., U.S. Pat. No. 4,816,567; Boss et al., U.S. Pat. No. 4,816,397; Tanaguchi et al., European Patent Publication EP171496; European Patent Publication 0173494, United Kingdom Patent GB 2177096B.

For additional antibody production techniques, see Antibodies: A Laboratory Manual, eds. Harlow et al., Cold Spring Harbor Laboratory, 1988. The present disclosure is not necessarily limited to any particular source, method of production, or other special characteristics of an antibody.

Some aspects of the present disclosure relate to host cells transformed with a polynucleotide or vector. Host cells may be a prokaryotic or eukaryotic cell. The polynucleotide or vector which is present in the host cell may either be integrated into the genome of the host cell or it may be maintained extrachromosomally. The host cell can be any prokaryotic or eukaryotic cell, such as a bacterial, insect, fungal, plant, animal or human cell. In some embodiments, fungal cells are, for example, those of the genus Saccharomyces, in particular those of the species S. cerevisiae. The term "prokaryotic" includes all bacteria which can be transformed or transfected with a DNA or RNA molecules for the expression of an antibody or the corresponding immunoglobulin chains. Prokaryotic hosts may include gram negative as well as gram positive bacteria such as, for example, E. coli, S. typhimurium, *Serratia* marcescens and *Bacillus* subtilis. The term "eukaryotic" includes yeast, higher plants, insects and vertebrate cells, e.g., mammalian cells, such as NSO and CHO cells. Depending upon the host employed in a recombinant production procedure, the antibodies or immunoglobulin chains encoded by the polynucleotide may be glycosylated or may be non-glycosylated. Antibodies or the corresponding immunoglobulin chains may also include an initial methionine amino acid residue.

In some embodiments, once a vector has been incorporated into an appropriate host, the host may be maintained under conditions suitable for high level expression of the nucleotide sequences, and, as desired, the collection and purification of the immunoglobulin light chains, heavy chains, light/heavy chain dimers or intact antibodies, antigen binding fragments or other immunoglobulin forms may follow; see, Beychok, Cells of Immunoglobulin Synthesis, Academic Press, N.Y. (1979). Thus, polynucleotides or vectors are introduced into the cells which in turn produce the antibody or antigen binding fragments. Furthermore, transgenic animals, preferably mammals, comprising the aforementioned host cells may be used for the large-scale production of the antibody or antibody fragments. Large-scale production typically refers to 250 liters or greater of bioreactor (e.g., cell culture), e.g., 250L, 500L, 1000L, 1500L, 2000L, 3000L, 4000L, 5000L, 6000L, or larger.

The transformed host cells can be grown in fermenters and cultured using any suitable techniques to achieve optimal cell growth. Once expressed, the whole antibodies, their dimers, individual light and heavy chains, other immunoglobulin forms, or antigen binding fragments, can be purified according to standard procedures of the art, including ammonium sulfate precipitation, affinity columns, column chromatography, gel electrophoresis and the like; see, Scopes, "Protein Purification", Springer Verlag, N.Y. (1982). The antibody or antigen binding fragments can then be isolated from the growth medium, cellular lysates, or cellular membrane fractions. The isolation and purification of the, e.g., microbially expressed antibodies or antigen binding fragments may be by any conventional means such as, for example, preparative chromatographic separations and immunological separations such as those involving the use of monoclonal or polyclonal antibodies directed, e.g., against the constant region of the antibody.

Aspects of the disclosure relate to a hybridoma, which provides an indefinitely prolonged source of monoclonal antibodies. As an alternative to obtaining immunoglobulins directly from the culture of hybridomas, immortalized hybridoma cells can be used as a source of rearranged heavy chain and light chain loci for subsequent expression and/or genetic manipulation. Rearranged antibody genes can be reverse transcribed from appropriate mRNAs to produce cDNA. In some embodiments, heavy chain constant region can be exchanged for that of a different isotype or eliminated altogether. The variable regions can be linked to encode single chain Fv regions. Multiple Fv regions can be linked to confer binding ability to more than one target or chimeric heavy and light chain combinations can be employed. Any appropriate method may be used for cloning of antibody variable regions and generation of recombinant antibodies, and antigen-binding portions thereof.

In some embodiments, an appropriate nucleic acid that encodes variable regions of a heavy and/or light chain is obtained and inserted into an expression vectors which can be transfected into standard recombinant host cells. A variety of such host cells may be used. In some embodiments, mammalian host cells may be advantageous for efficient processing and production. Typical mammalian cell lines useful for this purpose include CHO cells, 293 cells, or NSO cells. The production of the antibody or antigen binding fragment may be undertaken by culturing a modified recombinant host under culture conditions appropriate for the growth of the host cells and the expression of the coding sequences. The antibodies or antigen binding fragments may be recovered by isolating them from the culture. The expression systems may be designed to include signal peptides so that the resulting antibodies are secreted into the medium; however, intracellular production is also possible.

The disclosure also includes a polynucleotide encoding at least a variable region of an immunoglobulin chain of the antibodies described herein. In some embodiments, the variable region encoded by the polynucleotide comprises at least one complementarity determining region (CDR) of the VH and/or VL of the variable region of the antibody produced by any one of the above described hybridomas.

Polynucleotides encoding antibody or antigen binding fragments may be, e.g., DNA, cDNA, RNA or synthetically produced DNA or RNA or a recombinantly produced chimeric nucleic acid molecule comprising any of those polynucleotides either alone or in combination. In some embodiments, a polynucleotide is part of a vector. Such vectors may comprise further genes such as marker genes which allow for the selection of the vector in a suitable host cell and under suitable conditions.

In some embodiments, a polynucleotide is operatively linked to expression control sequences allowing expression in prokaryotic or eukaryotic cells. Expression of the polynucleotide comprises transcription of the polynucleotide into a translatable mRNA. Regulatory elements ensuring expression in eukaryotic cells, preferably mammalian cells, are well known to those skilled in the art. They may include regulatory sequences that facilitate initiation of transcription and optionally poly-A signals that facilitate termination of transcription and stabilization of the transcript. Additional regulatory elements may include transcriptional as well as translational enhancers, and/or naturally associated or heterologous promoter regions. Possible regulatory elements permitting expression in prokaryotic host cells include, e.g., the PL, Lac, Trp or Tac promoter in E. coli, and examples of regulatory elements permitting expression in eukaryotic host cells are the AOX1 or GAL1 promoter in yeast or the CMV-promoter, SV40-promoter, RSV-promoter (Rous sarcoma virus), CMV-enhancer, SV40-enhancer or a globin intron in mammalian and other animal cells.

Beside elements which are responsible for the initiation of transcription such regulatory elements may also include transcription termination signals, such as the SV40-poly-A site or the tk-poly-A site, downstream of the polynucleotide. Furthermore, depending on the expression system employed, leader sequences capable of directing the polypeptide to a cellular compartment or secreting it into the medium may be added to the coding sequence of the polynucleotide and have been described previously. The leader sequence(s) is (are) assembled in appropriate phase with translation, initiation, and termination sequences, and preferably, a leader sequence capable of directing secretion of translated protein, or a portion thereof, into, for example, the extracellular medium. Optionally, a heterologous polynucleotide sequence can be used that encode a fusion protein including a C- or N-terminal identification peptide imparting desired characteristics, e.g., stabilization or simplified purification of expressed recombinant product.

In some embodiments, polynucleotides encoding at least the variable domain of the light and/or heavy chain may encode the variable domains of both immunoglobulin chains or only one. Likewise, a polynucleotide(s) may be under the control of the same promoter or may be separately controlled for expression. Furthermore, some aspects relate to vectors, particularly plasmids, cosmids, viruses and bacteriophages used conventionally in genetic engineering that comprise a polynucleotide encoding a variable domain of an immunoglobulin chain of an antibody or antigen binding fragment; optionally in combination with a polynucleotide that encodes the variable domain of the other immunoglobulin chain of the antibody.

In some embodiments, expression control sequences are provided as eukaryotic promoter systems in vectors capable of transforming or transfecting eukaryotic host cells, but control sequences for prokaryotic hosts may also be used. Expression vectors derived from viruses such as retroviruses, vaccinia virus, adeno-associated virus, herpes viruses, or bovine papilloma virus, may be used for delivery of the polynucleotides or vector into targeted cell population (e.g., to engineer a cell to express an antibody or antigen binding fragment). A variety of appropriate methods can be used to construct recombinant viral vectors. In some embodiments, polynucleotides and vectors can be reconstituted into liposomes for delivery to target cells. The vectors containing the polynucleotides (e.g., the heavy and/or light variable domain(s) of the immunoglobulin chains encoding sequences and expression control sequences) can be transferred into the host cell by suitable methods, which vary depending on the type of cellular host.

### Modifications

Antibodies and antigen binding fragments of the disclosure may be modified with a detectable label, including, but not limited to, an enzyme, prosthetic group, fluorescent material, luminescent material, bioluminescent material, radioactive material, positron emitting metal, nonradioactive paramagnetic metal ion, and affinity label for detection and isolation of pro/latent-myostatin. The detectable substance may be coupled or conjugated either directly to the polypeptides of the disclosure or indirectly, through an intermediate (such as, for example, a linker) using suitable techniques. Non-limiting examples of suitable enzymes include horseradish peroxidase, alkaline phosphatase, β-galactosidase, glucose oxidase, or acetylcholinesterase; non-limiting examples of suitable prosthetic group complexes include streptavidin/biotin and avidin/biotin; non-limiting examples of suitable fluorescent materials include biotin, umbelliferone, fluorescein, fluorescein isothiocyanate, rhodamine, dichlorotriazinylamine fluorescein, dansyl chloride, or phycoerythrin; an example of a luminescent material includes luminol; non-limiting examples of bioluminescent materials include luciferase, luciferin, and aequorin; and examples of suitable radioactive material include a radioactive metal ion, e.g., alpha-emitters or other radioisotopes such as, for example, iodine (131I, 125I, 123I, 121I), carbon (14C), sulfur (35S), tritium (3H), indium (115min, 113min, 112In, 111In), and technetium (99Tc, 99mTc), thallium (201Ti), gallium (68Ga, 67Ga), palladium (103Pd), molybdenum (99Mo), xenon (133Xe), fluorine (18F), 153Sm, Lu, 159Gd, 149Pm, 140La, 175Yb, 166Ho, 90Y, 47Sc, 86R, 188Re, 142Pr, 105Rh, 97Ru, 68Ge, 57Co, 65Zn, 85Sr, 32P, 153Gd, 169Yb, 51Cr, 54Mn, 75Se, and tin (113Sn, 117Sn). The detectable substance may be coupled or conjugated either directly to the anti- pro/latent-myostatin antibodies, or antigen-binding portions thereof, of the disclosure or indirectly, through an intermediate (such as, for example, a linker) using suitable techniques. Anti- pro/latent-myostatin antibodies, or antigen-binding portions thereof, conjugated to a detectable substance may be used for diagnostic assays as described herein.

### Biological Effects of the Combination of Myostatin Pathway Inhibitors in Conjunction with GLP-1 pathway activators

Methods or treatments comprising administering a myostatin pathway inhibitor (such as a myostatin antibody) in conjunction with a GLP-1 pathway activator are encompassed by the present disclosure and can be used as a medicament to effectuate beneficial effects (e.g., therapeutic effects) in a subject when each is administered to the subject in an effective amount. Exemplary such biologically beneficial effects are provided herein. Beneficial biological effects in a subject can be achieved by administration of myostatin pathway inhibitors, e.g., antibodies, or antigen binding fragments thereof, as described herein, that specifically bind pro/latent myostatin. In some embodiments, the myostatin pathway inhibitor and the GLP-1 pathway activator are administered concurrently, simultaneously, or sequentially. In some embodiments, the myostatin pathway inhibitor and the GLP-1 pathway activator are administered in an amount effective to cause two or more of the biological effects described below. In some embodiments, the myostatin pathway inhibitor and the GLP-1 pathway activator are administered in an amount effective to cause three or more of the biological effects described below. In some embodiments, the myostatin pathway inhibitor and the GLP-1 pathway activator are administered in an amount effective to cause four or more of the biological effects described below. In some embodiments, the myostatin pathway inhibitor and the GLP-1 pathway activator are administered in an amount effective to cause five or more of the biological effects described below. In some embodiments, the myostatin pathway inhibitor and the GLP-1 pathway activator are administered in an amount effective to cause six or more of the biological effects described below. In some embodiments, the myostatin pathway inhibitor and the GLP-1 pathway activator are administered in an amount effective to cause seven or more of the biological effects described below. In some embodiments, the myostatin pathway inhibitor and the GLP-1 pathway activator are administered in an amount effective to cause eight, nine, ten, eleven, twelve, thirteen, fourteen, fifteen, or sixteen of the biological effects described below.

### Effect on Mass and/or Function of Muscle Tissue in the Human Subject

In some embodiments, administration of a myostatin pathway inhibitor in conjunction with a GLP-1 pathway activator increases mass and/or function of a muscle tissue in the human subject. In some embodiments, the muscle tissue is selected from the group consisting of a smooth muscle tissue, a skeletal muscle tissue and a cardiac muscle tissue. Smooth muscle tissue is made up from long tapering cells, generally involuntary and differs from striated muscle in the much higher actin/myosin ratio, the absence of conspicuous sarcomeres and the ability to contract to a much smaller fraction of its resting length. Smooth muscle cells are found particularly in blood vessel walls, surrounding the intestine and in the uterus. Cardiac muscle tissue is a striated but involuntary tissue responsible for the pumping activity of the vertebrate heart. The individual cardiac muscle cells are not fused together into multinucleate structures as they are in striated muscle tissue. Skeletal muscle tissue is under voluntary control. The muscle fibers are syncytial and contain myofibrils, tandem arrays of sarcomeres. There are two general types of skeletal muscle fibers: slow-twitch (type I) and fast-twitch (type II) according to the expression of their particular myosin heavy chain (MHC) isoform. Slow-twitch muscles are better equipped to work aerobically and help enable long-endurance feats such as distance running, while fast-twitch muscles fatigue faster but are better equipped to work anaerobically and are used in powerful bursts of movements like sprinting. The differentiation between slow and fast twitch muscle fibers is based on histochemical staining for myosin adenosine-triphosphatase (ATPase) and the type of myosin heavy chain. The slow twitch muscle fiber (type I fiber) is MHC isoform I and the three fast twitch isoforms (type II fibers) are MHC isoform Ila, MHC isoform Ild, and MHC isoform IIb (S. Schiaffino, J. Muscle Res. Cell. Motil., 10 (1989), pp. 197-205).

In some embodiments, the mass and/or function of a fast twitch muscle tissue in the human subject is increased. In other embodiments, the mass and/or function of a slow twitch muscle tissue in the human subject is increased. Maintaining muscle mass in the context of treating obesity is known to support weight loss, e.g., as observed by resistance training. Myostatin inhibition according to the invention preferentially preserves fast-twitch fibers.

Biological effects of an effective amount of the pharmaceutical compositions and therapeutic methods provided herein may be associated with a phenotypic change of muscle fiber types, which is a process referred to as fiber type switch. In some embodiments, fiber type switch is triggered by an event, such as an injury and starvation.

In one aspect, the disclosure provides a method for promoting fiber type switch in a subject. The method comprises administering to the subject a composition comprising a myostatin pathway inhibitor, e.g., an antibody, or antigen binding fragment thereof, that specifically binds pro/latent-myostatin and blocks release of mature myostatin in an amount effective to promote fiber type switch, thereby promoting fiber type switch in the subject.

In another aspect, the disclosure provides a method for preferentially increasing type II or fast twitch fibers over type I or slow twitch fibers in a subject. The method comprises administering to the subject a composition comprising a myostatin pathway inhibitor, e.g., an antibody, or antigen binding fragment thereof, that specifically binds pro/latent-myostatin and blocks release of mature myostatin in an amount effective to preferentially increase type II or fast twitch fibers over type I or slow twitch fibers fiber type switch, thereby preferentially increasing type II or fast twitch fibers over type I or slow twitch fibers in the subject.

In some embodiments, administration of an effective amount of each of a myostatin pathway inhibitor and a GLP-1 pathway activator as described herein to a subject can cause an increase in muscle mass and/or muscle function. Preferably, such an increase in muscle mass is clinically meaningful to benefit or otherwise improve the health status of the subject. For example, clinically meaningful changes in muscle mass may improve the patient's mobility, self-care, metabolism, etc. In some embodiments, the increase in muscle mass is an increase in lean muscle or lean muscles. In some embodiments, such increase in muscle mass is a systemic effect such that muscles in the whole body or substantially whole body show the measurable effect. In some embodiments, lean muscle is muscle that is densely packed with contractile tissue and has low fat and connective tissue content. In other embodiments, effects are localized to certain group/type of muscles. In some embodiments, the mass of the muscle tissue, e.g., lean muscle tissue, is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the mass of the muscle tissue, e.g., lean muscle tissue, is increased by at least 1-5%, 5-10%, 10-20%, 1-30%, 1-40%, 1-50%, 10-50%, 20-30%, 20-60%, 30-80%, 40-90%, or 50-100%. Such increase in muscle mass may be deduced or measured by any suitable known methods, including measurement of cross-sectional area via MRI (e.g., forearm cross section), circumference, diaphragm width (e.g., via ultrasound), qNMR, DXA, etc.

In some embodiments, administration of an effective amount of a combination of a myostatin pathway inhibitor and a GLP-1 pathway activator to a subject can cause an enhancement in muscle function. Muscle function may be assessed by a variety of measures, including, without limitation: force generation, grip strength (e.g., maximum grip strength), endurance, muscle oxidative capacity, dynamic grip endurance, etc. In some embodiments, serum creatinine levels are used as a validated biomarker indicative of muscle mass, albeit with limited sensitivity.

In some embodiments, the function of the muscle tissue is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the function of the muscle tissue is increased by at least about 1-5%, 5-10%, 10-20%, 1-30%, 1-40%, 1-50%, 10-50%, 20-30%, 20-60%, 30-80%, 40-90%, or 50-100%. In some embodiments, increased muscle function comprises improved rating, for example, from 1 to 2, 2 to 3, 3 to 4, 4 to 5, 5 to 6, 6 to 7, 7 to 8, 8 to 9, or 9 to 10.

In some embodiments, use of a myostatin pathway inhibitor alone or in conjunction with a GLP-1 pathway activator in the methods of the present invention may increase the mass and/or function of the muscle tissue in the subject suffering from a lesion, e.g., due to a spinal cord injury. In some embodiments, the subject is in an acute spinal cord injury phase immediately after injury, where diagnosis between complete and incomplete injury is generally difficult. In other embodiments, the subject is in a sub-acute spinal cord injury phase, where there is a distinction between complete and incomplete spinal cord injury, and recovery is possible through ongoing rehab. In yet another embodiment, the subject is in a chronic spinal cord injury phase. The chronic SCI phase occurs around 4 or 6 months from the date of injury, where patients have demonstrated substantial decrease in rate of recovery or when rehab efforts have reached a plateau despite the ongoing standard of care efforts.

In some embodiments, the mass and/or function of the muscle tissue below a lesion is increased in a subject suffering from a lesion, e.g., a spinal cord injury. In other embodiments, the mass and/or function of the muscle tissue above a lesion is increased in a subject suffering from a lesion, e.g., a spinal cord injury. In some embodiment, the muscle is selected from the group consisting of a soleus muscle, a gastrocnemius muscle, a bicep muscle and a tricep muscle. In some embodiments, the mass of the muscle tissue is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the mass of the muscle tissue is increased by at least about 1-5%, 5-10%, 10-20%, 1-30%, 1-40%, 1-50%, 10-50%, 20-30%, 20-60%, 30-80%, 40-90%, or 50-100%. In some embodiments, the function of the muscle tissue is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the function of the muscle tissue is increased by at least 1-5%, 5-10%, 10-20%, 1-30%, 1-40%, 1-50%, 10-50%, 20-30%, 20-60%, 30-80%, 40-90%, or 50-100%.

In some embodiments, administration of a myostatin pathway inhibitor, e.g., antibody, or antigen binding fragment thereof that specifically binds pro/latent myostatin, alone or in conjunction with a GLP-1 pathway activator increases locomotor function in the human subject, e.g., in a subject suffering from a lesion. In some embodiments, the locomotor function of the human subject is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the locomotor function of the human subject is increased by at least 1-5%, 5-10%, 10-20%, 1-30%, 1-40%, 1-50%, 10-50%, 20-30%, 20-60%, 30-80%, 40-90%, or 50-100%.

In some embodiments, administration of the myostatin pathway inhibitor, e.g., antibody, or antigen binding fragment thereof that specifically binds pro/latent myostatin, alone or in conjunction with a GLP-1 pathway activator increases the motor coordination and balance in the human subject, e.g., in a subject suffering from a lesion. In some embodiments, the motor coordination and balance of the human subject is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the motor coordination and balance of the human subject is increased by at least 1-5%, 5-10%, 10-20%, 1-30%, 1-40%, 1-50%, 10-50%, 20-30%, 20-60%, 30-80%, 40-90%, or 50-100%.

In another embodiment, administration of a myostatin pathway inhibitor alone or in conjunction with a GLP-1 pathway activator increases the muscle strength in the human subject, e.g., in a subject suffering from a lesion. In some embodiments, the muscle strength of the human subject is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the muscle strength of the human subject is increased by at least 1-5%, 5-10%, 10-20%, 1-30%, 1-40%, 1-50%, 10-50%, 20-30%, 20-60%, 30-80%, 40-90%, or 50-100%.

In some embodiments, administration of a myostatin pathway inhibitor alone or in conjunction with a GLP-1 pathway activator can cause clinically meaningful changes in muscle function which corresponds to enhanced functionality of the patient. In some embodiments, enhanced functionality includes improvement in the patient's mobility, self-care, metabolism, etc. In some embodiments, administration of an effective amount of the myostatin pathway inhibitor, e.g., an antibody, or antigen binding fragment thereof, that specifically binds pro/latent myostatin facilitates or accelerates recovery from a condition, such as injuries, surgeries, and other medical procedures. Suitable such conditions may involve a condition that is associated with a nerve damage (whether resulting from an injury or a surgical or other clinical procedure).

For example, suitable subjects include generally healthy individuals, such as a patient who: i) has sustained an acute injury involving a nerve damage that affects muscle function; ii) is scheduled to undergo a surgical procedure (therapeutic or corrective) that may cause an unintended nerve injury (e.g., motor neuron injury); iii) has undergone a surgical procedure that has caused an unintended muscle dysfunction; iv) receives a treatment that involves immobilization of a particular muscle or muscle groups (e.g., cast, etc.); v) is on ventilator (e.g., as a result of acute injury). The administration of a myostatin pathway inhibitor described herein may accelerate recovery in such patients. In some embodiments, such administration may be prophylactic. For example, prior to undergoing or immediately following a surgical procedure that may cause a nerve damage and associated muscle dysfunction, the antibody may be administered to prevent muscle dysfunction. Prevention includes alleviating or lessening the severity of such dysfunction. In these embodiments, administration may be a local administration at or near the site of the affected area, e.g., injury, surgery, etc.

### Effect on Muscle Catabolism of Protein and/or Muscle Release of Amino Acids in the Human Subject

In some embodiments, administration a myostatin pathway inhibitor (e.g., a myostatin inhibitor, e.g., a myostatin-selective inhibitor) alone or in conjunction with a GLP-1 pathway activator decreases muscle catabolism of protein and/or muscle release of amino acids in the human subject. In some embodiments, muscle catabolism of protein and/or muscle release of amino acids is decreased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, muscle catabolism of protein and/or muscle release of amino acids is decreased by at least 1-5%, 5-10%, 10-20%, 1-30%, 1-40%, 1-50%, 10-50%, 20-30%, 20-60%, 30-80%, 40-90%, or 50-100%.

### Effect on Preventing Muscle Loss or Atrophy in the Human Subject

In some embodiments, administration of an effective amount of the combination of a myostatin pathway inhibitor (e.g., a myostatin inhibitor, e.g., a myostatin-selective inhibitor) alone or in conjunction with a GLP-1 pathway activator prevents muscle loss or atrophy in the human subject at risk of developing muscle loss and/or atrophy. In some embodiments, muscle loss or atrophy is decreased or prevented by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, muscle loss or atrophy is decreased or prevented by at least 1-5%, 5-10%, 10-20%, 1-30%, 1-40%, 1-50%, 10-50%, 20-30%, 20-60%, 30-80%, 40-90%, or 50-100%. In some embodiments, muscle mass is retained (i.e., no muscle mass gain or loss) as a result of treatment, e.g., while losing fat mass. In some embodiments, a subject receiving the combination therapy may exhibit a gain in muscle mass. In some embodiments, treatment with the combination therapy maintains the gain in muscle mass relatively to muscle mass prior to treatment.

In some embodiments, a suitable subject is a subject who has not developed atrophy but is considered at risk of developing atrophy. In some embodiments, a subject is on a weight loss program, such as a program comprising diet restriction and/or an exercise regimen. In some embodiments, the subject is on a weight loss program comprising a diet restriction regimen of at least 20%, e.g., at least 30%, 35%, 40%, 45%, or 50%, calorie reduction. In some embodiments, the subject has type 2 diabetes. In some embodiments, the subject has cardiovascular disease. In some embodiments, a subject has a disease or condition associated with a neurological defect that impairs motor neuron function. In some embodiments, such conditions are caused by muscular dystrophy or atrophy. In some embodiments, the neurological defect is caused by a nerve injury. In some embodiments, the nerve injury involves partial denervation of motor neurons, which causes partial impairment of function in the affected muscle. In some embodiments, such condition is caused by SCI. In some embodiments, the subject with SCI is in an acute or sub-acute phase of SCI (e.g., not yet reached a chronic phase).

In some embodiments, when a composition comprising an effective amount of an inhibitor of myostatin signaling (e.g., a myostatin inhibitor, e.g., a myostatin-selective inhibitor) is administered to a population of patients who are at risk of developing muscle atrophy associated with partial denervation of motor neurons, the composition i) prevents manifestation or aggravation of the muscle atrophy in a statistically significant fraction of the patient population; or, ii) lessens the severity of the muscle atrophy in the statistically significant fraction of the patient population. In some embodiments, the myostatin signaling inhibitor is administered in conjunction with a GLP-1 pathway activator as described herein.

Prevention of muscle loss or atrophy by the use of a myostatin signaling inhibitor (i.e., a myostatin-selective inhibitor) alone or in conjunction with a GLP-1 pathway activator as described herein can be readily monitored or assessed by any suitable methods to evaluate muscle mass or motor function involving affected muscles.

In some embodiments, administration of an effective amount of a myostatin pathway inhibitor (i.e., a myostatin-selective inhibitor) also prevents or lessens neuropathy, e.g., diabetic neuropathy, or an early-onset axonal polyneuropathy in affected limbs.

### Effect on Intramuscular Fat Infiltration in the Human Subject

In some embodiments, administration of a myostatin pathway inhibitor (i.e., a myostatin-selective inhibitor) alone or in conjunction with a GLP-1 pathway activator decreases intramuscular fat infiltration in the human subject. In some embodiments, intramuscular fat infiltration is decreased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, intramuscular fat infiltration is decreased by at least 1-5%, 5-10%, 10-20%, 1-30%, 1-40%, 1-50%, 10-50%, 20-30%, 20-60%, 30-80%, 40-90%, or 50-100%.

### Effect on the Level of Adipose Tissue in the Human Subject

In some embodiments, administration of a myostatin pathway inhibitor (i.e., a myostatin-selective inhibitor) alone or in conjunction with a GLP-1 pathway activator affects the level of adipose tissue in the human subject. As used herein, the term "adipose tissue" refers to fat including the connective tissue that stores fat. Adipose tissue is derived from preadipocytes. Its main role is to store energy in the form of lipids, although it also cushions and insulates the body. The two types of adipose tissue are white adipose tissue (WAT), which stores energy, and brown adipose tissue (BAT), which generates body heat.

Brown adipose tissue (BAT) is known to function in the dissipation of chemical energy in response to cold or excess feeding, and also has the capacity to modulate energy balance. Activation of brown adipose tissue has been shown to improve glucose homeostasis and insulin sensitivity in humans suggesting that anyone with impaired insulin function might benefit from BAT activation (Stanford et al., J Clin Invest. 2013, 123(1): 215-223).

Beige adipose tissues are generated as a result of browning of WAT, also known as beiging. This occurs when adipocytes within WAT depots develop features of BAT. Beige adipocytes take on a multilocular appearance (containing several lipid droplets) and increase expression of uncoupling protein 1 (UCP1). In doing so, these normally energy-storing white adipocytes become energy-releasing adipocytes (Harms et al. Nature Medicine. 2013, 19 (10): 1252-63).

Visceral fat or abdominal fat (also known as organ fat or intra-abdominal fat) is located inside the abdominal cavity, packed between the organs (stomach, liver, intestines, kidneys, etc.). Visceral fat is different from subcutaneous fat underneath the skin, and intramuscular fat interspersed in skeletal muscles. Fat in the lower body, as in thighs and buttocks, is subcutaneous and is not consistently spaced tissue, whereas fat in the abdomen is mostly visceral and semi-fluid. An excess of visceral fat is known as central obesity, "central adiposity," or "belly fat", in which the abdomen protrudes excessively and new developments such as the Body Volume Index (BVI) are specifically designed to measure abdominal volume and abdominal fat. Excess visceral fat is also linked to type 2 diabetes, insulin resistance, inflammatory diseases and other obesity-related diseases (Mokdad et al., JAMA: The Journal of the American Medical Association. 2001, 289 (1): 76-9).

Mass of adipose tissue can be determined by any method known to a person of ordinary skill in the art. For example, adipose tissue may be measured by qNMR, dual-energy X-Ray absorptiometry (DXA),and other methods known in the art.

In some embodiments, administration of the myostatin pathway inhibitor (i.e., a myostatin-selective inhibitor) increases the level of brown adipose tissue and/or the level of beige adipose tissue in the human subject. In some embodiments, administration of the myostatin pathway inhibitor, e.g., anti-pro/latent myostatin antibody, or antigen-binding portion thereof, decreases the level of white adipose tissue and visceral adipose tissue in the human subject. In some embodiments, the administration comprises administering a GLP-1 pathway activator in conjunction with the myostatin inhibitor.

In some embodiments, the level of brown or beige adipose tissue is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the level of brown or beige adipose tissue is increased by at least 1-5%, 5-10%, 10-20%, 1-30%, 1-40%, 1-50%, 10-50%, 20-30%, 20-60%, 30-80%, 40-90%, or 50-100%.

In some embodiments, the level of white or visceral adipose tissue is decreased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the level of white or visceral adipose tissue is decreased by at least 1-5%, 5-10%, 10-20%, 1-30%, 1-40%, 1-50%, 10-50%, 20-30%, 20-60%, 30-80%, 40-90%, or 50-100%.

In some embodiments, myostatin inhibition has different effects during the weight loss phase as compared to the weight maintenance phase. In some embodiments, inhibiting the myostatin pathway promotes more fat mass loss in the weight maintenance phase as compared to fat mass loss in the active weight loss phase. In some embodiments, inhibiting the myostatin pathway promotes more fat mass loss under moderate calorie restriction (e.g., 30% or less calorie restriction, e.g., 20% calorie restriction) as compared to extreme calorie restriction (e.g., 30% or more calorie restriction).

In some embodiments, the rate of fat increase is slowed or stopped relative to the rate prior to treatment. In some embodiments, the treatment maintains the reduced rate of fat increase relative to the higher rate of fat increase prior to treatment.

### Effect on the Ratio of Adipose-To-Muscle Tissue in the Human Subject

In some embodiments, administration of a myostatin pathway inhibitor (i.e., a myostatin-selective inhibitor) alone or in conjunction with a GLP-1 pathway activator decreases the ratio of adipose-to-muscle tissue in the human subject. In some embodiments, the ratio of adipose-to-muscle tissue is decreased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the ratio of adipose-to-muscle tissue is decreased by at least 1-5%, 5-10%, 10-20%, 1-30%, 1-40%, 1-50%, 10-50%, 20-30%, 20-60%, 30-80%, 40-90%, or 50-100%. In some embodiments, administration of a myostatin pathway inhibitor (i.e., a myostatin-selective inhibitor) alone or in conjunction with a GLP-1 pathway activator maintains the reduction in the ratio between adipose-to-muscle tissue in a human subject who has previously achieved a reduced ratio of adipose-to-muscle tissue.

In some embodiments, administration of a myostatin pathway inhibitor (i.e., a myostatin-selective inhibitor) alone or in conjunction with a GLP-1 pathway activator increases the ratio of muscle tissue to adipose in the human subject. In some embodiments, the ratio of muscle tissue-to-adipose is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the ratio of muscle tissue-to-adipose is increased by at least 1-5%, 5-10%, 10-20%, 1-30%, 1-40%, 1-50%, 10-50%, 20-30%, 20-60%, 30-80%, 40-90%, or 50-100%. In some embodiments, administration of a myostatin pathway inhibitor (i.e., a myostatin-selective inhibitor) alone or in conjunction with a GLP-1 pathway activator maintains the increased ratio of muscle tissue to adipose tissue in a human subject who has previously achieved such an increased ratio.

### Hormonal Control

Leptin is a hormone produced and secreted by adipose tissue that plays a role in regulating food intake and stimulating energy expenditure. Defects in leptin production have been reported to cause severe hereditary obesity in rodents and humans. In addition to its effects on body weight, leptin has a variety of other functions, including the regulation of hematopoiesis, angiogenesis, wound healing, and the immune and inflammatory response. Leptin acts through the leptin receptor, a single-transmembrane-domain receptor of the cytokine receptor family, which is found in many tissues in several alternatively spliced forms. The LEP gene is the human homolog of the gene (ob) mutant in the mouse 'obese' phenotype. The leptin level in a subject refers to the amount of circulating leptin in the body of the subject, e.g., a mammalian subject, and a reduction in leptin levels refers to a reduction in the amount of circulating leptin as compared to a baseline measurement, e.g., as a result of treatment.

In some embodiments, the therapeutic efficacy of treating a subject (e.g., a mammalian subject) with a myostatin pathway inhibitor (e.g., alone or in conjunction with a GLP-1 pathway activator) may be determined by measuring a reduction in leptin levels in the blood of the subject after administering the myostatin inhibitor as compared to before the administration. In some embodiments, the therapeutic efficacy may be determined by (i) determining a blood level of leptin in the subject prior to administering the myostatin inhibitor; (ii) administering the myostatin inhibitor; and (iii) determining a blood level of leptin in the subject after administering the myostatin inhibitor; wherein a reduction in blood leptin level indicates therapeutic efficacy.

Adiponectin is an adipokine involved in the control of fat metabolism and insulin sensitivity with direct anti-diabetic, anti-atherogenic and anti-inflammatory activities. Adiponectin stimulates AMPK phosphorylation and activation in the liver and the skeletal muscle, enhancing glucose utilization and fatty-acid combustion and inhibits endothelial NF-kappa-B signaling through a cAMP-dependent pathway. Adiponectin may be involved in brown fat cell differentiation. As used herein, the term "adiponectin level" refers to the amount of circulating adiponectin, e.g., total adiponectin or glycosylated adiponectin. The plasma adiponectin level of a subject, e.g., a mammalian subject, may be measured by, e.g., the total adiponectin assay (ELISA kit EZHADP-61K, Millipore, St. Charles, Missouri, USA) which captures all forms of circulating adiponectin, with a sensitivity of 0.78 ng/mL and within-batch and between-batch coefficients of variation of 1.8% and 6.2%, respectively. An increase or decrease in adiponectin level refers to a higher or lower amount of circulating adiponectin detected in a subject (e.g., following a treatment for a metabolic disease or disorder) compared to a baseline measurement.

Ghrelin is an appetite-regulating hormone that has an appetite-stimulating effect, induces adiposity, and stimulates gastric secretion. Plasma ghrelin concentration is increased in fasting conditions and reduced after habitual feeding. The normal ghrelin concentration of plasma samples in humans is 10-20 fmol/ml for n-octanoyl ghrelin and 100-150 fmol/ml for total ghrelin, including both acyl-modified and des-acyl ghrelins. Ghrelin levels may be measured using, e.g., the Ghrelin Human ELISA kid, CAT# BMS2192 (Invitrogen).

### Effect on the Metabolic Rate of the Human Subject

In some embodiments, administration of a myostatin pathway inhibitor (i.e., a myostatin-selective inhibitor) alone or in conjunction with a GLP-1 pathway activator increases the metabolic rate of the human subject. In some embodiments, the administration can increase the basal metabolic rate in the subject. Metabolic rates can be calculated by any methods known in the art, for example, by examining the oxygen input and carbon dioxide output, or by indirect calorimetry. In some embodiments, the metabolic rate is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, the metabolic rate is increased by at least 1-5%, 5-10%, 10-20%, 1-30%, 1-40%, 1-50%, 10-50%, 20-30%, 20-60%, 30-80%, 40-90%, or 50-100%.

### Effect on Glucose Uptake in the Human Subject

In some embodiments, administration of a myostatin pathway inhibitor (i.e., a myostatin-selective inhibitor) alone or in conjunction with a GLP-1 pathway activator affects glucose uptake by tissues in the human subject. In some embodiments, glucose uptake by muscle tissue is increased. In some embodiments, glucose uptake by the muscle tissue is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In some embodiments, glucose uptake by the muscle tissue is increased by at least 1-5%, 5-10%, 10-20%, 1-30%, 1-40%, 1-50%, 10-50%, 20-30%, 20-60%, 30-80%, 40-90%, or 50-100%.

In other embodiments, glucose uptake by white adipose tissue, liver tissue and/or blood vessel tissue are reduced. In some embodiments, glucose uptake by white adipose tissue, liver tissue and/or blood vessel tissue are reduced by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, glucose uptake by white adipose tissue, liver tissue and/or blood vessel tissue are reduced by at least 1-5%, 5-10%, 10-20%, 1-30%, 1-40%, 1-50%, 10-50%, 20-30%, 20-60%, 30-80%, 40-90%, or 50-100%.

In some embodiments, treatment of a subject with a myostatin pathway inhibitor alone or in conjunction with a GLP-1 pathway activator reduces non-fasted glucose levels in the serum of the subject relative to baseline prior to treatment.

The term "non-fasted glucose levels" refers to glucose content in a subject's blood as measured without having to fast or refraining from eating or drinking for a prolonged period of time, e.g., 8 hours. Blood glucose can be measured using a hemoglobin A1C test, also known as an A1C, HbA1C, glycated hemoglobin, or glycosylated hemoglobin test. Conventional home glucose monitoring can also be used to measure blood sugar. The term "postprandial insulin level" refers to the insulin concentration in a subject's blood shortly after eating (e.g., two hours after eating). The term "postprandial glucose level" refers to glucose concentrations in a subject's blood shortly after eating (e.g., two hours after eating). The postprandial insulin and glucose levels may be affected by carbohydrate absorption, insulin and glucagon secretion, and their coordinated effects on glucose metabolism in the liver and peripheral tissues. The magnitude and time of the peak plasma insulin or glucose concentration depend on a variety of factors, including the timing, quantity, and composition of the meal. In non-diabetic human subjects, plasma glucose concentrations peak about 60 min after the start of a meal and return to preprandial levels within 2-3 hour. Postprandial insulin levels may be measured using a postprandial insulin test, such as a two-hour postprandial insulin test. Postprandial glucose levels may be measured using a postprandial blood glucose test, such as a two-hour postprandial blood glucose test.

### Effect on Insulin Sensitivity of the Human Subject

In some embodiments, administration of the myostatin pathway inhibitor (i.e., a myostatin-selective inhibitor) alone or in conjunction with a GLP-1 pathway activator increases insulin sensitivity of the human subject. Methods for measuring insulin sensitivity are known in the art, for example, glucose tolerance test, and fasting insulin or glucose test. During a glucose tolerance test, a fasting patient takes a 75-gram oral dose of glucose, and then blood glucose levels are measured over the following two hours. A glycemic level less than 7.8 mmol/L (140 mg/dl) is considered normal, a glycemic level of between 7.8 and 11.0 mmol/L (140 to 197 mg/dl) is considered as impaired glucose tolerance (IGT), and a glycemic level of greater than or equal to 11.1 mmol/L (200 mg/dl) is considered as diabetes mellitus. For a fasting insulin test, a fasting serum insulin level greater than 25 mIU/L or 174 pmol/L is considered insulin resistant. To measure postprandial insulin levels, a subject may follow a diet for two weeks comprising at least 150g carbohydrate per day. Following an overnight fast of more than 10 hours, a patient has an oral glucose tolerance test with samples obtained at baselines and again at 30, 60, 120, and 180 minutes. A sum of the 2nd and 3rd hour insulin less than 60 µU/mL is considered to be within the normal range. Borderline hyperinsulinemia or borderline insulin resistance is indicated when the sum of the 2nd and 3rd hour insulins are 60 or higher but less than 100. Hyperinsulinemia may be indicated when the sum of the 2nd and 3rd hour insulins are 100 or more, when there is a delayed insulin peak in hours 2 or 3, or when fasting insulin is above 50. (see, e.g., DiNicolantonio, JJ, et. al. Postprandial insulin assay as the earliest biomarker for diagnosing pre-diabetes, type 2 diabetes and increased cardiovascular risk. Published online November 27. Available at: http://openheart.bmj.com/content/openhrt/4/2/e000656.full.pdf Accessed November 28, 2017.)

### Effect on Insulin Dependent Glycemic Control in the Human Subject

In some embodiments, administration of a myostatin pathway inhibitor (i.e., a myostatin-selective inhibitor) alone or in conjunction with a GLP-1 pathway activator increases insulin dependent glycemic control in the human subject. In some embodiments, insulin dependent glycemic control is increased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, insulin dependent glycemic control is increased by at least 1-5%, 5-10%, 10-20%, 1-30%, 1-40%, 1-50%, 10-50%, 20-30%, 20-60%, 30-80%, 40-90%, or 50-100%.

### Effect on Preventing Development of a Metabolic Disorder in the Subject

In some embodiments, administration of an effective amount of a myostatin pathway inhibitor (i.e., a myostatin-selective inhibitor) alone or in conjunction with a GLP-1 pathway activator as described herein may prevent development of a metabolic disorder in the subject, e.g., a human subject. In some embodiments, development of a metabolic disorder is decreased by at least 1%, 2%, 3%, 4%, 5%, 6%, 7%, 8%, 9%, 10%, 15%, 20%, 25%, 30%, 35%, 40%, 45%, 50%, 60%, 70%, 80%, 90% or 100%. In other embodiments, development of a metabolic disorder is decreased by at least 1-5%, 5-10%, 10-20%, 1-30%, 1-40%, 1-50%, 10-50%, 20-30%, 20-60%, 30-80%, 40-90%, or 50-100%.

In some embodiments, administration of an effective amount of a myostatin pathway inhibitor (i.e., a myostatin-selective inhibitor) reduces a risk of a cardiovascular disease, e.g., by reducing levels of cholesterol and or the LDL/HDL ratio. Cholesterol encompasses both low-density lipoprotein (LDL) cholesterol and high-density lipoprotein (HDL) cholesterol. Total cholesterol level refers to a measure of the total amount of cholesterol (e.g., LDL and HDL) in a blood sample collected from a subject. LDL cholesterol contains low-density lipoprotein that makes up most of the cholesterol found in the body. A high level of LDL may cause lipid deposits (e.g., plaques) in arteries and may be associated with or linked to increased risk for cardiovascular conditions, such as heart disease and stroke. By contrast, HDL cholesterol contains high-density lipoprotein, which absorbs cholesterol and carries it back to the liver. Generally, a high level of HDL cholesterol is associated with lower risk of heart disease and stroke. Cholesterol levels can be measured using a blood test, including blood tests commonly known as complete cholesterol tests, lipid panels, or lipid profiles.

In some embodiments, a suitable subject is a subject who has not fully developed a metabolic disease but is considered at risk of developing such a condition. In some embodiments, a subject has a disease or condition associated with muscle dysfunction. In some embodiments, the muscle dysfunction is associated with partial denervation of motor neurons, which causes partial impairment of function in the affected muscle. In some embodiments, such conditions are caused by muscular dystrophy or atrophy. In some embodiments, such condition is caused by SCI. In some embodiments, the subject with SCI is in an acute or sub-acute phase of SCI (e.g., not yet reached a chronic phase).

In some embodiments, when a composition comprising an effective amount of an inhibitor of myostatin signaling described herein (i.e., a myostatin-selective inhibitor) is administered to a population of patients who are at risk of developing a metabolic disorder associated with muscle dysfunction, the composition i) prevents manifestation or aggravation of the metabolic disorder in a statistically significant fraction of the patient population; or, ii) lessens the severity of the metabolic disorder in the statistically significant fraction of the patient population. In some embodiments, the inhibitor of myostatin signaling is administered in conjunction with a GLP-1 pathway activator.

In some embodiments, effects on metabolism may be monitored or measured by insulin resistance, lipid panel/markers (e.g., leptin, adiponectin, ghrelin), inflammatory markers and oxidative stress markers, including, but are not limited to: IL-6, TNF, CRP, plasma total antioxidant status, lipid oxidation and erythrocyte glutathione peroxidase activity.

### Pharmaceutical Compositions

Myostatin pathway inhibitors and GLP-1 pathway activators described herein may be formulated into one or more pharmaceutical compositions suitable for administration in human or non-human subjects. In some embodiments, the myostatin pathway inhibitor and the GLP-1 pathway activator are co-formulated in one composition. For example, the myostatin pathway inhibitor and the GLP-1 pathway activator may be co-formulated for intravenous (i.v.) or subcutaneous (s.c.) administration. In other embodiments, the myostatin pathway inhibitor and the GLP-1 pathway activator are formulated for separate administration to human or non-human subjects (e.g., mammals). For example, a myostatin pathway inhibitor may be formulated for i.v. administration and a GLP-1 pathway activator may be formulated for s.c. or oral administration. In some embodiments, the myostatin pathway inhibitor is formulated for s.c. administration. In some embodiments, the dosing schedule for the myostatin pathway inhibitor and the GLP-1 pathway activator may require administration on different days or at different times. In some embodiments, the myostatin pathway inhibitor and GLP-1 pathway activator may be administered concurrently, separately, or simultaneously.

Such pharmaceutical compositions may be intended for therapeutic use or prophylactic use. One or more of the myostatin pathway inhibitors and/or GLP-1 pathway activators can be mixed with a pharmaceutically acceptable carrier (excipient), including buffer, to form a pharmaceutical composition for administering to a patient who may benefit from reduced myostatin signaling and GLP-1 pathway activation in vivo. "Pharmaceutically acceptable" means that the carrier must be compatible with the active ingredient of the composition (and preferably, capable of stabilizing the active ingredient) and not deleterious to the subject to be treated. Examples of pharmaceutically acceptable excipients (carriers), including buffers, would be apparent to the skilled artisan and have been described previously. See, e.g., Remington: The Science and Practice of Pharmacy 20th Ed. (2000) Lippincott Williams and Wilkins, Ed. K. E. Hoover. Acceptable carriers, excipients, or stabilizers are nontoxic to recipients at the dosages and concentrations used, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEENTM, PLURONICSTM or polyethylene glycol (PEG). Pharmaceutically acceptable excipients are further described herein.

In some examples, the pharmaceutical composition described herein comprises emulsion-based or lipid-based formulations, such as liposomes containing a myostatin inhibitor and/or a GLP-1 pathway activator, which can be prepared by any suitable method, such as described in Epstein, et al., Proc. Natl. Acad. Sci. USA 82:3688 (1985); Hwang, et al., Proc. Natl. Acad. Sci. USA 77:4030 (1980); and U.S. Pat. Nos. 4,485,045 and 4,544,545. Liposomes with enhanced circulation time are disclosed in U.S. Pat. No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

The myostatin inhibitor and/or the GLP-1 pathway activator may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Exemplary techniques have been described previously, see, e.g., Remington, The Science and Practice of Pharmacy 20th Ed. Mack Publishing (2000).

In other examples, the pharmaceutical composition described herein can be formulated in sustained-release format. Suitable examples of sustained-release preparations include semipermeable matrices of solid hydrophobic polymers containing the myostatin inhibitor and/or the GLP-1 pathway activator, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and 7 ethyl-L-glutamate, non-degradable ethylene-vinyl acetate, degradable lactic acid-glycolic acid copolymers such as the LUPRON DEPOTTM (injectable microspheres composed of lactic acid-glycolic acid copolymer and leuprolide acetate), sucrose acetate isobutyrate, and poly-D-(-)-3-hydroxybutyric acid.

The pharmaceutical compositions to be used for in vivo administration must be sterile. This is readily accomplished by, for example, filtration through sterile filtration membranes. Therapeutic antibody compositions are generally placed into a container having a sterile access port, for example, an intravenous solution bag or vial having a stopper pierceable by a hypodermic injection needle.

The pharmaceutical compositions described herein comprising the myostatin inhibitor and the GLP-1 pathway activator can be in unit dosage forms (either separately or together) such as tablets, pills, capsules, powders, granules, solutions or suspensions, or suppositories, for oral, parenteral, or rectal administration, or administration by inhalation or insufflation.

For preparing solid compositions such as tablets, the principal active ingredient can be mixed with a pharmaceutical carrier, e.g., conventional tableting ingredients such as corn starch, lactose, sucrose, sorbitol, talc, stearic acid, magnesium stearate, dicalcium phosphate or gums, and other pharmaceutical diluents, e.g., water, to form a solid preformulation composition containing a homogeneous mixture of a compound of the present disclosure, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 mg to about 500 mg of the active ingredient of the present disclosure. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Suitable surface-active agents include, in particular, non-ionic agents, such as polyoxyethylenesorbitans (e.g. TweenTM 20, 40, 60, 80 or 85) and other sorbitans (e.g. SpanTM 20, 40, 60, 80 or 85). Compositions with a surface-active agent will conveniently comprise between 0.05 and 5% surface-active agent and can be between 0.1 and 2.5%. It will be appreciated that other ingredients may be added, for example mannitol or other pharmaceutically acceptable vehicles, if necessary.

Suitable emulsions may be prepared using commercially available fat emulsions, such as IntralipidTM, LiposynTM, InfonutrolTM, LipofundinTM and LipiphysanTM. The active ingredient may be either dissolved in a pre-mixed emulsion composition or alternatively it may be dissolved in an oil (e.g. soybean oil, safflower oil, cottonseed oil, sesame oil, corn oil or almond oil) and an emulsion formed upon mixing with a phospholipid (e.g. egg phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%.

The emulsion compositions can be those prepared by mixing an anti-pro-myostatin antibody with IntralipidTM or the components thereof (soybean oil, egg phospholipids, glycerol and water).

Pharmaceutical compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. In some embodiments, the compositions are administered by the oral or nasal respiratory route for local or systemic effect.

Compositions in preferably sterile pharmaceutically acceptable solvents may be nebulised by use of gases. Nebulised solutions may be breathed directly from the nebulising device or the nebulising device may be attached to a face mask, tent or intermittent positive pressure breathing machine. Solution, suspension, or powder compositions may be administered, preferably orally or nasally, from devices which deliver the formulation in an appropriate manner.

### Kits

The present disclosure also provides kits (including disposable and/or kits for self-administration) for use in alleviating diseases/disorders associated with a metabolic disorder. Such kits can include one or more containers comprising a myostatin pathway inhibitor (i.e., a myostatin-selective inhibitor) and a GLP-1 pathway activator, e.g., any of those described herein. In some embodiments, the myostatin pathway inhibitor and the GLP-1 pathway activator are provided in the same container. In some embodiments, the container is a syringe. In some embodiments, the myostatin pathway inhibitor and the GLP-1 pathway activator are provided in separate containers.

In some embodiments, the kit can comprise instructions for use in accordance with any of the methods described herein. The included instructions can comprise a description of administration of the myostatin pathway inhibitor and the GLP-1 pathway activator to treat, delay the onset, or alleviate a target disease as those described herein. The kit may further comprise a description of selecting an individual suitable for treatment based on identifying whether that individual has the target disease. In still other embodiments, the instructions comprise a description of administering an antibody and a GLP-1 pathway activator to an individual at risk of the target disease.

The instructions relating to the use of a myostatin pathway inhibitor (i.e., a myostatin-selective inhibitor) and the GLP-1 pathway activator generally include information as to dosage, dosing schedule, and route of administration for the intended treatment. The containers may be unit doses, bulk packages (e.g., multi-dose packages) or sub-unit doses. Instructions supplied in the kits of the disclosure are typically written instructions on a label or package insert (e.g., a paper sheet included in the kit), but machine-readable instructions (e.g., instructions carried on a magnetic or optical storage disk) are also acceptable.

The label or package insert indicates that the composition is used for treating, delaying the onset and/or alleviating a disease or disorder associated with metabolic syndrome, diabetes, or obesity. Instructions may be provided for practicing any of the methods described herein.

The kits of this disclosure are in suitable packaging. Suitable packaging includes, but is not limited to, vials, bottles, jars, flexible packaging (e.g., sealed Mylar^{®} or plastic bags), and the like. Also contemplated are packages for use in combination with a specific device, such as an inhaler, nasal administration device (e.g., an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle). At least one active agent in the composition is an anti-pro/latent-myostatin antibody, or antigen binding fragment thereof, as those described herein.

Kits may optionally provide additional components such as buffers and interpretive information. Normally, the kit comprises a container and a label or package insert(s) on or associated with the container. In some embodiments, the disclosure provides articles of manufacture comprising contents of the kits described above.

### The Subject

Pharmaceutical compositions and therapeutic regimens described herein are suitable for administration in human or non-human subjects, e.g., mammalian subjects. Accordingly, treatment regimens comprising a myostatin inhibitor in conjunction with a GLP-1 pathway activator (e.g., GLP-1 analogs such as liraglutide) are useful for administering to a subject who is likely to benefit from reduced myostatin signaling and increased insulin production. Suitable subjects may include healthy individuals who may nonetheless benefit from enhanced muscle mass/function, as well as improved metabolism. In some embodiments, suitable subjects have an existing muscle condition and/or associated metabolic dysfunction. In some embodiments, suitable subjects are at risk of developing such condition(s). In some embodiments, suitable subjects are those on a therapy comprising another therapeutic agent to treat a muscle/metabolic condition, but which is associated with adverse effects or toxicity. In some embodiments, the subject is a human patient of between birth and <18 years of age. In some embodiments, the subject is a human subject (child or adolescent) aged 2-19 years, inclusive of endpoints. In some embodiments, the subject is a human subject aged 12 years or older (e.g., 12-17 years), inclusive of endpoints.

In some embodiments, preferred subjects include subjects (e.g., mammalian subjects) with obesity or who are overweight, e.g., those with at least one weight-related condition (such as diabetes mellitus type 2, high blood pressure, cardiovascular disease, or high cholesterol), including overweight subjects with a body mass index (BMI) of 27 kg/m² or greater. In some embodiments, a subject having obesity has a BMI of 28 to 40, inclusive of endpoints. In some embodiments, a subject having obesity has a BMI of 30 kg/m² or greater. In some embodiments, a subject having a BMI of 27 kg/m² or greater is considered overweight and a subject having a BMI of 30 kg/m2 or greater is considered obese according to European Medicines Agency Guideline on Clinical Evaluation of Medicinal Products Used in Weight Management. In some embodiments, the subjects are on a reduced calorie diet and/or an exercise regimen. In some embodiments, the subjects have a metabolic disorder. In some embodiments, the subject has an excess of abdominal fat.

Table 4 and Table 5 below show BMI ranges that may be used for overweight and obesity. An online tool for gauging the BMIs of adults can be found at: https://www.cdc.gov.

**Table 4. BMI of Adults Ages 20 and Older**

| **BMI of Adults Ages 20 and Older** | |
|---|---|
| **BMI** | **Classification** |
| 18.5 to <25 | Normal weight |
| 25 to <30 | Overweight |
| 30 or higher | Obesity (including extreme obesity) |
| 40 or higher | Severe obesity |

Younger individuals (e.g., children and adolescents) grow at different rates at different times, so it is not always easy to tell if a child is overweight. The CDC BMI growth charts may be used to compare a child's or adolescent's BMI with other children or adolescents of the same sex and age. An online tool for gauging the BMls of children and teens can be found at: https://www.cdc.gov/healthyweight/bmi/calculator.html.

**Table 5. BMI of Children and Adolescents Ages 2 to 19**

| **BMI of Children and Adolescents Ages 2 to 19** | |
|---|---|
| **BMI** | **Classification** |
| At or above the 85th percentile on the CDC growth charts | Overweight or obesity |
| At or above the 95th percentile on the CDC growth charts | Obesity (including extreme obesity) |
| At or above 120 percent of the 95th percentile on the CDC growth charts | Extreme obesity |

In some embodiments, a subject having obesity is obese and well, wherein the subject carries excess weight but does not have any comorbidities or risk factors for comorbid conditions and who does not experience any impairments in their daily feeling or functioning. In other embodiments, a subject having obesity is obese with risk factors, wherein the subject carries excess weight and does not yet have any comorbidities, but wherein the subject has measurable risk factors for comorbid conditions and/or impairments to their daily feeling or functioning. For instance, the subject may be at risk for insulin resistance, glucose intolerance, hypertension, cardiovascular disease, dyslipidemia, hyperuricemia, type 2 diabetes, stroke, fatty liver disease, kidney disease and other health issues. In other embodiments, a subject having obesity is obese and sick, wherein the subject carries excess weight and has one or more obesity-attributable comorbidities and impairments to their daily feeling or functioning.

In some embodiments, a subject having obesity is evaluated using the Edmonton Obesity Staging System (EOSS), a 5-point ordinal classification system that considers comorbidity and functional status, as shown in Table 6.

**Table 6. The Edmonton obesity staging system.**

| **Stage** | **Comorbidity and Functional Status** |
|---|---|
| 0 | No apparent risk factors (e.g., blood pressure, serum lipid and fasting glucose levels within normal range), physical symptoms, psychopathology, functional limitations and/or impairment of well-being related to obesity |
| 1 | Presence of obesity-related subclinical risk factors (e.g., borderline hypertension, impaired fasting glucose levels, elevated levels of liver enzymes), mild physical symptoms (e.g., dyspnea on moderate exertion, occasional aches and pains, fatigue), mild psychopathology, mild functional limitations and/or mild impairment of well-being |
| 2 | Presence of established obesity-related chronic disease (e.g., hypertension, type 2 diabetes, sleep apnea, osteoarthritis), moderate limitations in activities of daily living and/or well-being |
| 3 | Established end-organ damage such as myocardial infarction, heart failure, stroke, significant psychopathology, significant functional limitations and/or impairment of well-being |
| 4 | Severe (potentially end-stage) disabilities from obesity-related chronic diseases, severe disabling psychopathology, severe functional limitations and/or severe impairment of well-being |

| | |
|---|---|
| (See, e.g., Padwal et al, CMAJ. 2011 Oct 4; 183(14): e1059-e1066) | |

In some embodiments, a medicament disclosed herein is suitable for administration in a pediatric population, adult population, and/or an elderly population.

In some embodiments, such medicament is suitable for administration in a subject aged 2-19 years, inclusive of endpoints.

In some embodiments, such medicament is suitable for administration in a subject aged 12 years or older (e.g., 12-17 years), inclusive of endpoints.

The population in need for the myostatin inhibitor in combination with a GLP-1 pathway agonist as described herein, may range between 0 and 6 months of age, between 0 and 12 months of age, between 0 and 18 months of age, between 0 and 24 months of age, between 0 and 36 months of age, between 0 and 72 months of age, between 6 and 36 months of age, between 6 and 36 months of age, between 6 and 72 months of age, between 12 and 36 months of age, between 12 and 72 months of age. In some embodiments, the pediatric population suitable for receiving the myostatin inhibitor, e.g., antibody or antigen-binding fragment, described herein who is likely to benefit from such treatment may range between 0 and 6 years of age, between 0 and 12 years of age, between 3 and 12 years of age, between 0 and 17 years of age. In some embodiments, the population has an age of at least 5 years, e.g., 6, 7, 8, 9, 10, 11, 12, 13, 14, 15, 16, or 17 years. In some embodiments, the pediatric population may be aged below 18 years old. In some embodiments, the pediatric population may be (a) at least 5 years of age and (b) below 18 years of age.

The adult population in need for therapy comprising a myostatin inhibitor and a GLP-1 pathway activator as described herein may have an age of at least 20 years, e.g., at least 20, 25, 30, 35, 40, 45, 50, 55, 60 or 65 years. In some embodiments, the adult population may be below 65 years of age. In some embodiments, the adult population may be below 75 years of age.

The elderly population in need for therapy comprising a myostatin inhibitor and a GLP-1 pathway activator as described herein may have an age of 65 years or older (i.e., ≥ 65 years old), e.g., at least 70, 75 or 80 years.

In some embodiments, a human subject who is likely to benefit from the treatment comprising a myostatin inhibitor and a GLP-1 pathway activator may be a human patient having, at risk of developing, or suspected of having a metabolic disease/disorder. A subject having a metabolic disease or disorder (e.g., obesity, type 2 diabetes mellitus (T2DM), etc.) can be identified by routine medical examination, e.g., laboratory tests, organ functional tests, CT scans, or ultrasounds. A subject suspected of having any of such disease/disorder might show one or more symptoms of the metabolic disease/disorder. A subject at risk for the metabolic disease/disorder can be a subject having one or more of the risk factors for that metabolic disease/disorder.

In some embodiments, a human subject who is likely to benefit from the treatment comprising a myostatin inhibitor and a GLP-1 pathway activator may be a human subject having T2DM with a hemoglobin A1c (HbA1c) between 5% and 15% (e.g., between 6.5% and 10%, inclusive of endpoints).

In some embodiments, a human subject who is likely to benefit from the treatment comprising a myostatin inhibitor and a GLP-1 pathway activator may be a human subject having T2DM who has been on an anti-diabetic treatment for at least 3 months. In some embodiments, the subject has been on an anti-diabetic treatment for approximately 3 months. In some embodiments, the subject has been on an anti-diabetic treatment for at least 6 months.

In some embodiments, a human subject who is likely to benefit from the treatment comprising a myostatin inhibitor and a GLP-1 pathway activator may be a human subject having a body weight of at least 70 kg. In some embodiments, the subject has a body weight of at least 80 kg. In some embodiments, the subject has a body weight of 80 to 140 kg, inclusive of endpoints. In some embodiments, the subject has a body weight of more than 140 kg.

A control subject, as described herein, is a subject who provides an appropriate reference for evaluating the effects of a particular treatment or intervention of a test subject or subject. Control subjects can be of similar age, race, gender, weight, height, and/or other features, or any combination thereof, to the test subjects.

In some embodiments, a myostatin assay (e.g., myostatin ELISA) is used to identify a subject requiring treatment of a myostatin inhibitor and a GLP-1 pathway activator. Methods for assaying myostatin can be found in Lakshman et al. Molecular and Cell Endocrinology (2009) 302:26-32 (myostatin ELISA) and Bergen et al. Skeletal Muscle (2015) 5:21 (liquid chromatography with tandem mass spectrometry).

In some embodiments, a GLP-1 assay (e.g., a measurement of circulating GLP-1) is used to identify a subject requiring treatment of a myostatin inhibitor and a GLP-1 pathway activator. Circulating levels of GLP-1 and other incretin hormones may be determined by enzyme-linked immunosorbent assay (ELISA) and/or other methods known in the art. Circulating levels of GLP-1 level may be determined, e.g., by using a commercially available quantitative.

Total ELISA kit (EMD Millipore, Billerica, MA, USA), with the microplate absorbance reader (Bio-Rad) set to 450 nm. In some embodiments, methods are provided for improving muscle performance in a subject. The subject may or may not have or be at risk of having a condition associated with decreased muscle mass and/or decreased muscle function. As used herein, the term "muscle performance" generally refers to the capacity of the muscle to contract and/or to apply a force (e.g., to an external object). In some embodiments, muscle performance may relate to the capacity of the muscle to consume energy. For example, in some embodiments, muscle performance may relate to the capacity of the muscle to produce and/or consume adenosine triphosphate (ATP) molecules to facilitate muscle contraction. In some embodiments, muscle performance refers to the capacity of the muscle to contract repeatedly for a particular duration of time. In some embodiments, muscle performance refers to the capacity of the muscle to apply a force to an object, e.g., to move the object over a measurable distance. In some embodiments, muscle performance refers to the capacity of the muscle to apply a force to an object for a particular duration of time (e.g., to move the object over a measurable distance for a particular duration of time).

In some embodiments, the combination of a myostatin inhibitor and a GLP-1 pathway activator, described herein is administered to a subject in need of the treatment at an amount sufficient to inhibit the proteolytic activation of pro/latent-myostatin to active myostatin by at least 20% (e.g., 30%, 40%, 50%, 60%, 70%, 80%, 90% or greater) in vivo. In other embodiments, a myostatin inhibitor, e.g., antibody or antigen-binding portion thereof, is administered in an amount effective in reducing the pro/latent-myostatin or latent myostatin level by at least 20% (e.g., 30%, 40%, 50%, 60%, 70%, 80%, 90% or greater).

In some embodiments, the combination of a myostatin inhibitor and a GLP-1 pathway activator, described herein is administered to a subject in need of the treatment at an amount sufficient to inhibit the proteolytic activation of pro/latent-myostatin to active myostatin by at least 20% (e.g., 30%, 40%, 50%, 60%, 70%, 80%, 90% or greater) in vivo and/or at an amount sufficient to enhance insulin secretion or inhibit glucagon secretion by at least 0.5, 1.0, 1.5, 2.0. 2.5, 3.0, 3.5, 4.0, 4.5, 5.0, 5.5, 6.0, 6.5, 7.0, 7.5, 8.0, 8.5, 9.0, 9.5, or 10.0 fold.

In some embodiments, the myostatin inhibitor, e.g., anti-pro/latent-myostatin antibody or antigen-binding portion thereof, described herein is administered to a subject who will benefit from increased muscle mass. In some embodiments, the combination of a myostatin inhibitor and a GLP-1 pathway activator is administered to a subject who will benefit from increased muscle-to-fat ratios. In some embodiments, the combination described herein is administered to a subject who will benefit from increased muscle function. In some embodiments, the subject may or may not have or be at risk of having a condition associated with decreased muscle mass and/or decreased muscle function. In some embodiments, the subject has or is at risk of having a condition associated with decreased muscle mass and/or decreased muscle function.

The methods of the present invention further comprise selecting a patient (subject) or a patient population who is likely to benefit from the combination or adjunct (add-on) therapy described herein.

In some embodiments, the subject suffers from or is at risk of developing a muscle condition or disorder. In some embodiment, the subject suffers from or is at risk of developing a metabolic disorder. In some embodiment, the subject suffers or is at risk of developing a disease or disorder associated with impaired neurological signaling.

Patient selection may be based on the responsiveness of a patient to another therapy aimed to treat a metabolic disease such as obesity or type 2 diabetes. Patients who respond poorly to such therapy may have an enhanced response to the therapy when used in conjunction with, complementary to, as an adjunct therapy or as an add-on therapy with a myostatin pathway inhibitor. Thus, in some embodiments, a myostatin pathway inhibitor is used in the treatment of a metabolic disease in a patient, wherein the treatment comprises administration of the myostatin pathway inhibitor to the patient who has received another therapy aimed to treat the metabolic disease but has failed to achieve an intended therapeutic goal or expected therapeutic outcome, and wherein optionally the metabolic disease is obesity/overweight and/or type 2 diabetes. In some embodiments, said "another therapy aimed to treat the metabolic disease" is or comprises a GLP-1 receptor agonist. Examples of the other therapies, including those comprising GLP-1 receptor agonist, include, without limitation, albiglutide, taspoglutide, semaglutide, exenatide, BPI-3016, GW002, glutazumab, exendin-4, exenatide, GLP-1 (7-36)NH2, everestmab, liraglutide, lixisenatide, tirzepatide, dulaglutide, danuglipron (Pfizer). In some embodiments, other therapies comprising a GLP-1 receptor agonist include, but are not limited to, GLP-1 receptor agonist/GIP receptor antagonist combination such as AMG 133 (Amgen); GLP-1/GIP dual agonists such as tirzepatide (LY3298176; Eli Lilly) and CT-388; amylin/GLP-1 combination such as cagrilintide/semaglutide combination (Novo Nordisk); GLP-1/glucagon combination such as DD01 (Neuraly); GLP-1/glucagon combination such as ALT-801 (Altimmune), GLP-1/GIP such as CT-388 (Carmot); GLP-1/glucagon dual agonist such as IBI362 (LY-330567 (Innovent/Eli Lilly), danuglipron (PF-06882961) (Pfizer), MEDI0382; and GLP-1/GIP/Glucagon triple receptor agonist. In some embodiments, the intended therapeutic goal or expected therapeutic outcome is a reduction of body weight (percent change from baseline) of at least 5% or at least 10%, reduced BMI, reduced waist circumference, reduced fat mass, increased lean to fat mass ratio, and/or improved insulin sensitivity.

Patient selection may be based on a patient's ability to perform or adhere to a therapeutic regimen for a weight management treatment. In some embodiments, a myostatin pathway inhibitor is used in the treatment of a metabolic disease in a patient wherein the treatment comprises administration of the myostatin pathway inhibitor to the patient who is on a weight management therapy such as a GLP-1 pathway activator and who cannot or fails to perform or continue a reduced calorie regimen and/or an exercise regimen intended as part of the GLP-1 pathway activator therapy.

Patient selection may be based on background therapy or therapies that the patient is receiving. The background therapies may be standard-of-care medications for weight management and/or diabetes. In some embodiments, a myostatin-selective inhibitor is used in the treatment of obesity/overweight in a patient, wherein the treatment comprises administration of the myostatin-selective inhibitor to the patient who is on a background therapy containing a biguanide such as metformin or a derivative thereof. Non-limiting examples of metaformin include Fortamet, Glucophage, Glucophage XR, Glumetza, Riomet, Obimet, Gluformin, Dianben, Diabex, Diaformin, Metsol, Siofor, Metforgamma and Glifor, as well as metformin-containing medications that include additional active. Examples include, but are not limited to, thiazolidinediones (glitazones) and rosiglitazone.

In some embodiments, a myostatin selective inhibitor is used to treat an overweight or an obese patient wherein the treatment comprises administering the myostatin selective inhibitor to a patient who is on a medication for type 2 diabetes. The anti-diabetic medication may be an alpha-glucosidase inhibitor; a dipeptidyl peptidase-4 (DPP-4) inhibitor such as alogliptin, linagliptin, saxagliptin or sitagliptin; a sodium-glucose cotransporter-2 (SGLT-2) inhibitor such as dapagliflozin, canagliflozin, empagliflozin, or ertugliflozin; a sulfonylurea; or a thiazolidinedione.

### Routes of Administration

To practice the method disclosed herein, an effective amount of the pharmaceutical composition described above can be administered to a subject (e.g., a mammalian subject) in need of the treatment via a suitable route, such as intravenous administration, e.g., as a bolus or by continuous infusion over a period of time, by intramuscular, intraperitoneal, intracerebrospinal, subcutaneous, intra-articular, intrasynovial, intrathecal, oral, inhalation or topical routes. In some embodiments, the administration is subcutaneous. Commercially available nebulizers for liquid formulations, including jet nebulizers and ultrasonic nebulizers may also be useful for administration. Liquid formulations can be directly nebulized, and lyophilized powder can be nebulized after reconstitution. Alternatively, anti-pro/latent-myostatin antibodies can be aerosolized using a fluorocarbon formulation and a metered dose inhaler or inhaled as a lyophilized and milled powder.

Conventional methods, known to those of ordinary skill in the art of medicine, can be used to administer the pharmaceutical composition to the subject, depending upon the type of disease to be treated or the site of the disease. This composition can also be administered via other conventional routes, e.g., administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. In addition, it can be administered to the subject via injectable depot routes of administration such as using 1-, 3-, or 6-month depot injectable or biodegradable materials and methods.

In preferred embodiments, the composition comprising a myostatin pathway inhibitor is formulated for subcutaneous administration. In some embodiments, the composition contains both the myostatin pathway inhibitor and a GLP-1 pathway activator in the same formulation. In some embodiments, e.g. for adjunct therapy, separate formulations may be used.

Injectable compositions may contain various carriers such as vegetable oils, dimethylactamide, dimethyformamide, ethyl lactate, ethyl carbonate, isopropyl myristate, ethanol, and polyols (glycerol, propylene glycol, liquid polyethylene glycol, and the like). For intravenous injection, water soluble antibodies can be administered by the drip method, whereby a pharmaceutical formulation containing the antibody and a physiologically-acceptable excipient is infused. Physiologically acceptable excipients may include, for example, 5% dextrose, 0.9% saline, Ringer's solution or other suitable excipients. Intramuscular preparations, e.g., a sterile formulation of a suitable soluble salt form of the antibody, can be dissolved and administered in a pharmaceutical excipient such as water-for-injection, 0.9% saline, or 5% glucose solution.

In one embodiment, a myostatin-selective inhibitor, e.g., anti-pro/latent-myostatin antibody or antigen-binding portion thereof, is administered via site-specific or targeted local delivery techniques. Examples of site-specific or targeted local delivery techniques include various implantable depot sources of the myostatin inhibitor, e.g., anti-pro/latent-myostatin antibody or antigen-binding portion thereof, or local delivery catheters, such as infusion catheters, an indwelling catheter, or a needle catheter, synthetic grafts, adventitial wraps, shunts and stents or other implantable devices, site specific carriers, direct injection, or direct application. See, e.g., PCT Publication No. WO 00/53211 and U.S. Pat. No. 5,981,568.

The particular dosage regimen, e.g., dose, timing and repetition, used in the methods described herein will depend on the particular subject and that subject's medical history as well as the formulation and pharmacokinetic properties of each drug administered, e.g., the myostatin inhibitor and the GLP-1 pathway activator.

Treatment efficacy for a disease/disorder associated with metabolic syndrome can be assessed using any suitable methods.

In some embodiments, in the context of an increase in the level of pro-myostatin in the target muscle, the increase is at least 1-fold, 1.2-fold, 1.5-fold, 2-fold, 2.5-fold, 3-fold, 4-fold, 5-fold, 6-fold, 7-fold, 8-fold, 9-fold, or 10-fold or more (or any range bracketed by any of the values), compared to a control level of pro-myostatin. In one embodiment, the increase in the level of pro-myostatin in the target muscle is an increase in a range of 1-fold to 3-fold, 1.2-fold to 10-fold, 2-fold to 9-fold, 3-fold to 8-fold, 4-fold to 7-fold, 2-fold to 7-fold, etc. compared to the control level of pro-myostatin.

The term "control level" refers to an accepted or pre-determined level of a biological marker, e.g., a level of a marker obtained before treatment or the onset of disease or before administration of a drug, e.g., an antibody or an antigen-binding portion thereof. The level of a biological marker present in a subject or population of subjects having one or more particular characteristics, e.g., the presence or absence of a particular disease or condition.

In some embodiments, in the context of an increase in latent myostatin in the target muscle after the administering step, the increase is detectable within 4 hours, 24 hours, 48 hours, 7 days, 14 days, 21 days, 28 days or 30 days (or any time range bracketed by any of the listed duration of times) after the administering step. In one embodiment, an increase in latent myostatin in the target muscle after the administering step is detectable for at least 5 days, 7 days, 14 days, 21 days, 28 days, or 30 days (or any time range bracketed by any of the listed duration of times) after the administering step. In one embodiment, an increase in the level of latent myostatin in the target muscle after the administering step is at least 1-fold, 1.2-fold, 1.5-fold, 2-fold, 2.5-fold, 3-fold, 4-fold, 5-fold, 6-fold, 7-fold, 8-fold, 9-fold, or 10-fold or more (or any range bracketed by any of the values), compared to the level of latent myostatin in the target muscle before the administering step. In one embodiment, an increase in the level of latent myostatin in the target muscle after the administering step is an increase in a range of 1-fold to 3-fold, 1.2-fold to 10-fold, 2-fold to 9-fold, 3-fold to 8-fold, 4-fold to 7-fold, 2-fold to 7-fold, etc., compared to the level of latent myostatin in the target muscle before the administering step.

In some embodiments, in the context of an increase in latent myostatin in the circulation after the administering step, an increase is detectable within 4 hours, 24 hours, 48 hours, 7 days, 14 days, 21 days, 28 days, or 30 days (or any time range bracketed by any of the listed duration of times) after the administering step. In one embodiment, an increase in latent myostatin in the circulation after the administering step is detectable for at least 5 days, 7 days, 14 days, 21 days, 28 days, or 30 days (or any time range bracketed by any of the listed duration of times) after the administering step. In one embodiment, an increase in the level of latent myostatin in the circulation after the administering step is at least 1-fold, 2-fold, 3-fold, 5-fold, 10-fold, 15-fold, 20-fold, 25-fold, 30-fold, 35-fold, 40-fold, 45-fold, or 50-fold or more (or any range bracketed by any of the values), compared to the level of latent myostatin in the circulation before the administering step. In one embodiment, an increase in the level of latent myostatin in the target muscle after the administering step is an increase in a range of 1-fold to 3-fold, 1.2-fold to 10-fold, 2-fold to 9-fold, 3-fold to 8-fold, 4-fold to 7-fold, 2-fold to 7-fold, etc., compared to the level of latent myostatin in the target muscle before the administering step.

In some embodiments, in the context of a decrease in the level of latent myostatin in the circulation, the decrease is at least 1-fold, 1.2-fold, 1.5-fold, 2-fold, 2.5-fold, 3-fold, 4-fold, 5-fold, 6-fold, 7-fold, 8-fold, 9-fold, or 10-fold or more (or any range bracketed by any of the values), compared to a control level of latent myostatin. In one embodiment, a decrease in the level of latent myostatin in the circulation is a decrease in a range of 1-fold to 3-fold, 1.2-fold to 10-fold, 2-fold to 9-fold, 3-fold to 8-fold, 4-fold to 7-fold, 2-fold to 7-fold, etc. compared to the control level of latent myostatin. In the context of administering a myostatin inhibitor and a GLP-1 pathway activator, the myostatin inhibitor may be adjunct therapy, add-on therapy, used in combination with or be complementary to the GLP-1 pathway activator.

In some embodiments, in the context of administration of a combination therapy comprising a myostatin inhibitor and a GLP-1 pathway activator to a subject (e.g., a mammalian subject) an effective amount is an amount effective to increase mass of a target muscle in the subject compared with a control muscle mass. In some embodiments, the mass of a muscle treated with an effective amount of the combination therapy is increased by at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, etc. as compared with a control muscle that is not treated with an effective amount of the combination. In some embodiments, such a muscle mass increase is achieved in a select group or type of muscles in the subject. In some embodiments, an effective amount is an amount effective to maintain muscle mass in a subject (e.g., a mammal) throughout treatment with a myostatin inhibitor and a GLP-1 pathway activator. For example, a subject receiving the combination treatment may retain muscle mass as fat mass is reduced. In some embodiments, an effective amount is an amount effective to reduce loss of mass in a target muscle in a subject compared with a control muscle mass, during and/or after the treatment with the combination therapy.

The term "control" in reference to a control sample refers to any clinically or scientifically relevant comparative sample or counterpart, including, for example, a sample from a healthy subject, a sample from a subject having a deficiency that can cause or make the subject susceptible to a certain disease or condition, a subject with a disease or condition of interest, a sample from a subject treated with a pharmaceutical carrier, a sample from a subject prior to treatment, a sham or buffer treated subject or sample, an untreated subject or sample, and the like.

In some embodiments, in the context of administration of a myostatin inhibitor and a GLP-1 pathway activator to a subject, an effective amount is an amount effective to reduce overall body weight. In some embodiments, body weight of a subject treated with an effective amount of the combination therapy is decreased by at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, etc. as compared to a subject receiving a GLP-1 pathway activator alone. In some embodiments, the reduction in body weight of a subject treated with an effective amount of the combination therapy is predominantly due to reduction of fat mass as determined by qNMR.

In some embodiments, in the context of administration of the combination of a myostatin inhibitor in conjunction with a GLP-1 pathway activator to a subject, an effective amount is an amount effective to switch fiber types in the subject. In some embodiments, an effective amount of the combination therapy can promote a fiber type switch from type I to type II. In some embodiments, an effective amount of the combination therapy can promote a fiber type switch from type I to type IIB. In some embodiments, an effective amount of the combination therapy can promote type II fibers, relative to other types of fibers. In some embodiments, an effective amount of the combination therapy can promote type IIB fibers, relative to other types of fibers. In some embodiments, such phenotypic switch in fibers may occur without significant change in overall muscle mass. In other embodiments, such phenotypic switch in fibers may coincide an increase in overall muscle mass.

In some embodiments, in the context of administration of the combination of a myostatin inhibitor and a GLP-1 pathway activator to a subject, an effective amount is an amount effective to increase diameter of muscle fiber in the subject compared with a control muscle fiber. In some embodiments, the increase in the diameter of the muscle fiber is an increase of at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2-fold, at least 4-fold, at least 5-fold or more compared with a control muscle fiber. In some embodiments, the increase in the diameter of muscle fiber is an increase in a range of 1-fold to 5-fold, 2-fold to 10-fold, 1-fold to 1.5-fold, 1-fold to 2-fold, etc. compared with a control muscle fiber.

In some embodiments, in the context of administration of the combination of a myostatin inhibitor and a GLP-1 pathway activator (e.g., a GLP-1R agonist) to a subject, an effective amount is an amount effective to increase muscle-to-fat ratio in the subject compared with a control muscle mass. In some embodiments, the increase in the muscle-to-fat ratio is an increase of at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2-fold, at least 4-fold, at least 5-fold or more compared with a control subject. In some embodiments, the increase in the muscle-to-fat ratio is an increase in a range of 1-fold to 5-fold, 2-fold to 10-fold, 1-fold to 1.5-fold, 1-fold to 2-fold, etc. compared with a control subject.

In some embodiments, in the context of administration of a myostatin inhibitor and a GLP-1 pathway activator to a subject, an effective amount is an amount effective to decrease intramuscular fat infiltration in the subject compared with a control muscle mass. In some embodiments, the decrease in the intramuscular fat infiltration is a decrease of at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2-fold, at least 4-fold, at least 5-fold or more compared with a control subject. In some embodiments, the decrease in intramuscular fat infiltration is a decrease in a range of 1-fold to 5-fold, 2-fold to 10-fold, 1-fold to 1.5-fold, 1-fold to 2-fold, etc. compared with a control subject.

In some embodiments, a method of preventing a reduction of and/or increasing muscle mass in a human subject includes administering a myostatin inhibitor in conjunction with a GLP-1 pathway activator to a subject wherein the combination therapy inhibits proteolytic formation of mature myostatin by a tolloid protease. In one embodiment, inhibition of proteolytic cleavage of pro-myostatin or latent myostatin by a tolloid protease results in a progressive or sustained increase in muscle mass. In one embodiment, a subject exhibits a progressive increase in muscle mass for at least 2 weeks, 4 weeks, 6 weeks, 8 weeks, 10 weeks, 12 weeks, 14 weeks, 16 weeks, 18 weeks, or 20 weeks (or any range bracketed by any of the values). In some embodiments, a method of preventing a reduction of and/or increasing muscle mass in a human subject includes administering a myostatin inhibitor, e.g., a pro/latent-myostatin antibody, or antigen binding fragment thereof, to a subject comprising more than two doses. In one embodiment, administering the combination therapy comprises at least a first dose and a second dose, the first dose and the second dose are administered to the subject at least about one week apart, 2 weeks apart, 4 weeks apart, 6 weeks apart, 8 weeks apart, or 12 weeks apart.

As used herein, the term "control muscle mass" refers to a reference standard useful for evaluating effects of a condition (e.g., treatment with a myostatin inhibitor and a GLP-1 pathway activator on the mass of a target muscle in a subject. In some embodiments, the target muscle is a gastrocnemius muscle. In some embodiments, a control muscle mass is a predetermined value. In some embodiments, a control muscle mass is experimentally determined. In some embodiments, a control muscle mass is the mass of a target muscle in a subject who has not been administered the myostatin inhibitor, e.g., pro/latent-myostatin antibody, or antigen binding fragment thereof. In some embodiments, a control muscle mass is the mass (e.g., the average mass) of a target muscle in a population of subjects who have not been administered the combination therapy. In some embodiments, a control muscle mass is the mass of a target muscle in a subject prior to (e.g., immediately prior to) being administered the combination therapy. In some embodiments, a control muscle mass is the mass of a target muscle in a subject who has been administered, in place of the myostatin inhibitor, (e.g., an anti-myostatin antibody), a normal antibody (e.g., of the same isotype as the anti-myostatin antibody) that has been obtained from an animal that has not been exposed to the antigen to which the anti-myostatin antibody, or antigen binding fragment thereof, is directed. In some embodiments, a control muscle mass is the mass of a target muscle in a subject who has been administered, in place of the myostatin inhibitor, e.g., pro/latent-myostatin antibody, or antigen binding fragment thereof, a vehicle, e.g., saline.

### Dosages

Empirical considerations, such as the half-life, generally will contribute to the determination of the dosage. For example, antibodies and antigen-binding portions thereof that are compatible with the human immune system, such as humanized antibodies or fully human antibodies, may be used to prolong half-life of the antibody and to prevent the antibody being attacked by the host's immune system. The pharmacokinetic properties of small peptides, e.g., peptide hormones such as incretins, may be improved by tethering the peptide to a bulkier molecule (such as BSA) or modifying recognition sites for peptidases such as DPP-IV by, e.g., acylation or amino acid substitution. Frequency of administration may be determined and adjusted over the course of therapy, and is generally, but not necessarily, based on treatment and/or suppression and/or amelioration and/or delay of a disease/disorder associated with a metabolic disorder such as obesity, diabetes, or metabolic syndrome. Alternatively, sustained continuous release formulations of a myostatin inhibitor and/or a GLP-1 pathway activator, may be appropriate. Various formulations and devices for achieving sustained release would be apparent to the skilled artisan and are within the scope of this disclosure.

Dosage regimens for a GLP-1 pathway activator, such as a GLP-1 mimetic, are in part dependent on the mode of administration, and additionally may be dependent on the pharmacokinetics of the particular drug substance. For example, exenatide (based on exendin-4) may be administered to a mammal (e.g., a human) subcutaneously at an initial dose of 5 µg twice daily, with a maintenance dose of 5-10 µg twice daily, within 1 hour before morning and evening meals, and approximately 6 or more hours apart. Extended release exenatide (exenatide ER) may be administered to a mammal (e.g., a human) subcutaneously at a dose of 2 mg once daily with or without food. Lixisenatide (based on exendin-4) may be administered to a mammal (e.g., a human) subcutaneously at an initial dose of 10 µg once daily, with a maintenance dose of 20 µg once daily, within an hour before the first meal of the day. Liraglutide (a modified human GLP-1) may be administered to a mammal (e.g., a human) subcutaneously at a starting dose of 0.6 mg and a maintenance dose of 1.2 or 1.8 mg being given once daily with or without food. Dulaglutide (a modified human GLP-1) may be administered subcutaneously once daily (with or without food) at a starting dose of 0.75 mg and a maintenance dose of 0.75 or 1.5 mg. Semaglutide (a modified human GLP-1) may be administered subcutaneously once daily (with or without food) at a starting dose of 0.25 mg and a maintenance dose of 0.5 or 1.0mg. Several clinically approved GLP-1R agonists are reviewed in Cornell, J Clin Pharm Ther. 2020;45(Suppl 1):17-27.

In one embodiment, as part of a treatment regimen comprising a myostatin inhibitor and a GLP-1 pathway activator, an obese or overweight subject is given a GLP-1 mimetic once daily via subcutaneous injection. In another example, a GLP-1 pathway activator (e.g., a GLP-1 mimetic) is given once weekly. In some examples, the starting dose of a GLP-1 mimetic is lower than the maintenance dose of the GLP-1 mimetic. In some examples, the dose (one or both of a starting dose or maintenance dose) of the GLP-1 mimetic (or other GLP-1 pathway activator) administered in the combination therapy is lower than the FDA-approved dose.

In one embodiment, dosages for a myostatin inhibitor, e.g., an anti-pro/latent-myostatin antibody, or antigen binding fragment thereof, as described herein may be determined empirically in individuals who have been given one or more administration(s) of the myostatin inhibitor, e.g., antibody, or antigen binding fragment thereof. Individuals are given incremental dosages of the antagonist. To assess efficacy of the antagonist, an indicator of the disease/disorder can be followed.

Generally, for administration of any of the antibodies, or antigen binding fragment thereof, described herein, an initial candidate dosage can be about 2 mg/kg. For the purpose of the present disclosure, a typical daily dosage might range from about any of 0.1 µg/kg to 3 µg/kg to 30 µg/kg to 300 µg/kg to 3 mg/kg, to 30 mg/kg to 100 mg/kg or more, depending on the factors mentioned above. For repeated administrations over several days or longer, depending on the condition, the treatment is sustained until a desired suppression of symptoms occurs or until sufficient therapeutic levels are achieved to alleviate a disease or disorder associated with pro/latent-myostatin, or a symptom thereof. An exemplary dosing regimen comprises administering an initial dose of about 2 mg/kg, followed by a weekly maintenance dose of about 1 mg/kg of the antibody, or antigen binding fragment thereof, or followed by a maintenance dose of about 1 mg/kg every other week. However, other dosage regimens may be useful, depending on the pattern of pharmacokinetic decay that the practitioner wishes to achieve. For example, dosing from one to four times a week is contemplated. In some embodiments, dosing ranging from about 3 µg/kg to about 2 mg/kg (such as about 3 µg/kg, about 10 µg/kg, about 30 µg/kg, about 100 µg/kg, about 300 µg/kg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, every 4 months, every 5 months, every 6 months, every 8 months, every 10 months, every year, or longer. The progress of this therapy is easily monitored by conventional techniques and assays. The dosing regimen (including the antibody used) can vary over time.

In some embodiments, the administration of any of the myostatin inhibitors, e.g., antibodies, or antigen binding fragments thereof, described herein comprises a single dose. In some embodiments, the administration of any of the myostatin inhibitors, e.g., antibodies, or antigen binding fragments thereof, described herein comprises multiple doses (e.g., at least 2, 3, 4, 5, 6, 7, 8, 9, or 10 doses). Administering may comprise more than two doses. In some embodiments, the administration comprises at least a first dose and a second dose of a therapeutically effective amount of the myostatin inhibitor, e.g., antibody or antigen-binding portion thereof. In one embodiment, the first dose and the second dose are administered to the subject at least about 4 weeks apart, 6 weeks apart, 8 weeks apart, or 12 weeks apart.

For the purpose of the present disclosure, the appropriate dosage of a myostatin inhibitor and a GLP-1 pathway activator will depend on the specific myostatin inhibitor (or compositions thereof) and GLP-1 pathway activator employed, the type and severity of the disease/disorder, whether the combination therapy is administered for preventive or therapeutic purposes, previous therapy, current medication profile of the patient, the patient's clinical history and response to the combination therapy, potential synergy of the drug combination, and the discretion of the attending physician. In some embodiments, a clinician will administer the combination therapy (either together or each drug individually) until a dosage of the combination or of each drug individually is reached that achieves the desired result. Administration of the combination therapy can be continuous or intermittent, depending, for example, upon the recipient's physiological condition, whether the purpose of the administration is therapeutic or prophylactic, and other factors known to skilled practitioners. The administration of a myostatin inhibitor and a GLP-1 pathway activator may be essentially continuous over a preselected period of time or may be in a series of spaced dose, e.g., either before, during, or after developing a metabolic disease or disorder.

As used herein, the term "treating" refers to the application or administration of a composition including one or more active agents to a subject, who has a disease/disorder associated with myopathy, a symptom of the disease/disorder, or a predisposition toward the disease/disorder, with the purpose to cure, heal, alleviate, relieve, alter, remedy, ameliorate, improve, or affect the disorder, the symptom of the disease, or the predisposition toward the disease/disorder.

Alleviating a metabolic disease/disorder includes delaying the development or progression of the disease, or reducing disease severity. Alleviating the disease does not necessarily require curative results. As used therein, "delaying" the development of a metabolic disease/disorder means to defer, hinder, slow, retard, stabilize, and/or postpone progression of the disease. This delay can be of varying lengths of time, depending on the history of the disease and/or individuals being treated. A method that "delays" or alleviates the development of a disease, or delays the onset of the disease, is a method that reduces probability of developing one or more symptoms of the disease in a given time frame and/or reduces extent of the symptoms in a given time frame, when compared to not using the method. Such comparisons are typically based on clinical studies, using a number of subjects sufficient to give a statistically significant result. Measurement of improvement in conditions associated with a metabolic disease or disorder may be quantitated, e.g., by evaluation of circulating metabolic biomarkers by qNMR (examples of such biomarkers are discussed, e.g., in Robberecht et al., Metab Syndr Relat Disord. 2016 Mar;14(2):47-93, and Belhayara et al., Nutrients. 2020 Mar; 12(3): 727). "Development" or "progression" of a disease means initial manifestations and/or ensuing progression of the disease. Development of the disease can be detectable and assessed using standard clinical techniques. However, development also refers to progression that may be undetectable. For purpose of this disclosure, development or progression refers to the biological course of the symptoms. "Development" includes occurrence, recurrence, and onset. As used herein "onset" or "occurrence" of a disease/disorder associated with a metabolic disorder includes initial onset and/or recurrence.

### Combination Therapies

The invention encompasses pharmaceutical compositions and related methods used as combination therapies for treating subjects who may benefit from myostatin inhibition and GLP-1 pathway activation in vivo. In any of these embodiments, such subjects may receive combination therapies that include a first composition comprising at least one myostatin pathway inhibitor (i.e., a myostatin-selective inhibitor) in conjunction with a second composition comprising at least one additional therapeutic intended to treat the same or overlapping disease or clinical condition. The first and second compositions may both act on the same cellular target, or discrete cellular targets. In some embodiments, the first and second compositions may treat or alleviate the same or overlapping set of symptoms or aspects of a disease or clinical condition. In some embodiments, the first and second compositions may treat or alleviate a separate set of symptoms or aspects of a disease or clinical condition. Such combination therapies may be administered in conjunction with each other. The phrase "in conjunction with," in the context of combination therapies, means that therapeutic effects of a first therapy overlaps temporarily and/or spatially with therapeutic effects of a second therapy in the subject receiving the combination therapy. Thus, the combination therapies may be formulated as a single formulation for concurrent administration, or as separate formulations, for simultaneous or sequential administration of the therapies. The term "concurrent administration", as used herein, may include co-formulated therapeutics being administered together, or separately formulated therapeutics being administered at the same time, e.g., simultaneously, or, in some embodiments, on the same day. The term "sequential administration", as used herein, includes therapeutic regimens in which therapeutics are administered at separate times. In some embodiments, a subject receives concurrent administration of two or more therapies when subject is on one or more first therapeutic(s) at the beginning of the treatment regimen and one or more second therapeutic(s) is/are added to the regimen at a specific point during the treatment.

In some embodiments, combination therapies produce synergistic effects in the treatment of a disease. The term "synergistic" refers to effects that are greater than additive effects (e.g., greater efficacy) of each monotherapy in aggregate. In some embodiments, combination therapies produce additive effects in the treatment of a disease.

In some embodiments, combination therapies comprising a method described herein (e.g., administration of a myostatin pathway inhibitor (i.e., a myostatin-selective inhibitor) and a GLP-1 pathway activator) produce efficacy that is overall equivalent to that produced by another therapy (such as monotherapy of an additional agent) but are associated with fewer unwanted adverse effect or less severe toxicity associated with the second agent, as compared to the monotherapy of the second agent. In some embodiments, such combination therapies allow lower dosage of the second agent but maintain overall efficacy. Such combination therapies may be particularly suitable for patient populations where a long-term treatment is warranted and/or involving pediatric patients, patients aged 2-19 years, or patients aged 12 years or older (e.g., 12-17 years), inclusive of endpoints.

Accordingly, the invention provides pharmaceutical compositions and methods for use in combination therapies for the increase of lean mass to fat mass ratio, and for the treatment or prevention of metabolic diseases or diseases associated with an impaired neurological signaling, including metabolic syndrome, diabetes (e.g., T2DM), obesity, and spinal cord injury. In some embodiments, the methods or pharmaceutical compositions for use comprise a first therapy. In some embodiments, the methods or pharmaceutical compositions for use further comprise a second therapy. In some embodiments, the first therapy may be useful for increasing the ratio of lean mass to fat mass. In some embodiments, the second therapy may be useful in treating or preventing metabolic diseases or diseases associated with an impaired neurological signaling. In some embodiments, the second therapy may diminish or treat at least one symptom(s) associated with the targeted disease. The first and second therapies may exert their biological effects by similar or unrelated mechanisms of action. In some embodiments, either one or both of the first and second therapies may exert their biological effects by a multiplicity of mechanisms of action.

In some embodiments, the first therapy is a GLP-1 pathway activator and the second therapy is a myostatin pathway inhibitor (i.e., a myostatin-selective inhibitor). In some embodiments, the GLP-1 pathway activator is administered in conjunction with the myostatin pathway inhibitor (i.e., a myostatin-selective inhibitor). In some embodiments, the myostatin pathway inhibitor (i.e., a myostatin-selective inhibitor) is administered to a subject who is receiving a GLP-1 pathway activator therapy. In some embodiments, the myostatin pathway inhibitor (i.e., a myostatin-selective inhibitor) is administered to a subject who has not previously received a GLP-1 pathway activator therapy. In some embodiments, the GLP-1 pathway activator and the myostatin pathway inhibitor (i.e., a myostatin-selective inhibitor) are administered concurrently. In some embodiments, the GLP-1 pathway activator and the myostatin pathway inhibitor (i.e., a myostatin-selective inhibitor) are administered sequentially. In some embodiments, the GLP-1 pathway activator and the is administered myostatin pathway inhibitor (i.e., a myostatin-selective inhibitor) are administered separately.

It should be understood that the pharmaceutical compositions described herein may have the first and second therapies in the same pharmaceutically acceptable carrier or in a different pharmaceutically acceptable carrier for each described embodiment. It further should be understood that the first and second therapies may be administered simultaneously or sequentially within described embodiments.

The combination of a myostatin pathway inhibitor (i.e., a myostatin-selective inhibitor) and a GLP-1 pathway activator, according to the methods disclosed herein, may be used in combination with one or more of additional therapeutic agents. In some embodiments, the myostatin pathway inhibitor inhibits pro/latent myostatin. In some embodiments, the myostatin pathway inhibitor does not inhibit GDF11. In some embodiments, the myostatin pathway inhibitor inhibits myostatin and does not inhibit any other members of the TGFβ superfamily. Examples of the additional therapeutic agents which can be used with the combination therapy include, but are not limited to, additional diabetes mellitus-treating agents, diabetic complication-treating agents, cardiovascular diseases-treating agents, anti-hyperlipemic agents, hypotensive or antihypertensive agents, anti-obesity agents, chemotherapeutic agents, immunotherapeutic agents, immunosuppressive agents, and the like. Such combination therapies may advantageously utilize lower dosages of the administered therapeutic agents, thus avoiding possible toxicities or complications associated with the various monotherapies. In some embodiments, the additional therapeutic agent is administered in the same treatment regimen as the combination therapy comprising the myostatin pathway inhibitor and the GLP-1 pathway activator. In other embodiments, the myostatin pathway inhibitor (i.e., a myostatin-selective inhibitor) and the GLP-1 pathway activator are administered to a subject who is already receiving one or more therapeutic agents such as those described above. In some embodiments, treatment with myostatin pathway inhibitor (i.e., a myostatin-selective inhibitor) in conjunction with a GLP-1 pathway activator results in a reduction of symptoms of type 2 diabetes. In some embodiments, treatment with a myostatin pathway inhibitor (i.e., a myostatin-selective inhibitor) in conjunction with a GLP-1 pathway activator results in induction of remission of T2DM. In some embodiments, remission of T2DM refers to a return of blood sugar levels to normal or healthy levels, e.g., blood sugar level of someone without diabetes, e.g., an A1C level of less than 6.5%.

In some embodiments, the combination of a myostatin pathway inhibitor (i.e., a myostatin-selective inhibitor) and a GLP-1 pathway activator, according to the methods disclosed herein, may be used in combination with one or more diet and/or exercise treatment regimens. In some embodiments, the myostatin pathway inhibitor (i.e., a myostatin-selective inhibitor) and the GLP-1 pathway activator are administered to a subject who is on a diet and/or exercise regimen for weight loss or for improving or alleviating symptoms of one or more metabolic diseases or diseases associated with an impaired neurological signaling, including metabolic syndrome, diabetes (e.g., T2DM), obesity, and spinal cord injury. In some embodiments, the diet regimen includes, but is not limited to, caloric restriction (e.g., reduced calorie intake or reduced absorption of calories), diets with modified nutrient content (e.g., high protein, low fat, low carbohydrate, keto, paleo, etc.) or modified timing of food intake (e.g., intermittent fasting, increased frequency of feeding, etc.) or a combination of modified timing, portion size, and nutrient content (e.g., more frequent meals of smaller portions containing high protein, low fat, and/or other nutrient content restrictions).

Examples of agents for treating diabetes mellitus (e.g., T2DM) include insulin formulations (e.g., animal insulin formulations extracted from a pancreas of a cattle or a swine; a human insulin formulation synthesized by a gene engineering technology using microorganisms or methods), insulin sensitivity enhancing agents, pharmaceutically acceptable salts, hydrates, or solvates thereof (e.g., pioglitazone, troglitazone, rosiglitazone, netoglitazone, balaglitazone, rivoglitazone, tesaglitazar, farglitazar, CLX-0921, R-483, NIP-221, NIP-223, DRF-2189, GW-7282TAK-559, T-131, RG-12525, LY-510929, LY-519818, BMS-298585, DRF-2725, GW-1536, GI-262570, KRP-297, TZD18 (Merck), DRF-2655, and the like), alpha-glycosidase inhibitors (e.g., voglibose, acarbose, miglitol, emiglitate and the like), biguanides (e.g., phenformin, metformin, buformin and the like) or sulfonylureas (e.g., tolbutamide, glibenclamide, gliclazide, chlorpropamide, tolazamide, acetohexamide, glyclopyramide, glimepiride and the like) as well as other insulin secretion-promoting agents (e.g., repaglinide, senaglinide, nateglinide, mitiglinide, GLP-1 pathway activators and the like), amyrin agonist (e.g., pramlintide and the like), phosphotyrosin phosphatase inhibitor (e.g., vanadic acid and the like) and the like.

Examples of GLP1-R pathway activators include, but are not limited to, incretin mimetics such as albiglutide, taspoglutide, semaglutide, exenatide, BPI-3016, GW002 (GLP1 mimetic with albumin moiety to extend half-life; see, e.g., Eur J Pharmacol. 2021 Jan 5;890:173650 for discussion of half-life extension of GLP-1), glutazumab, exendin-4, exenatide, GLP-1 (7-36)NH2, everestmab, liraglutide, lixisenatide, tirzepatide, XW003, Noiiglutide, MEDI0382, or dulaglutide;

Additional examples of GLP1R pathway activators include DPPIV inhibitors, non-limiting examples of which are sitagliptin, vildagliptin, saxagliptin, linagliptin, gemigliptin, anagliptin, teneligliptin, alogliptin, trelagliptin, omarigliptin, evogliptin, gosogliptin, dutogliptin, and berberine. Examples of agents for treating diabetic complications include, but are not limited to, aldose reductase inhibitors (e.g., tolrestat, epalrestat, zenarestat, zopolrestat, minalrestat, fidareatat, SK-860, CT-112 and the like), neurotrophic factors (e.g., NGF, NT-3, BDNF and the like), PKC inhibitors (e.g., LY-333531 and the like), advanced glycation end-product (AGE) inhibitors (e.g., ALT946, pimagedine, pyradoxamine, phenacylthiazolium bromide (ALT766) and the like), active oxygen quenching agents (e.g., thioctic acid or derivative thereof, a bioflavonoid including flavones, isoflavones, flavonones, procyanidins, anthocyanidins, pycnogenol, lutein, lycopene, vitamins E, coenzymes Q, and the like), cerebrovascular dilating agents (e.g., tiapride, mexiletene and the like).

In one embodiment, remission of diabetes can be induced by administration of a myostatin pathway inhibitor (i.e., a myostatin-selective inhibitor) in combination with a GLP-1 pathway activator, optionally in combination with a caloric restriction diet, or other diet. In some embodiments, an exercise routine is included depending on the subject's physical capabilities. In some embodiments, a subject having T2DM that is in remission may be able to reduce or eliminate other diabetes medication.

In some embodiments, the combination of a myostatin pathway inhibitor (i.e., a myostatin-selective inhibitor) and a GLP-1 pathway activator may be used according to the methods disclosed herein in combination with one or more of a hyperlipidemic agent, an immunotherapeutic agent, an anti-obesity agent, a chemotherapeutic agent, an immunotherapeutic agent, an immunosuppressive agent or an agent that improves cachexia.

Anti-hyperlipemic agents include, for example, statin-based compounds which are cholesterol synthesis inhibitors (e.g., pravastatin, simvastatin, lovastatin, atorvastatin, fluvastatin, rosuvastatin and the like), squalene synthetase inhibitors or fibrate compounds having a triglyceride-lowering effect (e.g., fenofibrate, gemfibrozil, bezafibrate, clofibrate, sinfibrate, clinofibrate and the like), niacin, PCSK9 inhibitors, triglyceride lowing agents or cholesterol sequesting agents.

Hypotensive agents include, for example, angiotensin converting enzyme inhibitors (e.g., captopril, enalapril, delapril, benazepril, cilazapril, enalapril, enalaprilat, fosinopril, lisinopril, moexipril, perindopril, quinapril, ramipril, trandolapril and the like) or angiotensin II antagonists (e.g., losartan, candesartan cilexetil, olmesartan medoxomil, eprosartan, valsartan, telmisartan, irbesartan, tasosartan, pomisartan, ripisartan forasartan, and the like) or calcium channel blockers (e.g., amlodipine) or aspirin.

Anti-obesity agents include, for example, agents that increase energy expenditure and/or fat metabolism and weight loss by neural and chemical regulation. Anti-obesity agents include biologic agents, such as antibodies, fusion proteins, nanobodies, and the like, as well as small molecule agents. Agents include those targeting of GLP-1 and its receptor (GLP-1R), including GLP-1R agonists or other GLP-1 pathway activators, including biologics such as glutazumab, everestmab, exenatide, liraglutide, lixisenatide, tirzepatide, albiglutide, XW003, Noiiglutide, MEDI0382, and dulaglutide, and small molecules such as topiramate, dexfenfluramine, fenfluramine, phentermine, phenmetrazine, sibutramine, amfepramone, dexamphetamine, mazindol, diethylpropion, orlistat, cathine, oleoyl-estrone, perflubron, benfluorex, setmelanotide, aclimostat, cetilistat, amlintide, rimonabant, phenylpropanolamine, clobenzorex and the like. Agents also include insulin and insulin-secretion promoting agents, gastrointestinal lipase inhibitors (e.g., orlistat and the like), sodium glucose cotransporter 2 (SGLT-2) inhibitors, beta 3-adrenoceptor agonists (e.g., CL-316243, SR-58611-A, UL-TG-307, SB-226552, AJ-9677, BMS-196085 and the like), peptide-based appetite-suppressing agents (e.g., leptin, CNTF and the like), cholecystokinin agonists (e.g., lintitript, FPL-15849 and the like), oxytocin and oxytocin analogs (such as carbetocin),and the like.

Chemotherapeutic agents include, for example, alkylating agents (e.g., cyclophosphamide, iphosphamide and the like), metabolism antagonists (e.g., methotrexate, 5-fluorouracil and the like), anticancer antibiotics (e.g., mitomycin, adriamycin and the like), vegetable-derived anticancer agents (e.g., vincristine, vindesine, taxol and the like), cisplatin, carboplatin, etoposide and the like. Among these substances, 5-fluorouracil derivatives such as furtulon and neofurtulon are preferred.

Immunotherapeutic agents include, for example, microorganisms or bacterial components (e.g., muramyl dipeptide derivative, picibanil and the like), polysaccharides having immune potentiating activity (e.g., lentinan, sizofilan, krestin and the like), cytokines obtained by a gene engineering technology (e.g., interferon, interleukin (IL) and the like), colony stimulating factors (e.g., granulocyte colony stimulating factor, erythropoietin and the like) and the like, among these substances, those preferred are IL-1, IL-2, IL-12 and the like.

Immunosuppressive agents include, for example, calcineurin inhibitor/immunophilin modulators such as cyclosporine (Sandimmune, Gengraf, Neoral), tacrolimus (Prograf, FK506), ASM 981, sirolimus (RAPA, rapamycin, Rapamune), or its derivative SDZ-RAD, glucocorticoids (prednisone, prednisolone, methylprednisolone, dexamethasone and the like), purine synthesis inhibitors (mycophenolate mofetil, MMF, CellCept(R), azathioprine, cyclophosphamide), interleukin antagonists (basiliximab, daclizumab, deoxyspergualin), lymphocyte-depleting agents such as antithymocyte globulin (Thymoglobulin, Lymphoglobuline), anti-CD3 antibody (OKT3), and the like.

In addition, agents whose cachexia-improving effect has been established in an animal model or at a clinical stage, such as cyclooxygenase inhibitors (e.g., indomethacin and the like), progesterone derivatives (e.g., megestrol acetate), glucosteroid (e.g., dexamethasone and the like), metoclopramide-based agents, tetrahydrocannabinol-based agents, lipid metabolism improving agents (e.g., eicosapentanoic acid and the like), growth hormones, IGF-1, antibodies against TNF-α, LIF, IL-6 and oncostatin M may also be employed concomitantly with an anti-myostatin antibody according to the present invention. Additional therapeutic agents for use in the treatment of diseases or conditions related to metabolic disorders and/or impaired neurological signaling would be apparent to the skilled artisan and are within the scope of this disclosure.

In some embodiments, additional agents suitable for administration as a combination therapy in conjunction with the myostatin pathway inhibitor (i.e., a myostatin-selective inhibitor)/GLP-1 pathway activator combination described herein include anti-fibrotic agents, such as TGFβ1 inhibitors. In some embodiments, second agents suitable for administration as a combination therapy in conjunction with the antibodies described herein are modulators (e.g., agonists and antagonists) of certain members of the TGFβ super family of growth factors, such as BMP6, BMP7, GDF11, TGFβ2, TGFβ3, etc. In some embodiments, additional therapy comprises an inhibitor of RGMc, such as anti-RGMc antibodies.

Any of the above-mentioned agents can be administered in combination with the myostatin pathway inhibitor (i.e., a myostatin-selective inhibitor)/GLP-1 pathway activator to treat a metabolic disease or disorder, e.g., metabolic syndrome, obesity, T2DM, or T2DM associated with obesity. In some embodiments, any of the above-mentioned agents may be administered in combination with the myostatin pathway inhibitor (i.e., a myostatin-selective inhibitor) and/or GLP-1 pathway activator to treat a metabolic disease or disorder, e.g., metabolic syndrome, obesity, T2DM, or T2DM associated with obesity, wherein the agents are administered in conjunction with a diet and/or exercise regimen. Examples of the diet regimen include, but are not limited to, caloric restriction (e.g., reduced calorie intake or reduced absorption of calories), diets with modified nutrient content (e.g., high protein, low fat, low carbohydrate, keto, paleo, etc.) or modified timing of food intake (e.g., intermittent fasting, increased frequency of feeding, etc.) or a combination of modified timing, portion size, and nutrient content (e.g., more frequent meals of smaller portions containing high protein, low fat, and/or other nutrient content restrictions).

### Use of a Combination of a Myostatin Pathway Inhibitor and a GLP-1 Pathway Activator for Treating Metabolic Diseases/Disorders

Pharmaceutical compositions described herein are suitable for administration to human patients for the treatment or prevention of diseases and conditions where reduced myostatin signaling and increased insulin production is desirable. Such diseases and conditions include, but are not limited to: metabolic syndrome, obesity, type 2 diabetes mellitus (T2DM), and T2DM associated with obesity. Exemplary conditions for which the compositions and methods of the present invention may be useful are further described below.

### A. Metabolic Disorders and Diseases

Disclosed herein are methods for treating or preventing a metabolic disease in a subject. A metabolic disease (also referred to as a metabolic disorder) is generally associated with aberrant glucose, lipid/fat and/or protein/nitrogen metabolism, or osmotic dysregulation, and has pathological consequences arising from such condition. A number of metabolic disorders of the invention share certain characteristics, e.g., they are associated with a loss of fat-free or lean muscle mass, an excess of fat mass, a lower metabolic rate, insulin resistance, lack of ability to regulate blood sugar, weight gain, and/or increase in body mass index. In some cases, such metabolic conditions may be triggered or exacerbated by medication that the patients receive. As discussed in more detail herein, metabolic disorders can occur secondarily to, or occur as a result of, a muscle condition or disorder.

Metabolic disorders and diseases for treatment according to the methods provided herein include but are not limited to metabolic syndrome, obesity, overweight with at least one comorbidity, type 2 diabetes mellitus, and type 2 diabetes mellitus associated with obesity. In one embodiment, the metabolic disorder is T2DM. In another embodiment, the metabolic disorder is obesity.

The present invention is based, at least in part, on the discovery that administration of a combination of a myostatin pathway inhibitor (e.g., a myostatin inhibitor, e.g., a myostatin-selective inhibitor) and a GLP-1 pathway activator, to subjects having a metabolic disease significantly improves both the physiological and the functional characteristics of the injured subjects. In particular, the present inventors have surprisingly discovered that administration of a myostatin pathway inhibitor (e.g., a myostatin inhibitor, e.g., a myostatin-selective inhibitor) and a GLP-1 pathway activator significantly increases the metabolic rate or energy expenditure in subjects having metabolic disease. Administration of the combination therapy also significantly attenuated spinal cord injury (SCI)-induced reduction in sub-lesional muscle mass and overall body mass and, while at the same time reducing the mass of undesirable adipose tissue such as white and visceral adipose tissue. Subjects treated with the combination therapy may also exhibit a significant improvement in their locomotor function, muscle strength, as well as motor coordination and balance skills.

Accordingly, the present invention provides methods for treating or preventing metabolic diseases in a human subject. The methods include selecting a human subject suffering from a metabolic disease and administering to the human subject an effective amount of a combination of a myostatin pathway inhibitor (i.e., a myostatin-selective inhibitor) and a GLP-1 pathway activator, thereby treating or preventing the metabolic disease in the human subject. Preferably, the myostatin-selective inhibitor is an antibody, or antigen binding fragment thereof, that specifically binds to pro/latent myostatin, but does not bind to GDF11. Antibodies that specifically recognize pro/latent myostatin, but not GDF11, are beneficial and avoid undesirable toxicity caused by off-target binding of antibodies to GDF11 in the subject. In one embodiment, the subject is a pediatric subject. In one embodiment, the subject is a subject aged 2-19 years, inclusive of endpoints. In one embodiment, the subject is a subject aged 12 years or older (e.g., 12-17 years), inclusive of endpoints.

Examples of metabolic diseases that may be treated or prevented by the methods of the present invention include but are not limited to, type 2 diabetes, metabolic syndrome, pre-diabetes, obesity, cardiovascular diseases (such as congestive heart failure), spinal cord injury (SCI) (e.g., complete or incomplete/partial SCI), hypo-metabolic states, double diabetes, Cushing's disease (also referred to as Cushing's syndrome), obesity syndrome (e.g., diet-associated or diet-induced obesity), insulin resistance, insulin insufficiency, hyperinsulinemia, impaired glucose tolerance (IGT), abnormal glycogen metabolism, hyperlipidemia, hypoalbuminemia, hypertriglyceridemia, kidney disease, syndrome X, fatty liver disease and metabolic bone diseases. In some embodiments, metabolic diseases include diseases associated with impaired neurological signaling or partial denervation. In some embodiments, metabolic diseases include conditions triggered by or associated with certain medication (e.g., side effects).

Additional diseases or conditions related to metabolic disorders and/or body composition that would be apparent to the skilled artisan and are within the scope of this disclosure. Body composition may be measured by a variety of methods, including dual energy X-ray absorptiometry (DEXA). Total body scans using DEXA provide generally accurate and precise measurements of body composition, including bone mineral content, bone mineral density, lean tissue mass, fat tissue mass, and fractional contribution of fat.

Diabetes refers to a group of metabolic diseases characterized by high blood sugar (glucose) levels which result from defects in insulin secretion or action, or both. There are two most common types of diabetes, namely type 1 diabetes and type 2 diabetes, which both result from the body's inability to regulate insulin. Insulin is a hormone released by the pancreas in response to increased levels of blood sugar (glucose) in the blood.

In type 2 diabetes mellitus (T2DM, also referred to as noninsulin-dependent diabetes mellitus, NDDM), the pancreas continues to manufacture insulin, sometimes even at higher than normal levels. However, the body develops resistance to its effects, resulting in a relative insulin deficiency. Type 2 diabetes may occur in children and adolescents but usually begins after age 30 and becomes progressively more common with age: about 15 percent of people over age 70 have type II diabetes. Obesity is a risk factor for type 2 diabetes, and 80 to 90 percent of the people with this disorder are obese.

In some embodiments, diabetes includes pre-diabetes. "Pre-diabetes" refers to one or more early diabetic conditions including impaired glucose utilization, abnormal or impaired fasting glucose levels, impaired glucose tolerance, impaired insulin sensitivity and insulin resistance. Prediabetes is a major risk factor for the development of type 2 diabetes mellitus, cardiovascular disease, and mortality. Much focus has been given to developing therapeutic interventions that prevent the development of type 2 diabetes by effectively treating prediabetes.

In some embodiments, diabetes includes double diabetes, which is a combination of type 1 diabetes with features of insulin resistance and type 2 diabetes.

Diabetes can be diagnosed by the administration of a glucose tolerance test. Clinically, diabetes is often divided into several basic categories. Primary examples of these categories include, autoimmune diabetes mellitus, non-insulin-dependent diabetes mellitus (type 1 NDDM), insulin-dependent diabetes mellitus (type 2 IDDM), non-autoimmune diabetes mellitus, non-insulin-dependent diabetes mellitus (type 2 NIDDM), and maturity-onset diabetes of the young (MODY). A further category, often referred to as secondary, refers to diabetes brought about by some identifiable condition which causes or allows a diabetic syndrome to develop. Examples of secondary categories include, diabetes caused by pancreatic disease, hormonal abnormalities, drug- or chemical-induced diabetes, diabetes caused by insulin receptor abnormalities, diabetes associated with genetic syndromes, and diabetes of other causes. (see e.g., Harrison's (1996) 14th ed., New York, McGraw-Hill).

Obesity is another prevalent metabolic disease that can be treated or prevented by the methods of the present invention. "Obesity" refers to a chronic condition defined by an excess amount of body fat. The normal amount of body fat (expressed as percentage of body weight) is between 25-30% in women and 18-23% in men. Women with over 30% body fat and men with over 25% body fat are considered obese. Obesity can be defined using any clinically relevant definitions. For example, in adults, body mass index (BMI, kg/m2) is frequently used as a measure of overweight and obesity, with overweight being defined as a BMI 25-29.9 kg/m2, obesity as a BMI equal to or greater than 30 kg/m2, and morbid obesity being defined as BMIs over 40 kg/m2. Obesity can also be defined in adults by central adiposity as measured by waist circumference, with raised waist circumference defined as equal to or greater than 102 cm in men and equal to or greater than 88 cm in women. Subjects with obesity may exhibit other symptoms such as increased fasting plasma glucose, increased fasting plasma triglycerides, decreased fasting high density lipoprotein (HDL) level, and increased blood pressure. Obesity may also cause various orthopedic problems, skin disorders and swelling of the feet and ankles. Severe complications of obesity include a much higher risk of coronary artery disorder and of its major risk factors type II diabetes, hyperlipidemia and hypertension. Much of the morbidity associated with obesity is associated with type II diabetes, as poorly controlled diabetes and obesity lead to a constellation of symptoms that are together known as syndrome X, or metabolic syndrome. In some embodiments, the obesity is sarcopenic obesity. In some embodiments, the subject having obesity is on a caloric restriction regimen.

The methods of the present invention are also suitable for treating or preventing metabolic disease such as metabolic syndromes. As used herein, "metabolic syndrome" refers to the concept of a clustering of metabolic risk factors that come together in a single individual and lead to a high risk of developing diabetes and/or cardiovascular diseases. The main features of metabolic syndrome include insulin resistance, hypertension (high blood pressure), cholesterol abnormalities, dyslipidemia, triglyceride abnormalities, an increased risk for clotting and excess body weight, especially in the abdomen, or obesity. In some embodiments, metabolic syndrome can be diagnosed by the presence of three or more of the following components: (1) an elevated waist circumference (men, equal to or greater than 40 inches (102 cm); women, equal to or greater than 35 inches (88 cm)); (2) elevated triglycerides (equal to or greater than 150 mg/dL); (3) reduced High Density Lipoprotein cholesterol or HDL (men, less than 40 mg/dL; women, less than 50 mg/dL); (4) elevated blood pressure (equal to or greater than 130/85 mm Hg); and (5) elevated fasting glucose (equal to or greater than 100 mg/dL).

In another aspect, the methods of the present invention are suitable for treating or preventing metabolic disease such as obesity syndromes. The term "obesity syndrome" refers any disorder or conditions causing a subject to be grossly fat or overweight. Like other metabolic diseases, people with obesity syndrome are usually associated a loss of fat-free or lean muscle mass, an excess of fat mass, a lower metabolic rate, insulin resistance, lack of ability to regulate blood sugar, weight gain, and increase in body mass index. In some embodiments, the obesity syndrome is selected from the group consisting of Prader Willi, an obesity syndrome associated with a genetic disorder, and an obesity syndrome associated with a hypothalamic disorder.

The methods of the present invention are also suitable for treating or preventing metabolic diseases associated with a hypo-metabolic state. The term "a hypo-metabolic state" refers to a state of reduced metabolism or metabolic activity, where the body is not producing enough energy. Patients with a hypo-metabolic state generally have a lower metabolic rate, a loss of fat-free or lean muscle mass, an excessive gain of fat mass, insulin resistance, lack of ability to regulate blood sugar, weight gain, and an increase in body mass index. In some embodiments, the hypo-metabolic state is selected from the group consisting of a state associated with prolonged immobilization, a state associated with bed-rest, a state associated with casting, a state associated with a stroke, a state associated with amputation, and a post-surgery state. In some embodiments, the hypo-metabolic state is a post-surgery state, e.g., paraspinal muscle atrophy after lumbar spine surgery. In one embodiment, the paraspinal muscle atrophy is a nerve injury-dependent muscle atrophy. In one embodiment, the surgery is a spinal surgery. In one embodiment, the spinal surgery is a lumbar spine surgery or a lumbar spine procedure, e.g., a lumbar fusion procedure, a lumbar nonfusion procedure, a posterior lumbar fusion procedure, an anterior lumbar fusion procedure, a minimally invasive (MIS) posterior lumbar decompression procedure, a minimally invasive (MIS) posterior lumbar fusion procedure, a non-MIS equivalent procedure, etc.

In another aspect, the methods of the present invention are suitable for treating or preventing metabolic diseases such as Cushing's disease, which is also referred to as Cushing's syndrome or Cushing's syndrome. The term "Cushing's disease" refers to a collection of signs and symptoms due to prolonged exposure to cortisol. This may stem from endogenous causes, such as a condition in which the pituitary gland releases too much adrenocorticotropic hormone (ACTH), or exogenous causes, such as the use of oral corticosteroid medication. Some of the hallmark signs and symptoms of Cushing's disease may include: progressive obesity, such as weight gain and fatty tissue deposits, particularly around the midsection and upper back and between the shoulders (buffalo hump) (upper body obesity above the waist); thin arms and legs, round, red, full face (moon face); changes in the skin, such as pink or purple stretch marks (striae) on the skin of the abdomen, thighs, breasts and arms, thinning, fragile skin that bruises easily, low healing of cuts, insect bites and infections, and acne. Patients with Cushing's disease may also experience severe fatigue, muscle weakness, depression, anxiety and irritability, loss of emotional control, cognitive difficulties, new or worsened high blood pressure, headache, type 2 diabetes, and/or bone loss which may lead to fractures over time. In children, Cushing's disease may cause impaired growth (slow growth rate). In some embodiments, the Cushing's disease is selected from the group consisting of corticosteroid-induced Cushing's disease and tumor-induced Cushing's disease.

To date, standard treatments for Cushing's disease are designed to lower the high level of cortisol in the body, whether the source is endogenous overproduction of the hormones or due to medication. The best treatment for a particular patient depends on the cause of the syndrome. Treatment options that are currently available include, for example, reducing corticosteroid use, surgery, radiation therapy, and medications.

Thus, the use of the combination therapy described herein presents an alternative or additive treatment option for patients suffering from Cushing's disease.

Where the cause of Cushing's disease is long-term use of corticosteroid medications, controlled reduction of the dosage of the drug over a period of time, while still adequately managing the underlining disease or condition for which the drug is being administered, may be considered. Thus, in some embodiments, the patient who is on a corticosteroid therapy has one or more autoimmune or inflammatory diseases, such as rheumatoid arthritis, lupus and asthma. Corticosteroid may also be prescribed to patients to suppress the body's immunity in order to prevent the body from rejecting an allograft transplant, such as a transplanted organ or tissue.

In some embodiments, patients receiving a corticosteroid therapy include a sub-population of individuals who do not tolerate well, who are poorly responsive or not responsive, to other treatment options, such as non-corticosteroid medications. In such situations, the physician may continue to prescribe corticosteroid medication. In some embodiments, surgery may be considered as an alternative option.

If the cause of Cushing syndrome is a tumor, complete surgical removal and/or radiation therapy can be considered. In some embodiments, patients have a tumor in the pituitary, adrenal glands, lungs or pancreas. After the operation, cortisol replacement therapy is typically administered to provide the body with the correct amount of adrenal hormone production.

In some embodiments, patients with Cushing syndrome never experience a resumption of normal adrenal function and therefore may require lifelong replacement therapy. The inhibitors of myostatin activation described herein may be suitable to treat such patients.

In some embodiments, medication can be used to control cortisol production when surgery and/or radiation don't work. Medications may also be used before surgery in patients who have become very sick with Cushing syndrome. The inhibitors of myostatin activation encompassed by the present disclosure may be used to treat such patients prior to surgery to improve signs and symptoms and minimize surgical risk.

Medications currently used to control excessive production of cortisol at the adrenal gland include ketoconazole (Nizoral), mitotane (Lysodren) and metyrapone (Metopirone). Mifepristone (Korlym) is approved for patients with Cushing syndrome who have type 2 diabetes or glucose intolerance. Mifepristone does not decrease cortisol production, but it blocks the effect of cortisol on the tissues. Side effects from these medications may include fatigue, nausea, vomiting, headaches, muscle aches, high blood pressure, low potassium and swelling. Some have more serious side effects, such as neurological side effects and liver toxicity. The inhibitors of myostatin activation encompassed by the present disclosure may be used alone (in lieu of) or in combination with any of these therapeutics.

More recently, pasireotide (Signifor) has become available for the treatment of Cushings, which works by decreasing ACTH production from a pituitary tumor. This medication is given as an injection twice daily. It is typically recommended when pituitary surgery is unsuccessful or cannot be done. Side effects associated with this medication are fairly common, and may include diarrhea, nausea, high blood sugar, headache, abdominal pain and fatigue. The inhibitors of myostatin activation encompassed by the present disclosure may be used alone (in lieu of) or in combination with any of such therapeutics.

In some embodiments, the tumor or its treatment will cause other hormones produced by the pituitary or adrenal gland to become deficient, which may require hormone replacement therapy. In some embodiments, none of these currently available treatment options are appropriate or effective, surgical removal of the adrenal glands (bilateral adrenalectomy) may be considered, which will require lifelong replacement medications. Patients who are candidates for such option may benefit from a myostatin inhibition therapy described herein, before and/or after adrenalectomy.

In yet another aspect, the methods of the present invention are suitable for treating or preventing metabolic diseases such as cardiovascular disease, e.g., cardiovascular disease associated with metabolic syndrome. The term "cardiovascular disease" refers to any disease of the heart or blood vessels. Cardiovascular or heart disease includes but is not limited to, for example, angina, arrhythmia, coronary artery disease (CAD), coronary heart disease, cardiomyopathy (including dilated cardiomyopathy, restrictive cardiomyopathy, arrhythmogenic right ventricular cardiomyopathy, and diabetic cardiomyopathy), heart attack (myocardial infarction), heart failure (e.g., acute heart failure (AHF) or chronic heart failure (CHF)), hypertrophic cardiomyopathy, mitral regurgitation, mitral valve prolapse, pulmonary stenosis, etc. Blood vessel disease includes but is not limited to, for example, peripheral vascular disease, artery disease, carotid artery disease, deep vein thrombosis, venous diseases, and atherosclerosis. In some embodiments, a subject having heart failure is resistant to diuretic therapy. In another embodiment, a subject having heart failure responds poorly to diuretic therapy.

Obesity is a risk factor for the development of cardiovascular disease. Obese individuals experience cardiovascular disease events at an earlier age, live with cardiovascular disease for a greater proportion of their lifetime and have a shorter average lifespan than individuals with normal weight. Obesity contributes directly to cardiovascular risk factors, including dyslipidemia, type 2 diabetes, hypertension, and sleep disorders. Obesity accelerates atherosclerotic changes through multiple mechanisms, including insulin resistance and inflammation. Obesity also leads to the development of cardiovascular disease and cardiovascular disease mortality independently of other cardiovascular risk factors. Visceral adiposity promotes systemic and vascular inflammation, which is fundamental to the atherosclerotic process. Inflammation induced by obesity increases the likelihood of LDL oxidation, which in turn promotes atherogenesis. Insulin resistance is associated with dyslipidemia and metabolic syndrome, which are linked to atherosclerosis. Endothelial function in obesity, e.g., due to decreased bioavailability of nitric oxide in the setting of inflammation and oxidative stress also contributes to the progression of atherosclerosis. Obesity also has been liked to abnormalities in the coronary microvasculature and on epicardial coronary vessels. Another aspect of the disclosure includes a method of treating a subject having a metabolic disease or condition related to aging. Exemplary diseases and conditions related to ageing include, without limitation, sarcopenia (age-related muscle loss), frailty, and androgen deficiency.

Unlike non-specific agents that affect myostatin as well as additional pathway(s), in some embodiments the myostatin pathway inhibitor (i.e., a myostatin-selective inhibitor) for use in the methods disclosed herein is a monoclonal antibody selective for myostatin. In one embodiment, the monoclonal antibody specifically binds and inhibits the activation step of myostatin/GDF8. In some embodiments, such antibodies bind pro-myostatin and/or latent myostatin, thereby inhibiting activation and subsequent release of mature myostatin, but do not bind mature myostatin that is not associated with a latent (inactive) complex. In some embodiments, the antibodies or fragments thereof bind tethered forms (e.g., intramuscular) of inactive myostatin (e.g., pro-myostatin), which have the ability to locally act upon tissue-associated myostatin within a disease niche. In some embodiments, the antibodies or fragments thereof bind soluble forms (e.g., in circulation) of inactive myostatin (e.g., latent-myostatin), which have the ability to act upon circulating latent myostatin that may have endocrine or systemic effects. In any of such embodiments, preferred inhibitors of myostatin for carrying out the methods of the present invention are those that are selective for myostatin that do not antagonize other members of the TGFβ superfamily of growth factors/cytokines, such as GDF11. Such selectivity is advantageous particularly in pediatric patient populations and/or patient populations requiring a long-term care (e.g., chronic therapy), where inhibiting other pathways, such as GDF11, may produce harmful or unwanted side effects or adverse events.

### Weight Loss

The present invention further provides methods for promoting robust weight loss (e.g., loss of fat mass (i.e., the weight of fat in the body) without concomitant loss of lean muscle mass) in subjects having metabolic diseases, such as obesity, e.g., diet-induced obesity, metabolic syndrome, and/or type 2 diabetes mellitus (T2DM). As compared to dieting alone (e.g., dieting by caloric restriction, low-carbohydrate diet, ketogenic diet, vegan diet, etc.), where weight loss occurs in both fat stores and muscle during dieting, administration of a myostatin pathway inhibitor (e.g., a myostatin inhibitor, e.g., a myostatin-selective inhibitor) disclosed herein in combination with a GLP-1 pathway activator (e.g., an insulin secretion-promoting agent, e.g., liraglutide) leads to weight loss in fat stores, while sparing the muscle. In some embodiments, administration of the myostatin pathway inhibitor and the GLP-1 pathway activator results in about a 5%, about 10%, about 15%, or about 20% or greater loss of fat mass in the subject during the course of treatment

Body fat mass increases with age in both men and women through middle age. Abdominal fat in particular is associated with higher risk of cardiovascular disease, metabolic syndrome, hypertension, diabetes, dyslipidemia in a subject when compared to the risk in individuals without abdominal obesity. Fat mass or abdominal fat mass may be measured directly by dual energy X-ray absorptiometry, ultrasound, computed tomography, or magnetic resonance imaging (e.g., qNMR). Clinically, abdominal obesity is defined as a waist circumference of 102 cm or greater in men, and 88 cm or greater in women.

In some embodiments, administration of a myostatin pathway inhibitor (i.e., a myostatin-selective inhibitor) in conjunction with a GLP-1 pathway activator may result in more robust weight loss due to the maintenance of a higher metabolic rate; improved cardiometabolic benefits (such as lipid profile, glucose metabolism, cardiovascular risk, etc.); and higher reduction in fat (e.g., total fat or visceral fat and/or other deleterious fat levels) as compared to dieting or to the administration of the myostatin pathway inhibitor or the GLP-1 pathway activator alone. Additionally, administration of the combination therapy disclosed herein may prevent or reduce muscle atrophy and/or bone loss which can occur concomitantly with a diet, e.g., a caloric restriction diet, a low-carbohydrate diet, a ketogenic diet, etc. Overall, administration of a myostatin pathway inhibitor (i.e., a myostatin-selective inhibitor) and a GLP-1 pathway activator, optionally in combination with a diet, may increase the ratio of muscle to fat in the subject. In some embodiments, administration of a GLP-1 pathway activator yields a similar pharmacological effect as calorie restriction. In some embodiments, administering a GLP-1 pathway inhibitor in conjunction with a myostatin pathway inhibitor provides added benefit to weight loss and fat mass loss as compared to either agent administered alone.

In subjects with a metabolic disease, e.g., obesity, metabolic syndrome and/or diabetes, e.g., T2DM, the combination of a myostatin pathway inhibitor (i.e., a myostatin-selective inhibitor) with a GLP-1 pathway activator enables prevention or mitigation of lowering of the metabolic rate in a subject, and prevention or reduction of lean muscle loss. The combination of myostatin pathway inhibitor (i.e., a myostatin-selective inhibitor) and a GLP-1 pathway activator may optionally be combined with a diet, e.g., a caloric restriction diet. In some embodiments, a moderate calorie restriction diet is recommended, as this provides for better patient compliance and better long-term outcomes since subjects do not have to adhere to austere, aggressive diets, e.g., aggressive caloric restriction diets.

Such treatments are particularly useful for subjects who have limitations on physical activity, e.g., subjects having an orthopedic injury, spinal cord injury, musculoskeletal disease, a pulmonary disorder, a cardiovascular disorder, a neurologic disorder, severe obesity, etc. In such subjects, administration of a myostatin pathway inhibitor (e.g., a myostatin inhibitor, e.g., a myostatin-selective inhibitor) in combination with a GLP-1 pathway activator, and optionally a diet, e.g., a caloric restriction diet, prevents muscle atrophy and/or bone loss which are more prominent in these subjects due to their limitations on physical activity. In some embodiments, such a treatment enables subjects with limitations on physical activity to undergo a more robust diet, e.g., caloric restriction diet, because they are no longer limited by concerns regarding muscle loss or bone loss due to the administration of the myostatin pathway inhibitor. The combination therapy may also contribute to a reduction of feelings of hunger and unhealthy food behaviors in a patient due to the action of a GLP-1 pathway activator on the central nervous system.

In some embodiments, the subject is on a diet regimen, but is not on an exercise regimen. In some embodiments, the subject is on an exercise regimen, but is not on a diet regimen. In some embodiments, the subject is on a diet regimen, and an exercise regimen. Examples of a diet regimen include, but are not limited to, caloric restriction (e.g., reduced calorie intake or reduced absorption of calories), diets with modified nutrient content (e.g., high protein, low fat, low carbohydrate, keto, paleo, etc.) or modified timing of food intake (e.g., intermittent fasting, increased frequency of feeding, etc.) or a combination of modified timing, portion size, and nutrient content (e.g., more frequent meals of smaller portions containing high protein, low fat, and/or other nutrient content restrictions).

### Assessment and Measures of Body Composition

Clinical effects of the combination and adjunct therapies described herein may be measured by any suitable methods or criteria in order to assess benefits on pharmacologic management of excess adiposity and accompanying metabolic disturbances (Heymsfield et al. J Biol Chem. 2020 Apr 17;295(16):5404-5418.).

In some embodiments, various parameters of body composition can be measured, such as changes in total body fat mass, lean mass, waist circumference, HbA1c levels, and body weight. Any suitable techniques can be employed to assess body composition, including, without limitation, qNMR, dual energy x-ray absorptiometry (DXA), magnetic resonance imaging (MRI)-derived hepatic fat fraction, hydrodensitometry, air displacement plethysmography (ADP), bioelectrical impedance analysis (BIA), bioimpedance spectroscopy (BIS), electrical impedance myography (EIM), 3D body scanners, and multi-compartment models (e.g., 3-compartment and 4-compartment models).

In some embodiments, the combination and adjunct therapies described herein may result in a change in total body fat mass in a subject as compared to baseline. In some embodiments, total body fat mass is measured by dual energy x-ray absorptiometry (DXA) (Garito et al. Diabetes Obes Metab. 2018;20(1):94-102). In some embodiments, the combination and adjunct therapies described herein may result in a change in diabetes status in a subject as compared to baseline. In some embodiments, diabetes status can be determined by measuring the subject's HbA1c level, homeostatic model assessment, quantitative insulin sensitivity check, Matsuda Index (Yokoyama et al. J Clin Endocrinol Metab. 2004;89(3):1481-1484; Hrebícek et al. J Clin Endocrinol Metab. 2002;87(1):144-147; Matsuda et al. Diabetes Care. 1999;22(9):1462-1470). In some embodiments, the combination and adjunct therapies described herein may result in a change in a subject's body weight, BMI, waist circumference, and/or waist-to-hip ratio. In some embodiments, the combination and adjunct therapies described herein may result in a change in a subject's body composition. In some embodiments, the subject's body composition may be assessed by DXA-measurements of bone mineral-free lean mass, magnetic resonance imaging (MRI)-derived hepatic fat fraction (Mashood et al. J Magn Reson Imaging. 2013;37(6):1359-1370), and/or subcutaneous and abdominal visceral adipose tissue (Fallah et al. MAGMA. 2017;30 (2):139-151). In some embodiments, the combination and adjunct therapies described herein may result in a change in a subject's metabolic status (Garito et al. Diabetes Obes Metab. 2018;20(1):94-102; Goyal et al. N Am J Med Sci. 2012;4(4):180-184; Rossi et al. Obesity (Silver Spring). 2011;19(9):1747-1754). In some embodiments, the subject's metabolic status may be assessed using metabolic biomarkers, non-limiting examples of such markers are discussed in, e.g., in Robberecht et al., Metab Syndr Relat Disord. 2016 Mar;14(2):47-93, and Belhayara et al., Nutrients. 2020 Mar; 12(3): 727. In some embodiments, the subject's metabolic status may be assessed based on the subject's cardiovascular risk factors, including serum lipid levels, high-sensitivity C-reactive protein level, interleukin 6 level, leptin level, adiponectin level, and blood pressure. In some embodiments, the combination and adjunct therapies described herein may result in a change in a subject's physical performance. In some embodiments, the subject's physical performance may be assessed by measuring hand grip strength by dynamometry (Rooks et al. J AmGeriatr Soc. 2017;65(9):1988-1995).

The present invention is further illustrated by the following examples, which are not intended to be limiting in any way.

### EXAMPLES

Those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used. Those skilled in the art will recognize or be able to ascertain using no more than routine experimentation, many equivalents to the specific embodiments of the disclosure described herein. It is, therefore, to be understood that the foregoing embodiments are presented by way of example only and that, within the scope of the appended claims and equivalents thereto, the disclosure may be practiced otherwise than as specifically described and claimed. The present disclosure is directed to each individual feature, system, article, material, and/or method described herein. In addition, any combination of two or more such features, systems, articles, materials, and/or methods, if such features, systems, articles, materials, and/or methods are not mutually inconsistent, is included within the scope of the present disclosure.

### Example 1: Effects of myostatin inhibition coupled with calorie restriction in a diet-induced obesity model

Metabolic disorders such as obesity and diabetes can benefit from a reduction in calorie consumption in that they may experience a reduction in severity of their symptoms. However, ultra-low-calorie diets are impractical, and difficult for patients to maintain. Moreover, a substantial portion of the weight loss is in lean muscle rather than fat, some estimates show about 25% of the weight loss is muscle, although this may be as high as 33% in the context of more rapid weight loss, further complicated by the body's metabolic adaptations. The basal metabolic rate typically declines to compensate for the body's calorie deficit. With this lower basal metabolic rate, an individual would need to maintain the reduced calorie intake and increased physical activity to stay calorie budget neutral.

To determine whether the muscle building and/or muscle preserving effects of the myostatin inhibition could eliminate or reduce loss of muscle during weight loss, a study was performed in which mice having diet-induced obesity (DIO) were administered Ab2 with a calorie reduction diet after mice had been fed a high fat diet.

Male C57BL/6 DIO mice (n=55) were maintained for 12 weeks on a high fat diet (HFD) comprising 60% fat. On Study Day -2 all animals were weighed and assessed for whole body lean mass using qNMR techniques according to the method described in Jones et al. Int J Body Compos Res. 2009;7(2):67-72. Individual animals were assigned to treatment groups in such a way as to generate cohorts with no significant differences with regard to body weight and lean body mass. After the 12 weeks of high fat diet, the animals were divided into five groups and given diets as shown in Table 7 for the duration of the study. Group 1, a control group, was fed the high fat diet; the mice were allowed to eat at will and they received no antibody during the study. Groups 2 and 3 received 70% of the Group 1 amount (30% calorie reduction (CR)), and Groups 4-5 received 80% of the Group 1 amount (20% CR). Food consumption was quantitatively measured daily. A negative control antibody ("IgG") was administered to groups 2 and 4, and the myostatin inhibitor Ab2 was administered to groups 3 and 5. Each was administered via intraperitoneal (i.p.) injection once weekly. The first day of dosing was designated as Day 1.

**Table 7. Experimental Design**

| **Group No.** | **Diet** | **Antibody** | **Dose Level (mg/kg/dose)** | **Dose Volume (mL/kg)** | **Dose Concentration (mg/mL)** | **No. of Animals (Males)** |
|---|---|---|---|---|---|---|
| 1 | High fat diet ad lib | N/A | 20 mg/kg weekly | 10 | 2 | 10 |
| 2 | 70% of Group 1 ad lib amount | IgG | | | | 10 |
| 3 | | Ab2 | | | | 10 |
| 4 | 80% of Group 1 ad lib amount | IgG | | | | 10 |
| 5 | | Ab2 | | | | 10 |

The body weight of individual animals was measured 3X per week beginning on study day -2. Body composition analysis was performed on days -2, 14, 29, and 44. On scheduled days, animals were removed from their home cage and placed into a plastic restraint compatible with the body composition analysis machine (Bruker NMR LF90II). The restrainer was placed into the Bruker NMR, body composition was assessed, and the animals were returned to their home cage. Animals were maintained in the restrainer for no longer than five minutes.

On Study Day 42 the animals began an overnight fast. On day 43, fasting blood glucose levels were checked on all animals via glucometer (Abbott Alpha Trak) and fasting HbA1c levels were checked on all animals via a commercially available HbA1c reader.

At the close of the study (Day 44) whole blood samples (terminal, maximum obtainable volume) were collected via intracardiac puncture or into serum separator tubes. Blood samples were maintained at ambient temperature for 30 minutes following collection, and then processed to serum within 90 minutes of collection. The samples were centrifuged for 10 minutes in a refrigerated centrifuge (set to maintain a temperature of 4°C) at 1200 × g. The resultant serum was separated and analyzed for triglycerides, cholesterol, and insulin. Whole body weight for each animal was collected, and liver, inguinal fat pads (both sides), and calf muscle (gastrocnemius and soleus, both sides) were collected and frozen for analysis.

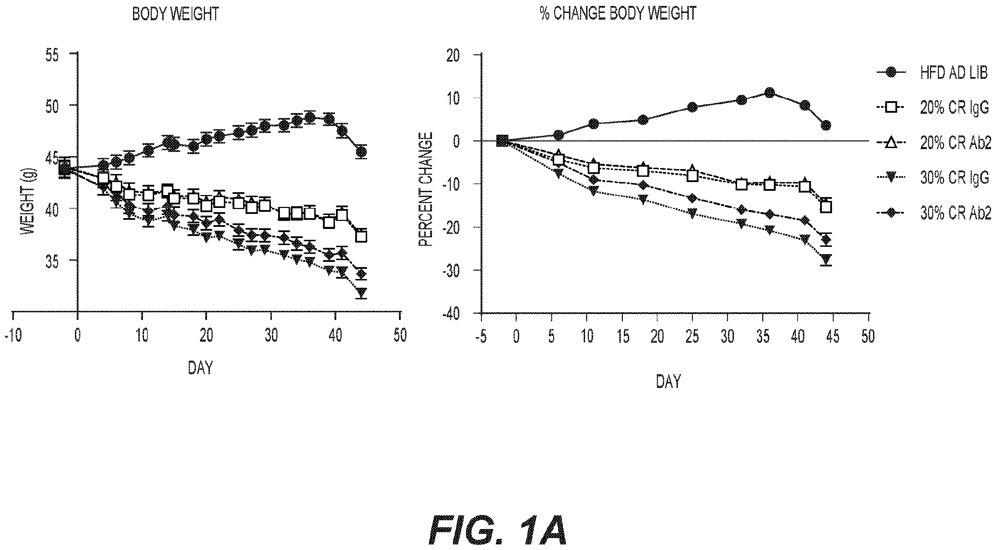

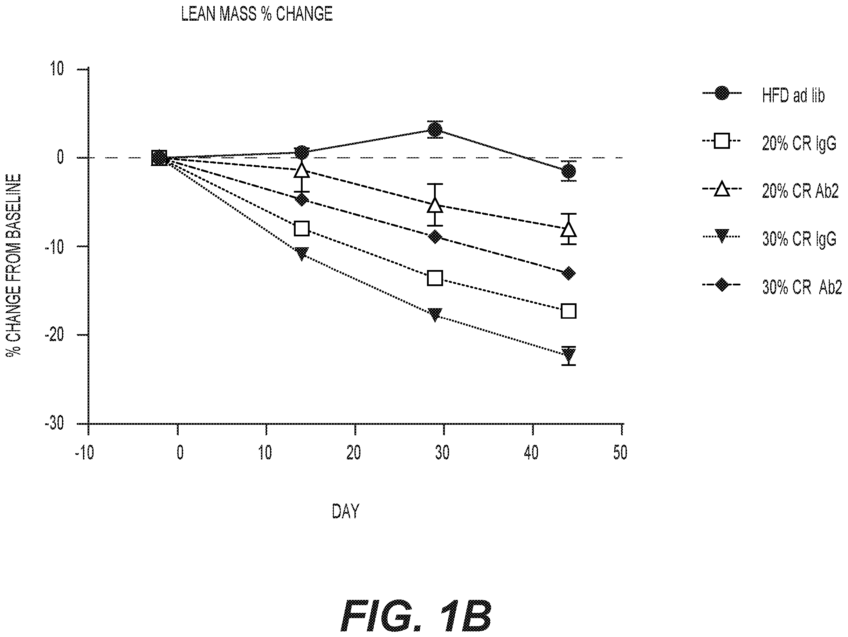

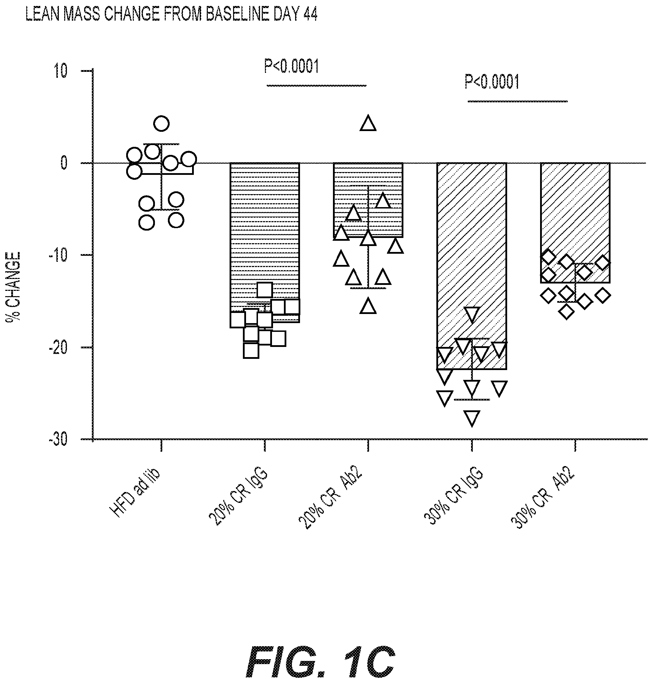

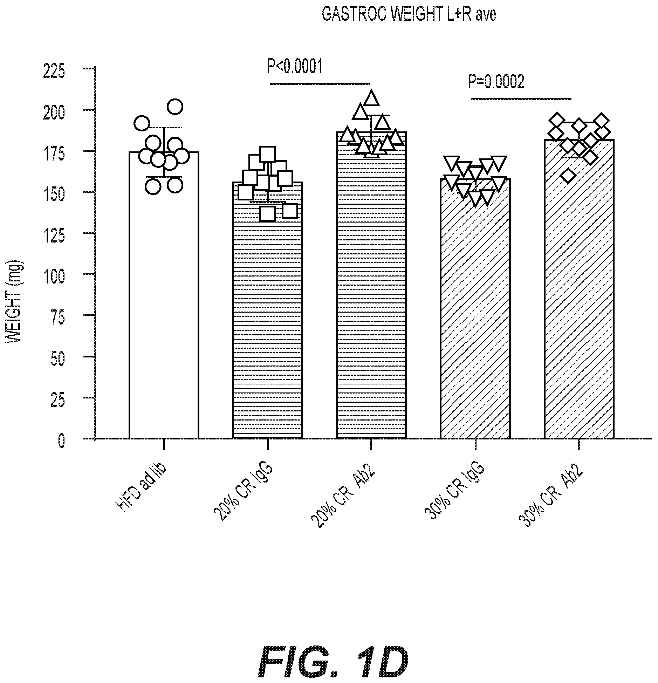

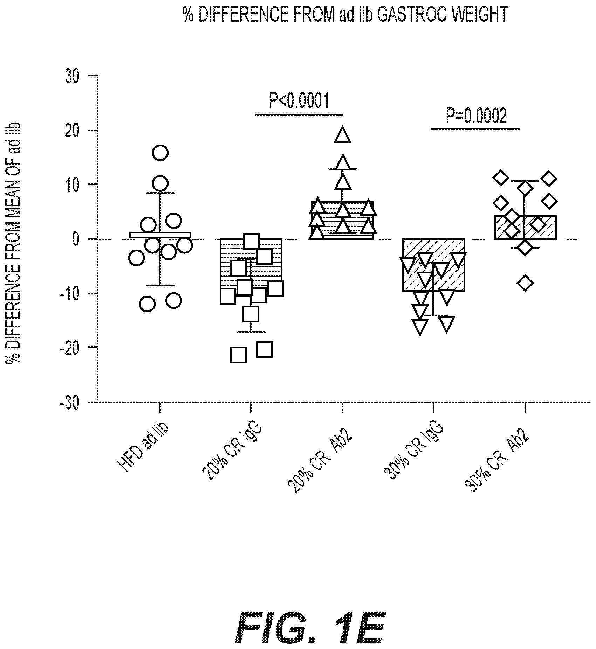

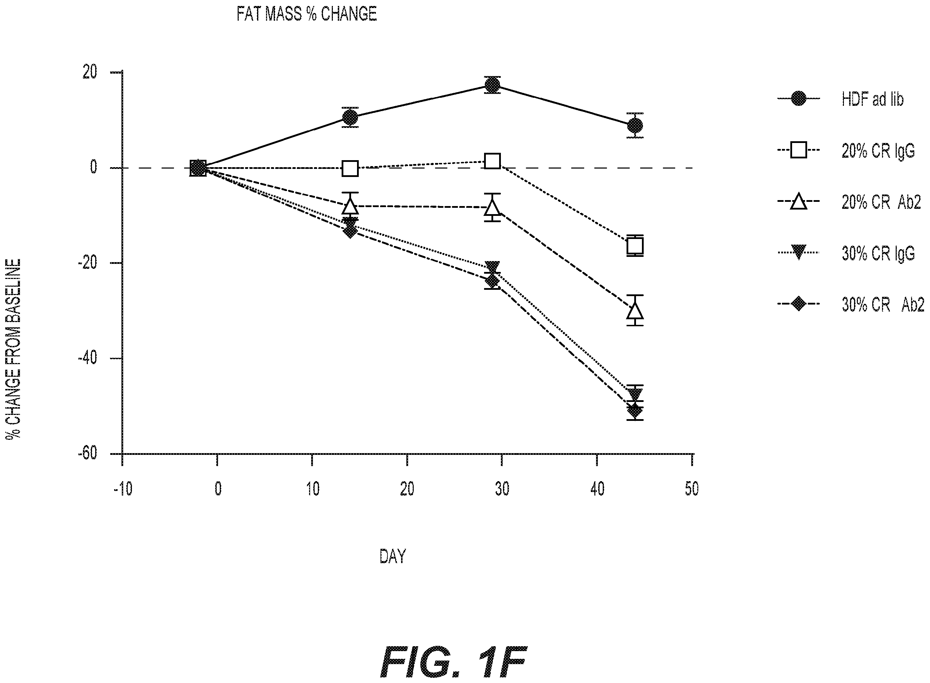

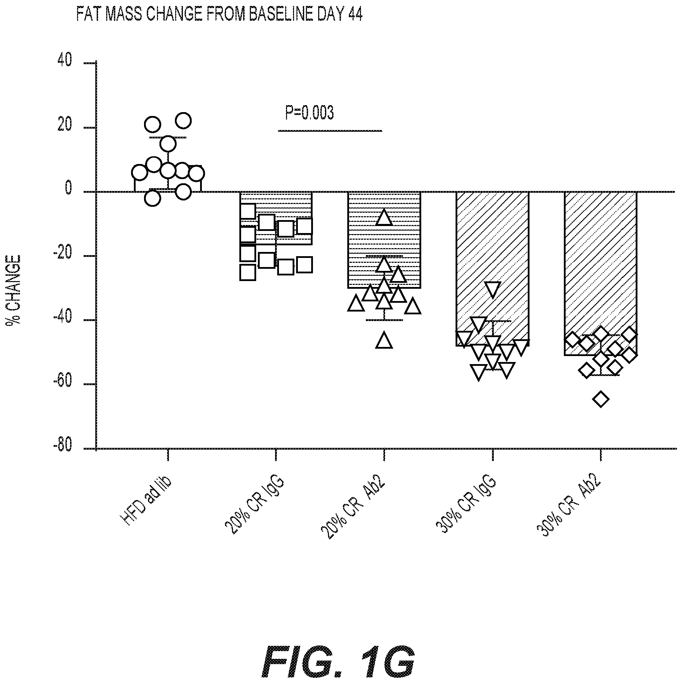

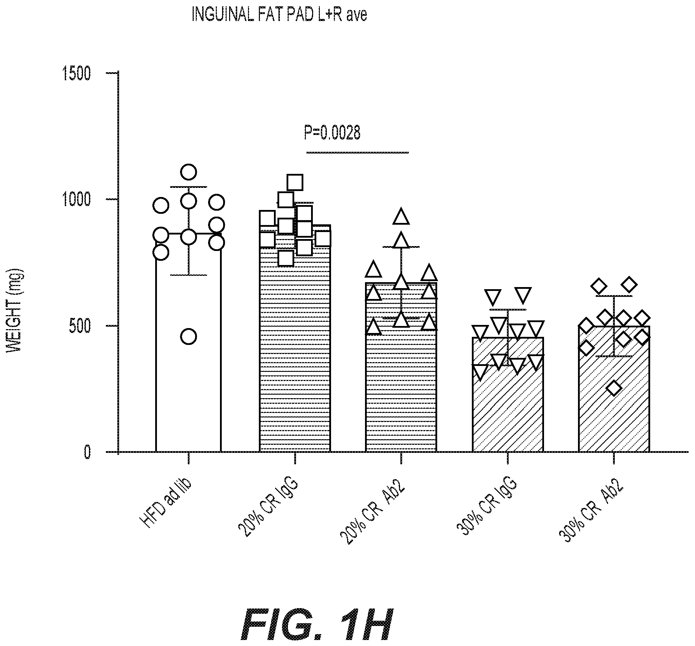

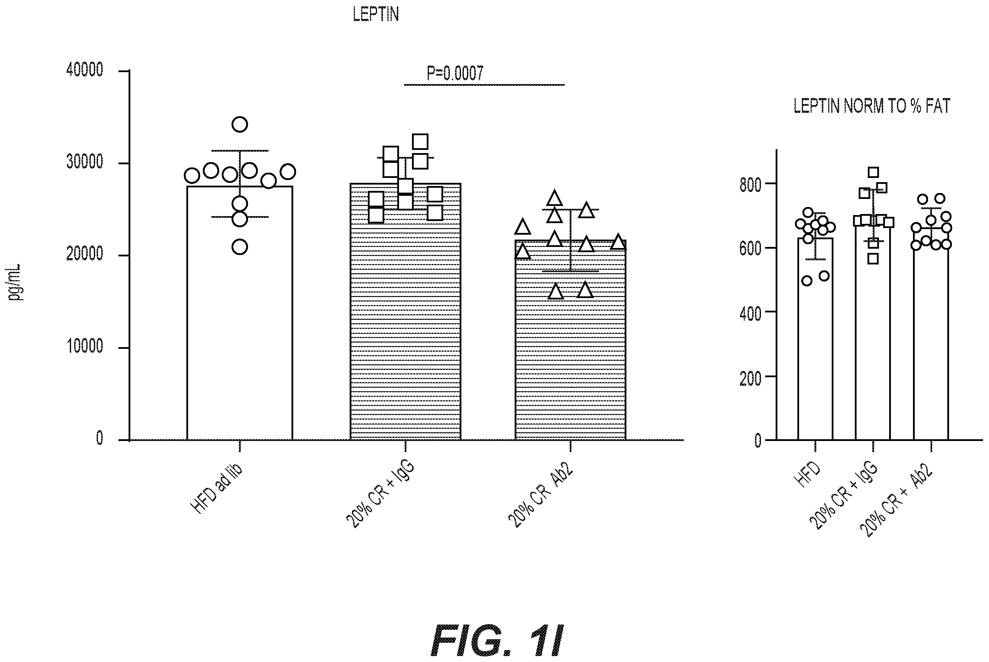

Results are shown in **FIG.** 1. With the exception of the animals fed a high fat diet ad lib, all of the all animals lost weight on the study, according to the whole-body measurements. The highest percentage of weight loss was observed in group 2 (30% CR, negative control antibody) and group 3 (30% CR, Ab2) (**FIG**. 1A). When looking at lean mass alone via qNMR, the highest percentage of muscle loss was observed in groups 2 (30% CR, negative control antibody) and 4 (20% CR, negative control antibody) showing that administration of the myostatin inhibitor Ab2 preserved some lean muscle mass over time compared to control (**FIG**. 1B). In both the 20% CR and 30% CR cohorts, administration of Ab2 resulted in a lower percentage of muscle loss compared to administration of the control IgG. A comparison of the right panel of **FIG.** 1A with **FIG.** 1B shows that when on a calorie restricted diet, anti-myostatin treatment resulted in less loss of muscle mass compared to a control.

**FIG.** 1C shows a comparison of the percent change of lean mass weights on day 44 as compared to baseline. Under conditions of either 20% or 30% calorie restriction, the percent loss of lean mass was highly significantly attenuated by Ab2 (p<0.0001). **FIG.** 1D (gastrocnemius weights, average of left & right gastrocnemius ("gastroc") shows that the gastrocnemius muscle mass did not decrease over time, even in the 30% CR cohort. This provides further evidence that myostatin inhibition is protective against muscle loss during calorie restriction. **FIG.** 1E shows the percent difference of these same gastrocnemius muscles compared to the gastrocnemius muscle weight of the control mice on calorie restriction but receiving the IgG control. Likewise, treatment with the anti-myostatin antibody highly significantly preserved gastrocnemius muscle weight as compared to IgG control (p<0.0001 for Group 5 (20% CR + Ab2); p=0.0002 for Group 3 (30% CR + Ab2)).

Notably, animals that were calorie restricted by 20% and treated with Ab2 (Group 5) showed a significant increase in the percentage of fat mass lost over time, measured by qNMR, compared to the animals receiving the IgG control (Group 4) **(****FIGs.** 1F and 1G). Similar results were obtained by measuring the average weight of the left and right inguinal fat pad **(****FIG.** 1H). Interestingly, while Ab2 helped to maintain lean mass in both CR cohorts, no significant difference was seen in the percentage of fat mass lost over time in the 30% cohort between Groups 2 and 3, possibly because the rapid weight loss in this cohort prevented Ab2 from having an additive effect with the 30% calorie reduction.

No difference was observed in blood glucose, insulin, or HbA1C between the groups. Additionally, Ab2 did not affect liver weight, triglyceride level, or cholesterol level (not shown).

At the end of the study serum levels of certain metabolic markers were measured in mice in the 20% cohort. Ten microliters of serum samples from Groups 1, 4, and 5 were analyzed in duplicate using the Milliplex^{®} Mouse Gut Hormone Magnetic Bead Panel (Cat. #MGTMAG-78K, EMD Millipore), which measures hormone regulators of appetite change with weight loss such as those released upon eating to reduce hunger (leptin, peptide YY, cholecystokinin, GLP-1), and those increased upon gastric emptying (ghrelin), as well as amylin, GIP, Insulin, and pancreatic peptide.

As shown in **FIG.** 1I (left panel), leptin levels were reduced by 22% in animals in Group 5 (20%CR + Ab2) as compared to animals in Group 4 (20% CR + IgG) and Group 1 (high fat diet ad lib). As leptin is produced by adipocytes, this may be due to the reduction in fat mass in the Group 5 animals compared to the control groups, resulting in fewer leptin-producing cells, and/or due to higher metabolic demand from greater muscle mass in the Group 5 animals compared to the control groups. When the results are normalized to the percentage of fat, Ab2 had no effect on leptin **FIG.** 1I (right panel).

This study demonstrated the potential beneficial effects of a myostatin inhibitor in conjunction with a reduced calorie diet by preventing muscle atrophy and loss allowing greater fat mass to be lost.

### Example 2: Effects of a myostatin inhibitor and a GLP-1 analog on body weight, body composition and liver lipid contents in a diet-induced obesity model

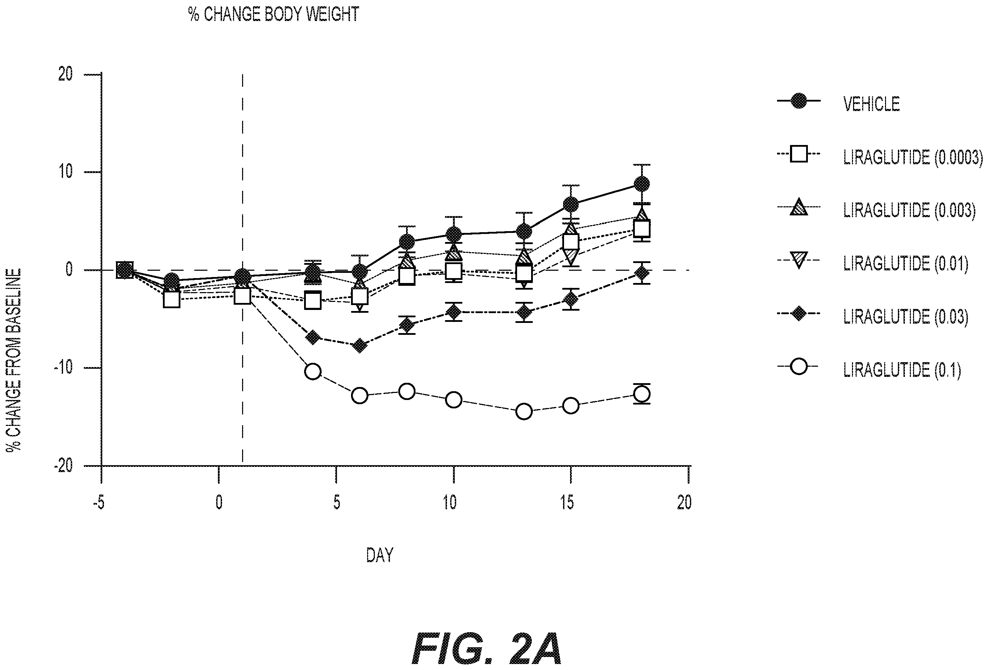

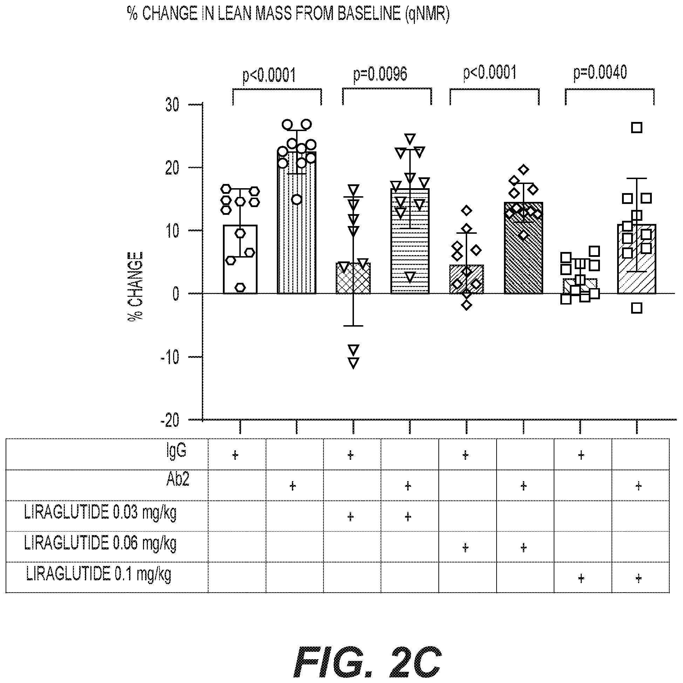

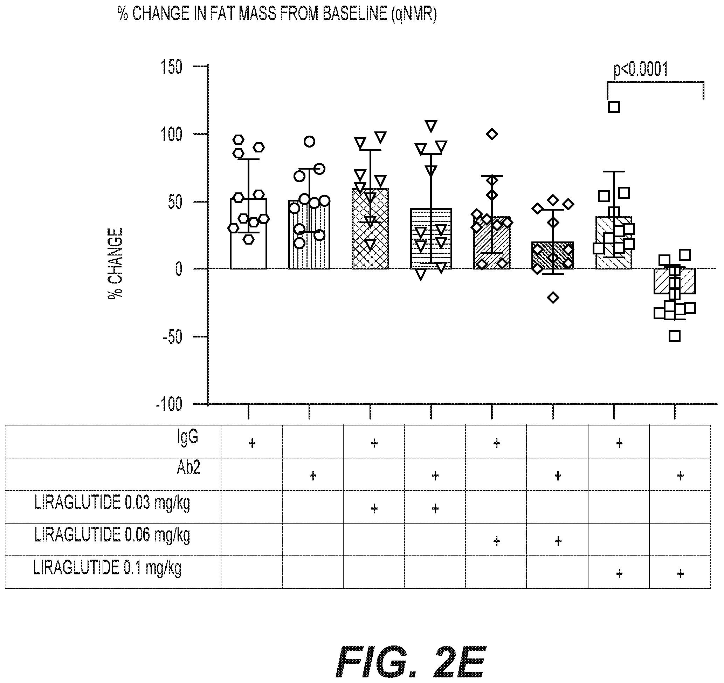

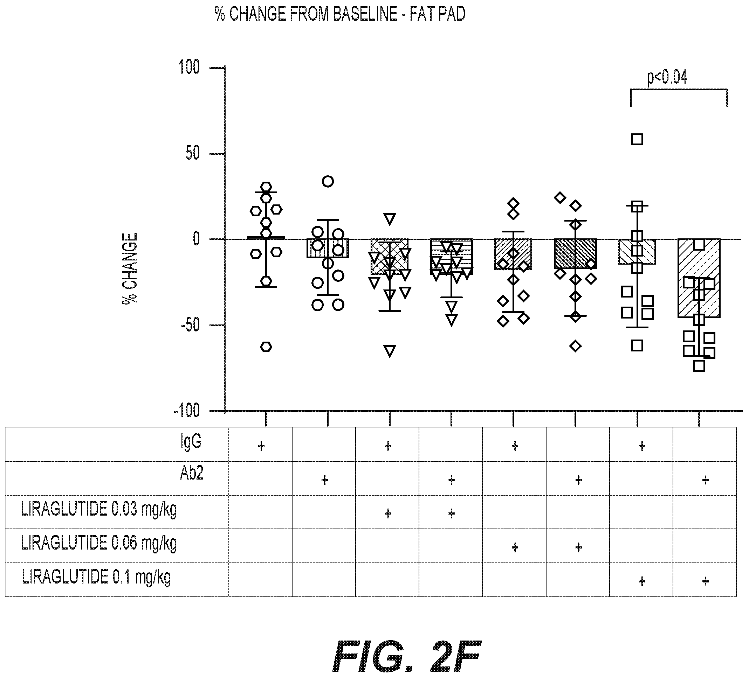

Evidence suggests that GLP-1 pathway activation may improve blood glucose control, pancreatic beta cell function, weight loss, and insulin sensitivity (see, e.g., Drugs Context. 2015; 4: 212283). To further evaluate the ability of a myostatin inhibitor (Ab2) to preserve lean muscle while losing fat mass, a study was designed in which a myostatin inhibitor was administered in conjunction with a GLP-1 pathway activator liraglutide (a GLP-1 analog). First, an 18-day dose determining study, as related to % change in body weight, was performed in C57BL/6NTac DIO mice to determine a useful treatment dose window for liraglutide in this mouse model, as shown in Table 8. **FIG.** 2A illustrates the dose-proportional change in weight observed over the 18-day study.

The C57BL/6NTac DIO (diet induced obesity) mouse model was chosen for this study as it is an accepted rodent species for nonclinical pharmacology testing of the choline deficient, L-amino acid-defined, high fat diet (CD-HFD) induced Non-Alcoholic Steatohepatitis (NASH). Eighty male mice at 16 weeks of age were fed the diet wherein 60% of their calories were from fat. This diet was maintained for 6-8 weeks prior to the start of the study; and the animals demonstrated increased weight, triglycerides, insulin resistance, and glucose intolerance.

All mice were maintained on the high fat diet throughout the study and received either control antibody migG1 or Ab2. On Day -2, the animals were weighed and assigned to treatment groups (Groups 1 - 8) in such a way as to generate groups with no significant differences with regards to body weight as recorded on Day -2.

Treatment groups 1-8 (5 mice each) are shown in Table 8.

**Table 8.**

| **Group** | **Antibody (20 mg/kg)** | **Liraglutide (mg/kg)** |
|---|---|---|
| 1 | mlgG1 | 0 |
| 2 | Ab2 | 0 |
| 3 | mlgG1 | 0.03 |
| 4 | Ab2 | 0.03 |
| 5 | mlgG1 | 0.06 |
| 6 | Ab2 | 0.06 |
| 7 | mlgG1 | 0.10 |
| 8 | Ab2 | 0.10 |

Mice were given control antibody mlgG1 or Ab2 by intraperitoneal injection once per week. Liraglutide was administered subcutaneously daily.

On Day 1 (prior to dose administration) body composition was measured by qNMR. Mice were placed into a plastic restrainer compatible with the body composition analysis machine Bruker NMR LF90II and body composition was assessed. Body composition analysis was also performed on days 15 and 28 of the study. Prior to dose administration of liraglutide on Study Day 28, non-fasted blood glucose was checked on all animals. Approximately 5 - 10 µL of blood was collected and blood glucose levels measured using a hand-held glucometer (Abbot Alpha Trak). At the end of the study, blood was collected via cardiac puncture. Left and right gastrocnemius muscles (soleus muscle intact) were collected from all groups and weighed. The left inguinal fat pad was collected and weighed. The entire liver was collected, blotted, and weighed and the right lateral lobe was cut into 3 mm thick slices for histological analysis.

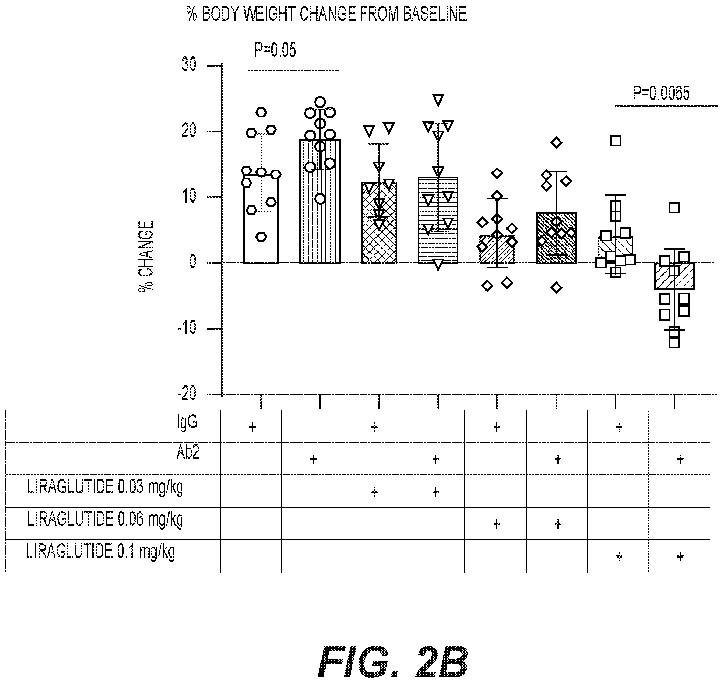

Results are shown in **FIGs.** 2B-2G. **FIG.** 2B shows the percentage of body weight change from baseline. The animals in Group 8 that received 0.1 mg/kg liraglutide + Ab2 lost a significant amount of weight as compared to the animals in Group 7 that received the control antibody (p=0.0065; **FIG.** 2B). Animals in Group 6, which received Ab2 and 0.06 mg/kg of liraglutide, had a higher percentage weight gain as compared to the animals in control Group 5, which received IgG instead of Ab2 (**FIG**. 2B). The weight gain observed in these animals is possibly due to an increase in muscle mass. Indeed, as seen in **FIG**. 2C, all mice treated with Ab2 showed a significant increase in the percentage change of lean mass as compared with mice treated with IgG control, thus demonstrating that Ab2 maintained lean mass better than liraglutide alone.

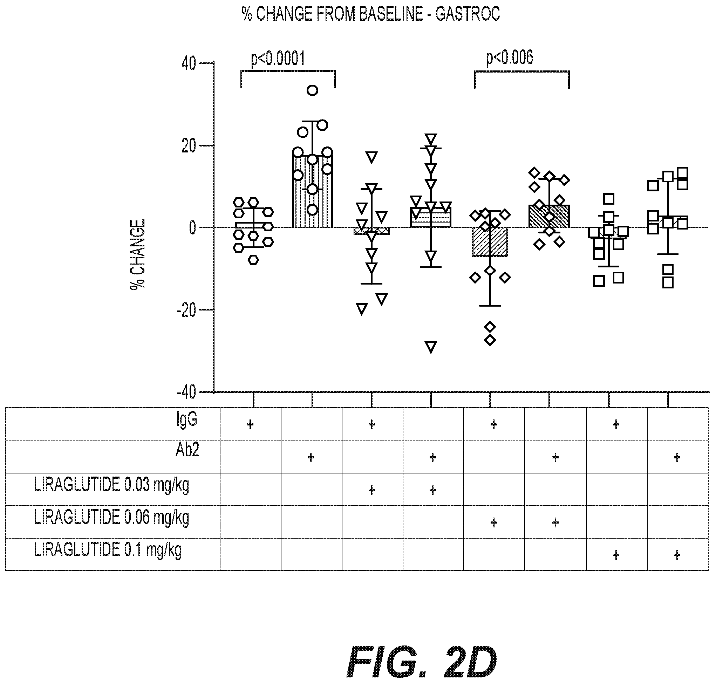

FIG. 2D demonstrates the effect of treating animals with both Ab2 and liraglutide on the gastrocnemius muscle. As expected, Ab2 significantly increased muscle mass when administered alone (p<0.0001). Mice treated with liraglutide at a dose of 0.06 mg/kg lost gastrocnemius muscle mass when administered a control IgG antibody. However, when treated with Ab2, these mice had increased muscle mass. The difference between gastrocnemius mass in the Ab2 treated mice and the control mice was highly significant (p<0.006).

In accordance with the observation of lean mass increase, animals receiving both Ab2 and liraglutide (Groups 4, 6, and 8) gained less fat mass as compared to the animals receiving control antibody. At the highest test dose of liraglutide, animals treated with the myostatin inhibitor Ab2 in conjunction with the GLP-1R agonist liraglutide actually lost weight over the course of the study, even while still on the high fat diet. This effect was observed in both percent change in overall fat mass from baseline as measured by qNMR (**FIG**. 2E) and in the percent change in the weight of the inguinal fat pad (**FIG**. 2F). As compared to treatment with IgG control, treatment with Ab2 significantly decreased fat mass (p<0.0001) and signficaintly decreased inguinal fat pad weight (p<0.04).

In summary, administering an inhibitor of the myostatin pathway and an activator of the GLP-1 pathway provided a strong beneficial effect on obesity. The myostatin inhibitor enhanced weight loss in animals on a high fat diet treated with the GLP-1 pathway activator. The myostatin inhibitor also maintained or increased lean muscle mass, including gastrocnemius mass, across the tested GLP-1 pathway activator dose range. The myostatin inhibitor supported fat mass loss with ascending doses of the GLP-1 pathway activator.

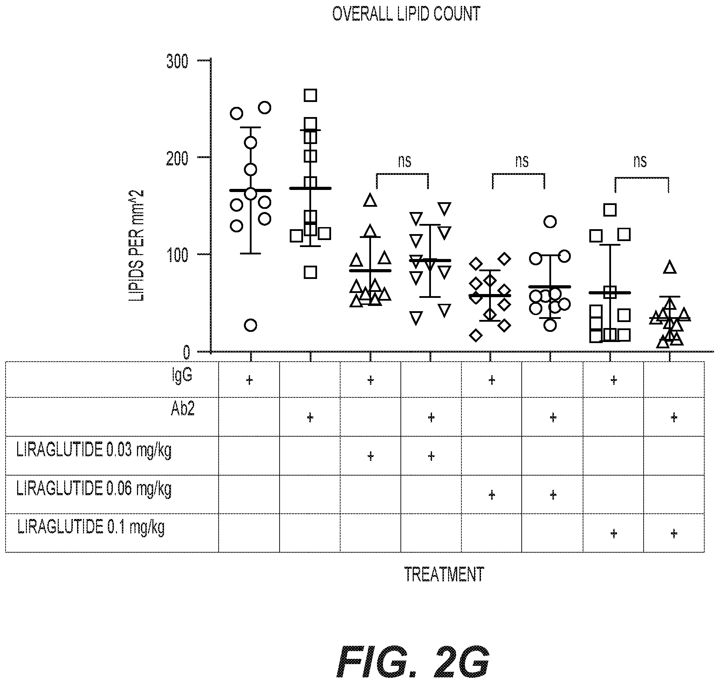

Livers from the mice were sectioned for histology and stained with hematoxylin and eosin, and lipid composition determined according to the method described in Nakano et al., J Clin Exp Hepatol. 2015 Sep;5(3):190-8, wherein unstained portions of the tissue section correspond to lipid droplets in the liver. Results are shown in **FIG.** 2G. A dose-dependent reduction in lipid droplets in the liver was seen in mice treated with liraglutide, regardless of whether combined with migG1 or Ab2. These results suggest that, in the DIO mouse model, liraglutide alone was sufficient to reduce fat droplets in the liver, and Ab2 did not have an additive effect to further reduce lipid droplet count. Without wishing to be bound by theory, it is possible that these effects are model-dependent or species-dependent.

### Example 3: Effects of a myostatin inhibitor on energy expenditure in a diet-induced obesity model

### Experiment 1

C57BL6/J DIO mice weighing approximately 42 g were maintained on a high fat diet comprising 60% fat for 10 weeks prior to beginning this study then divided into three groups of (1) high fat diet with no treatment; (2) high fat diet reduced in calories by 30%; and (3) high fat diet reduced in calories by 30% and treated with 20 mg/kg once weekly with the myostatin inhibiting antibody Ab2. Energy expenditure was calculated using Oxymax for Windows. O2 and CO2 measurements were taken every 25 to 40 minutes in a 24-hour cycle and raw data measurements were used to calculate AUC values.

Body weights of the mice were recorded throughout the study (n=10). Lean muscle and fat mass were measured by qNMR at baseline and multiple timepoints throughout the study. Inguinal fat pad weights were recorded at the end of the study, i.e., on day 44.

The animals in group 3 lost approximately 50% of their fat mass and about 15% of their lean mass and were in an active phase of weight loss during the study. Consistent with the results shown above, the calorie restricted animals lost both fat and lean mass. Energy expenditure is measured as heat and can be expressed as "calorific value" (CV), the relationship between heat and the volume of consumed oxygen. It is derived from empirical value and based on Equation 11 of The Elements of the Science of Nutrition, Grahm Lusk. CV=3.815 +1.232+RER. The rate of energy expenditure was then calculated using the subject's rate of oxygen consumption according to an equation standard for small to medium non-ruminant animals: Heat = CV + VO2.

Total energy expenditure (TEE) was determined by measuring oxygen consumption (VO₂) in metabolic cages at 24 hours. TEE can be calculated by dividing VO₂ by body weight. Lean mass and fat mass, however, do not contribute equally to TEE because lean mass is more metabolically active.

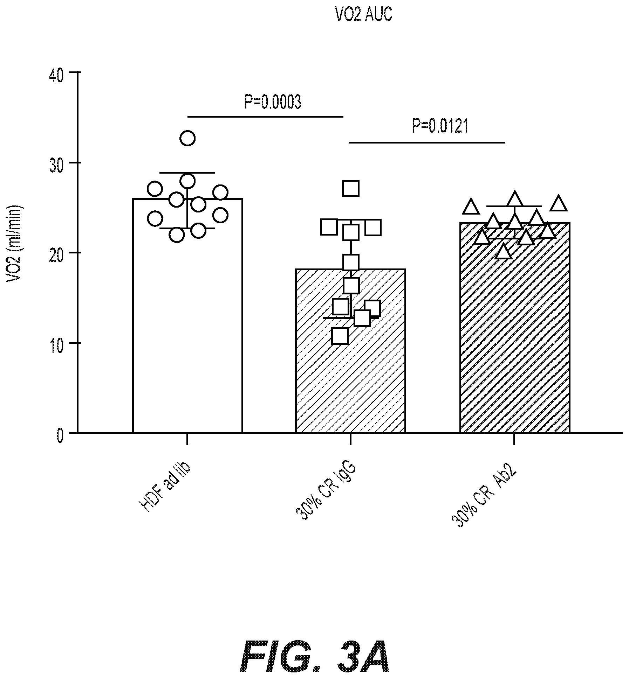

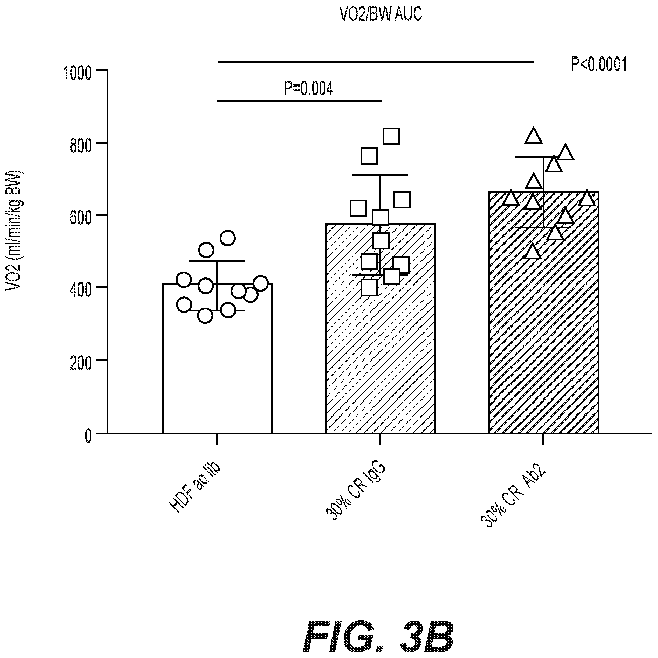

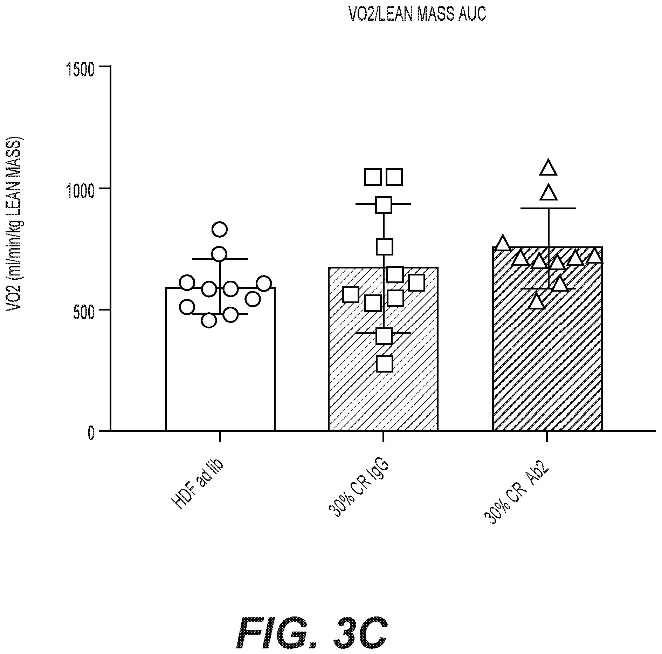

**FIG.** 3A shows the VO₂ of the 30% calorie restricted animals expressed in ml/min (data not normalized to body weight or lean mass). This calorie reduction lowered VO₂ (p=0.0003) as compared to the animals on the high fat diet that were not calorie restricted. Treatment with Ab2 increased VO₂ in the calorie restricted animals compared to the calorie restricted animals given a control antibody (p=0.0121). When the data shown in **FIG.** 3A were normalized to body weight, calorie reduction alone can be seen to increase VO₂ (p=0.004) and treatment with Ab2 further increases VO₂ (p<0.0001) compared to animals on the high fat diet **(****FIG.** 3B). When the data shown in **FIG.** 3A were normalized to lean mass **(****FIG.** 3C), the results showed that animals treated with Ab2 maintained VO₂ levels despite drastic losses in weight (approximately 50% weight loss). Similar changes were also observed with VCO₂ levels measured in the mice (data not shown).

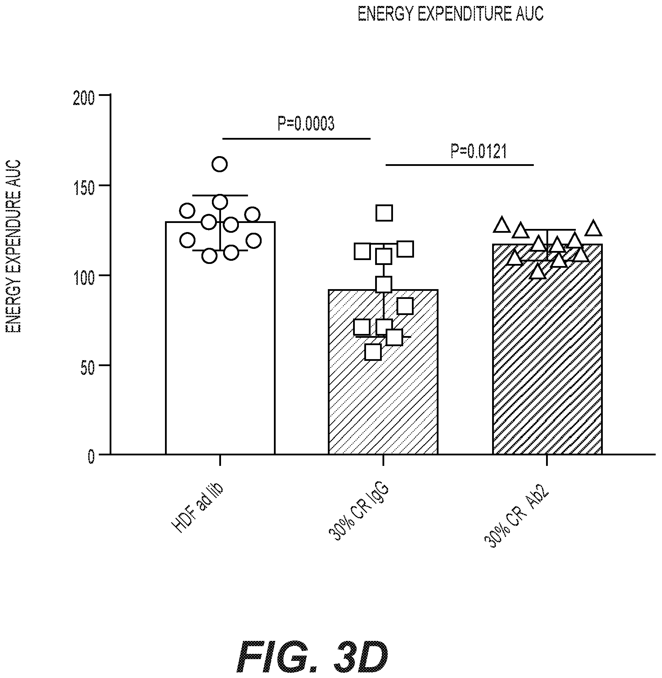

As shown in **FIG.** 3D, treatment with Ab2 increased energy expenditure in the 30% calorie restricted animals compared to untreated calorie restricted animals (p=0.0121), when measured as area under the curve (AUC). Energy expenditure was calculated using Oxymax for Windows. O₂ and CO₂ measurements were taken every 25 to 40 minutes in a 24-hour cycle and raw data measurements were used to calculate AUC values. The energy expenditure increase by the myostatin inhibitor was due to increased oxygen consumption, presumably due to the action of myostatin inhibition to preserve lean mass.

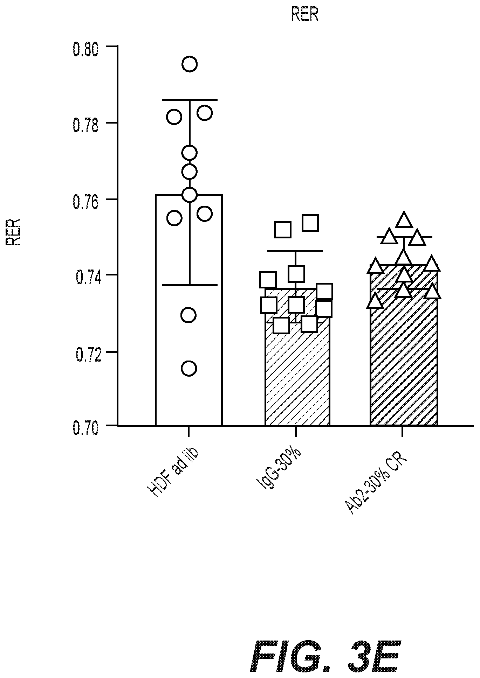

The Respiratory Exchange Ratio (RER) is the ratio between the metabolic production of CO₂ and the uptake of O₂. A low RER indicates that fat is the predominant fuel source, and a high RER indicates that carbohydrates are the predominant fuel source. **FIG.** 3E shows that, while calorie restriction lowered RER, indicating that calorie restriction increased lipid oxidation as a fuel source, RER remained unchanged by myostatin inhibition, i.e., equal changes in VO₂ and VCO₂ were observed.

In summary, the Ab2 myostatin inhibitor increased VO₂ consumption, VCO₂ output and total energy expenditure in mice that were calorie restricted by 30%. No change in VO₂ or VCO₂ were observed when normalized to lean mass, indicating that the observed increases are due to increased metabolic demand from a greater muscle mass. The antibody treatment did not change the RER in the calorie restricted mice, indicating that fuel type utilization, i.e., lipid vs. carbohydrate, was not affected by specific myostatin inhibition.

### Experiment 2

C57BL/6/ DIO mice weighing approximately 36 g were maintained on a high fat diet comprising 60% fat for 14 weeks prior to beginning the study then divided into three groups of ten mice each and fed (1) a high fat diet ad lib; (2) a high fat diet reduced in calories by 20% and treated once weekly with 20 mg/kg of a placebo control monoclonal IgG; and (3) a high fat diet reduced in calories by 20% and treated once weekly with 20 mg/kg of the myostatin inhibiting antibody Ab2. Mice were treated with control IgG antibody or Ab2 for six weeks. Body weight was measured three times per week, body composition was measured by qNMR pre-dose at week 0 and at weeks 2, 4 and 6. The animals were placed in metabolic cages at the end of the study for 72 hours. The animals were allowed to acclimate to the metabolic cage for the first 24 hours, following which metabolic cage data was collected for the next 48 hours. The left and right gastrocnemius muscles were then weighed, and the left gastrocnemius muscle was snap frozen. Inguinal fat pads were weighed, and the left inguinal fat pad was snap frozen.

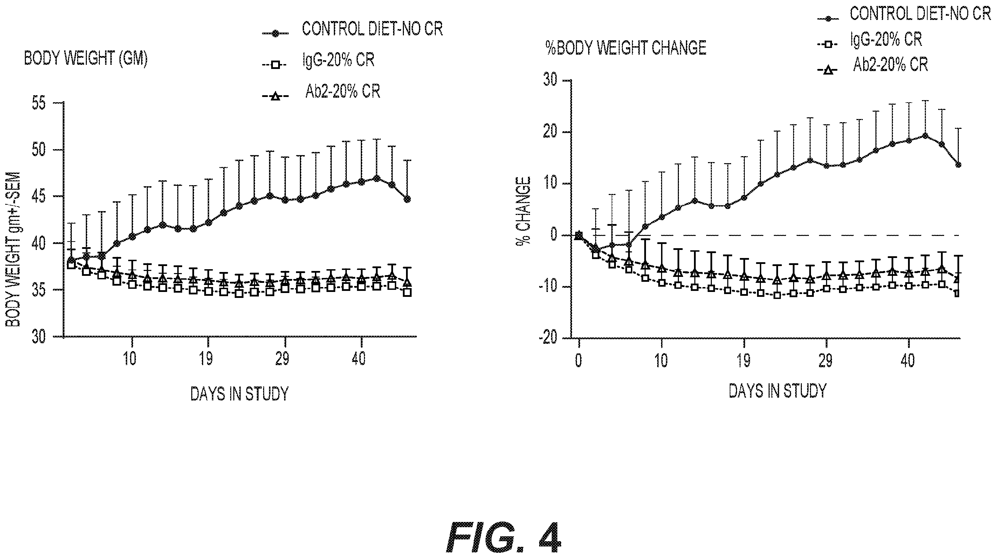

Mice that consumed 20% fewer calories per day lost weight, fat, and lean mass. There was no significant difference in the amount of weight lost or the percent body weight change between the calorie restricted mice treated with Ab2 and control IgG. Mice in Experiment 2 started with lower body weights compared to those in Experiment 1 and body weights of these mice in Experiment 2 plateaued rapidly **(****FIG.** 4). Thus, Experiment 2 was conducted in the plateau, or equilibrium, phase of body weight loss.

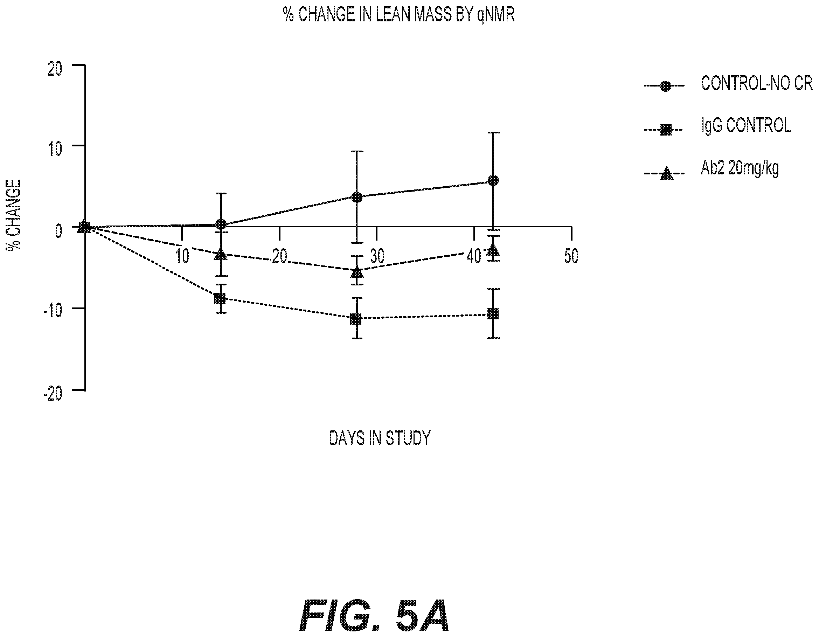

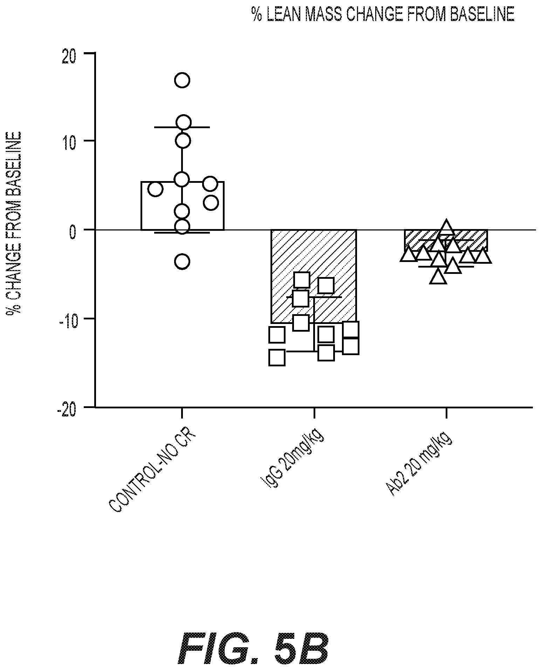

The myostatin inhibitor Ab2 preserved lean body mass, measured by qNMR, in the animals that were calorie restricted by 20%. As shown in **FIG.** 5A, lean body mass increased in animals fed the high fat diet ad lib and decreased in the calorie restricted animals. Consistent with results presented above, the anti-myostatin antibody preserved lean body mass. **FIG.** 5B shows the same data expressed as a change from the baseline mass. The animals treated with the anti-myostatin antibody Ab2 lost significantly less lean body mass than the animals treated with the control antibody.

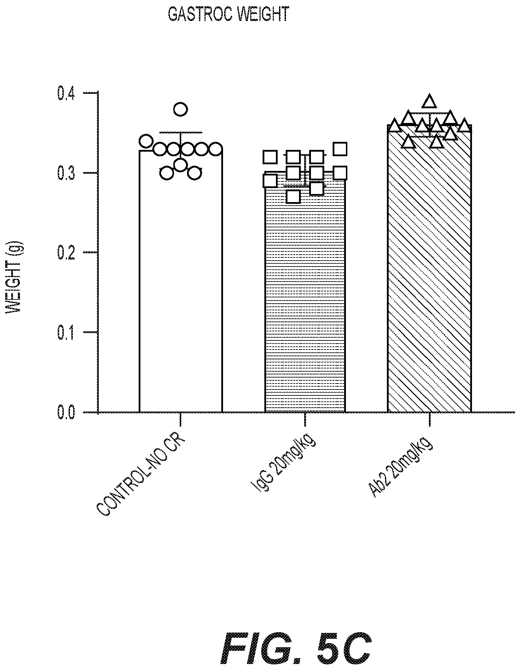

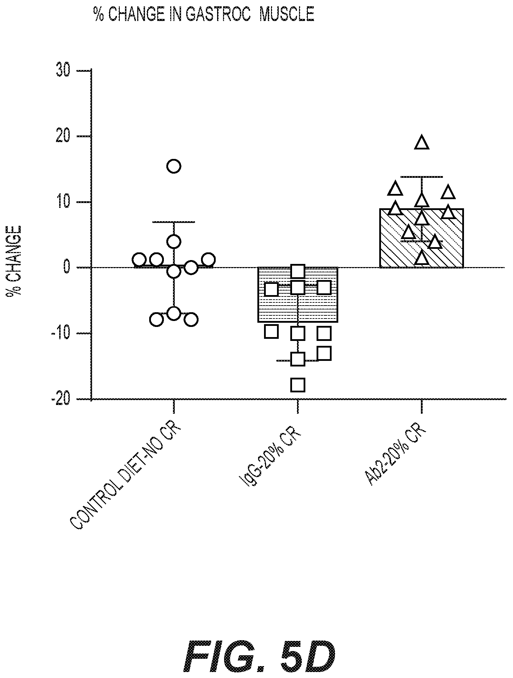

The myostatin inhibitor Ab2 also increased gastrocnemius muscle weight in the animals that were calorie restricted by 20%. As shown in **FIG.** 5C, gastrocnemius weight increased in animals treated with Ab2 compared to those treated with the control antibody. **FIG.** 5D shows the same data expressed as a change from the baseline mass. The calorie restricted animals treated with the control antibody lost gastrocnemius muscle weight while the calorie restricted animals treated with Ab2 gained gastrocnemius muscle weight.

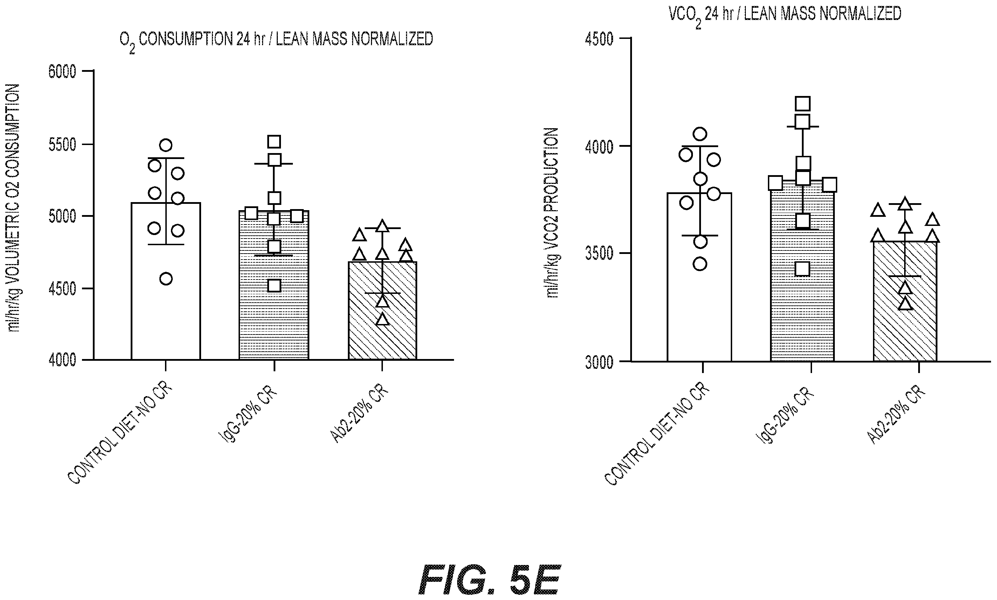

The myostatin inhibitor Ab2 decreased energy expenditure as measured by oxygen consumption and carbon dioxide output compared to mice treated with control antibody in the animals that were calorie restricted by 20%. The control antibody did not affect either O₂ consumption or CO₂ output compared to animals that were not calorie restricted. **FIG.** 5E shows these decreases as measured by area under the curve and normalized to lean mass.

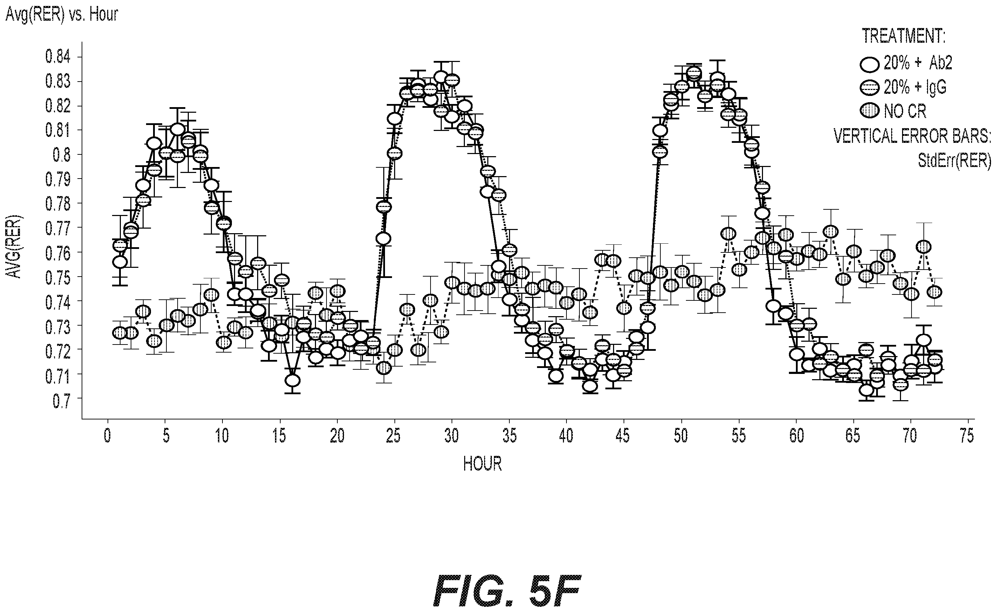

While the area under the curve data indicated that Ab2 decreased energy output, a detailed time analysis suggested that the energy expenditure was similar between the calorie restricted animals treated with Ab2 and control antibody. **FIG.** 5F shows the average respiratory exchange ratio as a function of time over the course of the 72 hours that the mice spent in the metabolic cage. Compared to mice that were not calorie restricted, the calorie restricted mice displayed a marked circadian rhythm that cycled between nocturnal and diurnal periods. No difference was detected between the mice treated with Ab2 and those administered the control antibody.

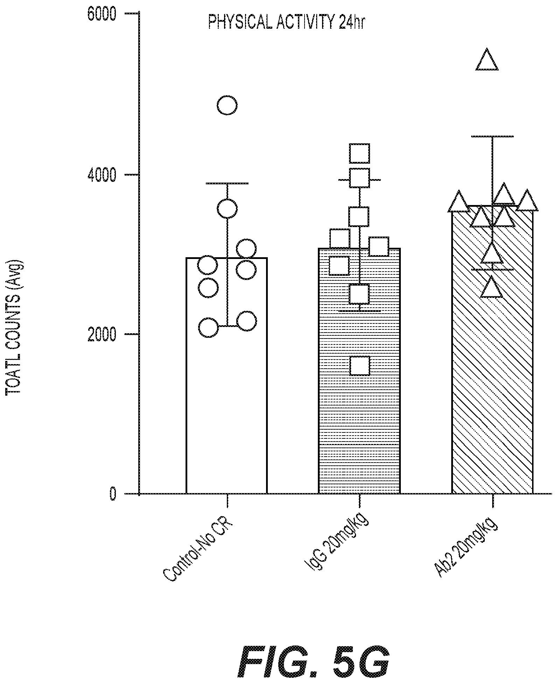

Physical activity levels of the mice were determined by measuring the number of times the mice crossed infrared beams inside the metabolic cages. During the 72-hour period, the physical activity of mice treated with Ab2 showed a trend toward increased activity during all 72 hours in the cages as compared to the physical activity of mice treated with control antibody. This trend in increased physical activity was also clear when compared to the mice that were not calorie restricted **(****FIG.** 5G). This trend in increased activity in the Ab2-treated mice was observed in both nocturnal and diurnal periods.

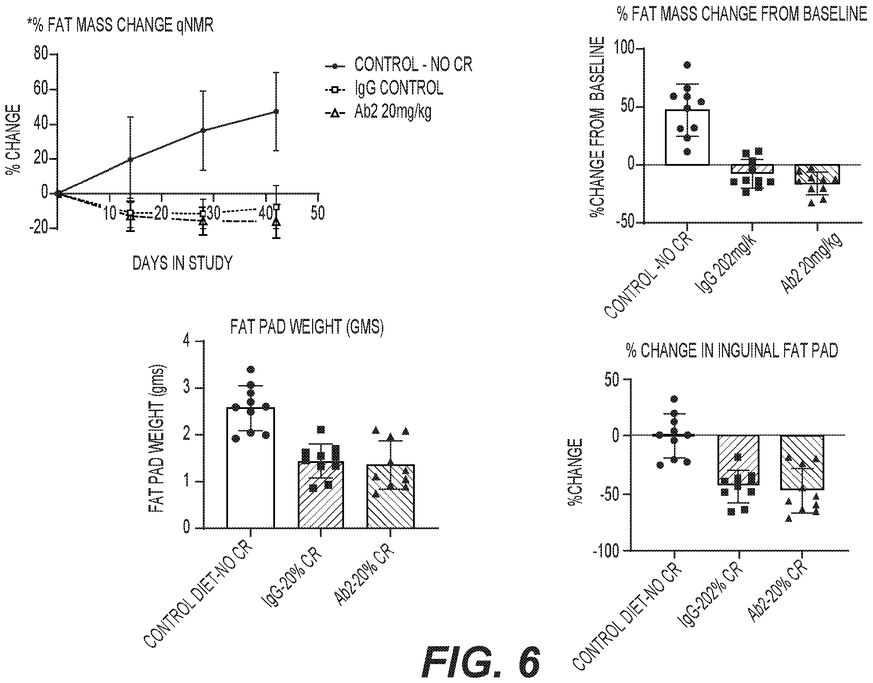

As expected, caloric restriction by 20% led to a significant reduction in fat mass . The animals treated with Ab2 showed a trend towards fat mass loss compared to the animals treated with control antibody when measured as percent fat mass change from baseline, fat pad weight and percent change in the inguinal fat pad (FIG. 6).

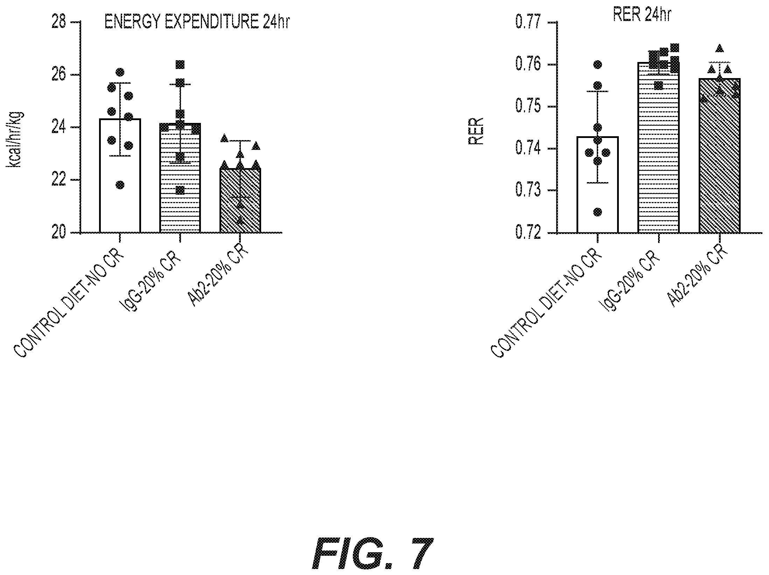

**FIG.** 7 shows the energy expenditure parameters of the mice that were 20% calorie restricted and treated with either the myostatin inhibitor antibody Ab2 or a control antibody. Ab2 decreased the energy expenditure of the 20% calorie restricted mice and had little or no effect on the respiratory exchange rate.

In summary, Experiment 2 demonstrated the ability of the myostatin inhibiting antibody Ab2 to maintain lean mass. In animals that were moderately (20%) calorie restricted, myostatin inhibition supported the maintenance of lean mass but did not dramatically reduce body weight compared to the control antibody, although a trend towards reducing fat mass was observed. The treated animals showed a trend towards greater activity. One difference between Experiment 1 and Experiment 2 is that the animals in Experiment 1 were subjected to 30% calorie restriction and animals in Experiment 2 were subjected to 20% calorie restriction. Another difference between Experiment 2 and Experiment 1 is that Experiment 2 used animals that were weight-stable (i.e., not in an active weight loss phase), whereas Experiment 1 used animals that were in active weight loss. Thus, the parameters measured in Experiment 2, e.g., energy expenditure, were measured during weight maintenance as opposed to the parameters measured in Experiment 1, which were measured during active weight loss.

Treatment with the myostatin inhibiting antibody preserved lean mass in the animals that were 20% and 30% calorie restricted. Treatment with the myostatin inhibiting antibody increased fat mass loss in the animals that were 20% calorie restricted but not those that were 30% calorie restricted, the latter of which were in an active stage of weight loss and under extreme calorie restriction. These results demonstrate that myostatin inhibition supports lean mass maintenance under calorie restriction and supports additional fat loss under moderate calorie restriction.

By preventing muscle loss during dieting, the myostatin inhibitor helped to block a compensatory decline in basal metabolic rate by maintaining muscle mass. In conclusion, treatment with a myostatin inhibitor preserves muscle when combined with moderate diet in DIO mice.

## Claims

1. A myostatin-selective inhibitor for use in the treatment of a metabolic disorder in a subject, wherein the treatment comprises administration of the myostatin-selective inhibitor to the subject in conjunction with a GLP-1 analog, wherein the myostatin-selective inhibitor and the GLP-1 analog are administered in amounts sufficient to treat the metabolic disorder; wherein, optionally, the metabolic disorder is type 2 diabetes mellitus (T2D), obesity, obesity associated with T2D, or metabolic syndrome,
wherein the myostatin-selective inhibitor is a myostatin-selective antibody or antigen-binding fragment thereof that binds to myostatin and exhibits no detectable binding or potency towards GDF11 or Activin A; and
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117).

2. A GLP-1 analog for use in the treatment of a metabolic disorder in a subject, wherein the treatment comprises administration of the GLP-1 analog to the subject in conjunction with a myostatin-selective inhibitor, wherein the myostatin-selective inhibitor and the GLP-1 analog are administered in amounts sufficient to treat the metabolic disorder; wherein, optionally, the metabolic disorder is type 2 diabetes mellitus (T2D), obesity, obesity associated with T2D, or metabolic syndrome,
wherein the myostatin-selective inhibitor is a myostatin-selective antibody or antigen-binding fragment thereof that binds to myostatin and exhibits no detectable binding or potency towards GDF11 or Activin A; and
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117).

3. A myostatin-selective inhibitor and a GLP-1 analog for use in the treatment of a metabolic disorder in a subject, wherein the treatment comprises administration of the myostatin-selective inhibitor to the subject in conjunction with the GLP-1 analog, wherein the myostatin-selective inhibitor and the GLP-1 analog are administered in amounts sufficient to treat the metabolic disorder; wherein, optionally, the metabolic disorder is type 2 diabetes mellitus (T2D), obesity, obesity associated with T2D, or metabolic syndrome,
wherein the myostatin-selective inhibitor is a myostatin-selective antibody or antigen-binding fragment thereof that binds to myostatin and exhibits no detectable binding or potency towards GDF11 or Activin A; and
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117).

4. The myostatin-selective inhibitor and/or GLP-1 analog for use according to any one of claims 1-3, wherein:
a. the GLP-1 analog is an Fc conjugate or multi-functional molecule comprising the GLP-1 analog; and/or
b. the GLP-1 analog is semaglutide, exenatide ER, liraglutide, lixisenatide, tirzepatide, XW003, Noiiglutide, MEDI0382, dulaglutide, albiglutide, AMG 133, CT-388, cagrilintide/semaglutide combination, DD01, ALT-801, or IBI362.

5. The myostatin-selective inhibitor and/or GLP-1 analog for use according to any one of claims 1-4, wherein:
a. the myostatin-selective inhibitor is trevogrumab, GYM329, MST1032, or an antibody or antigen-binding fragment that competes or cross-competes with trevogrumab, GYM329, MST1032, or apitegromab; and/or
b. the myostatin-selective inhibitor comprises at least one amino acid mutation that increases binding affinity for FcRn.

6. The myostatin-selective inhibitor and/or GLP-1 analog for use according to any one of claims 1-5, wherein the myostatin-selective inhibitor is an antibody or antigen-binding fragment thereof that binds an epitope that comprises one or more amino acid resides of KALDEN (SEQ ID NO: 118) and/or FVQILRLIKPMKDGTRYTGIRSLK (SEQ ID NO: 57); wherein the antibody is not apitegromab.

7. The myostatin-selective inhibitor and/or GLP-1 analog for use according to any one of claims 1-5, wherein the myostatin-selective inhibitor is an antibody or antigen-binding fragment thereof that binds selectively to pro- and/or latent-myostatin; wherein, optionally, the antibody or antigen-binding fragment thereof does not bind to mature myostatin.

8. The myostatin-selective inhibitor and/or GLP-1 analog for use according to any one of claims 1-7, wherein:
a. the subject is an adult subject with a BMI of 25 or greater or a child or adolescent aged 2-19 years (e.g., 12 years or older, e.g., 12-17 years) with a BMI of at or above the 85th percentile on the CDC growth charts;
b. the subject has received at least one therapy for obesity, overweight, or a weight-related condition, but failed to achieve an intended clinical outcome, wherein the intended clinical outcome is a reduction of body weight by at least 5% or 10% as compared to baseline body weight prior to the start of the at least one therapy, wherein, optionally, the subject failed to perform or continue with a diet regimen and/or an exercise regimen as part of weight management;
c. the subject has one or more of central adiposity, cardiovascular disease, kidney disease, fatty liver disease, sleep apnea, high blood pressure, high blood triglyceride levels, high blood cholesterol levels, low high-density lipoprotein (HDL) levels, and a hemoglobin A1c (HbA1c) level of 6% or greater (e.g., 6.5%-10%), wherein, optionally, the subject has a body mass index (BMI) of greater than 25; wherein, further optionally, the subject has a BMI of between 28 and 40, inclusive of endpoints; and/or
d. the subject is on a calorie restriction diet and/or an exercise regimen.

9. The myostatin-selective inhibitor and/or GLP-1 analog for use according to any one of claims 1-8, wherein:
a. the myostatin-selective inhibitor and GLP-1 analog are administered concurrently, simultaneously, or sequentially; wherein, optionally, the myostatin-selective inhibitor and GLP-1 analog are formulated as a single formulation, as part of a single molecular construct, or as separate formulations; and/or
b. the myostatin-selective inhibitor is administered subcutaneously or intravenously.

10. The myostatin-selective inhibitor and/or GLP-1 analog for use according to any one of claims 1-9, wherein:
a. the treatment reduces triglyceride, total cholesterol, LDL cholesterol, and/or non-fasted glucose levels relative to a baseline level as measured prior to starting the treatment, optionally wherein the treatment reduces triglyceride, total cholesterol, LDL cholesterol, and/or non-fasted glucose levels relative to treatment with a GLP-1 analog alone; and/or
b. the treatment reduces fat mass, preserves lean body mass, or improves insulin sensitivity and/or insulin secretion relative to a baseline level as measured prior to starting the treatment, optionally wherein the treatment improves insulin sensitivity and/or insulin secretion relative to treatment with a GLP-1 analog alone.

11. A composition comprising a myostatin-selective inhibitor and a GLP-1 analog;
wherein the myostatin-selective inhibitor is a myostatin-selective antibody or antigen-binding fragment thereof that binds to myostatin and exhibits no detectable binding or potency towards GDF11 or Activin A; and
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117), optionally wherein the GLP-1 analog is semaglutide, exenatide ER, liraglutide, lixisenatide, tirzepatide, XW003, Noiiglutide, MEDI0382, dulaglutide, or albiglutide.

12. The composition according to claim 11, wherein:
a. the myostatin-selective inhibitor is an antibody or antigen-binding fragment thereof that binds an epitope that comprises one or more amino acid resides of KALDEN (SEQ ID NO: 118) and/or FVQILRLIKPMKDGTRYTGIRSLK (SEQ ID NO: 57); wherein the antibody is not apitegromab; or
b. the myostatin-selective inhibitor is an antibody or antigen-binding fragment thereof that competes or cross-competes with apitegromab for antigen binding, wherein the antibody is not apitegromab; wherein, optionally, the antibody or antigen-binding fragment comprises an HCDR3 paratope containing up to two amino acid substitutions as compared to SEQ ID NO: 10; wherein, further optionally, the antibody or antigen-binding fragment binds to an epitope comprising one or more of the amino acid residues F147, Q149, L151, Y186, S168, K170, K205, and/or L207, as numbered according to SEQ ID NO: 52.

13. A myostatin-selective inhibitor for use as an adjunct therapy for weight management in the treatment of obesity or overweight in a subject, wherein the therapy comprises administration of the myostatin-selective inhibitor according to any one of claims 1-10 in an amount effective to treat obesity or overweight, wherein the subject is treated with a GLP-1 analog;
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117);
wherein, optionally, the subject has at least one weight-related condition; wherein, further optionally, the subject has been treated with the GLP-1 analog for at least 3 months.

14. A GLP-1 analog for use as an adjunct therapy for weight management in the treatment of obesity or overweight in a subject, wherein the treatment comprises administration of an effective amount of the GLP-1 analog to treat obesity or overweight, wherein the subject is treated with the myostatin-selective inhibitor according to any one of claims 1-10;
wherein the GLP-1 analog is capable of binding to and activating the GLP-1 receptor and comprises the amino acid sequence EGTFTSD (SEQ ID NO: 116) and/or the amino acid sequence HXXGXFTXD, wherein X is any amino acid residue (SEQ ID NO: 117);
wherein, optionally, the subject has at least one weight-related condition; wherein, further optionally, the subject has been treated with the myostatin-selective inhibitor for at least 3 months.

15. The myostatin-selective inhibitor and/or GLP-1 analog for use according to any one of claims 1-5, 7-10, 13 or 14, or the composition according to claim 11, wherein the myostatin-selective inhibitor is not apitegromab.

## Patentansprüche

1. Myostatin-selektiver Inhibitor zur Verwendung bei der Behandlung einer metabolischen Störung in einem Subjekt, wobei die Behandlung das Verabreichen des Myostatin-selektiven Inhibitors an das Subjekt in Verbindung mit einem GLP-1-Analogon umfasst, wobei der Myostatin-selektive Inhibitor und das GLP-1-Analogon in Mengen verabreicht werden, die dazu ausreichen, die metabolische Störung zu behandeln; wobei optional die metabolische Störung Diabetes Mellitus Typ 2 (T2D), Fettleibigkeit, Fettleibigkeit in Verbindung mit T2D oder metabolisches Syndrom ist,
wobei der Myostatin-selektive Inhibitor ein Myostatin-selektiver Antikörper oder ein antigenbindendes Fragment davon ist, der/das an Myostatin bindet und keine nachweisbare Bindung oder Potenz in Bezug auf GDF11 oder Activin A aufweist; und
wobei das GLP-1-Analogon in der Lage ist, an den GLP-1-Rezeptor zu binden und diesen zu aktivieren und die Aminosäuresequenz EGTFTSD (SEQ ID NO: 116) und/oder die Aminosäuresequenz HXXGXFTXD, wobei X ein beliebiger Aminosäurerest ist (SEQ ID NO: 117), umfasst.

2. GLP-1-Analogon zur Verwendung bei der Behandlung einer metabolischen Störung in einem Subjekt, wobei die Behandlung das Verabreichen des GLP-1-Analogons an das Subjekt in Verbindung mit einem Myostatin-selektiven Inhibitor umfasst, wobei der Myostatin-selektive Inhibitor und das GLP-1-Analogon in Mengen verabreicht werden, die dazu ausreichen, die metabolische Störung zu behandeln; wobei optional die metabolische Störung Diabetes Mellitus Typ 2 (T2D), Fettleibigkeit, Fettleibigkeit in Verbindung mit T2D oder metabolisches Syndrom ist,
wobei der Myostatin-selektive Inhibitor ein Myostatin-selektiver Antikörper oder ein antigenbindendes Fragment davon ist, der/das an Myostatin bindet und keine nachweisbare Bindung oder Potenz in Bezug auf GDF11 oder Activin A aufweist; und
wobei das GLP-1-Analogon in der Lage ist, an den GLP-1-Rezeptor zu binden und diesen zu aktivieren und die Aminosäuresequenz EGTFTSD (SEQ ID NO: 116) und/oder die Aminosäuresequenz HXXGXFTXD, wobei X ein beliebiger Aminosäurerest ist (SEQ ID NO: 117), umfasst.

3. Myostatin-selektiver Inhibitor und GLP-1-Analogon zur Verwendung bei der Behandlung einer metabolischen Störung in einem Subjekt, wobei die Behandlung das Verabreichen des Myostatin-selektiven Inhibitors an das Subjekt in Verbindung mit dem GLP-1-Analogon umfasst, wobei der Myostatin-selektive Inhibitor und das GLP-1-Analogon in Mengen verabreicht werden, die dazu ausreichen, die metabolische Störung zu behandeln; wobei optional die metabolische Störung Diabetes Mellitus Typ 2 (T2D), Fettleibigkeit, Fettleibigkeit in Verbindung mit T2D oder metabolisches Syndrom ist,
wobei der Myostatin-selektive Inhibitor ein Myostatin-selektiver Antikörper oder ein antigenbindendes Fragment davon ist, der/das an Myostatin bindet und keine nachweisbare Bindung oder Potenz in Bezug auf GDF11 oder Activin A aufweist; und
wobei das GLP-1-Analogon in der Lage ist, an den GLP-1-Rezeptor zu binden und diesen zu aktivieren und die Aminosäuresequenz EGTFTSD (SEQ ID NO: 116) und/oder die Aminosäuresequenz HXXGXFTXD, wobei X ein beliebiger Aminosäurerest ist (SEQ ID NO: 117), umfasst.

4. Myostatin-selektiver Inhibitor und/oder GLP-1-Analogon zur Verwendung nach einem der Ansprüche 1-3, wobei:
a. das GLP-1-Analogon ein Fc-Konjugat oder ein multifunktionales Molekül, umfassend das GLP-1-Analogon ist; und/oder
b. das GLP-1-Analogon Semaglutid, Exenatid ER, Liraglutid, Lixisenatid, Tirzepatid, XW003, Noiiglutid, MEDI0382, Dulaglutid, Albiglutid, AMG 133, CT-388, Cagrilintid/Semaglutid-Kombination, DD01, ALT-801 oder IBI362 ist.

5. Myostatin-selektiver Inhibitor und/oder GLP-1-Analogon zur Verwendung nach einem der Ansprüche 1-4, wobei:
a. der Myostatin-selektive Inhibitor Trevogrumab, GYM329, MST1032 oder ein Antikörper oder ein antigenbindendes Fragment ist, der/das mit Trevogrumab, GYM329, MST1032 oder Apitegromab konkurriert oder kreuzkonkurriert; und/oder
b. der Myostatin-selektive Inhibitor mindestens eine Aminosäuremutation umfasst, die die Bindungsaffinität für FcRn erhöht.

6. Myostatin-selektiver Inhibitor und/oder GLP-1-Analogon zur Verwendung nach einem der Ansprüche 1-5, wobei der Myostatin-selektive Inhibitor ein Antikörper oder ein antigenbindendes Fragment davon ist, der/das an ein Epitop bindet, das einen oder mehrere Aminosäurereste aus KALDEN (SEQ ID NO: 118) und/oder FVQILRLIKPMKDGTRYTGIRSLK (SEQ ID NO: 57) umfasst; wobei der Antikörper nicht Apitegromab ist.

7. Myostatin-selektiver Inhibitor und/oder GLP-1-Analogon zur Verwendung nach einem der Ansprüche 1-5, wobei der Myostatin-selektive Inhibitor ein Antikörper oder ein antigenbindendes Fragment davon ist, der/das selektiv an Pro- und/oder latentes Myostatin bindet; wobei optional der Antikörper oder das antigenbindende Fragment davon nicht an reifes Myostatin bindet.

8. Myostatin-selektiver Inhibitor und/oder GLP-1-Analogon zur Verwendung nach einem der Ansprüche 1-7, wobei:
a. das Subjekt ein erwachsenes Subjekt mit einem BMI von mindestens 25 oder ein Kind oder ein Jugendlicher im Alter von 2-19 Jahren (z. B. mindestens 12 Jahre alt, z. B. 12-17 Jahre alt) mit einem BMI auf mindestens dem 85. Perzentil der CDC-Wachstumstabellen ist;
b. das Subjekt mindestens eine Therapie für Fettleibigkeit, Übergewicht oder eine gewichtsbezogene Erkrankung erhalten hat, ohne aber ein beabsichtigtes klinisches Ergebnis zu erzielen, wobei das beabsichtigte klinische Ergebnis eine Verringerung des Körpergewichts um mindestens 5 % oder 10 % im Vergleich zum Baseline-Körpergewicht vor dem Start der mindestens einer Therapie ist, wobei optional das Subjekt einen Ernährungsplan und/oder ein Trainingsprogramm als Teil der Gewichtskontrolle nicht ausführt oder nicht fortführt;
c. das Subjekt eines oder mehrere hat aus zentraler Adipositas, Herz-Kreislauf-Erkrankung, Nierenerkrankung, Fettlebererkrankungen, Schlafapnoe, Bluthochdruck, hohen Triglyceridwerten im Blut, hohen Cholesterinwerten im Blut, niedrigen High-Density-Lipoprotein(HDL)-Werten und einen Hämoglobin-A1c-Wert (HbA1c) von mindestens 6 % (z. B. 6,5-10 %), wobei optional das Subjekt einen Body-Mass-Index (BMI) von über 25 aufweist; wobei ferner optional das Subjekt einen BMI von zwischen 28 und 40, jeweils einschließlich, aufweist; und/oder
d. das Subjekt einer kalorieneingeschränkte Ernährung und/oder einem Trainingsprogramm unterliegt.

9. Myostatin-selektiver Inhibitor und/oder GLP-1-Analogon zur Verwendung nach einem der Ansprüche 1-8, wobei:
a. der Myostatin-selektive Inhibitor und das GLP-1-Analogon parallel, gleichzeitig oder nacheinander verabreicht werden; wobei optional der Myostatin-selektive Inhibitor und das GLP-1-Analogon als eine einzige Formulierung, als Teil eines einzigen molekularen Konstrukts oder als separate Formulierungen formuliert sind; und/oder
b. der Myostatin-selektive Inhibitor subkutan oder intravenös verabreicht wird.

10. Myostatin-selektiver Inhibitor und/oder GLP-1-Analogon zur Verwendung nach einem der Ansprüche 1-9, wobei:
a. die Behandlung Triglyceride, Gesamtcholesterin, LDL-Cholesterin und/oder Nicht-Nüchtern-Glukosespiegel in Bezug auf einen Baseline-Spiegel verringert, der vor Beginn der Behandlung gemessen wurde, optional wobei die Behandlung Triglyceride, Gesamtcholesterin, LDL-Cholesterin und/oder Nicht-Nüchtern-Glukosespiegel in Bezug auf die Behandlung nur mit einem GLP-1-Analogon verringert; und/oder
b. die Behandlung, im Vergleich zu einem vor Beginn der Behandlung gemessenen Baseline-Spiegel, Fettmasse verringert, fettfreie Körpermasse beibehält oder Insulinsensitivität und/oder Insulinsekretion verbessert, optional wobei die Behandlung Insulinsensitivität und/oder Insulinsekretion im Vergleich zu Behandlung nur mit einem GLP-1-Analogon verbessert.

11. Zusammensetzung, umfassend einen Myostatin-selektiven Inhibitor und ein GLP-1-Analogon;
wobei der Myostatin-selektive Inhibitor ein Myostatin-selektiver Antikörper oder ein antigenbindendes Fragment davon ist, der/das an Myostatin bindet und keine nachweisbare Bindung oder Potenz in Bezug auf GDF11 oder Activin A aufweist; und
wobei das GLP-1-Analogon in der Lage ist, an den GLP-1-Rezeptor zu binden und diesen zu aktivieren und die Aminosäuresequenz EGTFTSD (SEQ ID NO: 116) und/oder die Aminosäuresequenz HXXGXFTXD, wobei X ein beliebiger Aminosäurerest ist (SEQ ID NO: 117), umfasst, optional wobei das GLP-1-Analogon Semaglutid, Exenatid ER, Liraglutid, Lixisenatid, Tirzepatid, XW003, Noiiglutid, MEDI0382, Dulaglutid oder Albiglutid ist.

12. Zusammensetzung nach Anspruch 11, wobei:
a. der Myostatin-selektive Inhibitor ein Antikörper oder ein antigenbindendes Fragment davon ist, der/das an ein Epitop bindet, das einen oder mehrere Aminosäurereste aus KALDEN (SEQ ID NO: 118) und/oder FVQILRLIKPMKDGTRYTGIRSLK (SEQ ID NO: 57) umfasst; wobei der Antikörper nicht Apitegromab ist; oder
b. der Myostatin-selektive Inhibitor ein Antikörper oder ein antigenbindendes Fragment davon ist, der/das mit Apitegromab bei der Antigenbindung konkurriert oder kreuzkonkurriert, wobei der Antikörper nicht Apitegromab ist; wobei optional der Antikörper oder das antigenbindende Fragment davon ein HCDR3-Paratop umfasst, das im Vergleich zu SEQ ID NO: 10 bis zu zwei Aminosäuresubstitutionen umfasst; wobei ferner optional der Antikörper oder das antigenbindende Fragment davon an ein Epitop bindet, umfassend einen oder mehrere der Aminosäurereste F147, Q149, L151, Y186, S168, K170, K205 und/oder L207, jeweils nummeriert gemäß SEQ ID NO: 52.

13. Myostatin-selektiver Inhibitor zur Verwendung als Zusatztherapie zur Gewichtskontrolle bei der Behandlung von Fettleibigkeit oder Übergewicht in einem Subjekt, wobei die Therapie das Verabreichen des Myostatin-selektiven Inhibitors nach einem der Ansprüche 1-10 in einer Menge umfasst, die wirksam ist, Fettleibigkeit oder Übergewicht zu behandeln, wobei das Subjekt mit einem GLP-1-Analogon behandelt wird;
wobei das GLP-1-Analogon in der Lage ist, an den GLP-1-Rezeptor zu binden und diesen zu aktivieren und die Aminosäuresequenz EGTFTSD (SEQ ID NO: 116) und/oder die Aminosäuresequenz HXXGXFTXD, wobei X ein beliebiger Aminosäurerest ist (SEQ ID NO: 117), umfasst;
wobei optional das Subjekt mindestens eine gewichtsbezogene Erkrankung hat, wobei ferner optional das Subjekt über mindestens 3 Monate mit dem GLP-1-Analogon behandelt worden ist.

14. GLP-1-Analogon zur Verwendung als eine Zusatztherapie zur Gewichtskontrolle bei der Behandlung von Fettleibigkeit oder Übergewicht in einem Subjekt, wobei die Behandlung das Verabreichen einer wirksamen Menge des GLP-1-Analogons umfasst, um Fettleibigkeit oder Übergewicht zu behandeln, wobei das Subjekt mit dem Myostatin-selektiven Inhibitor nach einem der Ansprüche 1-10 behandelt wird;
wobei das GLP-1-Analogon in der Lage ist, an den GLP-1-Rezeptor zu binden und diesen zu aktivieren und die Aminosäuresequenz EGTFTSD (SEQ ID NO: 116) und/oder die Aminosäuresequenz HXXGXFTXD, wobei X ein beliebiger Aminosäurerest ist (SEQ ID NO: 117), umfasst;
wobei optional das Subjekt mindestens eine gewichtsbezogene Erkrankung hat, wobei ferner optional das Subjekt über mindestens 3 Monate mit dem Myostatin-selektiven Inhibitor behandelt worden ist.

15. Myostatin-selektiver Inhibitor und/oder GLP-1-Analogon zur Verwendung nach einem der Ansprüche 1-5, 7-10, 13 oder 14, oder Zusammensetzung nach Anspruch 11, wobei der Myostatin-selektive Inhibitor nicht Apitegromab ist.

## Revendications

1. Inhibiteur sélectif de la myostatine pour le traitement d'un trouble métabolique chez un sujet, le traitement comprenant l'administration de l'inhibiteur sélectif de la myostatine au sujet en association avec un analogue de GLP-1, l'inhibiteur sélectif de la myostatine et l'analogue de GLP-1 sont administrés en quantités suffisantes pour traiter le trouble métabolique ; facultativement, le trouble métabolique étant le diabète de type 2 (DT2), l'obésité, l'obésité associée au DT2 ou le syndrome métabolique,
l'inhibiteur sélectif de la myostatine étant un anticorps sélectif contre la myostatine ou un fragment de liaison à l'antigène de celui-ci, qui se lie à la myostatine et ne présente aucune liaison ni activité détectables envers GDF11 ou l'activine A ; et
l'analogue de GLP-1 pouvant se lier au récepteur de GLP-1 et activer celui-ci, et comprenant la séquence d'acides aminés EGTFTSD (SEQ ID NO : 116) et/ou la séquence d'acides aminés HXXGXFTXD, X représentant un résidu d'acide aminé (SEQ ID NO : 117).

2. Analogue de GLP-1 pour le traitement d'un trouble métabolique chez un sujet, le traitement comprenant l'administration de l'analogue de GLP-1 au sujet en association avec un inhibiteur sélectif de la myostatine, l'inhibiteur sélectif de la myostatine et l'analogue de GLP-1 étant administrés en quantités suffisantes pour traiter le trouble métabolique ; facultativement, le trouble métabolique étant le diabète de type 2 (DT2), l'obésité, l'obésité associée au DT2 ou le syndrome métabolique,
l'inhibiteur sélectif de la myostatine étant un anticorps sélectif contre la myostatine ou un fragment de liaison à l'antigène de celui-ci, qui se lie à la myostatine et ne présente aucune liaison ni activité détectables envers GDF11 ou l'activine A ; et
l'analogue de GLP-1 pouvant se lier au récepteur de GLP-1 et activer celui-ci, et comprenant la séquence d'acides aminés EGTFTSD (SEQ ID NO : 116) et/ou la séquence d'acides aminés HXXGXFTXD, X représentant un résidu d'acide aminé (SEQ ID NO : 117).

3. Inhibiteur sélectif de la myostatine et analogue de GLP-1 pour le traitement d'un trouble métabolique chez un sujet, le traitement comprenant l'administration de l'inhibiteur sélectif de la myostatine au sujet en association avec l'analogue de GLP-1, l'inhibiteur sélectif de la myostatine et l'analogue de GLP-1 sont administrés en quantités suffisantes pour traiter le trouble métabolique ; facultativement, le trouble métabolique étant le diabète de type 2 (DT2), l'obésité, l'obésité associée au DT2 ou le syndrome métabolique,
l'inhibiteur sélectif de la myostatine étant un anticorps sélectif contre la myostatine ou un fragment de liaison à l'antigène de celui-ci, qui se lie à la myostatine et ne présente aucune liaison ni activité détectables envers GDF11 ou l'activine A ; et
l'analogue de GLP-1 pouvant se lier au récepteur de GLP-1 et activer celui-ci, et comprenant la séquence d'acides aminés EGTFTSD (SEQ ID NO : 116) et/ou la séquence d'acides aminés HXXGXFTXD, X représentant un résidu d'acide aminé (SEQ ID NO : 117).

4. Inhibiteur sélectif de la myostatine et/ou analogue de GLP-1 selon l'une quelconque des revendications 1 à 3, **caractérisés en ce que** :
a. l'analogue de GLP-1 est un conjugué de Fc ou une molécule multifonctionnelle comprenant l'analogue de GLP-1 ; et/ou
b. l'analogue de GLP-1 est le sémaglutide, l'exénatide ER, le liraglutide, le lixisénatide, le tirzépatide, XW003, le noiiglutide, MEDI0382, le dulaglutide, l'albiglutide, AMG 133, CT-388, l'association cagrilintide/sémaglutide, DD01, ALT-801 ou IBI362.

5. Inhibiteur sélectif de la myostatine et/ou analogue de GLP-1 selon l'une quelconque des revendications 1 à 4, **caractérisés en ce que** :
a. l'inhibiteur sélectif de la myostatine est le trévogrumab, GYM329, MST1032, ou un anticorps ou un fragment de liaison à l'antigène en compétition ou compétition croisée avec le trévogrumab, GYM329, MST1032 ou l'apitégromab ; et/ou
b. l'inhibiteur sélectif de la myostatine comprend au moins une mutation d'acide aminé qui augmente l'affinité de liaison pour FcRn.

6. Inhibiteur sélectif de la myostatine et/ou analogue de GLP-1, selon l'une quelconque des revendications 1 à 5, l'inhibiteur sélectif de la myostatine étant un anticorps ou un fragment de liaison à l'antigène de celui-ci, qui se lie à un épitope qui comprend un ou plusieurs résidus d'acides aminés de KALDEN (SEQ ID NO : 118) et/ou de FVQILRLIKPMKDGTRYTGIRSLK (SEQ ID NO : 57) ; l'anticorps n'étant pas l'apitégromab.

7. Inhibiteur sélectif de la myostatine et/ou analogue de GLP-1 selon l'une quelconque des revendications 1 à 5, l'inhibiteur sélectif de la myostatine étant un anticorps ou un fragment de liaison à l'antigène de celui-ci, qui se lie sélectivement à la promyostatine et/ou à la myostatine latente ; facultativement, l'anticorps ou un fragment de liaison à l'antigène de celui-ci ne se liant pas à la myostatine mature.

8. Inhibiteur sélectif de la myostatine et/ou analogue de GLP-1 selon l'une quelconque des revendications 1 à 7, **caractérisés en ce que** :
a. le sujet est un adulte ayant un IMC supérieur ou égal à 25, ou un enfant ou un adolescent âgé de 2 à 19 ans (par exemple, d'au moins 12 ans, par exemple, de 12 à 17 ans) ayant un IMC égal ou supérieur au 85e centile sur les courbes de croissance du CDC ;
b. le sujet a reçu au moins un traitement contre l'obésité, le surpoids ou une affection liée au poids, sans obtenir le résultat clinique escompté, ce résultat étant une réduction du poids corporel d'au moins 5 % ou 10 % par rapport au poids corporel de référence mesuré avant le début du ou des traitements, facultativement, le sujet n'ayant pas suivi ou poursuivi un régime alimentaire et/ou un programme d'exercice physique dans le cadre de la gestion de son poids ;
c. le sujet présente une ou plusieurs affections parmi une adiposité centrale, une maladie cardiovasculaire, une maladie rénale, la stéatose hépatique, l'apnée du sommeil, l'hypertension artérielle, une hypertriglycéridémie, une hypercholestérolémie, un faible taux de lipoprotéines de haute densité (HDL) et un taux d'hémoglobine A1c (HbA1c) supérieur ou égal à 6 % (par exemple, de 6,5 % à 10 %), facultativement, le sujet présentant un indice de masse corporelle (IMC) supérieur à 25 ; facultativement, le sujet présentant un IMC compris entre 28 et 40, limites incluses ; et/ou
d. le sujet suit un régime hypocalorique et/ou un programme d'exercice physique.

9. Inhibiteur sélectif de la myostatine et/ou analogue de GLP-1 selon l'une quelconque des revendications 1 à 8, **caractérisés en ce que** :
a. l'inhibiteur sélectif de la myostatine et l'analogue de GLP-1 sont administrés concomitamment, simultanément ou séquentiellement ; facultativement, l'inhibiteur sélectif de la myostatine et l'analogue de GLP-1 étant formulés sous forme de formulation unique, en tant que composant d'une construction moléculaire unique ou de formulations distinctes ; et/ou
b. l'inhibiteur sélectif de la myostatine est administré par voie sous-cutanée ou intraveineuse.

10. Inhibiteur sélectif de la myostatine et/ou analogue de GLP-1 selon l'une quelconque des revendications 1 à 9, **caractérisés en ce que** :
a. le traitement réduit les taux de triglycérides, de cholestérol total, de cholestérol LDL et/ou la glycémie à jeun par rapport à une valeur de référence mesurée avant le début du traitement, facultativement, le traitement réduisant les taux de triglycérides, de cholestérol total, de cholestérol LDL et/ou la glycémie à jeun par rapport à un traitement par un analogue de GLP-1 seul ; et/ou
b. le traitement réduit la masse grasse, préserve la masse maigre ou améliore l'insulinosensibilité et/ou la sécrétion d'insuline par rapport à une valeur de référence mesurée avant le début du traitement, facultativement, le traitement améliorant l'insulinosensibilité et/ou la sécrétion d'insuline par rapport à un traitement par un analogue de GLP-1 seul.

11. Composition comprenant un inhibiteur sélectif de la myostatine et un analogue de GLP-1 ;
l'inhibiteur sélectif de la myostatine étant un anticorps sélectif contre la myostatine ou un fragment de liaison à l'antigène de celui-ci, qui se lie à la myostatine et ne présente aucune liaison ni activité détectables envers GDF11 ou l'activine A ; et
l'analogue de GLP-1 pouvant se lier au récepteur de GLP-1 et activer celui-ci, et comprenant la séquence d'acides aminés EGTFTSD (SEQ ID NO : 116) et/ou la séquence d'acides aminés HXXGXFTXD, X représentant un résidu d'acide aminé (SEQ ID NO : 117), facultativement, l'analogue de GLP-1 étant le sémaglutide, l'exénatide ER, le liraglutide, le lixisénatide, le tirzépatide, XW003,
le noiiglutide, MEDI0382, le dulaglutide ou l'albiglutide.

12. Composition selon la revendication 11, dans laquelle :
a. l'inhibiteur sélectif de la myostatine est un anticorps ou un fragment de liaison à l'antigène de celui-ci qui se lie à l'antigène, qui se lie à un épitope comprenant un ou plusieurs résidus d'acides aminés de KALDEN (SEQ ID NO : 118) et/ou de FVQILRLIKPMKDGTRYTGIRSLK (SEQ ID NO : 57) ; l'anticorps n'étant pas l'apitégromab : ou
b. l'inhibiteur sélectif de la myostatine est un anticorps ou un fragment de liaison à l'antigène de celui-ci, qui entre en compétition ou en compétition croisée avec l'apitégromab pour la liaison à l'antigène, l'anticorps n'étant pas l'apitégromab ; facultativement, l'anticorps ou le fragment de liaison à l'antigène celui-ci comprenant un paratope HCDR3 contenant jusqu'à deux substitutions d'acides aminés par rapport à SEQ ID NO : 10 ; dans laquelle, facultativement, l'anticorps ou le fragment de liaison à l'antigène se lie à un épitope comprenant un ou plusieurs des résidus d'acides aminés F147, Q149, L151, Y186, S168, K170, K205 et/ou L207, numérotés selon SEQ NO : 52.

13. Inhibiteur sélectif de la myostatine pour un traitement d'appoint pour la gestion du poids dans le traitement de l'obésité ou du surpoids chez un sujet, le traitement comprenant l'administration de l'inhibiteur sélectif de la myostatine selon l'une quelconque des revendications 1 à 10 en une quantité efficace pour traiter l'obésité ou le surpoids, le sujet étant traité par un analogue de GLP-1 ;
l'analogue de GLP-1 pouvant se lier au récepteur de GLP-1 et activer celui-ci, et comprenant la séquence d'acides aminés EGTFTSD (SEQ ID NO : 116) et/ou la séquence d'acides aminés HXXGXFTXD, X représentant un résidu d'acide aminé (SEQ ID NO : 117) ;
facultativement, le sujet présentant au moins une affection liée au poids ; facultativement, le sujet ayant été traité par l'analogue de GLP-1 pendant au moins 3 mois.

14. Analogue de GLP-1 pour un traitement d'appoint pour la gestion du poids dans le traitement de l'obésité ou du surpoids chez un sujet, le traitement comprenant l'administration d'une quantité efficace de l'analogue de GLP-1 pour traiter l'obésité ou le surpoids, le sujet étant traité par l'inhibiteur sélectif de la myostatine selon l'une quelconque des revendications 1 à 10 ;
l'analogue de GLP-1 pouvant se lier au récepteur de GLP-1 et activer celui-ci, et comprenant la séquence d'acides aminés EGTFTSD (SEQ ID NO : 116) et/ou la séquence d'acides aminés HXXGXFTXD, X représentant un résidu d'acide aminé (SEQ ID NO : 117) ;
facultativement, le sujet présentant au moins une affection liée au poids ; facultativement, le sujet ayant été traité par l'inhibiteur sélectif de la myostatine pendant au moins 3 mois.

15. Inhibiteur sélectif de la myostatine et/ou analogue de GLP-1 selon l'une quelconque des revendications 1 à 5, 7 à 10, 13 ou 14, ou composition selon la revendication 11, l'inhibiteur sélectif de la myostatine n'étant pas l'apitégromab.
